# EUROPEAN PATENT APPLICATION

(11) **EP 2 607 374 A1**
(43) Date of publication of application: **26.06.2013**
(21) Application number: 11306712.8
(22) Date of filing: 21.12.2011
(51) Int. Cl.: C07K 14/36, C12N 15/76, A61K 38/16

(54) **Method for recombinant production of labyrinthopeptins and functional derivatives thereof**

(71) Applicant: SANOFI, 75008 Paris (FR)
(72) Inventor: The designation of the inventor has not yet been filed
(74) Representative: Schneider, Rudolf

(57) **Abstract**

The present invention relates to a method for recombinant production of Labyrinthopeptins and functional derivatives thereof. Moreover, the present invention relates to novel functional derivatives of Labyrinthopeptins.

## Description

The present invention relates to a method for recombinant production of Labyrinthopeptins and functional derivatives thereof. Moreover, the present invention relates to novel functional derivatives of Labyrinthopeptins.

Lantibiotics are peptides that are ribosomally synthesized from bacteria such as staphylococci, lactobacilli, and actinomycetes. The common structural characteristic of lantibiotics is the noncanonical amino acid lanthionine (Lan), which confers conformational stability to the peptide. Labyrinthopeptins are class III lantibiotics isolated from a desert bacterium *Actinomadura namibiensis.* They are first representatives of a new type of lantibiotics with a unique carbacyclic post-translationally modified amino acid named labionin. The chemical structre of Labionin is as follows:

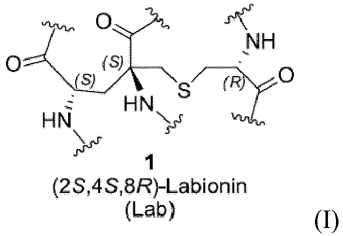

The biological characterization showed that Labyrinthopeptin A2 (LabA2) revealed an efficacy in an *in vivo* neuropathic pain model. Since these compounds can be considered as new leads for drug discovery there is a strong need to perform the structure-function relationship studies to identify motifs essential for their bioactivity. However, due to the presence of complex post-translational modifications, a chemical synthesis of lantibiotic analogues is complicated and not efficient, or like it is for Labyrinthopeptins not known at all.

There is therefore a need for methods allowing recombinant production of Labyrinthopeptins, as well as for the generation of functional derivatives of Labyrinthopeptins.

The present invention relates to a nucleic acid comprising at least one Labyrinthopeptin *(lab)* gene cluster, in particular a gene cluster having a sequence according to SEQ ID No. 139, of an actinomycete, in particular of *Actinomaduras namibiensis,* wherein:
(a) the nucleic acid further comprises a constitutive promoter for expression in *Streptomyces* cells, in particular an *ermE* promotor, and wherein the gene cluster is under control of said promoter, and/or
(b) the nucleic acid encoding LabA1 and/or LabA2 or a functional derivative thereof is mutated, such that the C-terminal amino acid of the leader peptide of LabA1 and/or LabA2 is substituted with a methionine, and/or
(c) the nucleic acid further comprises at least one hybrid gene sequence,
   wherein the hybrid gene sequence:
   (i) encodes a pre-pro-peptide comprising a LabA2 leader sequence and labA1 pre-peptide or functional derivative thereof and/or
   (ii) encodes a pre-pro-peptide comprising a LabA1 leader sequence and labA2 pre-peptide or functional derivative thereof.

In a preferred embodiment, the *Streptomyces* cells are selected from *Streptomyces lividans* and *Streptomyces albus* cells, in particular the cells are *Streptomyces lividans* cells.

It was found that LabA1 and/or LabA2 and functional derivatives thereof can be successfully produced by recombinant production, as shown in Examples 2 to 4. In particular, the expression was sucessfully performed using a consitutive promotor *ermE* for expression in *Streptomyces.*

The *lab* gene cluster comprises the genes *labKC*, *labA1*, *labA2*, *labT1* and *labT2.* Structural genes *labA1* and *labA2* code for labyrinthopeptin A1/A3 and labyrinthopeptin A2 prepropeptides, respectively. *labT1* and *labT2* encode proteins with homology to transporter proteins. The gene *labKC* codes for a trifunctional protein with an N-terminal lyase domain, Ser/Thr kinase domain and a C-terminal putative lanthionine cyclase domain necessary for posttranslational modification of Labyrinthopeptins, and therefore for generation of mature Labyrinthopeptin.

The native *labA1* and/or *labA2* genes have a sequence according to SEQ ID No. 140 and SEQ ID No. 141, respectively. The expression of these genes resulting in mature LabA1 and LabA2 could be performed successfully by recombinant expression in *Streptomyces* cells according to the Examples. Moreover, functional derivatives could be generated.

Thus, in a further embodiment, the nucleic acid encoding LabA1 and/or LabA2 has a sequence according to SEQ ID No. 140 or SEQ ID No. 141, or has a sequence exhibiting at least 85%, preferably at least 90%, even more preferably at least 95% sequence identity to SEQ ID No. 140 and/or SEQ ID No. 141.

Moreover, it could be shown in the examples, that mutating the last, C-terminal amino acid of the leader peptide to methionine suprisingly results in the production of mature LabA1 and LabA2, without remaining amino acids of the leader peptide.

Also, it could suprisingly be shown that it is possible to transfer the nucleic acid encoding the pro-peptide sequence LabA2 to the nucleic acid encoding the leader peptide of LabA1, and to transfer the nucleic acid encoding the pro-peptide sequence LabA1 to the nucleic acid encoding the leader peptide of LabA2, thereby generating hybrid pre-pro-peptides.

In a further embodiment, the invention relates to a vector, in particular a plasmid, comprising a nucleic acid construct as described above. In the examples, the vectors pUWLab and pLab were successfully used for expression of Labyrinthopeptins and derivatives thereof in *Streptomyces* species. The vectors were constructed starting from the known vector pUWLoriT, as described in the Examples, by inserting a *lab* gene cluster, such that the gene cluster is under control of the constitutive promotor *ermE.*

Thus, in a preferred embodiment, the vector of the invention is:
(a) pLab, having a sequence according to SEQ ID No. 143, or pUWLab, having a sequence according to SEQ ID No. 143, or pLabAmp, having a sequence according to SEQ ID No. 144, and/or
(b) obtainable from pUWLoriT by inserting at least one *lab* gene cluster, such that the gene cluster is under control of the constitutive promotor.

In particular, the vector is a plasmid.

After several unsuccessful attempts, as described in Example 1, expression of Labyrinthopeptins and functional derivatives thereof could be achieved by transformation of *Streptomyces lividans* and *Streptomyces albus* cells (Examples 2 to 4), using the above vectors. Notably, the vectors are bifunctional and can therefore also be used for replication and propagation in *E. coli.*

Therefore, the present invention further relates to a bacterial cell, in particular a *Streptomyces* or *E. coli* cell, more preferably a *Streptomyces lividans* or *Streptomyces albus* cell, transformed with a vector according to the invention.

Methods for transforming *Streptomyces* or *E. coli* cells are known to a skilled person. In particular, the transformation method as explained in the Materials and Methods section of the Examples may be used.

In another embodiment, the present invention relates to the use of at least one *Streptomyces lividans* and/or *Streptomyces albus* cell for the recombinant expression of at least one Labyrinthopeptin or a functional derivative thereof.

In a further embodiment, the invention relates to a method for producing at least one Labyrinthopeptin or functional derivative thereof, comprising the steps:
(a) culturing a *Streptomyces* cell, preferably a *Streptomyces lividans* or *Streptomyces albus* cell, which cell is transformed with a vector according to the invention, in culture medium,
(b) harvesting the culture medium comprising at least one Labyrinthopeptin or functional derivative thereof, and
(c) optionally purifying at least one Labyrinthopeptin or functional derivative thereof from the culture medium.

Methods for culturing bacterial cells are known to a skilled person. In particular, the culturing methods and media as explained in the Materials and Methods section of the Examples may be used. *A. namibiensis* and *Streptomyces* cells may be cultured in suitable media known to a skilled person. In particular, agar media, preferably selected from MS, R2YE, R5 or KM4, as disclosed in the Examples, may be used. In another preferred embodiment, liquid media, in particular selected from YEME, CRM, KM4 or M5294 may be used, as for example disclosed the Examples. The *A. namibiensis* and *Streptomyces* cells may be cultured at about 10°C to about 40°C, preferably at about 20°C to about 35°C, more preferably at about 25°C to 30°C, most preferably at about 28°C. Methods for culturing *E. coli* are known in the art. In particular, LB medium, as described in the Examples may be used. The cell cultures may be supplemented with antibiotics, where appropriate for selection.

Cells may in particular be cultured for about 30 minutes to 6 about months, in particular for 1 about day to about 3 months, for preferably for about 3 days to about 6 weeks depending on the used species or type of cells. E. coli cells for example are usually grown for 1 to 2 days, and Streptomyces spec. cells are usually grown for 3 days to 3 weeks.

In another preferred embodiment, the vector of the invention comprises a nucleic acid encoding LabA1 and/or LabA2 or functional derivative thereof, wherein the nucleic acid is mutated such that the C-terminal amino acid the leader peptide of LabA1 and/or LabA2 is substituted with a methionine. Such constructs can allow for expression of mature peptides without amino acids from the leader peptide.

In another preferred embodiment, the vector of the invention comprises at least one hybrid nucleic acid selected from:
(i) a hybrid nucleic acid encoding a pre-pro-peptide comprising a LabA2 leader sequence and LabA1 pre-peptide or functional derivative thereof and/or
(ii) a hybrid nucleic acid encoding a pre-pro-peptide comprising a LabA1 leader sequence and LabA2 pre-peptide or functional derivative thereof, preferably (ii).

Such hybrid molecules are in particular suitable for expressing higher amounts of LabA2 or functional derivatives thereof, as shown in Example 4.

Methods for introducing mutations in a nucleic acid are well-known in the art. For example, site-directed mutagenesis as described in the Materials and Methods section of the Examples may be performed.

Also, methods for detecting Labyrinthopeptin or functional derivatives thereof are known in the art. For example, immunological methods, like Western Blotting, and/or LC-MS and/or MS/MS and/or GC-MS, as shown in the Materials and Methods section of the Examples and the Examples 2 to 4 may be used.

For cloning and DNA amplification experiments, various methods are described in the prior art. In particular, PCR may be used for amplification and cloning as described in the Materials and Methods section of the Examples. Also, methods for cloning including the use of restriction endonucleases and/or ligases are known in the art. Preferred methods are described in the Materials and Methods section of the Examples.

In some embodiments, a bifunctional vector is used, which can be propagated both in *E. coli* and in *Streptomyces* cells.

Methods for transforming bacteria are well known in the art. For example, *E. coli* cells may be transformed by electroporation or chemical transformation, as disclosed in the Examples. *Streptomyces* strains may be transformed in particular using protoplasts. In particular, protoplasts may be prepared, followed by PEG-assisted transformation. Also, conjugation may be used. Such protocols are disclosed in the Examples.

The invention further relates to a mixture of more than one Labyrinthopeptin or functional derivatives thereof, obtainable by the method of the invention.

A number of novel Labyrinthopeptin derivatives could be generated according to the examples.

Therefore, in one embodiment, the present invention relates to a compound selected from:
(a) a Labyrinthopeptin pre-pro-peptide Labyrinthopeptin or a functional derivative thereof, wherein the C-terminal amino acid of the leader sequence is a methionine,
   or
(b) a nucleic acid encoding a Labyrinthopeptin pre-pro-peptide or a functional derivative thereof, wherein the C-terminal amino acid of the leader sequence of the pre-pro-peptide is a methionine.

In a further embodiment, the present invention relates to a nucleic acid comprising a sequence according to any of SEQ ID No. 1 to 24, preferably, it is consisting of a sequence according to any of SEQ ID No. 1 to 24.

In a further embodiment, the present invention relates to a functional derivative of a Labyrinthopeptin pre-pro-peptide encoded by a nucleic acid comprising a sequence according to any of SEQ ID No. 1 to 24, and which pre-pro-peptide has a sequence different from LabA1 pre-pro-peptide according to SEQ ID No. 132 or LabA2 pre-pro-peptide according to SEQ ID No. 135.

As shown in Examples 3 and 4, it was surprisingly possible to express a number of mutated forms of Labyrinthopeptins in *Streptomyces* cells using the nucleic acid molecules according to SEQ ID No. 1 to 24. These cassettes could be inserted into an appropriate vector, for transformation and expression in *Streptomyces* cells.

Particularly preferred is the use of a nucleic acid comprising a sequence according to SEQ ID No. 1 (SG2), and SEQ ID No. 5 (SG6). These cassettes were in particluar useful for performing site-directed mutagenesis of *labA1* and/or *labA2* genes.

In another preferred embodiment, nucleic acids nucleic acid comprising a sequence according to SEQ ID No. 3 and/or SEQ ID No. 6 may be used. Such constructs have been useful for expressing LabA2 and functional derivatives thereof.

In another embodiment, the present invention relates to a functional derivative of a Labyrinthopeptin pre-peptide encoded by a nucleic acid comprising a sequence according to any of SEQ ID No. 1 to 24, and which has pre-peptide has a sequence different from LabA1 pre-peptide according to SEQ ID No. 134 or LabA2 pre-peptide according to SEQ ID No. 137, or LabA3 pre-peptide according to SEQ ID No. 138, and which pre-peptide lacks the leader sequence or at least the N-terminal 80%, in particular 90%, more preferably 95% of the leader sequence, in particular wherein the leader sequence has a sequence according to SEQ ID No. 133 or SEQ ID No. 136.

In another embodiment, the invention relates to a derivative, in particular functional derivative, of a Labyrinthopeptin peptide, in particular selected from: LabA1_N2A, LabA1_V5A, LabA1_E7A, LabA1_T11A, LabA1_V15A, LabA1_P16A, LabA1_F17A, LabA2_D2A, LabA2_W3A, LabA2_L5A, LabA2_W6A, LabA2_E7A, LabA2_T11A, LabA2_G12A, LabA2_L14A, LabA2_F15A, LabA1_V5del, LabA1_W6insV, LabA1_P16del, LabA1_P16insV, LabA1_S4T, M-LabA1_S4T, AM-LabA1_S4T, NR-LabA2_M (SG20), R-LabA2_M (SG20), LabA1_V15S, M-LabA1_V15S, LabA1_T11S (Ser), LabA1_W6Y, LabA1_A3H, LabA1_C20insA, LabA1_S10insA, LabA1_C20del, LabA1_S1insC, D-LabA1/A2 (SG11), AD-LabA1/A2 (SG11), R-LabA2/A1 (SG11), NR-LabA2/A1 (SG11), and ENR-LabA2/A1 (SG11).

In another embodiment, the present invention relates to a *S. lividans* cell deposited with Accession number DSM24184. In a further embodiment, the present invention relates to the use of a *S. lividans* cell deposited with Accession number DSM24184 for producing LabA1, LabA2 and/or LabA3 and/or derivatives thereof, in particular functional derivatives thereof.

In another embodiment, the present invention relates to a *S. lividans* cell deposited with Accession number DSM24580. In a further embodiment, the present invention relates to a LabA2 derivative produced by the cell, in particular to a LabA2_L14A molecule produced by the cell.

In another embodiment, the present invention relates to a *S. lividans* cell deposited with Accession number DSM24581. In a further embodiment, the present invention relates to a LabA2 derivative produced by the cell, in particular to a LabA2 _F 15A produced by the cell.

According to the invention, "LabA#_§X&" is understood as meaning a LabA# variant, wherein amino acid No. X of the pre-peptide of LabA# is mutated from § to &. For example, in LabA1_N2A, amino acid No. 2 of LabA1 pro-peptide is mutated from N to A.

The number of amino acids is understood as to be calculated starting from the N-terminus.

According to the invention, "LabA#_§Xdel" is understood as meaning a LabA# variant, wherein amino acid No. X of the pre-peptide of LabA1, which is a §, is deleted. For example, "LabA1_V5del" is understood as meaning a LabA1 variant, wherein amino acid No. 5 of the pre-peptide of LabA1, which is a Valine, is deleted.

According to the invention, "LabA# _9Xins&" is understood as meaning a LabA# variant, wherein amino acid & is inserted before amino acid No. X of the pre-peptide of LabA#, pushing original amino acid No. X (&) to position X+1. For example, "LabA1_P16insV" is understood as meaning a LabA1 variant, wherein amino acid Valine is inserted before amino acid No. 16 of the pre-peptide of LabA1, pushing original amino acid No. 16 (Proline) to position 17.

According to the invention, "§-LabA#" is understood as LabA#, having an additional amino acid § at the N-terminus. Such additional amino acid may origin from the leader sequence which is not completely processed.

According to the invention, "LabA2/A1" is understood as hybrid consisting of a LabA2 leader sequence and a LabA1 pre-peptide sequence. Accordingly, LabA1/A2 is understood as hybrid consisting of a LabA1 leader sequence and a LabA2 pre-peptide sequence.

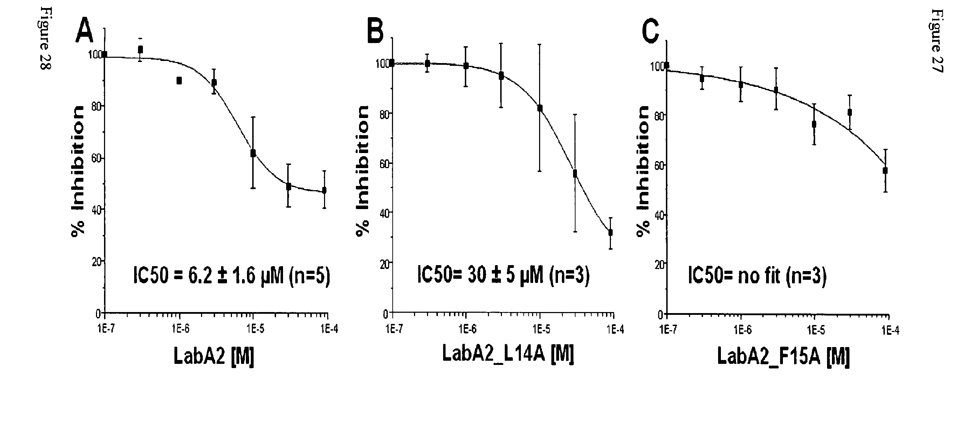

Labyrinthopeptins LabA1 and LabA2 were shown to have antimicrobial, antiviral and anti-pain effects. Moreover, LabA2 (A) and LabA2_L14A are shown to exhibit anti-pain activity in the present application (Figure 27). Therefore, the present invention also relates to a mixture or a compound of the invention, for use in the treatment and/or prevention of bacterial infections, HIV infections or pain, in particular neuropathic pain. Further, the present invention also relates to the use of a mixture or a compound of the invention, for the preparation of a medicament for the treatment and/or prevention of bacterial infections, HIV infections or pain, in particular neuropathic pain. Also, the present invention relates to a method for treating and/or preventing bacterial infections, HIV infections or pain, in particular neuropathic pain in a mammal, in particular in a human, comprising administering to said mammal an effective amount of a mixture or a compound of the invention.

The peptides may be formulated according to methods skilled in the art. Preferably, the compounds and mixtures are present as a pharmaceutical composition, together with a pharmaceutically acceptable carrier, like e.g. water or saline.

The Labyrinthopeptins LabA1 and LabA2 are disclosed in WO2008/040469A1. Moreover, the chemical structure and activity of the Labyrinthopeptins is summarized in Meindl et al. (2010). The term "Labyrinthopeptin" is understood as encompassing LabA1, LabA2, and LabA3 peptides.

The term "mature Labyrinthopeptin" is understood as Labyrinthopeptin functional derivative thereof, which lacks the leader sequence, or a part of it, and which has undergone posttranslational modification. In particular, a mature Labyrinthopeptin contains one or more labionine amino acids.

The structure of mature LabA1 is as follows: 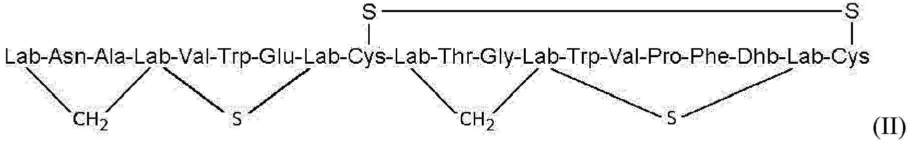 wherein Dhb is didehydrobutyrine, and Lab is Labionin.

The structure of mature LabA2 is as follows: 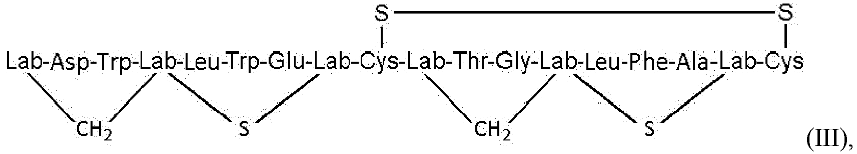 wherein Lab is Labionin.

Labionin rings A and A' are formed by a methylene group between αC atoms of the amino acid No. 1, starting from the N-terminus (Lab 1) and the amino acid No. 4 (Lab 4) for ring A, and by a methylene group between αC atoms of the amino acid No. 10, starting from the N-terminus (Lab 10) and the amino acid No. 13 (Lab 13) for ring A'. The rings B and B' rings are formed by a thioether bridge.

The structure of Dhb is as follows:

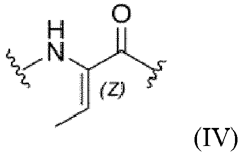

Also LabA3 has been described (Meindl et al., supra). LabA3 carries an additional Asp residue at the N-terminus compared to mature LabA1.
LabA1: C₉₂H₁₁₉N₂₃O₂₅S₄ (2073.7624 amu)
LabA2: C₈₅H₁₁₀N₂₀O₂₄S₄ (1922.6930 amu)
Labionin: C₉H₁₆N₃O₆S

"Functional derivatives" ofLabyrinthopeptins are understood peptides exhibiting at least 80%, more preferably at least 90%, more preferably at least 95% sequence identity compared to pre-peptide sequence of LabA1 according to SEQ ID No. 134, of LabA2 according to SEQ No. 137, or LabA3 according to SEQ No. 138, and/or to a pre-pro-peptide sequence of LabA1 according to SEQ ID No. 132, or LabA2 according to SEQ ID No. 135. Preferably, the functional derivatives exhibit at least about 10%, more preferably at least about 50%, even more preferably at least about 90% of the antibacterial, antiviral and/or anti-pain activity of LabA1, LabA2 and/or LabA3, as determined according to Examples 15, 16 and 17 of WO2008/040469A1. In particular, functional derivatives encompass Labyrinthopeptins which contain 1 or more, in particular 1, 2, 3, or 4 amino acids of the leader sequence, but not the complete leader sequence. Functional derivatives are also orthologs of *labA1* or *labA2* from other actinomycetes.

A "pre-pro-peptide" according to the present invention is understood as peptide having the length of the complete translated coding region and which can be obtained by translation by the ribosomal machinery. A pre-pro-peptide includes leader sequences and does not contain post-translational modifications.

A "pro-peptide" according to the present invention is understood as peptide which lacks the leader sequence and which does not contain post-translational modifications.

A "leader sequence" according to the present invention is understood as peptide sequence released during peptide processing in *A. namibiensis* cells. The leader sequence is therefore not present in mature LabA1 or LabA2. The native leader sequences of LabA1 and LabA2 are depicted in SEQ ID No. 133 and SEQ ID No. 136, respectively.

"About" according to the present invention is understood as meaning the experimental error range, in particular ± 5% or ± 10%.

DSM24184 relates to a *Streptomyces lividans* strain deposited with the DSMZ Deutsche Sammlung von Mikroorganismen und Zellkulturen GmbH with Accession number DSM24184. The strain comprises the *lab* gene cluster.

DSM24580 relates to a *Streptomyces lividans* strain deposited with the DSMZ Deutsche Sammlung von Mikroorganismen und Zellkulturen GmbH with Accession number DSM24580. The strain expresses the derivative LabA2_L14A.

DSM24581: relates to a *Streptomyces lividans* strain deposited with the DSMZ Deutsche Sammlung von Mikroorganismen und Zellkulturen GmbH with Accession number DSM24581. The strain expresses the derivative LabA2_F15A.
- SEQ No. 132: represents the sequence of LabA1 pre-pro-peptide.
- SEQ No. 133: represents the sequence of LabA1 leader peptide. SEQ No. 134 represents the sequence of LabA1 pre-peptide, lacking the leader sequence.
- SEQ No. 135: represents the sequence of LabA2 pre-pro-peptide.
- SEQ No. 136: represents the sequence of LabA2 leader peptide.
- SEQ No. 137: represents the sequence of LabA2 pre-peptide, lacking the leader sequence.
- SEQ ID No. 138: represents the sequence of LabA3 pre-peptide.
- SEQ ID No. 139: represents the sequence of the *lab* gene cluster.
- SEQ ID No. 140: represents the sequence of the labA1 gene of *A. namibiensis.*
- SEQ ID No. 141: represents the sequence of the labA2 gene of *A. namibiensis.*
- SEQ ID No. 142: represents the sequence of pUWLab.
- SEQ ID No. 143: represents the sequence of pLab.
- SEQ ID No. 144: represents the sequence of pLabAmp.
- SEQ ID No. 162: represents the sequence of pUWLoriT.

The remaining sequences are described in the Examples below.

### Description of Figures:

- Figure 1:: gene clusters hypothetically coding for Labyrinthopeptin-like peptides.
- Figure 2:: A) A picture represents which part of the *lab* gene cluster was amplified by the use of primer pair I-VI. B) DNA fragments obtained after PCR with primers I-VI for *S. coelicolor* integration mutant. C) Southern blotting, 1-cosmid KH, 2- *S. coelicolor* wt, 3- cl. 3A*, 4) Clone 3B*, 5- Clone 3C*, 6-Clone 3D*, 7- Clone 3E*, 8- Clone 4A*, 9- Clone 4B*, 10- DigVII marker, * *S.coelicolorlcosmid* KH.
- Figure 3:: Primers used for amplification of the *lab* gene cluster for cloning to a vector pUWLoriT.
- Figure 4:: Cloning strategy to generate a vector pUWLab carrying the whole *lab* gene cluster.
- Figure 5:: Verification of a correctness of a vector pUWL by restriction digestion.
- Figure 6:: Detection of LabA2 by a Western-blot. Lanes: 1 and 4) Molecular size marker (10-170 kDa), 2) *S. lividans* (extract of bacterial cells from the MS agar plate), 3) *S. lividans*/*pUWLab* (extract of bacterial cells from the MS agar plate), 5) *A. namibiensis* (supernatant obtained from the liquid culture medium M5294).
- Figure 7:: Detection of LabA1 and LabA2 in a liquid culture of *A. namibiensis* (9 d, medium KM4). A) Total ion chromatogram (TIC), B) MS spectrum of LabA1 and LabA2 (LabA1: [M+2H]²⁺=1038.3; LabA2: [M+2H]²⁺=962.9), C) Extracted Ion Chromatogram over the range *m*/*z* 1037.5-1038.5 corresponding to the doubly-charged LabA1, D) Extracted Ion Chromatogram over the range *m*/*z* 962.0-963.0 corresponding to the doubly-charged LabA2.
- Figure 8:: Detection of LabA1 and LabA2 derivatives in a liquid culture of S. *lividans*/*pUWLab* (9 d, medium YEME). A) Total ion chromatogram (TIC), B) Extracted Ion Chromatogram over the range *m*/*z* 1095.5-1096.5 corresponding to the doubly-charged D-LabA1, C) Extracted Ion Chromatogram over the m/z range 1097.5-1098.5 corresponding to doubly-charged NR-LabA2, D) MS spectrum of D-LabA1 and AD-LabA2 (D-LabAl: [M+2H]²⁺=1096.1; AD-LabA1: [M+2H]²⁺=1131.7), E) MS spectrum of NR-LabA2 (NR-LabAl: [M+2H]²⁺=1098.2).
- Figure 9:: Strategy to obtain construct pLab. pLab is suitable for preparation of different Labyrinthopeptin variants.
- Figure 10:: Cloning strategy to obtain pLabAmp.
- Figure 11:: Cloning strategy used to obtain pLab_SG2/SG3/SG4/SG5/SG6 vectors. A. Restriction digestion of pLabAmp with PasI and Eco47III; isolation of 13.4 kbp fragments from an agarose gel B. Restriction digestion of pMK/pMA carrying synthetic gene (SG) with PasI and Eco47III; isolation of 374 bp (for pMK_SG2)/ 371 bp (pMA_SG3)/ 212bp (pMA_SG4) fragment from a gel C. Ligation of synthetic gene to pLabAmp D. Verification of clones correctness by PCR and sequencing.

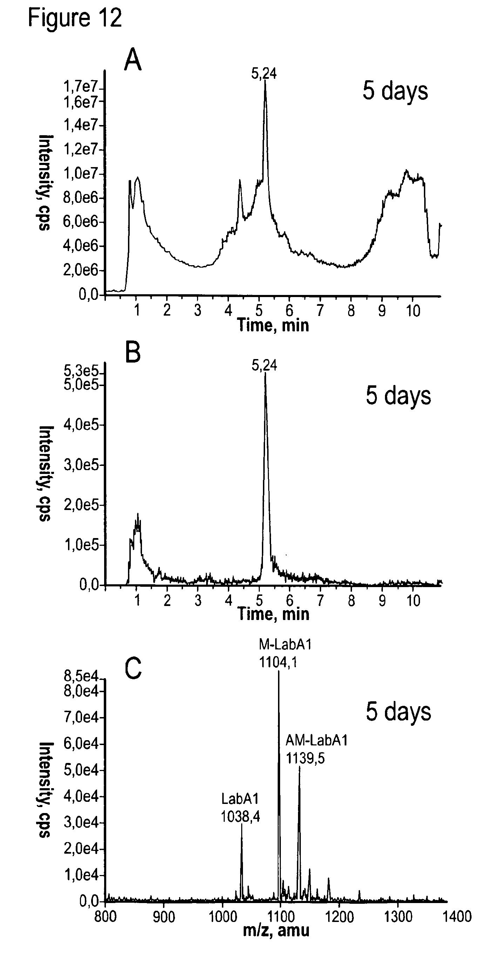

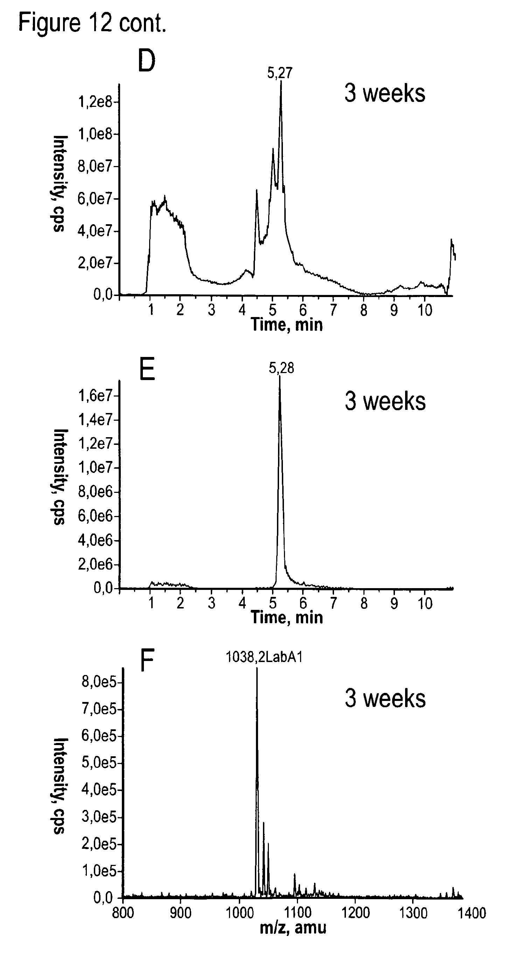

- Figure 12:: Detection of LabA1 and its derivatives in a liquid culture (medium R2YE) of *S. lividans* carrying the vector pLab_SG2 after 5 d and 3 weeks of growth. A) Total ion chromatogram (5 d old culture), B) Extracted Ion Chromatogram over the range *m*/*z* 1037.5-1038.5 corresponding to the doubly-charged LabA1 (5 d old culture), C) MS spectrum of LabA1 and LabA1 derivatives (LabA1: [M+2H]²⁺=1038.4; M-LabA1: [M+2H]²⁺=1104.1; AM-LabA1: [M+2H]²⁺=1139.5) (5 d old culture), D) Total ion chromatogram (3 weeks old culture), E) Extracted Ion Chromatogram over the range *m*/*z* 1037.5-1038.5 corresponding to the doubly-charged LabA1 (3 weeks old culture), F) MS spectrum of LabA1 (LabA1: [M+2H]²⁺=1038.2) (3 weeks old culture).
- Figure 13:: Comparison of amino acid sequences of labyrinthopeptin prepropeptides present in the wild type (pLab), and in synthetic genes SG2 and SG3.
- Figure 14:: Detection of LabA1 and LabA2 derivatives in a liquid culture of *S. lividans* carrying the vector pLab_SG3 (3 weeks, medium R2YE). A) Total ion chromatogram (TIC), B) Extracted Ion Chromatogram over the range *m*/*z* 1037.5-1038.5 corresponding to the doubly-charged LabA1, C) MS spectrum of LabA1 and LabA2 derivatives (LabA1: [M+2H]²⁺=1038.1; A-LabA1: [M+2H]²⁺=1073.7; AA-LabA1: [M+2H]²⁺=109.5; AD-LabA2: [M+2H]²⁺=1055.6).
- Figure 15:: Graphic presentation of synthetic genes SG3, SG3(M) and SG6.
- Figure 16:: Detection of LabA2 and LabA1 derivatives in a liquid culture of *S. lividans*/SG6 (NZ Amine medium, 3 weeks old culture). A) Total ion chromatogram (TIC), B) Extracted Ion Chromatogram over the range *m*/*z* 962.0-963.0 corresponding to the doubly-charged LabA2, C) MS spectrum of LabA2 (LabA2: [M+2H]²⁺=962.13; LabA2: [M+H]⁺=1922.30). D) Extracted Ion Chromatogram over the range *m*/*z* 1115.5-1116.5 corresponding to the doubly-charged R-LabA1, E) MS spectrum of LabA1 derivatives (R-LabAl: [M+2H]²⁺=115.43; NR-LabA1: [M+2H]²⁺=1172.35; ENR-LabA1: [M+2H]²⁺ =1236.74).
- Figure 17:: Comparison of amino acid sequences of labyrinthopeptin prepropeptides present in the wild type (pLab), and in synthetic genes SG4 and SG5.
- Figure 18:: Strategy of preparing different Labyrinthopeptin derivatives. A) Introduction of desired mutation to synthetic oligonucleotide by means of site-directed mutagenesis B, C) Amplification of a fragment between PasI and Eco47III restriction sites (Infusion primers) D) Restriction digestion of plasmid pLab (with enzymes PasI and Eco47III) containing the whole *lab* gene cluster D) Ligation independent cloning (LIC) to obtain a construct for expression of new Labyrinthopeptin derivatives; SG - synthetic genes, * - mutation.
- Figure 19:: LabA1 (A) and LabA2 (B) alanine-scanning mutants prepared for further SAR studies.
- Figure 20:: Comparison of production yields for LabA1 (A, B) and LabA2 (C, D) alanine mutants after 8 (A, C) and 21 (B, D) days of growth in NZ Amine medium. A,B) pLab carrying: 1. SG2, 2. LabA1_N2A, 3. LabA1_W6A, 4. LabA1_E7A, 5. LabA1_T11A, 6. LabA1_V15A, 7. LabA1_P16A, 8. LabA1_F17A; C, D) pLab carrying: 1. SG6, 2. LabA2_D2A, 3. LabA2_E7A, 4. LabA2_T11A, 5. LabA2_L14A, 6. LabA2_F15A.
- Figure 21:: LabA1 mutants prepared to verify A- and A'-ring flexibility. None of labyrinthopeptin A1 derivatives with changes in the A- or A'-ring size (peptide: LabA1_A2del, LabA1_N2insN, LabA1_N2insD, LabA1_A3insA, LabA1_G12del) could be detected by HPLC-MS analysis.
- Figure 22:: LabA1 mutants prepared to verify flexibility of B- and B'-rings. Production of all labyrinthopeptin derivatives with changes in the B- or B'-ring size (peptide: LabA1_V5del, LabA1_W6insV, LabA1/A2, LabA1_P16del, LabA1_P16insV) could be detected by HPLC-MS analysis.
- Figure 23:: Additional substitutions. The HPLC-ESI-MS analysis revealed the production of peptides: LabA1_T11S, LabA1_V15S, LabA1_A3H, LabA1_W6Y. No production of peptide: LabA1_E7R, LabA1_S4A, LabA1_S13A was observed.
- Figure 24:: Mutants prepared to assess the flexibility of the C-ring. The HPLC-ESI-MS analysis revealed the production of peptides: LabA1_C20del, LabA1_SlinsC, LabA1_S10insA and LabA1_C20insA.
- Figure 25:: Graphical representation of LabA1 and LabA2 hybrids. The HPLC-ESI-MS analysis revealed the presence of labyrinthopeptin hybrids: LabA/A2 and LabA2/A1.
- Figure 26:: Graphical presentation of LabA1_ABA'B'AB and LabA1_ABA'B'ABA'B'. Neither production of LabA_ABA'B'AB, nor of LabA1_ABA'B'ABA'B' could be detected by HPLC-ESI-MS analysis.
- Figure 27:: Evaluation of anti-pain activity of labyrinthopeptin A2 and its structural analogs in in vitro assay: IC50 determination for LabA2 (A), LabA2_L14A (B) and LabA2_F 15A (C). IC50 is a concentration of a substance that blocks 50% of channels.
- Figure 28:: Structure of LabA2_L14A.
- Figure 29:: ¹H NMR spectrum of LabA2_L14A.
- Figure 30:: Comparison of the HSQC spectra of LabA2 and its mutant LabA2_L14A: alpha protons region and methyl groups region. Experimental conditions: Tris buffer pD 8.0 at 285 K. A, B) HSQC spectra - alphas area (A: LabA2, B: LabA2_L14A); C, D) HSQC spectra - methyls area (C: LabA2, D: LabA2_L14A).

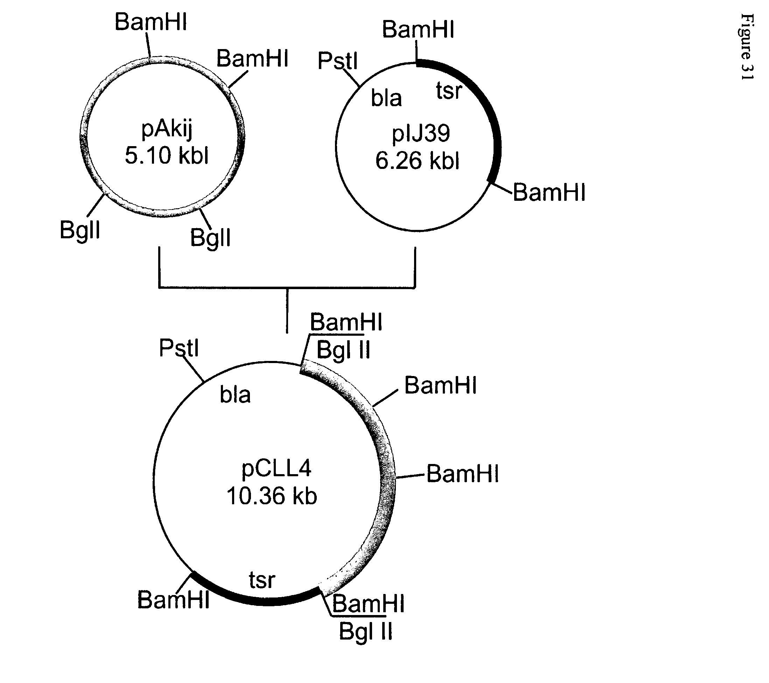

- Figure 31:: Construction of vector pCLL4. pCLL4 is comprising two functional origins of replication: from plasmid pAkijl (*Actinomadura kijanita*) and from plasmid pBR322. Figure from (*90*).

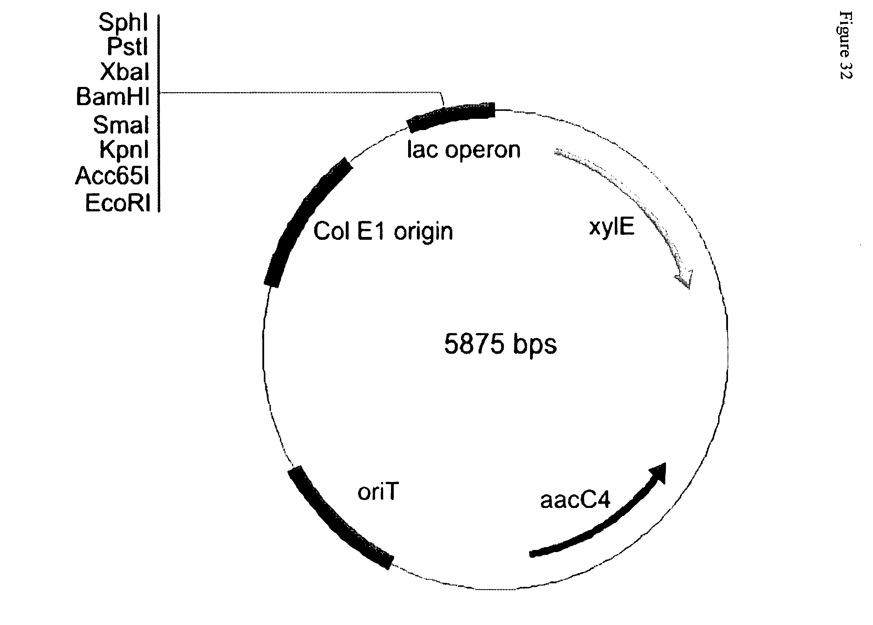

- Figure 32:: Map of pK18mobXylE. pK18mobXylE is a vector for gene inactivation experiments. It was constructed by cloning the gene xylE into pK18mob2.

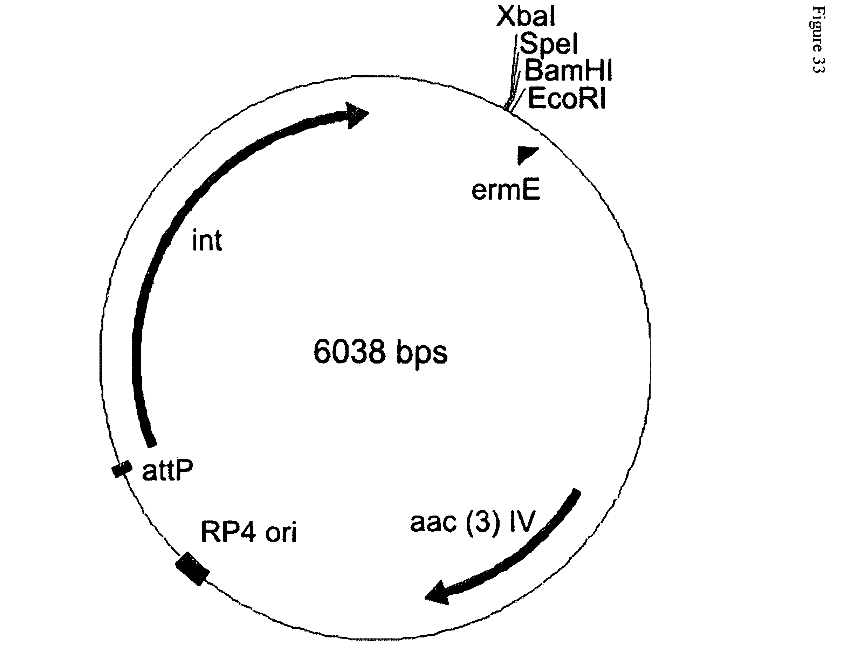

- Figure 33:: Map of pSET152ermE. pSET152ermE can integrate site-specifically at the bacteriophage ΦC31-attachment site (91)(92).

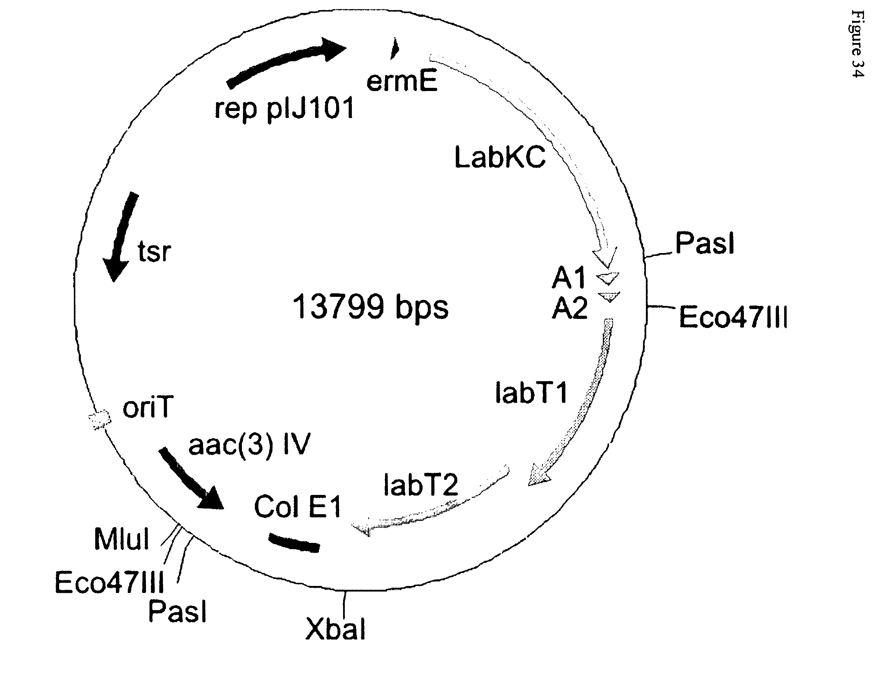

- Figure 34:: Map of pUWLab, carrying the lab gene cluster under a control of the *ermE** promoter.
- Figure 35:: A) Map of pMK_SG2, a vector pMK carrying a synthetic gene SG2, that was used for site-directed mutagenesis of LabA1. B) Map of pMK_SG6, a vector pMK carrying a synthetic gene SG6, that was used for site-directed mutagenesis of LabA2.
- Figure 36:: A) LabA1 isolated from A. namibiensis, MS/MS of m/z 1038.9 [M+2H]2+, B) D-LabA1 produced by S. *lividans*/pUWLab (supernatant from a 9 d culture in YEME medium), MS/MS of m/z 1096.4 [M+2H]2+, C) calculated and found fragment masses for LabA1, D) calculated and found fragment masses for D-LabA1, produced by *S. lividans*/pUWLab (supernatant from a 9 d culture in YEME medium), MS/MS of m/z 1096.4 [M+2H]2+.

### Examples Materials:

### Bacterial strains

| Strain | Genotype/ Phenotype | Reference/ Source |
|---|---|---|
| *E. coli* DH5α | *supE44 ΔlacU169* | (*1*) |
| | (Φ80*lac*ZΔM15) *hsdR17* | |
| | *recA1 endA1 gyrA* | |
| | *thi-1 relA1* | |
| *E. coli* BW25113 | *lacI*q *rrnB*_{T14} *ΔlacZ*_{WJ16} | (*2*) |
| | *hsdR514 ΔaraBA*-*D*_{AH33} | |
| | *ΔrhaBAD*_{LD78} | |
| *E coli* ET12567 | F⁻ *dam-13::Tn9 dmc-6* | (*3*) |
| | *hsdM hsdR lacY1* | |
| *S. coelicolor* M145 | | John Innes Centre, Norwich, UK |
| *S. albus* CB89 | | Combinature Biopharm AG, Berlin, Germany |
| *S. avermitilis* DSM 46492 | | DSMZ, Braunschweig, Germany |
| *S. lividans* ZX7 | | Combinature Biopharm AG, Berlin, Germany |
| *S. griseus* DSM 40236 | | DSMZ, Braunschweig, Germany |
| *A. namibiensis* | Labyrinthopeptins producer | Sanofi-Aventis, Frankfurt am Main, Germany |

### Antibodies

Primary antibody: Polyclonal antibodies produced in rabbits against labyrinthopeptin A2 and labyrinthopeptin A2 leader peptide were generated by BioGenes (Berlin, Germany) according to standard methods.

Secondary antibody: Commercially available Anti-Rabbit IgG Alkaline Phosphatase antibody produced in goat (Sigma-Aldrich Chemie GmbH, München).

### Material:

### Plasmid isolation from Streptomyces

- TESLR: 25 mM Tris-HCl pH 8
25 mM EDTA pH 8
0.3 M sucrose
0.02 % bromocresol green
2 mg/ml lysozyme
5 µg/ml pre-boiled RNase A
- NaOH/SDS: 0.3 N NaOH
2 % SDS
- Acid phenol/ chloroform: 5 g phenol
5 ml chloroform
1 ml H₂O
5 mg 8-hydroxyquinoline

### Genomic DNA isolation from Streptomyces and A. namibiensis

- SET buffer: 75 mM NaCl
25 mM EDTA pH 8.0
20 mM Tris-HCl pH 7.5

### Plasmids

| Plasmid | Characteristic | Reference/ Source |
|---|---|---|
| pK18mob2 | vector for genes inactivation; replicates in *E. coli* but not in *Streptomyces*; *aac(3)IV* | (*4*) / Combinature Biopharm AG, Berlin, Germany |
| pK18mobXylE | vector for genes inactivation; replicates in *E. coli* but not in *Streptomyces*; *xylE*; *aac(3)IV* see Figure 31 | Combinature Biopharm AG, Berlin, Germany |
| pUZ8002 | non-transmissible oriT mobilising plasmid; *tra*, *neo*, RP4 | (*6*) / John Innes Centre, Norwich, UK |
| pSET152ermE | contains *oriT* RK2 for conjugation from *E. coli* to *Streptomyces; ermE** promoter; *attP*, *int*, *aac(3)IV* See Figure 33 | (*7*) / Combinature Biopharm AG, Berlin, Germany |
| pUWLoriT | bifunctional plasmid that replicate in *E. coli* and *Streptomyces*; *ermE** promoter; *aac(3)IV*, *tsr* | (*8*) / Combinature Biopharm AG, Berlin, Germany |
| pMK | vector used for cloning of synthetic genes and for site-directed mutagenesis; *neo* | Geneart AG, Regensburg, Germany |
| pMA | vector used for cloning of synthetic genes and for site-directed mutagenesis; *bla* | Geneart AG, Regensburg, Germany |
| pCLL4 | vector comprising two functional origins of replication: from plasmid pAkijl (*A. kijanita*) and from plasmid pBR322 see Figure 31 | (*9*) / ATTC |
| pK18mob2_labKC | vector pK18mob2 carrying a 1.5 kb fragment of *labKC* gene; replicates in *E. coli* but no in *Streptomyces*; *aac(3)IV* | This studies |
| pSETermEΔHindIII_oriC LL4 | vector pSET152 with an additional 0.9 kb from a vector pCLL4 carrying an origin of replication from *A. kijanita*; *aac(3)IV* | This studies |
| pK18mobXylE_oripCLL4 | vector pK18mobXylE with an additional 0.9 kb from a vector pCLL4 carrying an origin of replication from *A. kijanita*; *aac(3)IV* | This studies |
| pCLL4_ori_apra | vector pCLL4 with an additional fragment containing *oriT* and *aac(3)IV* gene (from a vector pK18mob2); *aac(3)IV*, *tsr* | This studies |
| pUWLab | plasmid carrying the *lab* gene cluster under control of *ermE** promoter; vector replicates in *E*. *coli* and *Streptomyces*; *aac(3)IV*, *tsr* see Figure 34 | This study |

Selective markers: Am, apramycin; Ap, ampicillin; Cm, chloramphenicol; Km, kanamycin; Th, thiostrepton.

### Oligonuceotides and synthetic genes

All the primers were synthesized by biomers.net GmbH (Ulm, Germany). Lyophilized primers were dissolved in sterile H₂Odd to reach a concentration 100 pmol/µl.

| Mutant | | Primers pair | Sequence (5'→3') |
|---|---|---|---|
| Alanine scanning mutagenesis of LabA1: | | | |
| LabA1_N2A | | LabA1_N2A_fw | CCGCCATGAGCGCGGCCAGCGTCTGG (SEQ ID No. 26); |
| | | LabA1N2A_rv | CCAGACGCTGGCCGCGCTCATGGCGG (SEQ ID No. 27) |
| LabA1_V5A | | LabA1_V5A_fw | GCAACGCCAGCGCCTGGGAGTGCTG (SEQ ID No. 28); |
| | | LabA1_V5A_rv | CAGCACTCCCAGGCGCTGGCGTTGC (SEQ ID No. 29) |
| LabA1_W6A | | LabA1_W6A_fw | CAACGCCAGCGTCGCGGAGTGCTGCAG (SEQ ID No. 30); |
| | | LabA1_W6A_rv | CTGCAGCACTCCGCGACGCTGGCGTTG (SEQ ID No. 31) |
| LabA1_E7A | | LabA1_E7A_fw | CAGCGTCTGGGCCTGCTGCAGCACG (SEQ ID No. 32); |
| | | LabA1_E7A_rv | CGTGCTGCAGCAGGCCCAGACGCTG (SEQ ID No. 33) |
| LabA1_T11A | | LabA1_T11A_fw | GAGTGCTGCAGCGCGGGCAGCTGGG (SEQ ID No. 34); |
| | | LabA1_T11A_rv | CCCAGCTGCCCGCGCTGCAGCACTC (SEQ ID No. 3 5) |
| LabA1_V15A | | LabA1_V15A_fw | CACGGGCAGCTGGGCACCCTTCACCTGCT G (SEQ ID No. 36); |
| | | LabA1_V15A_rv | CAGCAGGTGAAGGGTGCCCAGCTGCCCGT G (SEQ ID No. 37) |
| LabA1_F17A | | LabA1_F17A_fw | GCTGGGTTCCCGCCACCTGCTGCTG (SEQ ID No. 3 8); |
| | | LabA1_F17A_rv | CAGCAGCAGGTGGCGGGAACCCAGC (SEQ ID No. 39) |

| Alanine scanning mutagenesis of LabA2: | | | |
|---|---|---|---|
| LabA2_D2A | | LabA2_D2A_fw | CCGCCATGTCCGCCTGGAGCCTGTG (SEQ ID No. 40); |
| | | LabA2_D2A_rv | CACAGGCTCCAGGCGGACATGGCGG (SEQ ID No. 41) |
| LabA2_W3A | | LabA2_W3A_fw | CGCCATGTCCGACGCCAGCCTGTGGGAG (SEQ ID No. 42); |
| | | LabA2_W3A_rv | CTCCCACAGGCTGGCGTCGGACATGGCG (SEQ ID No. 43) |
| LabA2_L5A | | LabA2_L5A_fw | CCGACTGGAGCGCGTGGGAGTGCTG (SEQ ID No. 44); |
| | | LabA2_L5A_rv | CAGCACTCCCACGCGCTCCAGTCGG (SEQ ID No. 45) |
| LabA2_W6A | | LabA2_W6A_fw | CGACTGGAGCCTGGCCGAGTGCTGTAGCA C (SEQ ID No. 46); |
| | | LabA2_W6A_rv | GTGCTACAGCACTCGGCCAGGCTCCAGTC G (SEQ ID No. 47) |
| LabA2_E7A | | LabA2_E7A_fw | GGAGCCTGTGGGCCTGCTGTAGCACG (SEQ ID No. 48); |
| | | LabA2_E7A_rv | CGTGCTACAGCAGGCCCACAGGCTCC (SEQ ID No. 49) |
| LabA2_G12A | | LabA2_G12A_fw | GCTGTAGCACGGCCAGCCTGTTCGCC (SEQ ID No. 50); |
| | | LabA2_G1A_rv | GGCGAACAGGCTGGCCGTGCTACAGC (SEQ ID No. 51) |
| LabA2_L14A | | LabA2_L14A_fw | GCACGGGAAGCGCGTTCGCCTGCTG (SEQ ID No. 52); |
| | | LabA2_L14A_rv | CAGCAGGCGAACGCGCTTCCCGTGC (SEQ ID No. 53) |
| LabA2_F15A | | LabA2_F15A_fw | CACGGGAAGCCTGGCCGCCTGCTGCTG (SEQ ID No. 54); |
| | | LabA2_F15A_rv | CAGCAGCAGGCGGCCAGGCTTCCCGTG (SEQ ID No. 55) |

| Ser/Ser/Cys motif (Ring A and A'): | | | |
|---|---|---|---|
| LabA1_N2insD | | LabA1_N2insD_fw | CCGCCATGAGCGACAACGCCAGCGTC (SEQ ID No. 56); |
| | | LabA1_N2insD_rv | GACGCTGGCGTTGTCGCTCATGGCGG (SEQ ID No. 57) |
| LabA1_N2insN | | LabA1_N2insN_fw | CCATGAGCAACAACGCCAGCGTCTG (SEQ ID No. 58); |
| | | LabA1_N2insN_rv | CAGACGCTGGCGTTGTTGCTCATGG (SEQ ID No. 59) |
| LabA1_A3insA | | LabA1_A3insA_fw | CATGAGCAACGCCGCCAGCGTCTGGGAG (SEQ ID No. 60); |
| | | LabA1_A3insA_rv | CTCCCAGACGCTGGCGGCGTTGCTCATG |
| | | | (SEQ ID No. 61) |
| LabA1_A3del | | LabA1_A3del_fw | CGCCATGAGCAACAGCGTCTGGGAG (SEQ ID No. 62); |
| | | LabA1_A3del_rv | CTCCCAGACGCTGTTGCTCATGGCG (SEQ ID No. 63) |
| LabA1_G12insA | | LabA1_G12insA_fw | GTGCTGCAGCACGGCCGGCAGCTGGGTTC (SEQ ID No. 64) |
| | | LabA1_G12insA_rv | GAACCCAGCTGCCGGCCGTGCTGCAGCAC (SEQ ID No. 65) |
| LabA1_G12del | | LabA1_G12del_fw | GTGCTGCAGCACGAGCTGGGTTCCC (SEQ ID No. 66; |
| | | LabA1_G12del_rv | GGGAACCCAGCTCGTGCTGCAGCAC (SEQ ID No. 67) |

| Ser/Ser/Cys motif (Ring B and B'): | | | |
|---|---|---|---|
| LabA1_W6insV | | LabA1_W6insV_fw | CAACGCCAGCGTCGTCTGGGAGTGCTGC (SEQ ID No. 68); |
| | | LabA1_W6insV_rv | GCAGCACTCCCAGACGACGCTGGCGTTG (SEQ ID No. 69) |
| LabA1_W6insL | | LabA1_W6insL_fw | CAACGCCAGCGTCCTGTGGGAGTGCTGC (SEQ ID No. 70); |
| | | LabA1_W6insL_rv | GCAGCACTCCCACAGGACGCTGGCGTTG (SEQ ID No. 71) |
| LabA1_V5del | | LabA1_V5del_fw | GAGCAACGCCAGCTGGGAGTGCTGC (SEQ ID No. 72); |
| | | LabA1_V5del_rv | GCAGCACTCCCAGCTGGCGTTGCTC (SEQ ID No. 73) |
| LabA1_P16del | | LabA1_P16del_fw | GGGCAGCTGGGTTTTCACCTGCTGC (SEQ ID No. 74); |
| | | LabA1_P16del- rv | GCAGCAGGTGAAAACCCAGCTGCCC (SEQ ID No. 75) |
| LabA1_T18del | | LabA1_T18del_fw | CTGGGTTCCCTTCTGCTGCTGACGC (SEQ ID No. 76); |
| | | LabA1_T18del_rv | GCGTCAGCAGCAGAAGGGAACCCAG (SEQ ID No. 77) |
| LabA1_P16insV | | LabA1_P16insV_fw | GCAGCTGGGTTGTCCCCTTCACCTG (SEQ ID No. 78); |
| | | LabA1_P16insV_rv | CAGGTGAAGGGGACAACCCAGCTGC (SEQ ID No. 79) |
| LabA1_VP15del | | LabA1_VP15del_fw | CACGGGCAGCTGGTTCACCTGCTGC (SEQ ID No. 80); |
| | | LabA1_VP15del_rv | GCAGCAGGTGAACCAGCTGCCCGTG (SEQ ID No. 81) |

| Substitute Ser with Thr: | | | |
|---|---|---|---|
| LabA1_S1T | | LabA1_S1T_fw | GGCCGCCATGACGAACGCCAGCGTC (SEQ ID No. 82); |
| | | LabA1_S1T_rv | GACGCTGGCGTTCGTCATGGCGGCC (SEQ ID No. 83) |
| LabA1_S4T | | LabA1S10T_fw | CATGAGCAACGCCACCGTCTGGGAGTGC (SEQ ID No. 84); |
| | | LabA1_S10T_rv | GCACTCCCAGACGGTGGCGTTGCTCATG (SEQ ID No. 85) |
| LabA1_S13T | | LabA_S13T_fw | GCAGCACGGGCACCTGGGTTCCCTTC (SEQ ID No. 86); |
| | | LabA1_S13T_rv | GAAGGGAACCCAGGTGCCCGTGCTGC (SEQ ID No. 87) |

| Express only east part of LabA1: | | | |
|---|---|---|---|
| LabA1_C9tga | | LabA1_C9tga_fw | CGTCTGGGAGTGCTGAAGCACGGGCAGCT G (SEQ ID No. 88); |
| | | LabA1_C9tga_rv | CAGCTGCCCGTGCTTCAGCACTCCCAGAC G (SEQ ID No. 89) |

| Additional substitutions: | | | |
|---|---|---|---|
| LabA1_S4A | | LabA1_S4A_fw | CATGAGCAACGCCGCCGTCTGGGAGTG (SEQ ID No. 90); |
| | | LabA1_S4A_rv | CACTCCCAGACGGCGGCGTTGCTCATG (SEQ ID No. 91) |
| LabA1_S13A | | LabA1_S13A_fw | GTGCTGCAGCACGGGCGCCTGGGTTCCCT (SEQ ID No. 92) |
| | | LabA1_S13A_rv | TCAC;GTGAAGGGAACCCAGGCGCCCGTG |
| | | | CTGCAGCAC (SEQ ID No. 93) |
| LabA1_V5T | | LabA1_V5T_fw | GAGCAACGCCAGCACCTGGGAGTGCTG (SEQ ID No. 94); |
| | | LabA1_V5T_rv | CAGCACTCCCAGGTGCTGGCGTTGCTC (SEQ ID No. 95) |
| LabA1_V15S | | LabA1_V15S_fw | CACGGGCAGCTGGTCCCCCTTCACCTGC (SEQ ID No. 96); |
| | | LabA1_V15S_rv | GCAGGTGAAGGGGGACCAGCTGCCCGTG (SEQ ID No. 97) |
| LabA1_W6Y | | LabA1_W6Y_fw | CAACGCCAGCGTCTACGAGTGCTGCAGCA C (SEQ ID No. 98); |
| | | LabA1_W6Y_rv | GTGCTGCAGCACTCGTAGACGCTGGCGTT G (SEQ ID No. 99) |
| LabA1_A3H | | LabA1_A3H_fw | GCCATGAGCAACCACAGCGTCTGGGAG (SEQ ID No. 100); |
| | | LabA1_A3H_rv | CTCCCAGACGCTGTGGTTGCTCATGGC (SEQ ID No. 101) |
| LabA1_E7R | | LabA1_E7R_fw | CCAGCGTCTGGCGGTGCTGCAGCAC (SEQ ID No. 102); |
| | | LabA1_E7R_rv | GTGCTGCAGCACCGCCAGACGCTGG (SEQ ID No. 103) |

| Ring C: | | | |
|---|---|---|---|
| LabA1_C20insA | | LabA1_C20insA_fw | CCTTCACCTGCGCCTGCTGACGCCC (SEQ ID No. 104); |
| | | LabA1_C20insA_rv | GGGCGTCAGCAGGCGCAGGTGAAGG (SEQ ID No. 105) |
| LabA1_S10insA | | LabA1_S10insA_fw | GGGAGTGCTGCGCCAGCACGGGCAG (SEQ ID No. 106); |
| | | LabA1_S10insA_rv | CTGCCCGTGCTGGCGCAGCACTCCC (SEQ ID No. 107) |
| LabA1_C20del | | LabA1_C20del_fw | GTTCCCTTCACCTGCTGACGCCCGCACAC (SEQ ID No. 108); |
| | | LabA1_C20del_rv | GTGTGCGGGCGTCAGCAGGTGAAGGGAA C (SEQ ID No. 109) |
| LabA1S1insC | LabA1_S1insC_fw | CGGCCGCCATGTGCAGCAACGCCAG (SEQ ID No. 110); | |
| | LabA1_S1insC_rv | CTGGCGTTGCTGCACATGGCGGCCG (SEQ ID No. 111) | |
| Spacer: | | | |
| LabA1_C9insV | LabA1_C9insV_fw | GTCTGGGAGTGCGTCTGCAGCACGGG (SEQ ID No. 112); | |
| | LabA1_C9insV_rv | CCCGTGCTGCAGACGCACTCCCAGAC (SEQ ID No. 113) | |
| LabA1_C9insV N | LabA1_C9insVN_fw | GGGAGTGCGTCAACTGCAGCACGGG (SEQ ID No. 114); | |
| | LabA1_C9insVN_rv | CCCGTGCTGCAGTTGACGCACTCCC (SEQ ID No. 115) | |

| Primer | Sequence (5'→3') | | Description |
|---|---|---|---|
| attB_For | CGGTCTCGAAGCCGCGGTGC (SEQ ID No. 116) | | verification of a presence of phi att sites in *A. namibiensis* |
| attB_Rev | GCCCGCCGTGACCGTCGAG (SEQ ID No. 117) | | |
| p18mob_EcoRI_for | CATCTCGAATTCCGCTCATGAG CTCAG (SEQ ID No. 118) | | amplify a fragment containing *oriT* and aac(3)IV; used to |
| p18mob_EcoRI_rev | GTTATCGAGATCTGCAGGAGC TCTTTGG (SEQ ID No. 119) | | generate a vector pCLL4_ori_apra |
| pSETproof_for | CGAGCCGGAAGCATAAAGTG (SEQ ID No. 120) | | verification of a correctness of an exconjugant *A. namibiensis*/ |
| pSETproof_rev | GCTTGGAGCGAACGACCTAC (SEQ ID No. 121) | | pSET152 |
| STK_RTPCR_fw | agcagcaagtacgccgaacg (SEQ ID No. 122) | | amplify a fragment of *labKC* gene; used to generate a vector |
| STK_RTPCR_rv | gcgaagtggagctggttgag (SEQ ID No. 123) | | pDrive_labKC |
| pMA_seq | tgtgctgcaaggcgattaag (SEQ ID No. 124) | | sequencing primer; confirmation of correctness of pMK_SGm after site-directed mutagenesis |
| Infusion 1 (fw) | | gtcgaggcggccctgggcggccaccccctgag ac (SEQ ID No. 125) | |
| Infusion 2 (rv) | | gcggcctcggtcagcgctgttcagcagcaggcg aacagg (SEQ ID No. 126) | |
| Infusion 3 (rv) | | tcggcggcctcggtcagcgctgttcagcagcagg tg (SEQ ID No. 127) | |
| Infusion SG17 (rv) | | CGGCGGCCTCGGTCAGCGCTG TTCAGCAGCACCAG (SEQ ID No. 128) | Primers used for amplification of an insert SG(M) for ligation independent cloning |
| Infusion SG18 (rv) | | TCGGCGGCCTCGGTCAGCGCT GTTCAGCAGCAGAG (SEQ ID No. 129) | |
| Infusion SG19 (rv) | | TTCGGCGGCCTCGGTCAGCGC TGTTCAGCAACAGG (SEQ ID No. 131) | |
| Infusion SG20 (rv) | | CGGCGGCCTCGGTCAGCGCTG TTCAGCAGGCGAAC (SEQ ID No. 131) | |

All synthetic genes were synthesized by Geneart AG (Regensburg, Germany). Sequences of synthetic genes are summarized below.
aac(3)IV

ACGCGTCGATTATCTCGAGAATGACCACTGCTGTGAGCGGTTTGCCTTGGCGGACAG
GTGGCTCAAGGAGAAGAGCCTTCAGAAGGAAGGTCCAGTCGGTCATGCCTTTGCTC
GGTTGATCCGCTCCCGCGACATTGTGGCGACAGCCCTCGGTCAACTGGGCCGAGATC
CGTTGATCTTCCTGCATCCGCCAGAGGCGGATGCGAAGAATGCGATGCCGCTCGCC
AGTCGATTGGCTGAGCTCATGAGCGGAGAACGAGATGACGTTGGAGGGGCAAGGTC
GCGCTGATTGCTGGGGCAACACGTGGAGCGGATCGGGGATTGTCTTTCTTCAGCTCG
CTGATGATATGCTGACGCTCAATGCGCCTCACTGATTAAGCATTGGTAACTGTCAGA
CCAAGTTTACTCATATATACTTTAGATTGATTTAAAACTTCATTTTTAATTTAAAAGG

ATCTAGGTGAAGATCCTTTTTGATAATCTCATGACCAAAATCCCTTAACGTGAGTTTT
CGTTCCACTGAGCGTCAGACCCCGTAGAAAAGATCAAAGGATCTTCTTGAGATCCTT
TTTTTCTGCGCGTAATCTGCTGCTTGCAAACAAAAAAACCACCGCTACCAGCGGTGG
TTTGTTTGCCGGATCAAGAGCTACCAACTCTTTTTCCGAAGGTAACTGGCTTCAGCA
GAGCGCAGATACCAAATACTGTTCTTCTAGTGTAGCCGTAGTTAGGCCACCACTTCA
AGAACTCTGTAGCACCGCCTACATACCTCGCTCTGCTAATCCTGTTACCAGTGGCTG
CTGCCAGTGGCGATAAGTCGTGTCTTACCGGGTGGACTCAAGACGATAGTTACCGG
ATAAGGCGCAGCGGTCGGGCTGAACGGGGGGTTCGTGCACACAGCCCAGCTTGGAG
CGAACGACCTACACCGAACTGAGATACCTACAGCGTGAGCTATGAGAAAGCGCCAC
GCTTCCCGAAGGGAGAAAGGCGGACAGGTATCCGGTAAGCGGCAGGGTCGGAACA
GGAGAGCGCACGAGGGAGCTTCCAGGGGGAAACGCCTGGTATCTTTATAGTCCTGT
CGGGTTTCGCCACCTCTGACTTGAGCGTCGATTTTTGTGATGCTCGTCAGGGGGGCG
GAGCCTATGGAAAAACGCCAGCACGCGGCCTTTTTACGGTTCCTGGCCTTTTGCTG
GCCTTTTGCTCACATGTTCTTTCCTGCGTTATCCCCTGATTCTGTGGATAACCGTATT
ACCGCCTTTGAGTGAGCTGATACCGCTCGCCGCAGCCGAACGACCGAGCGCAGCGA
GTCAGTGAGCGAGGAAGCGGAAGAGCGCCCAATACGCAAACCGCCTCTCCCCGCGC
GTTGGCCGATTCATTAATGCAGAGCTTCTAGA (SEQ ID No. 25)

XbaI (TCTAGA) and MluI (ACGCGT) are underlined. Changed PasI (CCCTCGG) and Eco47III (AGCGGT) restriction sites are underlined.

In the following sequences, the respective pre-peptide sequence is shown in bold. PasI (CCCTGGG) and Eco47III (AGCGCT) restriction sites are underlined. labA1 gene is shown in black letters, *labA2* gene is marked with white letters with black background. Propeptide sequences are shown in bold.

SG2 (SEQ ID No. 1)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCTGCAGCACGGGCAGCTGGGTTCCCTTCACCTGCTGCTGACGCCCGCACACCG
TTCCACCGATGAGAGGTGACAGTCCCATGGCGTCAATATTGGAACTCCAGAACCTG
GACGTCGAGCACGCCCGCGGCGAGAACCGCATGTCCGACTGGAGCCTGTGGGAGTG
CTGTAGCACGGGAAGCCTGTTCGCCTGCTGCTGAACAGCGCT

SG3 (SEQ ID No. 2)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCGCCAGCAACGCCAGCGTCTGGG
AGTGCTGCAGCACGGGCAGCTGGGTTCCCTTCACCTGCTGCTGACGCCCGCACACCG
TTCCACCGATGAGAGGTGACAGTCCCATGGCATCCATCCTTGAGCTCCAGGACCTGG
AGGTCGAGCGCGCCAGCTCGGCCGCCGACTCCGACTGGAGCCTGTGGGAGTGCTGT
AGCACGGGAAGCCTGTTCGCCTGCTGCTGAACAGCGCT

SG4 (SEQ ID No. 3)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCGACAGCAACGCCAGCGTCTGGG
AGTGCTGCAGCACGGGCAGCTGGGTTCCCTTCACCTGCTGCTGAACAGCGCT

SG5 (SEQ ID No. 4)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGTCCGACTGGAGCCTGTGGG
AGTGCTGTAGCACGGGAAGCCTGTTCGCCTGCTGCTGAACAGCGCT

SG6 (SEQ ID No. 5)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGTCCGACTGGAGCCTGTGGG
AGTGCTGTAGCACGGGAAGCCTGTTCGCCTGCTGACGCCCGCACACCGTTCCAC
CGATGAGAGGTGACAGTCCCATGGCGTCGATCCTGGAACTCCAGAACCTGGACGTC
GAGCACGCCCGCGGCGAGAACCGCAGCAACGCCAGCGTCTGGGAGTGCTGCAGCAC
GGGCAGCTGGGTTCCCTTCACCTGCTGCTGAACAGCGCT

SG7 (SEQ ID No. 6)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA

GGACCTGGAGGTCGAGCGCCAGCTCGGCCGCCATGTGCAGCACGGGCAGCTGGG
TTCCCTTCACCTGCTGCTGACGCCCGCACACCGTTCCACCGATGAGAGGTGACAGTC
CCATGGCGTCGATCCTGGAACTCCAGAACCTGGACGTCGAGCACGCCCGCGGCGAG
AACCGCATGTCCGACTGGAGCCTGTGGGAGTGCTGTAGCACGGGAAGCCTGTTCGC
CTGCTGCTGAACAGCGCT

SG8 (SEQ ID No. 7)
CCCTGGGCGGCCACCCCCTGAGACTCCCCTTCCTCCATCCGGAGGAAGGGGCGAGC
ACGCGACCCCGGGGGAGGAGGTGAACATCCACCatggcacttctcgacctgcagacgatggaagccgac
gagacgaccggtaccggcgggcccagctccctgagcgtgctgtcctgtgtgagcgcggccagcatcacgctctgcctctgaCGCCC
GCACACCGTTCCACCGATGAGAGGTGACAGTCCCATGGCGTCAATATTGGAACTCC
AGAACCTGGACGTCGAGCACGCCCGCGGCGAGAACCGCATGTCCGACTGGAGCCTG
TGGGAGTGCTGTAGCACGGGAAGCCTGTTCGCCTGCTGCTGAACAGCGCT

RamS2 prepropeptide sequence from *S. scabiei* is marked in small letters.

SG9 (SEQ ID No. 8)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCTGCAGCACGGGCAGCTGGGTTCCCTTCACCTGCTGCAGCAACGCCAGCGTCT
GGGAGTGCTGACGCCCGCACACCGTTCCACCGATGAGAGGTGACAGTCCCATGGCG
TCAATATTGGAACTCCAGAACCTGGACGTCGAGCACGCCCGCGGCGAGAACCGCAT
GTCCGACTGGAGCCTGTGGGAGTGCTGTAGCACGGGAAGCCTGTTCGCCTGCTGCTG
AACAGCGCT

SG10 (SEQ ID No. 9)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCTGCAGCACGGGCAGCTGGGTTCCCTTCACCTGCTGCAGCAACGCCAGCGTCT
GGGAGTGCGCCAGCACGGGCAGCTGGGTTCCCTTCACCTGCGCCTGACGCCCGCAC
ACCGTTCCACCGATGAGAGGTGACAGTCCCATGGCGTCAATATTGGAACTCCAGAA
CCTGGACGTCGAGCACGCCCGCGGCGAGAACCGCATGTCCGACTGGAGCCTGTGGG
AGTGCTGTAGCACGGGAAGCCTGTTCGCCTGCTGCTGAACAGCGCT

SG11 (SEQ ID No. 10)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCGACAGCAACGCCAGCGTCTGGG
AGTGCTGTAGCACGGGAAGCCTGTTCGCCTGCTGCTGACGCCCGCACACCGTTCCAC
CGATGAGAGGTGACAGTCCCATGGCGTCGATCCTGGAACTCCAGAACCTGGACGTC
GAGCACGCCCGCGGCGAGAACCGCTCCGACTGGAGCCTGTGGGAGTGCTGCAGCAC
GGGCAGCTGGGTTCCCTTCACCTGCTGCTGAACAGCGCT

SG12 (SEQ ID No. 11)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCTGGTGCT
GCAGCACGGGCAGCTGGGTTCCCTTCACCTGCTGCTGACGCCCGCACACCGTTCCAC
CGATGAGAGGTGACAGTCCCATGGCGTCGATCCTGGAACTCCAGAACCTGGACGTC
GAGCACGCCCGCGGCGAGAACCGCAGCAACGCCAGCGTCGTCGCCTGGGAGTGCTG
CAGCACGGGCAGCTGGGTTCCCTTCACCTGCTGCTGAACAGCGCT

SG13 (SEQ ID No. 12)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCTGCAGCACGGGCAGCTGGACCTGCTGCTGACGCCCGCACACCGTTCCACCG
ATGAGAGGTGACAGTCCCATGGCGTCGATCCTGGAACTCCAGAACCTGGACGTCGA
GCACGCCCGCGGCGAGAACCG AGCAACGCCAGCGTCTGGGAGTGCTGCAGCACGG
GCAGCTGGGTTGCCGTCCCCTTCACCTGCTGCTGAACAGCGCT

SG14 (SEQ ID No. 13)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCGCCAGCACGGGCAGCTGGGTTCCCTTCACCTGCGCCTGACGCCCGCACACCG
TTCCACCGATGAGAGGTGACAGTCCCATGGCGTCGA

AACGCCGTCAGCTGGGAGAGCTG
CAGCACGGGCAGCTGGGTTCCCTTCACCTGCTGCTGAACAGCGCT

SG15 (SEQ ID No. 14)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCGTCA
GCGCCTGGGAGTGCTGCAGCACGGGCAGCTGGGTTCCCTTCACCTGCTGCTGACGCC
CGCACACCGTTCCACCGATGAGAGGTGACAGTCCCATGGCGTCGATCCTGGAACTCC
AGAACCTGGACGTCGAGCACGCCCGCGGCGAGAACCGCAGCAACGCCAGCGTCTGG
GAGTGCTGCAGCACGGGCAGCTGGGTTAGCCCCTTCACCTGCTGCTGAACAGCGCT

SG16 (SEQ ID No. 15)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCTGCAGCAGCGGCAGCTGGGTTCCCTTCACCTGCTGCTGACGCCCGCACACCG
TTCCACCGATGAGAGGTGACAGTCCCATGGCGTCGATCCTGGAACTCCAGAACCTG
GACGTCGAGCACGCCCGCGGCGAGAACCGCAGCAACGCCAGCTGCTGCAGCACGGG
CAGCTGGGTTCCCTTCACCTGCTGCTGAACAGCGCT

SG17 (SEQ ID No. 16)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCTGCAGCACGGGCAGCTGGGTTCCCTTCAGCTGCTGCTGACGCCCGCACACCG
TTCCACCGATGAGAGGTGACAGTCCCATGGCGTCGATCCTGGAACTCCAGAACCTG
GACGTCGAGCACGCCCGCGGCGAGAACCGCAGCAACGCCAGCGTCTGGGAGTGCTG
CAGCACGGGCAGCTGGTGCTGCTGAACAGCGCT

SG18 (SEQ ID No.17)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGagctccctgagcgtgctgtcctgtgtgagcg
cggccagcatcacgctctgcctctgaCGCCCGCACACCGTTCCACCGATGAGAGGTGACAGTCCCA

TGGCGTCGATCCTGGAACTCCAGAACCTGGACGTCGAGCACGCCCGCGGCGAGAAC
CGCagctccctgagcgtgctgtcctgttgcagcgcggccagcatcacgctctgctgctgaACAGCGCT

RamS2 prepeptide sequence from *S. scabiei* is marked in small letters.

SG19 (SEQ ID No. 18)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGaccggcagccgcgcgagcctcctgctctgc
ggcgacagcagcctgagcatcaccacctgtaactgaCGCCCGCACACCGTTCCACCGATGAGAGGTGAC
AGTCCCATGGCGTCGATCCTGGAACTCCAGAACCTGGACGTCGAGCACGCCCGCGG
CGAGAACCGCaccggcagccgcgcgagcctcctgctctgctgcagcagcctgagcatcaccacctgttgctgaACAGCGC
T

SapB prepeptide sequence from *S. coelicolor* is marked in small letters.

SG20 (SEQ ID No. 19)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCATGTGCAGCACGGGCAGCTGGGTTCCCTTCACCTGCTGCTGACGCCCGCACA
CCGTTCCACCGATGAGAGGTGACAGTCCCATGGCGTCAATATTGGAACTCCAGAAC
CTGGACGTCGAGCACGCCCGCGGCGAGAACCGCTCCGACTGGAGCCTGTGGGAGTG
CATGAGCACGGGAAGCCTGTTCGCCTGCTGAACAGCGCT

LabA2_T11A (SEQ ID No. 20)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGTCCGACTGGAGCCTGTGGG
AGTGCTGTAGCGCCGGAAGCCTGTTCGCCTGCTGCTGACGCCCGCACACCGTTCCAC
CGATGAGAGGTGACAGTCCCATGGCGTCGATCCTGGAACTCCAGAACCTGGACGTC
GAGCACGCCCGCGGCGAGAACCGCAGCAACGCCAGCGTCTGGGAGTGCTGCAGCAC
GGGCAGCTGGGTTCCCTTCACCTGCTGCTGAACAGCGCT

LabA1_G12A (SEQ ID No. 21)

CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCTGCAGCACGGCCAGCTGGGTTCCCTTCACCTGCTGCTGACGCCCGCACACCG
TTCCACCGATGAGAGGTGACAGTCCCATGGCGTCAATATTGGAACTCCAGAACCTG
GACGTCGAGCACGCCCGCGGCGAGAACCGCATGTCCGACTGGAGCCTGTGGGAGTG
CTGTAGCACGGGAAGCCTGTTCGCCTGCTGCTGAACAGCGCT

LabA1_W14A (SEQ ID No. 22)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCTGCAGCACGGGCAGCGCCGTTCCCTTCACCTGCTGCTGACGCCCGCACACCG
TTCCACCGATGAGAGGTGACAGTCCCATGGCGTCAATATTGGAACTCCAGAACCTG
GACGTCGAGCACGCCCGCGGCGAGAACCGCATGTCCGACTGGAGCCTGTGGGAGTG
CTGTAGCACGGGAAGCCTGTTCGCCTGCTGCTGAACAGCGCT

LabA1_P16A (SEQ ID No. 23)
CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCTGCAGCACGGGCAGCTGGGTTGCCTTCACCTGCTGCTGACGCCCGCACACCG
TTCCACCGATGAGAGGTGACAGTCCCATGGCGTCAATATTGGAACTCCAGAACCTG
GACGTCGAGCACGCCCGCGGCGAGAACCGCATGTCCGACTGGAGCCTGTGGGAGTG
CTGTAGCACGGGAAGCCTGTTCGCCTGCTGCTGAACAGCGCT

LabA1_T18A (SEQ ID No. 24)

CCCTGGGCGGCCACCCCCTGAGACTCCCCTTCCTCCATCCGGAGGAAGGGGCGAGC
ACGCGACCCCGGGGGAGGAGGTGAACATCCACCATGGCATCCATCCTTGAGCTCCA
GGACCTGGAGGTCGAGCGCGCCAGCTCGGCCGCCATGAGCAACGCCAGCGTCTGGG
AGTGCTGCAGCACGGGCAGCTGGGTTCCCTTCGCCTGCTGCTGACGCCCGCACACCG
TTCCACCGATGAGAGGTGACAGTCCCATGGCGTCAATATTGGAACTCCAGAACCTG
GACGTCGAGCACGCCCGCGGCGAGAACCGCATGTCCGACTGGAGCCTGTGGGAGTG
CTGTAGCACGGGAAGCCTGTTCGCCTGCTGCTGAACAGCGCT

### Methods

Standard molecular biology methods were used according to Sambrook and colleagues (1989) and are not described here in detail. When temperature conditions are not specified then it means that an experiment was performed at room temperature.

### Microbiological methods:

### Growth conditions and preservation of E. coli

*E. coli* strains were grown in LB medium supplemented with appropriate antibiotics. The cultures were incubated at 37 °C with shaking at 160 rpm. *E. coli* were stored at -80 °C in 10% glycerol.

### Growth conditions and preservation of Streptomyces and A. namibiensis

*Streptomyces* and *Actinomadura namibiensis* were grown on different agar media (like MS, R2YE, R5, KM4) at 28 °C and in liquid media (like YEME, CRM, KM4, M5294) at 28 °C shaking at 160 rpm. 50 ml bacterial culture was cultivated in 250 ml or 300 ml flask with a coil for a better aeration. *Streptomyces* containing recombinant plasmids were grown in presence of apramycin (25 µg/ml or 50 µg/ml). For first cultures prepared after conjugation *Streptomyces* were grown also with nalidixic acid (25 µg/ml). For cultivation of *A. namibiensis* no antibiotics were used. *Streptomyces* as well as *A. namibiensis* were stored at -80 °C in 10 % glycerol with addition of 5 % sucrose.

### Molecular biological methods:

### Preparation of plasmid DNA from E. coli

*E. coli* plasmid DNA was purified using the GeneJET™ Plasmid Miniprep Kit (Fermentas) according to the producer's protocol.

### Preparation of plasmid DNA from Streptomyces

Plasmid DNA from *Streptomyces* was isolated by alkaline lysis and phenol precipitation according to the protocol described by Kieser et al., 2000 (10). First step is resuspension of mycelium from 5 ml culture in a total volume of 500 µl TESLR and incubation for 30 min at 37 °C. Then 250 µl ofNaOH.3/SDS is added and the mixture is immediately vortex. After 15 min incubation of open tubes at 70 °C they need to be cooled to 37 °C. The next step is addition of 80 III acid phenol/chloroform, emulsification by vortexing and centrifugation for 10 min. Now supernatant (700 µl) is transferred to a new centrifuge tube containing 50 µl sodium acetate and 450 µl isopropanol. Such mixture is vortex and centrifuge for 10 min. The obtained precipitate is dissolved now in 70 µl TE. This is followed by addition of 3M potassium acetate (14 µl), acid phenol/ chloroform (14 µl), emulsification and vortexing. Then upper aqueous phase is transferred to a new tube containing 56 µl of 3M potassium acetate and 630 µl of TE. Afterwards 460 µl isopropanol is added and the mixture is centrifuged for 5 min to get a pellet which should be washed with 1.2 ml 0 °C ethanol and dissolved in 150 µl TE. 150 µl 5M ammonium acetate is added to DNA solution which is then mixed and centrifuged. After that supernatant (pellet should be discarded) is transferred to a new tube. This is followed by addition of 660 µl ethanol and centrifugation for 10 min. At the very end obtained pellet is washed with 1 ml 70% ethanol, dried and dissolved in 50 µl TE.

### Preparation of genomic DNA from Streptomyces

For preparation of genomic DNA from *Streptomyces* salting out procedure described by Kieser et al., 2000 (*10*) was applied. 30 ml of bacterial culture is grown in standard *Streptomyces* medium (like TSB, CRM, YEME) with glycine (end conc. 0.5 %) in 100 ml flasks. At the beginning mycelium from 7 ml of culture needs to be resuspended in 1.25 ml SET buffer. Then 25 µl of lysozyme solution is added and such mixture is incubated for 60 min at 37 °C (final lysozyme concentration is 2 mg/ml). The next step is addition of 35 µl proteinase K solution (final proteinase conc. is 0.5 mg/ml), mixing, addition of 150 µl 10 % SDS (final SDS conc. is 1 %), mixing by inversion and incubation for 2 h at 55 °C. It is important to invert occasionally the sample during the whole incubation time. Afterwards 500 µl of 5 M NaCl is added, the sample is mixed thoroughly by inversion and cooled to 37 °C. Next 1.25 ml of chloroform is added. Such prepared mixture needs to be mixed by inversion for 30 min at 20 °C. Then after 15 min centrifugation (4500 x g at 20 °C) the supernatant is transferred to a fresh tube. This is followed by addition of 0.6 vol isopropanol. After 3 min of mixing by inversion DNA can be spooled onto a sealed Pasteur pipette, rinsed in 1.25 ml 70 % ethanol and air dried. At the end DNA is dissolved in 100 µl water at 55 °C.

### Measuring DNA concentration

DNA concentration was measured by means of UV spectrophotometry at λ=260 nm (Ultrospec 2100 pro Classic; Amersham Pharmacia Biotech, Freiburg, Germany). Samples were 16 times diluted (5µl sample in 75 µl water). Pure DNA yields an A260/A280 ratio of 1.8 - 1.9.

### Agarose gel electrophoresis

Agarose gel electrophoresis was used for separation of mixed population of DNA fragments by length and for estimation of their size. Nucleic acid molecules are separated by applying an electric field to move the negatively charged molecules through an agarose matrix. Shorter molecules move faster and migrate farther than longer ones because shorter molecules migrate more easily through the pores of the gel. Samples were prepared by addition of 10 % of a loading buffer. The loading buffer gives color and density to the sample to make it easy to load into the wells. Different agarose concentrations were employed: 2 % agarose gels for DNA fragments smaller that 1 kp, 0.8% agarose gels for DNA fragments obtained after restriction digestion of cosmids. For all the other purposes 1 % agarose gels were prepared. Gels were run in a horizontal tank (Mini-Sub Cell GT; Bio-Rad Laboratories GmbH, München, Germany) in TAE buffer (Tris/Acetate/EDTA). Separation of DNA fragments was achieved by using a voltage between 50 V and 90 V. After electrophoresis the gel was incubated in an ethidium bromide bath. DNA bands were visualized by the use of UV light (λ=315 nm). As a molecular weight marker 2-log DNA marker (New England Biolabs, Frankfurt am Main, Germany) was used.

### DNA extraction from agarose gels

DNA fragments were isolated from an agarose gel by the use of GeneJet Gel Extraction kit from Fermentas.

### Restriction enzyme digestion of DNA

Restriction digestion enzymes from Fermentas and New England BioLabs were used. Restriction digests were performed according to manufacturer's recommendations.

### Introduction of DNA into E. coli

### Electroporation of E. coli ET12567/ pUZ8002

*E. coli* is grown overnight at 37 °C in 10 ml LB medium containing kanamycin (50 µg/ml) and chloramphenicol (34 µg/ml). The next day 10 ml LB medium containing kanamycin (50 µg/ml), chloramphenicol (34 µg/ml) and 20 mM MgSO₄ is inoculated with 100 µl *E. coli* from the overnight culture. Bacteria are grown at 37 °C shaking at 160 rpm to an OD₆₀₀ of ~ 0.4. Afterwards cells are recovered by centrifugation at 4,000 rpm for 5 min at 4 °C and the pellet is resuspended by gentle mixing in 10 ml ice-cold 10 % glycerol. Then the cell suspension is again centrifuged and the pellet is resuspended in 5 ml ice-cold 10 % glycerol. After another centrifugation step the pellet is resuspended in the remaining ~ 100 µl 10 % glycerol. For electroporation 50 µl of such obtained cell suspension is mixed with 100-300 ng of plasmid DNA. Experiment is carried out in a 0.2 cm ice-cold electroporation cuvette using a power supply unit (BioRad) set to: 200 Ω, 25 µF and 2,5 kV. Just after 1 ml ice cold LB is added to shocked cells. Bacteria are incubated shaking for 1h at 37 °C and then they are spread onto LB agar containing apramycin (100 µg/ml), kanamycin (50 µg/ml) and chloramphenicol (34 µg/ml). Plates are incubated overnight at 37 °C.

### Chemical transformation

### E. coli stocks should be thawed on ice. Then to an aliquot bacterial suspension (50 µl or

100 µl per tube) 1 to 5 µl of DNA mix is added (e.g., isolated plasmid, ligation mix) and bacteria are incubated 30 min on ice. Afterwards a heat shock takes place: samples are incubated at 42 °C for 2 min. Then samples are immediately transferred on ice. After 10 min 1 ml of LB medium is added. Samples are incubated at 37 °C for 1 h with shaking at 160 rpm. The last step is platting of bacterial suspension on an agar plate with appropriate antibiotic. Plates are incubated overnight at 37 °C.

### Introduction of DNA into Streptomyces and A. namibiensis

### Preparation and transformation of protoplasts

### Preparation of protoplasts

25 ml of medium (YEME and TSB for *Streptomyces*; KM4 and CRM for *A. namibiensis*; with 0.5 % of glycine) is inoculated with bacteria from a cryostock or agar plate culture, and grown for few days. When the culture reaches the appropriate growth phase then 20 ml of a culture should be centrifuged (2,000 rpm, 10 min). Then pellet is resuspended in 15 ml 10.3 % sucrose and spun in a centrifuge with the same conditions as described above. This step is repeated twice. After the centrifugation step mycelium is resuspended in 4 ml of P buffer or L buffer containing for *Streptomyces* 1-2 mg/ml of lysozyme and for *A. namibiensis* a mixture of cellosyl (1 mg/ml) and lysozym (0,5 mg/ml) solubilized in P-buffer (containing 20 % of sacharose instead of a commonly used 10 % solution). Such suspension is incubated either at 28 °C, 30 °C or 37 °C until protoplasts are observed under the microscope. Later cells are draw in and out three times and incubated for a further 15 min. This is followed by addition of P buffer and again cells are draw in and out and incubated for a further 15 min. Next protoplasts are filtered through cotton wool (using a filter tube), transferred to a plastic tube and gently centrifuged (1,000 g, 7 min). Now supernatants is discarded and protoplasts are suspended in 1 ml P buffer. Such prepared protoplasts can be frozen or directly transformed.

### PEG-assisted transformation of protoplasts

For one transformation 50 µl of protoplast sample should be used. It is recommended to spin protoplasts down immediately before a transformation experiment. First, 5 µl of DNA solution should be added to protoplasts and mixed immediately by tapping the tube. Second, 200 µl T buffer (*) is added and mixed by pipetting up and down. Then protoplasts are spread on two dried R2YE plates and incubated at 28 °C. The next day (after 14-20 h) plates are overlayed for selection. (*) For preparation of Buffer T two different PEGs were used: PEG 1000 (Roth) and PEG 3350 (Sigma Aldrich).

### Conjugation from E. coli

First, competent cells of *E. coli* ET12567/ pUZ8002 need to be prepared in the presence of kanamycin (50 µg/ml) and chloramphenicol (34 µg/ml). These cells are transformed with the *oriT*-containing vector by electroporation. Obtained colonies should be inoculated into 10 ml LB medium containing kanamycin (50 µg/ml), chloramphenicol (34 µg/ml) and apramycin (100 µg/ml) each and grown overnight. The next day 10 ml LB medium (for one conjugation) containing kanamycin (50 µg/ml), chloramphenicol (34 µg/ml), apramycin (100 µg/ml) is inoculated with the overnight culture (the overnight culture is diluted 1:100). Bacteria are grown at 37 °C to OD₆₀₀ of 0.4-0.6. Then *E. coli* cells are washed twice with 10 ml of LB medium. After centrifugation step the supernatant is discarded and the pellet is resuspended in 500 µl LB medium. While washing the *E. coli* cells, for each conjugation approximately 10⁸ *Streptomyces* spores are added to 500 µl LB medium and such suspension is heat shocked at 50°C for 10 min. (If mycelial fragments are used for conjugation, then bacteria is harvested from a 3-4 d old culture growing on MS agar using 3 ml 20 % glycerol. Approximately 0.5 ml of the mycelial fragments is used for each conjugation. In a case of mycelial fragments the heat shock is omitted.) Afterwards 500 µl of *E. coli* cells are added to 500 µl heat-shocked spores or mycelial fragments. After mixing most of the supernatant is poured off and the pellet is resuspended in the residual medium. Now the bacteria suspension is plated out on

MS agar + 10 mM MgCl₂. The plate is incubated at 28 °C for 16-20 h. The next day the plate is overlayed with 1 ml water or 4 ml of a soft agar containing 0.5 mg nalidixic acid and 1 mg apramycin. Incubation is continued at 28 °C for the next few days until potential exconjugants are observed on the plate.

### Direct transformation attempts with A. namibiensis

Experiments were performed according to the protocol described by Madoń and Hütter, 1991 (*11*).

### Direct transformation of A. namibiensis mycelium

100 ml of a *Actinomadura namibiensis* preculture in KM4 media is inoculated with bacteria growing on a KM4 agar plate and incubated shaking (160 rpm) at 28 °C for one day. The next day 100 ml of KM4 medium is inoculated with preculture and incubated in an orbital shaker at 160 rpm for three days at 28 °C. Aliquots (10 ml) of the culture are centrifuged in 15 ml Falcon tubes at 5,000 rpm for 15 min. Afterwards the mycelium (from one Falcon tube) is resuspended in 4 ml of 20 % aqueous glycerol solution and stored at -28 °C or it is directly used for a transformation experiment. Directly before transformation, a mycelial suspension is thawed, washed three times in 25 ml of TE buffer. Then a pellet is resuspended in 0.6 ml TE buffer. Mycelium and transformation mixtures should be prepared at room temperature. To 100 µl of a TE mycelium suspension are added: 10 µl of 0.2 M MgCl₂, 60 µl of 4.17 M CsCl, 4 µl of calf thymus DNA, up to 10 µl of plasmid DNA (pSET152: 96 ng, 320 ng; pUWLoriT: 117 ng, 390 ng), and TE buffer to final volume of 100 µl. This is followed by the addition of 200 µl of 70 % (w/v) PEG-3350 in TE buffer. After each constituent is added, the mixture is mixed thoroughly. The transformation mixture is incubated at 28 °C for 90 min and then at 42 °C for 5 min and finally cooled to room temperature. The whole content of the transformation mixture is added directly to 3 ml of R2L- overlay medium and plated on S27M agar plates. Plates are dried by incubation in a laminar flow cabinet for 15 min. After incubation at 28 °C for 18 h each plate is overlayed with 1.5 ml water containing 1 mg of apramycin. It is important to use fresh S27M agar plates. Preferably prepare them on the day of transformation and dry in a laminar flow cabinet for 3 h.

### Direct transformation of A. namibiensis protoplasts

Protoplasts prepared in the way described in section 4.2.3.1 are used. To 100 µl of refrozen protoplasts in P buffer is added: 10 µl of 0.2 M MgCl₂, 60 µl of 4.17 M CsCl, 4 µl of calf thymus DNA, up to 10 µl of a plasmid DNA (96 ng, 160 ng of pSET152 and 117 ng, 195 ng of pUWLoriT), and TE buffer to final volume of 100 µl. This is followed by the addition of 200 µl of 70 % (w/v) PEG in TE buffer. After each constituent is added, the mixture is mixed thoroughly. The transformation mixture is incubated at 28 °C for 40 min and then at 42 °C for 5 min and finally cooled to room temperature. The whole content of the transformation mixture is added directly to 3 ml of R2L- overlay medium and plated on S27M agar plates. Plates are dried by incubation in a laminar flow cabinet for 15 min. After incubation at 28 °C for 18 h each plate is overlayed with 1.5 ml water containing 1 mg of apramycin.

### Electroporation of mycelium

A slightly modified protocol has been used as previously published for *Streptomyces ramous* (*10*).

A preculture *of A. namibiensis* is inoculated with bacteria from a KM4 agar plate and is incubated in KM4 medium shaking (160 rpm) at 28 °C for one day. The next day three different cultures are inoculated with 5 ml of preculture: 50 ml of CRM medium, 50 ml of CRM medium with glycine (end conc. 0.5 %), 100 ml of KM4 medium. These cultures are incubated in an orbital shaker at 160 rpm for four days at 28 °C. Then mycelium is harvest by centrifugation (4 °C, 10,000 rpm) and cells are resuspended in 50 ml ice-cold 10.32 % sucrose and recentrifuged. This step is repeated twice. Afterwards the mycelium is resuspended in 4 ml 15 % glycerol containing 15 mg of cellosyl. After incubation at 37 °C for 3 h the mycelium is washed twice with 7 ml ice-cold 15 % glycerol. Later the pellet is resuspended in 2 ml of 30 % PEG 1000, 10 % glycerol, 5 % sucrose. For electroporation, 50 µl of such suspension with plasmid DNA is mixed by pipetting (96 ng and 192 ng of pSET152; 117 ng and 234 ng of pUWLoriT). Sample must be placed on ice. Electroporation is carried out in a 0.2 cm ice-cold electroporation cuvette using 2 kV electric pulse from a Gene Pulser (Bio-Rad), connected to a Pulse Controller (parallel resistance 400 Ω, 25 µF capacitor). Pulsed mycelium is immediately diluted with 1 ml ice-cold KM4 medium and it is incubated with shaking for 3 h at 28 °C. Bacteria dilutions are plated on KM4 medium. The next day each plate is overlayed with 1 ml water containing 1 mg of apramycin.

### Polymerase chain reaction (PCR)

During these studies two kinds of DNA polymerases were used: Taq (Qiagen) and Herculase II (Stratagene). Because Taq DNA polymerase has no 3' to 5' exonuclease activity which could cause misincorporations it was used only for colony PCR, verification of positive transformants by PCR and preparation of DIG-labeled DNA probes. For all the other applications like site-directed mutagenesis, amplification of fragments used for cloning and sequencing Herculase II DNA polymerase was used. Standard reaction composition and Thermal Cycler conditions are summarized below:

| Component | Taq polymerase | Component | Herculase II polymerase |
|---|---|---|---|
| | Quantity per reaction | | Quantity per reaction |
| dH₂O | To final volume of 50.0 µl | dH₂O | To final volume of 50.0 µl |
| 10x CoralLoad PCR | 5 | 5 x Herculase II | 10 µl |
| buffer | | reaction buffer | |
| dNTP mix | 1 | dNTP mix | 0.5 µl |
| (10 mM each) | | (25mM each) | |
| DNA template | 1 | DNA template | 1 µl |
| (100 ng/ µl) | | (100 ng/ µl) | |
| Primer fw | 1 | Primer fw | 1.25 µl |
| Primer rv | 1 | Primer rv | 1.25 µl |
| Taq polymerase | 0.25 | Herculase II polymerase | 1 µl |
| 25 mM MgCl₂ | variable | DMSO | 2 µl |
| Total reaction volume | 50 µl | Total reaction volume | 50 µl |

| | Taq polymerase | | Herculase II polymerase | |
|---|---|---|---|---|
| Initial denaturation 3-step cycling | 3 min | 94 °C | 2 min | 95 °C |
| Denaturation | 0.5-1 min | 94 °C | 30 s | 95 °C |
| Annealing | 0.5-1 min | 50-68 °C | 30 s | 50-70 °C |
| Extension | 1 min/kb | 72 °C | 40 s/kb | 72 °C |
| Number of cycles | 30 | | 30 | |
| Final extension | 10 min | 72 °C | 3 min | 72° C |

### Site-directed mutagenesis

Labyrinthopeptins mutants were prepared by means of site-directed mutagenesis. For all experiments a procedure described previously (*12*), which is a slightly modified QuickChange Site-Directed Mutagenesis System (QCM) developed by Stratagene (La Jolla, CA, USA) was used. This two-stage procedure allows the efficient introduction of point mutation, deletions and insertions to a sequence of interest. For design of primers the program PrimerX was used. The procedure consists of two stages. In stage one, two extension reactions were performed in separate tubes; one containing the forward primer and the other containing the reverse primer. Subsequently, the two reactions were mixed, one microliter of polymerase was added and another PCR was carried out (2^{nd} PCR). Following the PCR, 1 µl ofDpnI was added and incubated at 37 °C for one hour. Five microliter of the final PCR products were transformed into 50 µl of DH5α cells, and appropriate volumes were spread on LB agar plates containing either ampicillin or kanamycin.

| | 1^{ST} PCR | | 2^{ND} PCR | |
|---|---|---|---|---|
| Initial denaturation | 3 min | 95 °C | 3 min | 95 °C |
| 3-step cycling | | | | |
| Denaturation | 20 s | 95 °C | 20 s | 95 °C |
| Annealing | 30 s | 55-65 °C | 30 s | 55-65 °C |
| Extension | 3 min 30 s | 72 °C | 3 min 30 s | 72 °C |
| Number of cycles | 5 | | 20 | |
| Final extension | 10 min | 72 °C | 10 min | 72 °C |

### Cloning experiments

### Ligation of DNA fragments

For ligation of DNA fragments T4 DNA ligase (from Fermentas or New Englan BioLabs) was used. In most of the performed reactions the molecular ration of insert to vector was 3:1 1 or 5:1. Reaction mixtures were incubated at 16 °C for 2 to 16 hours.

### LIC (ligation-independent cloning)

All LIC experiments were performed according to a protocol established for In Fusion® HD Cloning System (Clontech laboratories, Inc. a Takara Bio Company, Madison, WI, USA). LIC is a form of molecular cloning (*13-14*), which enables directional cloning of PCR fragment or multiple fragments into a linearized vector. Importantly, no additional treatment of the PCR fragment is required (such as restriction digestion, phosphorylation). The main idea which stays behind this method is the use of the 3'--> 5'-activity of T4 DNA polymerase to create very specific 10-15 base single overhangs in the expression vector, and also to create complementary overhangs in PCR product. Those 15 bp overlaps in PCR products can be engineered by designing primers for amplification of the desired sequences. The annealing of the insert (PCR product) and the vector is performed in the absence of ligase by simple mixing of the DNA fragments.

| | Thermal Cycler conditions | |
|---|---|---|
| Initial denaturation | 3 min | 95 °C |
| 3-step cycling | | |
| Denaturation | 20 sec | 95 °C |
| Annealing | 20 sec | 70 °C |
| Extension | 10 sec | 72 °C |
| Number of cycles | 30 | |
| Final extension | 10 min | 72 °C |

| Quantity per reaction | Component |
|---|---|
| To final volume of 50.0 µl | dH₂O |
| 10 µl | 5 x Herculase II reaction buffer |
| 0.5 µl | dNTP mix (25mM each) |
| 1 µl | DNA template (100 ng/ µl) |
| 1.3 µl | Primer fw |
| 1.3 µl | Primer rv |
| 0.5 µl | Herculase II polymerase |
| 2 µl | DMSO |

### Southern blotting

A Southern-blot is a method for detection of a specific DNA sequence in DNA samples. Southern-blotting combines the transfer of electrophoresis-separated DNA fragments to a filter membrane and subsequent fragment detection by probe hybridization. In these studies Southern-blotting was used to prove the integration of a cosmid M1104 to a genomic DNA of *Streptomyces.* The very first step was restriction digestion of genomic DNA of *Streptomyces* wild type strains (negative control) and *Streptomyces* carrying the cosmid M1104. As a positive control the cosmid M1104 isolated from *E. coli* DH5α/cosmid M1104 was used. Cosmid M1104 and genomic DNA of *S. lividans* and *S. coelicolor* were fragmented with BamHI. For digestion of genomic DNA of *S. albus* the enzyme SacI was used. Obtained DNA fragments were separated by electrophoresis on an agarose gel (60 V, 90 min). To estimate the size of DNA fragments DIG-labeled DNA molecular weight marker VII (Roche) was used. The gel was dyed by ethidium bromide which after tacking a picture was washed away with water. Afterwards the gel was incubated in different solutions shaking at 4 °C: first for 20 min in Southern I, then for 30 min in Southern II, and finally for 30 min in Southern III. Such prepared DNA was transferred overnight on a nylon membrane (Hybond-N, Amersham Pharmacia Biotech). To do this a sheet of nylon membrane was placed on top of the gel. Pressure was applied evenly to the gel by placing a stack of paper towels and a weight (500 g) on top of the membrane and gel, to ensure good and even contact between gel and membrane. 20X SSC buffer was used to ensure a seal and prevent drying of the gel. Buffer transferred by capillary action from a region of high water potential to a region of low water potential (paper tissues) was then used to move the DNA from the gel onto the membrane; ion exchange interactions bound the DNA to the membrane due to the negative charge of the DNA and positive charge of the membrane. The next day the membrane was exposed to UV radiation (2 min) to permanently attach the transferred DNA to the membrane. A membrane was incubated with 100 ml Church Buffer (preheated to 65 °C) for 4 h at 65 °C. Then Church Buffer was removed and a membrane was exposed to a hybridization probe. As a probe a DIG-labeled cosmid M1104 was used (see below). The use of digoxigenin gave a possibility for detection of probe-target hybrids by a color reaction with an alkaline-phosphatase-conjugated antibody. As a substrate for alkaline phosphatase the NBT/BCIP mix was used. First a cosmid M1104 (8 µg) was digested with a restriction enzyme MlyI to obtain fragments with a size of 800-1000 bp. After restriction digestion 4 µl water was added and DNA fragments were denatured by incubation at 100 °C for 10 min, later cooled on ice. Then DIG-labeled nucleotides were incorporated to a cosmid M1104 sequence by mixing with 4 µl DIG High Prime Solution (Roche) which contained Klenow enzyme, randomized oligonucleotides and DIG-11-dUTP. Such mix was incubated for 16 h at 37 °C. Later the concentration of labeled DNA was calculated by blot membrane and comparison with a standard. Just before the use the probe was denatured (100 °C, 5 min) and added to a 50 ml DIG Easy Hyb buffer (Roche) to reach an end concentration of 25 ng/ml. After an overnight incubation at 65 °C the probe solution was removed and the membrane was incubated shaking first with 2 x SSC+0.1 % SDS for 15 min (twice), second with 0.5 x SSC+0.1 % SDS for 15 min (twice). Later the membrane was washed for 5 min with 50 ml Washing Buffer which was followed by incubation in 20 ml anti-DIG-antibody solution for 30 min. Then the membrane was washed twice with 100 ml Washing Buffer (each time 15 min). Afterwards the membrane was incubated with 100 ml Detection Buffer for 5 min and then an additional portion of Detection Buffer (10 ml) containing 200 µl NBT/BCIP was added. The membrane was incubated with a substrate until a violet color appeared. The reaction was stopped by washing with water.

### Tris-glycine SDS-PAGE and Western blotting

SDS-PAGE (sodium dodecyl sulfate polyacrylamide gel electrophoresis) is a technique widely used to separate proteins according to their electrophoretic mobility (a function of length of polypeptide chain or molecular weight). SDS gel electrophoresis of samples has identical charge per unit mass due to binding of SDS results in fractionation by size. This method was used for detection of LabA2 in heterologous strains. Because LabA2 is a small peptide (2.1 kDa) it was not possible to reach a proper separation by the use of the popular and commonly used glycine-SDS-PAGE system. In this situation a tris-glycine SDS-PAGE (under denaturing conditions) was performed according to the protocol described by Schägger and von Jagow, 1987 (*15*). Samples were prepared by incubation of either agar plate extracts or supernatants and pellets from liquid cell cultures in sample loading buffer (in 1:1 ratio). Then samples were heated at 95 °C for 5 min and cooled down to RT. A stacking and resolving gel was run under a constant electric current 15 mA and 20 mA per gel, respectively. To estimate protein size the PageRuler Prestained Protein Ladder (Fermentas) was used.

| Reagent | Resolving gel (16.5%) | Stacking gel (5%) |
|---|---|---|
| Rotiphorese Gel 30 (acrylamide:bisacrylamide=37.5:1) | 3.33 ml | 330 µl |
| Buffer | 1.125 ml | 200 µl |
| H₂O_{dd} | 1.41 ml | 1.44 ml |
| 10% SDS | 120 µl | 20 µl |
| TEMED | 15 µl | 4 µl |
| APS (100 mg/ml) | 30 µl | 10 µl |

After separation of protein by gel electrophoresis a semi-dry Western-blot was performed. During Western-blotting experiment proteins were transferred to a membrane, where they were probed using antibodies specific to the target protein. First proteins and peptides were moved from the gel onto a membrane made of nitrocellulose (Whatman). The membrane was placed on top of the gel, and a stack of filter papers placed on top of that. The entire stack was placed in a buffer solution which moved up the paper by capillary action, bringing the proteins with it. As a result of this "blotting" process, the proteins were exposed on a thin surface layer for detection. There was a need to optimize a transfer step (25 min, 50 mA) since LabA2 is a small peptide and can go through the membrane when a standard transfer conditions are used. The blocking of nonspecific binding was achieved by placing the membrane in a blocking buffer for one hour. Afterwards membrane was incubated overnight at 4 °C in 30 ml TNT buffer containing 1 % BSA and a primary antibody. Polyclonal antibodies against mature LabA2 or LabA2 leader peptide were used. Different concentrations of antibodies were tested but the best results for both of them were obtained when antibodies were diluted 1: 2500. The next day membrane was washed three times with 30 ml TNT buffer with 1 % BSA (each time for 10 min). After washing step a membrane was incubated in 30 ml TNT buffer with 1 % BSA and 6 µl of a secondary antibody for two hours. As a secondary antibody commercially available Anti-Rabbit IgG Alkaline Phosphatase antibody was used. Then membrane was washed three times with 30 ml TNT buffer (each time for 10 min) and incubated with a detection solution until the colored precipitate was observed. Development of the blot was stopped by washing away the soluble dye with water.

### Analytical methods:

### Sample preparation for MS analysis

### Sample preparation from solid agar cultures

For bacteria which were grown on solid media a small piece of an agar were cut (around 1 cm²) and was extracted with 500 µl methanol or methanol:acetone (1:1) by sonication for 30 min. Later a sample was centrifuged and a soluble fraction was used for MS analysis. When a content of whole plate was extracted then 15 ml of methanol or methanol:acetone (1:1) was used.

### Sample preparation from liquid cultures

First step was separation of bacterial cells from a supernatant. The supernatant was either directly used for MS measurements or it was diluted before in methanol (1:1). The mycelium was extracted with methanol or with methanol:acetone (1:1), sonicated for 30 min and centrifuged. The soluble fraction was used for MS analysis. The amount of the solvent used for mycelium extraction was dependent on the weight of the pellet.

### SPE (solid phase extraction)

For some of the samples SPE was performed. Each time Chromabond C18 cartridge (1 ml/100 mg) from Macherey-Nagel were used. SPE procedure conditions are summarized as follows:

| | | |
|---|---|---|
| Column conditioning | 3 ml | Methanol |
| | 3 ml | H₂O |
| | | |
| Sample aspiration | 1 ml | Crude extract |
| | | |
| Washing | 1 ml | Dichloromethane |
| | 1 ml | Acetone |
| | 1 ml | EtOAc |
| | 1 ml | Acetonitrile |
| | | |
| Elution | 1 ml | 10 % Methanol |
| | 1 ml | 20 % Methanol |
| | 1 ml | 50 % Methanol |
| | 1 ml | 80 % Methanol |
| | 1 ml | 100 % Methanol |
| | 1 ml | 0.1 % HCOOH in H₂O |

### Mass spectrometry analysis of Labyrinthopeptin variants

### HPLC-ESI

Labyrinthopeptins were detected by the use of HPLC-ESI-MS. LC-MS system was composed with 1100 Series HPLC System (Agilent) coupled either to QTRAP 2000 (Applied Biosystems) or to Q-Tof II (The Micromass). Gradients timetables are presented below:

| Time [min] | % Solvent A | % Solvent B | Flow [µl/min] |
|---|---|---|---|
| 0.00 | 60.0 | 40.0 | 60 |
| 10.00 | 20.0 | 80.0 | 60 |
| 10.10 | 0.0 | 100.0 | 60 |
| 13.00 | 0.0 | 100.0 | 60 |
| 13.10 | 95.0 | 5.0 | 60 |
| 15.00 | 95.0 | 5.0 | 60 |

The gradient timetable for LC-ESI-MS (QTRAP 2000). Solvent A: H₂O + 0.1 % HCOOH, Solvent B: ACN + 0.1 % HCOOH. Injection volume 5 µl.

| Time [min] | % Solvent A | % Solvent B | Flow [ml/min] |
|---|---|---|---|
| 0.00 | 95.0 | 5.0 | 0.300 |
| 20.00 | 0.0 | 100.0 | 0.300 |
| 25.00 | 0.0 | 100.0 | 0.300 |
| 26.00 | 95.0 | 5.0 | 0.300 |
| 30.00 | 95.0 | 5.0 | 0.300 |

The gradient timetable for LC-ESI-MS (Q-Tof II). Solvent A: H₂O + 0.1 % HCOOH, Solvent B: ACN + 0.1 % HCOOH. Injection volume 20 µl.

### LTQ-Orbitrap

The Orbitrap is a electrostatic ion trap using fast fourier transformation (FFT) to obtain mass spectra. It provides high mass accuracy (2-5 ppm) and mass resolution (150,000) which significantly reduces false positive peptide identifications. Masses were obtained after measurements with LTQ Orbitrap XL (Thermo Scientific). The gradient timetable is shown below:

| LC-MS parameters | |
|---|---|
| Column: | XDB C-18, 5 µL, 4,6*150 mm (Agilent) |
| Inj ection volume: | 10 µL |
| Gradient: | 0 min 5:95 % (MeOH + 0.1 % HCOOH):(H₂O + 0.1 % HCOOH), |
| | 25 min 100:0 % |
| | 38 min 100:0 % |
| | 40 min 5:95 % |

LC-MS parameters for the high-resolution Orbitrap-ESI-MS. Injection volume: 10µl.

### MS/MS

In these studies MS/MS experiment was used to compare Labyrinthopeptins obtained from heterologous strain *S. lividans*/ pUWLab with Labyrinthopeptins isolated from a natural producer *A. namibiensis*. To obtain better fragmentation a disulfide bridge in Ring C of LabA1 was disrupted. Sample was incubated with NaHCO₃ (20 mM) and DTT (0.5 M) solution. Such mixture was incubated for 1 h at 50 °C. After centrifugation a soluble fraction was ready to use for MS/MS experiment. Experiments were recorded using a LTQ-Orbitrap XL (Thermo Scientific, Bremen, Germany) coupled to an Agilent 1260 HPLC system (Agilent Technologies, Waldbronn, Germany). For chromatographic separation, a Vydac 218M C18 5u, 150 mm x 2.1 mm column (Grace, Deerfield, USA) with a linear solvent gradient from 5 % to 100 % ACN + 0.1 % HCOOH (solvent B) in 20 min were used. Solvent A was water (0.1 % HCOOH). The MS/MS spectra were recorded in FTMS-mode with fragmentation in the HCD cell (a normalized collision energy of 23 % was used).

### Example 1: Introduction of DNA in A. namibiensis

It was aimed at establishing a protocol for generation of Labyrinthopeptins variants. Since the total synthesis is not a viable approach for generating preparative amounts of Labyrinthopeptins, biological approach was chosen. Initially mutations of the *A. namibiensis* genome were planned to enable generation of Labyrinthopeptin diversity. An important argument to choose this strategy was based on the fact that chemical synthesis of lantibiotics is very challenging and remain industrially impractical. For instance even for the best studied lantibiotic nisin a commercial production relies on biosynthesis using its natural producer (*Lactococcus lactis*) although its chemical synthesis has also been described previously (*16*).

*Actinomadura* belong to the genus which is the most dominant among rare Actinomycetes (*17*). This is the reason why this genus is one of the most important targets in screening programs for pharmacologically active compounds. However, in contrast to other actinomycete strains, there is little information available on the basic aspects of gene expression mainly due to the lack of versatile gene manipulation systems. In this respect the lack of suitable cloning vectors and methods of transformation negatively impacts genetic work with *Actinomadura.* For the molecular analysis of biosynthetic genes in certain actinomycetes, different transformation systems have been reported (e.g. polyethylene glycol induced protoplast transformation, transformation by electroporation and direct transformation). In presented study methods elaborated for *Streptomyces* were used to establish the method for DNA transfer to *A. namibiensis*. This choice was based on the fact that both *Actinomadura* and *Streptomyces* belong to Actinomycetes and methods for genetic manipulations in *Streptomyces* are very well described.

| Antibiotic | Conc. [µg/ml] | Growth after 4 days | Growth after 7 days |
|---|---|---|---|
| Apramycin | 25 | - | + |
| Apramycin | 50 | - | - |
| Streptomycin | 30 | + | ++ |
| Phosphomycin | 400 | +++ | +++ |
| Thiostrepton | 25 | - | + |
| Kanamycin | 50 | +++ | +++ |
| Viomycin | 30 | + | + |
| Hydromycin | 50 | ++ | +++ |
| Spectinomycin | 100 | +++ | +++ |

Testing selection markers suitable for genetic manipulations *of A. namibiensis.* Resistance markers. Legend: - no growth, + slight growth, ++ medium growth, +++ strong growth.

### Plasmids

An apramycin resistance gene was used as selectable marker for vectors used in this study because from all eight tested antibiotics *A. namibiensis* was sensitive only to apramycin (50 µg/ml). Initially for establishing a way in which DNA can be introduced into *A. namibiensis* the typical cloning vectors for *Streptomyces* strains were used. Plasmid pUWLoriT which can replicate autonomously in *Streptomyces* and plasmid pSET152 which can integrate site-specifically at the bacteriophage ΦC31 attachment site (*18*) (*7*) were used. In order to prove the presence of ΦC31 attachment sites in *A. namibiensis* PCR-experiment was performed (primers: attB_For (5'-CGGTCTCGAAGCCGCGGTGC-3') and attB_Rev (5'-GCCCGCCGTGACCGTCGAG-3'), expected PCR product: 260 bp). Although *Actinomaduras* and *Streptomyces* are related it is still possible that origin of replication (*ori*) for *Streptomyces* is not recognized by *A. namibiensis* (*9*). Description of similar difficulties can be found in the literature (*17*). This led to the use of vectors harboring replicons of *Actinomadura* strains: pSETermEΔHindIII_oriCLL4, pK18mobXy1E_oripCLL4 and pCLL4_ori_apra. Also a non-replicating plasmid pK18mob2 with a fragment coding for the LabKC modifying enzyme was used for transformation *of A. namibiensis.*

To generate a vector pK18mob2_labKC a fragment of *labKC* gene was amplified with primers STK_RTPCR_fw and STK_RTPCR_rv to obtain 1547 bps long PCR fragment. PCR product was cloned into the vector pDrive to obtain pDrive_labKC. pDrive_labKC was cut with EcoRI and the fragment containing *labKC* was cloned to a vector pK18mob2.

The vector pCLL4 contains two functional origins of replication (*9*). One is delivered from plasmid pAkijl (isolated from *Actinomadura kijanita* ATCC 31588) and the other from plasmid pBR322 (purified from *E. coli* DH10B). pCLL4 was capable of replication in *Escherichia coli* (providing resistance to ampicillin) and expression of resistance to ampicillin, and of replication in *Streptomyces lividans* and expression of resistance to thiostrepton. The lack of information about pCCL4 sequence makes the use of this vector difficult. Moreover as mentioned above *A. namibiensis* is not sensitive to thiostrepton. Three constructs were prepared to allow usage of origin of replication from the genus *Actinomadura* together with the apramycin resistance gene: pSETermEΔHindIII_oriCLL4, pK18mobXy1E_oripCLL4 and pCLL4_ori_apra.

To construct pSETermEΔHindIII_oriCLL4 and pK18mobXy1E_oripCLL4 plasmids pUC18 and pCLL4 were digested with KpnI. Then a 0.9 kbp fragment from pCLL4 was cloned into pUC18 to get pUC18_oripCLL4. pUC18_oripCLL4 was subsequently digested with EcoRI and XbaI and cloned to vectors pSETermEΔHindIII and pK18mobXy1E previously digested with the same restriction enzymes, to obtain pSETermEΔHindIII_oriCLL4 and pK18mobXy1E_oripCLL4, respectively.

The vector pCLL4_ori_apra was prepared by cloning of a fragment containing *oriT* and an apramycin resistance gene to EcoRI and PstI restriction sites. This fragment was amplified by means of PCR with primers pK18mob_EcoRI_for (5'-CATCTCGAATTCCGCTCATG AGCTCAG-3') and pK18mob_EcoRI_rev (5'-AGTTATCGAGATCTGCAGGAGCTCTTTGG-3'). pCLL4_ori_apra was then transformed into ET12567/pUZ8002 and used for conjugation with *S. lividans.* This experiment showed that pCLL4_ori_apra is successfully transferred by conjugation and its presence in bacteria can be verified by selection with apramycin.

### PEG-assisted transformation of A. namibiensis protoplasts with plasmid DNA:

Many authors have investigated the parameters influencing regeneration and transformation frequency and attempted to optimize the conditions for individual *Streptomyces* species (*19-20*). Of special importance are the growth phase of the mycelium at the time of protoplasting, the temperature at which the mycelium and the regenerating protoplasts are incubated, the number of protoplasts used per transformation, the dryness of the regeneration plates, and composition of the medium. For generation, transformation and regeneration of protoplast standard protocol for *Streptomyces* (see above) with some modifications was used. *A. namibiensis* was grown each time at 28 °C rotating 160 rpm in two different kinds of media CRM and YEME. Different modifications of media composition were tested. Mycelia were grown in the presence of 0.5 %, 1 % and 1.5 % of glycine. Protoplasting was the most effective when glycine (1 %) was present in the CRM growth medium. For efficient preparation of protoplasts the age and physiological state of the mycelium is important. It was described that the best transformation frequencies in *S. lividans* are obtained with protoplasts prepared from mycelium grown to "late exponential" phase, whereas for transfection of *S. parvulus* protoplast needed to come from much younger mycelium. To estimate when *A. namibiensis* is in exponential phase a growing curve was prepared. Because *A. namibiensis* cells form clusters it was not possible to simply measure the OD, instead every 6 hours 1 ml of culture was centrifuged and pellet was weighted. Another important step is a use of lytic enzymes. For *Streptomyces* the use of lysozyme give satisfying results. Surprisingly, no *A. namibiensis* protoplasts were observed after incubation with lysozyme. Neither prolonged incubated time (1, 2, 3 hours) nor increased incubation temperature (37°C) and lysozyme concentration (2, 10, 50 and 100 mg/ml) helped. Although *A. namibiensis* is extraordinarily resistant to lysozyme it was possible to develop the protocol for protoplast formation. Protoplasts were formed when *A. namibiensis* was treated with cellosyl or cellosyl/ lysozyme mixture. Cellosyl is a bacterial lysozyme from *Streptomyces coelicolor*. Cellosyl is of considerable interest because it is able to degrade cell walls of *Staphylococcus aureus* and other bacteria which are not hydrolyzed by chicken-, goose- or phage-type lysozyme (*21*). The best conditions for formation of protoplasts were obtained when *A. namibiensis* was grown in CRM medium with 0.5 % or 1 % final concentration of glycine, and cells were incubated for 2 h at 28 °C with a mixture of cellosyl (1 mg/ml) and lysozyme (0.5 mg/ml) solubilized in P-buffer (containing 20 % of saccharose instead of standard 10 %). To determine the optimal conditions for regeneration of viable cells from protoplasts R2YE and PWP overlay medium were tested. Only growth on PWP plates with an overlay of soft agar resulted in a lawn, which is necessary for successful regeneration. Work on the transfection of protoplasts led to the discovery that the presence of polyethylene glycol (PEG) in the transformation mixture is absolutely necessary for high frequency plasmid DNA transformation (*22*). Most often PEG 3350 and PEG 1000 are used. In order to examine the transformation ability of the *A. namibiensis* protoplasts, PEGs with differences in polymerisation degree (3350, 1000, 6000) and also PEGs from different suppliers (Sigma and Roth) were tested since it was reported before, that batches of PEG from different sources are not necessarily equivalent. As a plasmid DNA the following vectors were used: pSET152, pUWLoriT and pK18mob2_labKC. Since unsuccessful transformation may be the result of an active restriction systems in the host, plasmids were isolated from a dam- donor such as *E. coli* ET12567. Transformation experiments were performed with the use of frozen and also freshly prepared protoplasts. Although different transformation conditions and vectors were used no transformants were obtained.

### Intergeneric conjugation between E. coli and A. namibiensis

One of the reasons for low transformation frequencies may be the result of active restriction systems in the host. This barrier can be overcome by the use of intergeneric conjugation (*23*) to introduce DNA into the host in a single-stranded form. Another advantage of conjugation is that it is simple and doesn't rely on the development of procedures for protoplasts formation and regeneration. The initial conjugative transfer of a shuttle plasmid between *Escherichia coli* and Gram-positive bacteria was reported by Trieu-Cuot *et al.* (*24*). The intergeneric transfer of plasmids from *E. coli* to *Streptomyces* was first described by Mazodier et al. (1989) (*25*) (*26*). Later, this method has been successfully applied to a number of different *Streptomyces* strains and other Actinomycetes as *Amycolatopsis* (*27*), *Actinoplanes* (*28*), *Nonomuraea* (*29*), *Saccharopolyspora*, *Actinomadura*, *Micromonospora*, *Nocardia* and *Rhodococcus* (*30*).

Conjugation experiments were performed according to the protocol described above. As shuttle plasmid pSET, pUWLoriT, pCLL4_ori_apra from non-methylating *E. coli* donor ET12567 were used. First experiments were performed with the use of spores. Although *A. namibiensis* was described as a sporulating strain (*31*) there was a need to test many different media to obtain reasonable amount of spores. The following media were used ISP1, ISP2, ISP3, ISP4, ISP5, ISP7, MS, SM and sporulation medium. Sporulation was observed only on ISP4 and on sporulation medium, but only growth on sporulation medium gave satisfactory results. Spores as well as mycelial fragments were used for conjugation since mycelial fragments although less convenient for preparation may give higher numbers of recombinants. Mycelial fragments were prepared from bacteria which were grown on agar plates (AS1 and MS agar plates) or in a liquid culture (KM4 and TSB medium).

An exconjugant was obtained only after conjugation from *E. coli* ET12567/ pUZ8002/ pSET152. The correctness of a clone was verified by PCR reaction. Primer pSETproof_for (5'-CGAGCCGGAAGCATAAAGTG-3') and pSETproof_rev (5'-GCTTGGAGCGAACGACCTAC-3') were used to amplify the 591 bps fragment of pSET152. This result demonstrates that vectors characteristic for *Streptomyces* are able to replicate in *A. namibiensis* and can be used for further genetic manipulations in this strain. Although an exconjugant was obtained there is still a strong need to establish the protocol which allows conjugation with much higher yield.

### Direct transformation of A. namibiensis

Another method used for finding a way *of A. namibiensis* transformation was the direct transformation described before for *Amycolatopsis japonicum* (*27*). It is an efficient method for various *Amycolatopsis* strains (*11*, *32*), which were not prone to transformation with standard techniques. This method is based on the observation that bacteria can be transformed by incubation of protoplasts together with plasmid DNA in the presence of polyethylene glycol, calcium ions and calf thymus DNA, which is working as a carrier. It has been shown that the most critical parameter of this procedure is the age of the culture. For instance *Amycolatopsis japonicum* (*27*) showed the highest transfection efficiency if early stationary phase mycelia were used, but the optimal culture age of *Nocardia lactamdurans* was when the culture was in the exponential phase (*33*). Experiments were performed when *A. namibiensis* was in the late exponential phase according to the protocol described above with plasmid pSET and pUWLoriT. No transformants were obtained.

### Electroporation of mycelium

An application of a brief, high voltage pulse to a suspension of cells and DNA, which results in the formation of transient membrane pores and uptake of DNA is called electroporation. It may enable to omit the need to develop conditions for protoplasts formation and regeneration, but on the other hand appropriate conditions for electroporation can be strain-specific. Electrotransformation was developed for *Actinomyces* spp. (*34*) for example for transformation of mycelium of several *Streptomyces* species (*35-36*), *Streptomyces lividans* protoplasts (*37*) and even *Streptomyces* germinating spores (*38*). For electroporation *of A. namibiensis* the slightly modified protocol for *Streptomyces rimosus* (*10*) was applied. Electroporation (with a plasmid pSET152ermE and pUWLoriT) was performed on mycelial fragments as well as on protoplasts. No transformants were obtained.

### Summary

The work with *Actinomadura namibiensis* was impeded due to the unavailability of genetic transfer method to this strain. Although in this study several transformation protocols were tested none of them gave a satisfying solution to this problem. However, it was possible to optimize partially a protocol for conjugation and transformation of protoplasts. A method for formation of *Actinomadura namibiensis* protoplasts was established by the use of cellosyl or cellosyl and lysozyme mixture. The lack of transformants after protoplasts transformation can be due to an insufficient regeneration of protoplasts. A conjugation from mycelium resulted in one exconjugant. Such low efficiency shows that this method is not reproducible and reliable, but on the other hand it is a proof that it is possible to introduce foreign DNA into *Actinomadura namibiensis.* Another important information from this experiment is that vectors characteristic for *Streptomyces* (e.g. pSET152) can be used for genetic manipulations in *A. namibiensis.* It is important since it is not always the case for the *Actinomadura* genera (*9*). These studies show that although different transformation methods were established for many actinomycetes they are still far from universal since optimal conditions for different strains may vary significantly.

### Example 2: Heterologous expression of Labyrinthopeptins in Streptomyces

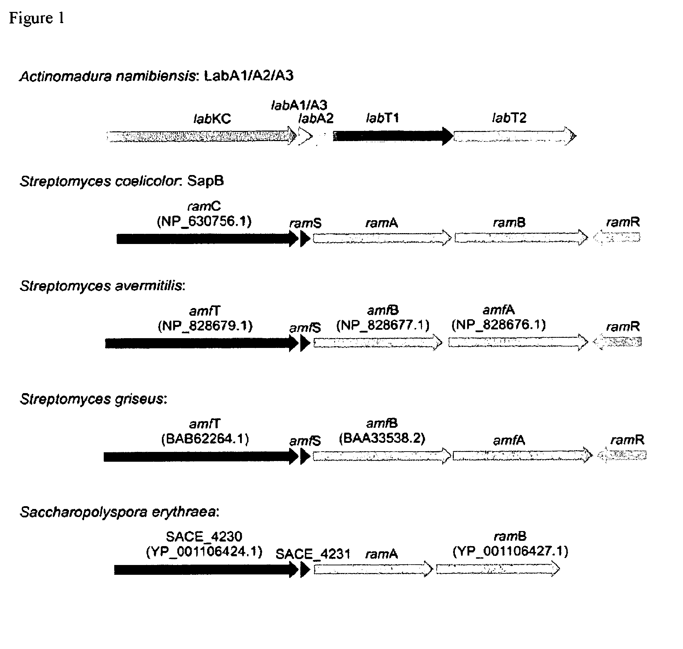

The development of efficient protocols for genetic manipulation *of A. namibiensis* gave unsatisfactory results which basically impeded a possibility of genetic engineering of Labyrinthopeptins in their natural producer. When the organism of interest is slow-growing or genetically intractable, the common strategy is to establish expression of the entire biosynthetic gene clusters in a more suitable host (*39-40*). The same strategy was followed. As heterologous hosts for Labyrinthopeptins expression *Streptomyces* were chosen. Strains used for these studies are easy to handle and genetic manipulation techniques are well-described (*10*). Several characteristics make *Streptomyces* a good candidate as a heterologous hosts. Importantly *Streptomyces* spp. and *Actinomadura* spp. belong to actinomycetes, they are both GC reach strains with similar codon usage. In addition the genus *Streptomyces* exhibits the ability to produce a wide variety of secondary metabolites (*41*). Although the information concerning genetic manipulations in *Actinomaduras* is very limited, the complementation of the biosynthetic gene clusters from *A. madurae* in two *Streptomyces* strains (*S. carzinostaticus*, *S. globisporus*) was reported (*42*). Another important issue is due to the fact that to obtain mature Labyrinthopeptins the leader peptide needs to be removed. No candidate gene has been identified in the *lab* gene cluster which may code for the protease responsible for the cleavage of the leader peptide. It is possible that the Labyrinthopeptin leader peptide is cut off by an extracellular nonspecific protease like it has been already described for subtilisin from *Bacillus subtilis* (*43*). *In silico* analysis revealed that gene clusters homologous to the *lab* gene cluster are present also in other actinomycetes (Figure 1) like *Streptomyces coelicolor*, *Streptomyces avermitilis*, *Streptomyces griseus* and *Saccharopolyspora erythraea*. This finding is of special interest because it seems that proteases from *Streptomyces* strains chosen as heterologous hosts could be able to complement the protease from *A. namibiensis.* All these features, together with a non-pathogenic nature and established fermentation technology, have made *Streptomyces* an obvious choice for producing Labyrinthopeptins.

### Heterologous expression of a cosmid M1104 bearing the lab gene cluster:

### Integration of cosmid M1104 into a Streptomyces chromosome

The construct allowing heterologous expression (cosmid M1104) of Labyrinthopeptins was prepared already during PhD work of Dr. Timo Schmiederer (*44*). By means of λ-Red-mediated recombination (*2*, *5*), the integrase gene (*int*) and the attachment site (*attP*) of phage ΦC31 (*45*) (*46*) were introduced into cosmid 1104 containing the complete *lab* gene cluster. The cosmid M1104 was site-specifically integrated into the chromosome of the heterologous hosts: *S. lividans*, *S. albus* and *S. coelicolor.* Transformation of a cosmid M1104 was performed by means of PEG-assisted transformation of protoplasts described by Kieser et al., 2000 (10). From obtained clones resistant to apramycin seven clones for *S. coelicolor,* three clones for *S. albus* and two clones for *S. lividans* were chosen for further experiments.

### Verification of obtained integration mutants by PCR and Southern-blot

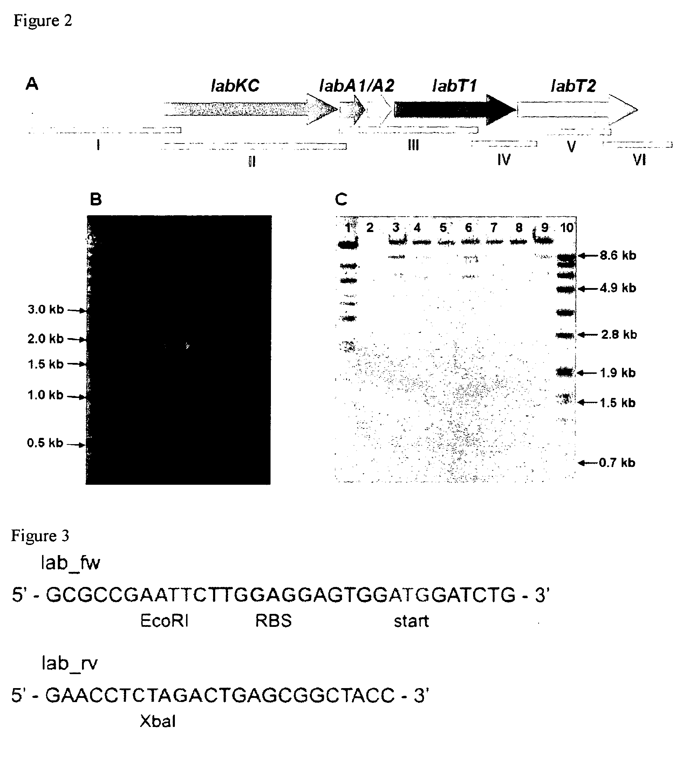

Integration mutants were verified by PCR and Southern-blot. All clones were treated in the same manner. Genomic DNA was isolated by slightly modified salting out procedure described by (*10*). Cosmid M1104 and genomic DNA of the *Streptomyces* host strain were fragmented either with BamHI (*S. lividans*, *S. coelicolor*) or SacI (*S. albus*). The DNA blot was probed with the cosmid M1104 digested with MlyI and DIG-labeled. The profiles of wild type strains and heterologous hosts carrying the cosmid M1104 were compared. Southern hybridization experiments with total DNA from *S. coelicolor* with cosmid M1104 did not reveal hybridization signals, indicating that *S. coelicolor* did not have DNA homologous to the introduced cosmid. This finding eliminates the possibility of either activation or complementation of cryptic genes in the host. Similar results were obtained for two other strains *S. lividans* and *S. albus* used as heterologous hosts. To verify integration events into mutants by means of PCR six primer pairs were design. Sequences of primers and the length of expected PCR products are described below. This experiment showed that all the expected DNA fragments could be observed after PCR with primer pairs I-VI for integration mutants but not for wild types. An example is shown in the Figure 2B. PCR and Southern blot analysis proved that no significant deletions and rearrangements had taken place within integration mutants.

### Primers used for PCR verification of integration mutants:

| Primer | Sequence | Expected PCR product [bps] |
|---|---|---|
| I: Iko Fw1+ Iko Rev1 | Iko Fw1: 5'-GTTCGTTCGACGGACCAATG-3' (SEQ ID No. 145) | 3004 |
| | Iko Rev1: 5'-CCTGCTCGACGCAGTATTTG-3' (SEQ ID No. 146) | |
| II: Iko Fw2 + Iko Rev2 | Iko Fw2: 5'-CGCAGGACGAACGGTTTCAG-3' (SEQ ID No. 147) | 3032 |
| | Iko Rev2: 5'-CCATGGGACTGTCACCTCTC-3' (SEQ ID No. 148) | |
| III: Iko Fw3 + Iko Rev3' | Iko Fw3: 5'-CATCCACCATGGCATCCATC-3' (SEQ ID No. 149) | 1866 |
| | Iko Rev3': 5'-GCGTCGTCGAGGATGATCAG-3' (SEQ ID No. 150) | |
| IV: Iko Fw3' + Iko Rev3 | Iko Fw3': 5'-ACTACCGGGCGATGTTCGAG-3' (SEQ ID No. 151) | 567 |
| | Iko Rev3: 5'-AGCAGCCGGGAGAGCAGCAG-3' (SEQ ID No. 152) | |
| V: Iko Fw4 + Iko Rev4' | Iko Fw4: 5'-ACTACCGGGCGATGTTCGAG-3' (SEQ ID No. 153) | 1302 |
| | Iko Rev4': 5'-CTGAAGACGTACGCCTCCTG-3' (SEQ ID No. 154) | |
| VI: Iko Fw4' + Iko Rev4 | Iko Fw4': 5'-CAGGAGGCGTACGTCTTCAG-3' (SEQ ID No. 155) | 1751 |
| | Iko Rev4: 5'-AGATGAAGCGGGCGATCGAG-3' (SEQ ID No. 156) | |

### Cultivation of heterologous strains and detection of Labyrinthopeptins

Integration mutants and parental host strains were cultured in few different production media summarized below. Because the production levels can vary between clones, more that one independent integrant were isolated and tested for Labyrinthopeptins production. Production was tested by LC-ESI-MS and Western-blotting analysis. For Western blotting antibody against LabA2 leader peptide and also mature LabA2 were used. This allowed detection of not only fully modified LabA2 but also its prepropeptide and fully modified LabA2 with an attached leader peptide. The possibility to detect LabA2 with an attached leader peptide was very important because of the lack of the protease responsible for the leader peptide cleavage in the *lab* gene cluster (also no protease fulfilling this function could be identified in the boundaries of a cosmid M1104). The Western blot protocol, LC-MS method and the way of samples preparation are described above. The analysis of metabolites showed that, the integration mutants did not produce any Labyrinthopeptins or Labyrinthopeptins derivatives.

Strains and growth conditions used for heterologous expression of a cosmid M1104:

| Heterologous host | Number of tested clones | Culture conditions Liquid cultures | Solid media |
|---|---|---|---|
| | | TSB | R2YE |
| *S. coelicolor* | 7 | CRM | KM4 |
| *S. lividans* | 2 | KM4 | MS |
| *S. albus* | 3 | Glu-Nutrient | SMMS |
| | | Broth | R5 |

### Heterologous expression of Labyrinthopeptin by using the vector pUWLab:

### Construction and verification of a vector pUWLab

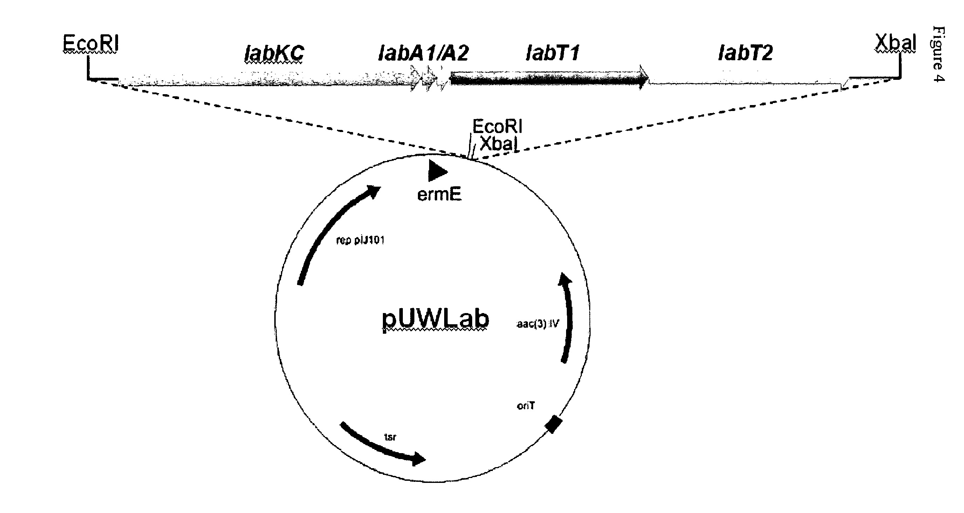

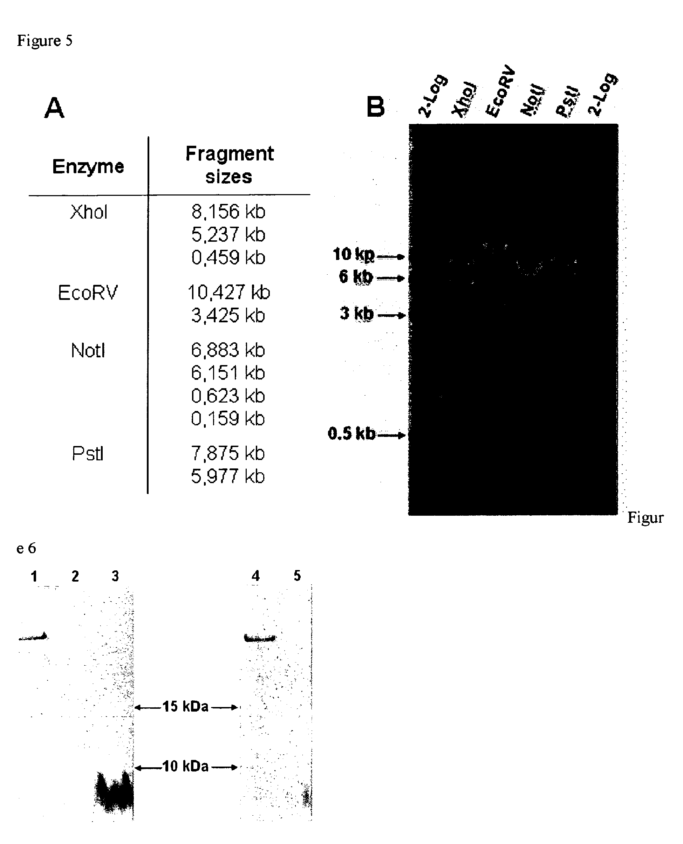

The whole *lab* gene cluster was amplified from a cosmid 1104 by means of PCR with the use of primers *lab-fw*/ rev (Figure 3) and Herculase-II-Fusion-DNA polymerase. Importantly primer *lab-fw* allowed an addition of a RBS characteristic for *Streptomyces* (GGAGG sequence five bp upstream the start codon of *labKC* gene). Thus obtained PCR product (6.4 kb) was digested with enzyme EcoRI and XbaI and directly cloned under the control of a constitutive *ermE* promoter (*47*) into an *Escherichia coli - Streptomyces* shuttle vector pUWLoriT, yielding a new vector pUWLab (13.8 kb) (Figure 4). Correctness of a construct was confirmed by restriction digestion and sequencing reactions (primers: IkoRev1, LabKC_Fw2, LabKC_Fw3, LabKC_Fw4, LabKC_Fw5, LabKC_Fw6, IkoRev3', IkoRev4', IkoFw4'). pUWLab was digested with four different restriction enzymes (XhoI, EcoRV, NotI, PstI). For all of them expected DNA fragments could be observed like it is shown in Figure 5. Also sequencing reaction did not reveal any mistakes (primers used for sequencing are summarized below). Plasmid pUWLab was then transformed to the methylation-defective *E. coli* strain ET12567/pUZ8002. Finally, the desired construct was successfully transformed to few *Streptomyces* strains (*S. coelicolor* M145, S. *lividans* ZX7, *S. albus* CB 89, *S. avermitilis* DSM 46492, *S. griseus* DSM 40236) by conjugation.

### Primers used for amplification of a lab gene cluster and sequencing of pUWLab:

| Primer name | Primer sequence |
|---|---|
| IkoRev1 | CCTGCTCGACGCAGTATTTG (SEQ ID No. 146) |
| LabKC_Fw2 | CCTGCCGGACGGCTGGGAAC (SEQ ID No. 157) |
| LabKC_Fw3 | GGGAGAACGGGACCGTCGAG (SEQ ID No. 158) |
| LabKC_Fw4 | CGCCCGACTACACCGGGTTC (SEQ ID No. 159) |
| LabKC_Fw5 | CCCGCGAGCTCATGGAGCAC (SEQ ID No. 160) |
| LabKC_Fw6 | CCGGCATCCTCGCCTACCTG (SEQ ID No. 161) |
| III- IkoRev3' | GCGTCGTCGAGGATGATCAG (SEQ ID No. 150) |
| IkoRev4' | CTGAAGACGTACGCCTCCTG (SEQ ID No. 154) |
| IkoFw4' | CAGGAGGCGTACGTCTTCAG (SEQ ID No. 155) |

### Cultivation of heterologous strains and detection of Labyrinthopeptins

Mutants transformed with a plasmid pUWLab and parental host strains were cultured in different production media. For each strain two types of liquid cultures (YEME, M5294) and solid agar plates (R2YE, KM4) were prepared. Expression of Labyrinthopeptins was tested by Western blotting experiments and mass spectrometry (HPLC-ESI-MS, HPLC-ESI-MS/MS, LTQ-Orbitrap-MS). All performed experiments are summarized below:

| Expression vector | Heterologous host | Culture conditions | Detection of Labyrinthopeptins |
|---|---|---|---|
| | *S. coelicolor* | Liquid cultures | Western blotting |
| | *S. lividans* | (M5294, YEME) | |
| pUWLab | *S. albus* | | HPLC-ESI-MS |
| | | | LTQ-Orbitrap-MS |
| | *S. avermitilis* | Plates | MS/MS |
| | *S. griseus* | (R2YE, KM4) | |

Although at least three clones were tested and many different growth conditions were applied, no expression of Labyrinthopeptins was observed for *S. coelicolor*, *S. avermitilis* and *S. griseus* carrying the vector pUWLab. Fortunately, the analysis of secondary metabolites showed that, in contrast to the untransformed host strains, *S. lividans* and *S. albus* containing pUWLab accumulated Labyrinthopeptins, but only expression in *S. lividans* gave sufficient amounts of desired products. In the following, results from expression in *S. lividans* are shown.

Western blotting was performed with antibodies which were raised either against mature LabA2 or LabA2 leader peptide. LabA2 is a peptide with a mass of only 2.1 kDa. The small molecular mass may induce difficulties with a good detection by the use of Western blotting technique. As a positive control pure LabA2 or supernatant from a liquid culture *of A. namibiensis* was used. As a negative control served a wild type culture extract. Signals were observed only after incubation with antibody against fully modified LabA2 for *S. lividansl* pUWLab cultures (liquid and agar media) but not for a wild type (Figure 6).

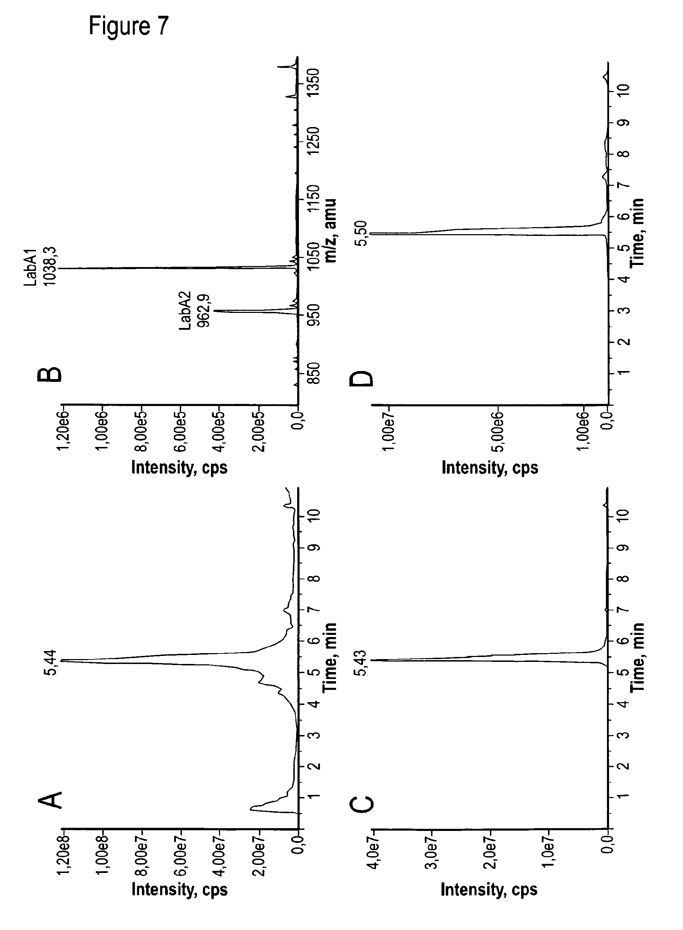

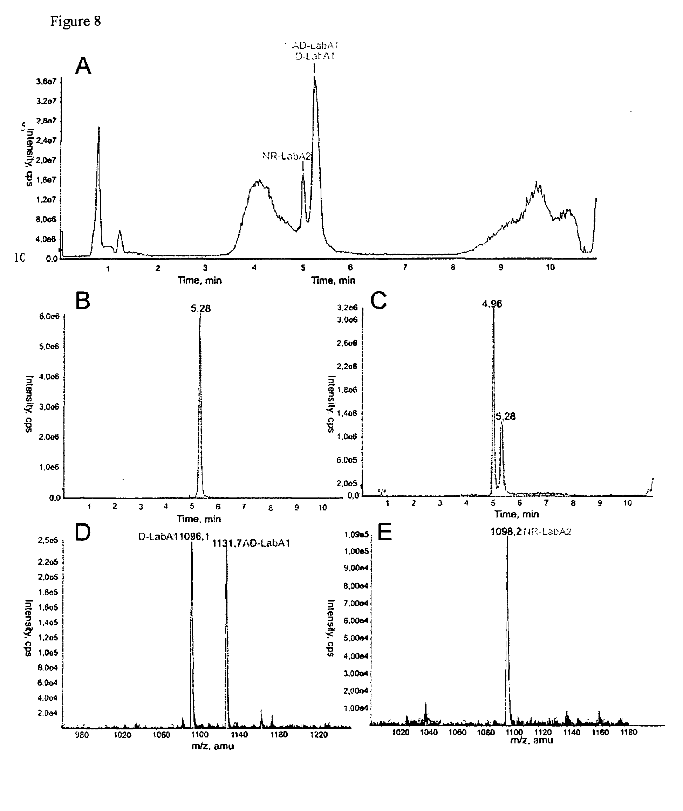

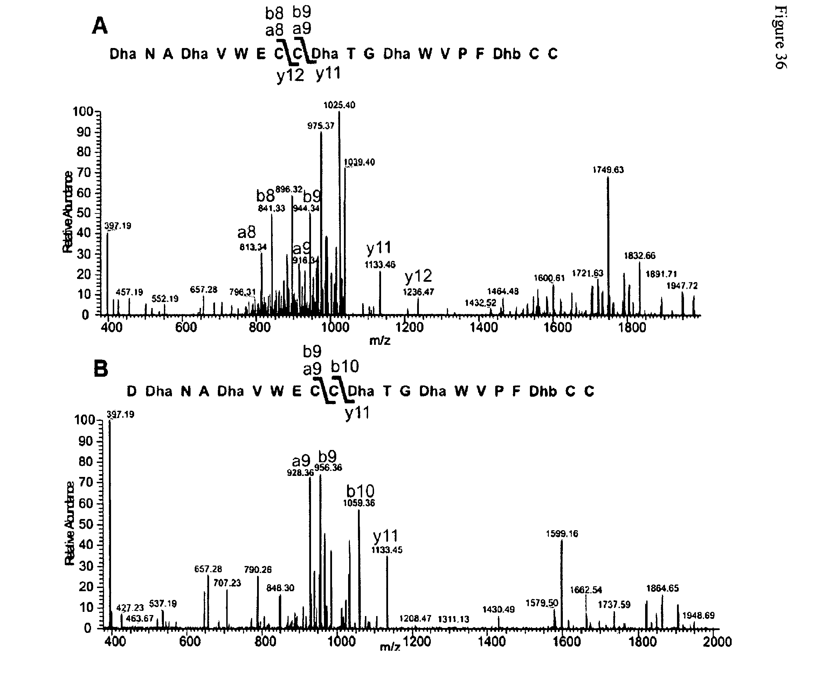

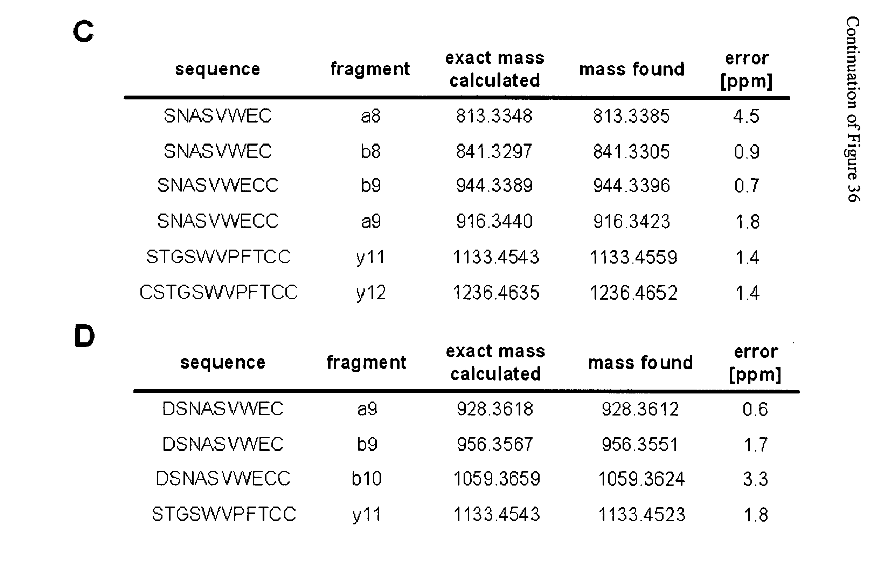

Comparison of LC-MS spectra of *S. lividans* wild type and *S. lividans*/ pUWLab revealed unknown compounds in the culture filtrates of the latter. It was obvious from the evaluation of chromatograms that clones containing the *lab* gene cluster were producing substances which were not present in a wild type. New compounds were detected under all tested growth conditions: in liquid cultures (in the supernatant and in the pellet) and also on agar plates. These metabolites have similar retention time like Labyrinthopeptins (see Figures 7, 8). Surprisingly their masses did not correspond to the masses characteristic for LabA1 and LabA2. Further analysis revealed that those mysterious products were Labyrinthopeptins derivatives with additional amino acids derived from the leader peptide. *S. lividans* was able to express mature LabA1 with an N-terminal Ala or Ala-Asp overhang (Figure 8). The ratio between observed LabA1 derivatives depends on the culture stage. At the beginning of growth LabA1 with two additional amino acids is the main product. In two weeks old cultures of *S. lividans*/pUWLab LabA1 with only one additional *N*-terminal amino acid is the major product. Also expression of fully modified LabA2 was successful. LabA2 derivatives with N-terminal Asn-Arg overhang were detected (Figure 8). These results suggest that the leader sequence may be processed by a non specific protease contributed from the heterologous strain *S. lividans*, although the sequence of the cleavage site appears different from the natural producer. Additionally, LC-ESI-MS/MS and LC-ESI-MS Orbitrap experiments were performed in order to support the observed results. To confirm the correctness of our assumption additional experiments were performed like MS/MS and LTQ/Trap measurements. Tandem mass spectrometry (MS/MS) analysis was used to further confirm the identity of LabA1 observed in cultures of *S. lividans*/pUWLab. Two samples were measured and compared. The control sample was LabA1 isolated from culture broths of *A. namibiensis*, which was referred to a supernatant from a 9 d liquid culture (medium YEME) of *S. lividans*/pUWLab. Molecular masses observed in the LC-MS spectra allowed the prediction that the heterologous host strain can produce D-LabA1 and AD-LabA1. Because the level of production of D-LabA1 in older cultures was higher MS/MS experiments focused on this derivative. Before performing MS/MS measurements disulphide bridges in Labyrinthopeptins were reduced. Fragmentation pattern is in agreement with proposed ring topology and resemble the MSMS fragmentation of the wild type LabA1. High-resolution LTQ Orbitrap mass spectrometry was used to investigate supernatant from a 9 d liquid culture (medium M5294) of *S. lividans*/pUWLab. It was possible to identify and to calculate the molecular formula of singly charged ions which belong to D-LabA1, AD-LabA1 and NR-LabA2. Obtained relative errors have fully acceptable values. These results are in agreement with an outcome from LC-MS (Figure 8) and MS/MS experiments, as shown in Figure 36.

Molecular formula proposals by means of accurate molecular mass measurement compared to experimental data obtained from LC-ESI-OrbiTrap-MS.

| Mutant | Formula | Exact mass calc. | Found mass (Charge state) | Error [ppm] |
|---|---|---|---|---|
| D-LabA1 | C₉₆H₁₂₄O₂₈N₂₄S₄ | 2188.7901 | 2189.9103 (+1) | 3.532 |
| AD-LabA1 | C₉₉H₁₂₉0₂₉N₂₅S₄ | 2259.8271 | 2260.8334 (+1) | 2.515 |
| NR-LabA2 | C₉₅H₁₂₅O₂₇N₂₆S₄ | 2192.8325 | 2193.8210 (+1) | 2.544 |

### Summary

The heterologous expression of Labyrinthopeptins by the use of the cosmid M1104 failed. One explanation is that promoters from *A. namibiensis* were not recognized by *Streptomyces* (*9*). However, it is possible that the use of other strains could result in finding of an efficient method for the heterologous expression of the *lab* gene cluster. Even through many production media were tested, the lack of Labyrinthopeptins expression can be caused by the use of not optimal growth conditions. Interestingly, the *lab* gene cluster does not contain regulatory genes which seem to play an important role for lantibiotics showing homology to Labyrinthopeptins. For example the *ram* gene cluster responsible for an aerial mycelium formation in *S. coelicolor* contains two-component response regulator (RamR) disruption of which results in strains that were 12 to 24 hours delayed in the onset of differentiation compared to the wild-type strains (*48*).

Heterologous expression of a vector pUWLab in *S. albus* and *S. lividans* resulted in a production of LabA1 and LabA2 derivatives. The lack of Labyrinthopeptins in cultures of *S. coelicolor*, *S. griseus* and *S. avermitilis* shows that heterologous expression is an unpredictable method. Labyrinthopeptins expressed in *S. albus* and *S. lividans* contain additional amino acids from a leader peptide, which is not surprising since no candidate gene coding for the protease has been identified in the *lab* gene cluster. Such phenomena has been already described for other lantibiotics e.g. actagardine (*49*) which also doesn't posses a protease in the boundaries of the gene cluster. Expression of the actagardine gene cluster in *Streptomyces lividans* resulted in the production of ala(0)-actagardine (actagardine with additional N-terminal alanine). The presence of additional amino acids in Labyrinthopeptins is the main drawback of the pUWLab construct in a heterologous host. This could negatively influence their biological properties and in any case cause problems for separation by chromatographic methods. Fortunately, this problem was solved in the following example.

### Example 3: Establishing methods for efficient generation of novel Labyrinthopeptins

Lantibiotics can be used as templates for the generation of novel compounds with altered amino acid side chains. This is due to the fact that they are ribosomally synthesized and therefore can be modified by means of site directed mutagenesis. The ribosomal origin of lantibiotics - in contrast to multienzyme complexes - makes them excellent candidates for bioengineering according to current state-of-the-art technologies. Two main strategies have been developed for the production of engineered lantibiotics. They have been already applied for generation of for example nisin (*50-51*), mutacin II (*52*), epidermin (*53*) and Pep5 (*54*) variants.

The first method involves a plasmid-borne, complementary copy of a structural gene in the host strain, and the second involves replacement of the wild-type gene with a mutated copy by using gene replacement procedures. To employ the complementation strategy it is desirable to first inactivate the structural gene encoding the wild type prepropeptide. In all these systems the structural gene is plasmid-encoded and its expression occurs independently of the other enzymes involved in lantibiotic biosynthesis. The advantage of this system is that the sequences involved are more readily manipulated by a variety of molecular biology techniques. Problems may arise when the structural gene is expressed on a multicopy plasmid, because overproduction of the mature lantibiotic could result in higher processing error rates, or in higher antimicrobial activity than the hosts immunity system can cope with. Even if the natural producer is used for expression of novel lantibiotic often the yield of mutated peptide is much lower than the yield observed for the wild type peptide.

The second strategy is based on the replacement of the wild type structural gene by a new prepropeptide. In this case the new structural gene is expressed at its natural genomic location. The advantages of a gene replacement approach are that unchanged gene-dosage and regulatory responses are maintained. It also has the advantage of retaining the balance between structural, biosynthetic and immunity genes. However it is a very time consuming procedure.

Generally, better results are obtained by gene replacement than by complementation. This phenomenon was observed also in case of nisin (*55*). The possible reason for this is that the uncoupling of the transcription of the structural *nisA* gene from downstream genes might have a negative effect on biosynthetic genes expression and in consequence the production level. Because neither complementation nor replacement strategy give a possibility of easy and fast generation of novel lantibiotics without significant dropps in production yield, scientists are still looking for new solutions. Lin et al., 2011 (*56*) described semi-*in vitro* biosynthesis (SIVB) of bovicin HJ50 totally based on *E. coli* expression system. Although expression in *E. coli* facilitates an easy genetic manipulation this method is still not perfect. First it is not possible to obtain high amount of novel lantibiotics, second it increases costs since for each reaction enzymes and substrate need to be purified.

### Construction of the vector pLab for heterologous expression in Streptomyces

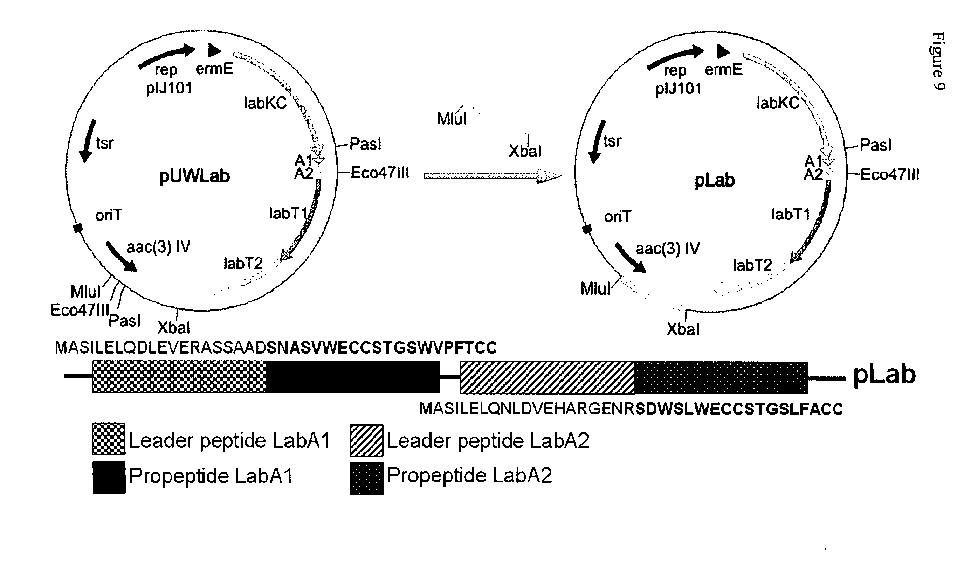

The lack of a method for DNA transfer to *A. namibiensis* impedet the use of this strain for generation of Labyrinthopeptins derivatives. However, the vector pUWLab enables an expression of satisfying amounts of Labyrinthopeptins in *S. lividans* which opened surprisingly a way for expression of novel peptides in a heterologous strain. Because of the ribosomal origin of the peptides, variant generation can be accomplished by mutagenesis of structural genes only. Furthemore, if only a part of pUWLab coding for prepropeptides needs to be modified then there are at least two possible ways of generation of novel peptides. The first strategy is the knock-out of the prepropeptide sequence in pUWLab and then complementation with a small plasmid carrying only genes coding for Labyrinthopeptins pre-pro-peptides. In this situation mutants could be easily generated by means of site-directed mutagenesis. However, this method was rejected because it is very likely that the expression of Labyrinthopeptins would be much lower than when pUWLab is used. First the knock-out could have a polar effect on downstream genes. Second, an inverted repeat located in the short noncoding segment between *labA2* and *labT1* was detected. The presence of a stem-loop structure between the structural gene and the downstream biosynthetic genes is a feature of a number of other lantibiotics (*57*) (*58*). It has been established that the stem-loop structure can stabilize mRNA (*59*) by protecting them from degradation by ribonucleases influencing yields of lantibiotics production. In order to maintain high expression levels another method for generation of mutants was considred. This would involve site directed mutagenesis using expression vector pUWLab as a template. However, one serious complication arose since pUWLab is a comparatively big plasmid with a size of 13.8 kbp. In this situation site directed mutagenesis experiments cannot be performed in one step which complicates the whole procedure and significantly prolongs the time of an experiment. In this situation it was important to determine restriction sites in the pUWLab sequence which could be used to specifically excise *labA1* and *labA2* genes. Then these genes could be subcloned into a smaller plasmid which allows a convenient site-directed mutagenesis experiment. After introduction of a mutation a sequence coding for a novel labyrinthopeptin prepropeptide could be cloned back to pUWLab. Fortunately, it was possible to find suitable restriction enzymes. PasI and Eco47III could be used to precisely excise the prepropeptide encoding region. Restriction sites for these enzymes appear twice in the pUWLab sequence. To remove PasI and Eco47III restriction sites from an undesired region a synthetic gene aac(3)IV was used. It contains a part of *acc3(IV)* gene with two silent point mutations (CGC→CGG (Arg), CTG→CTC (Leu)). PasI recognition sequence CCCTGGG was changed to CCCTCGG which cannot be recognized anymore by the enzyme. Also the Eco47III recognition sequence AGCGCT was disrupted by a single point mutation, which generated AGCGGT sequence. A synthetic gene aac(3)IV was cloned to a pUWLab via MluI and XbaI restriction sites to obtain pLab (cloning strategy is presented in Figure 9).

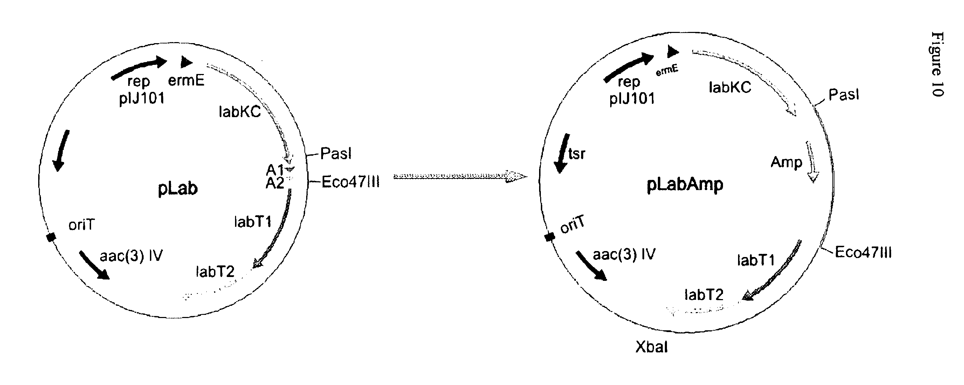

Although pLab allows cloning of new prepropeptide sequences by the use of PasI and Eco47III sites it is still not an optimal construct. After digestion of pLab with these two restriction enzymes two DNA fragments are obtained: 13431 bp and 368 bp. To clone new prepeopeptide sequences to pLab first the digested vector needs to be isolated. However, it is difficult to distinguish on an preparative agarose electrophoresis gel between digested and not digested pLab. In this situation one could overdigest the pLab and use excess of prepared insert for ligation. Unfortunately this was not possible since PasI displays star activity (capability of a restriction enzyme to cleave sequences which are not identical to their defined recognition sequence). It means that after each cloning experiment obtained plasmids need to be sequenced to eliminate these which are carrying wild type sequences. To overcome this problem *labA1* and *labA2* were cut out with PasI and Eco47III, and then these enzymes were used to clone DNA fragment coding for ampicillin resistance from a construct pMA_SG3 (Figure 10). Such obtained pLabAmp vector has several advantages. First, because Amp has a size of 2359 bp it is possible to distinguish on the gel between digested and non digested vector (13428 bp). Second, it gives a possibility to eliminate false positives coming from a non digested vector by preparation of ampicillin replica plate. Bacteria carrying mutated sequences are resistance only to apramycin while bacteria with pLabAmp are resistance to apramycin and also ampicillin.

### Optimization of a template for engineering of LabA1

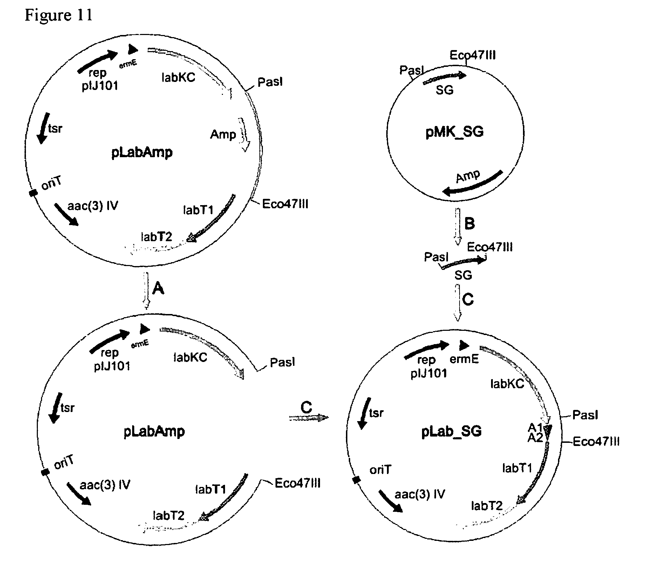

As it was described in Example 2 the drawback of pLab construct is that expressed Labyrinthopeptins contain additionally one or two amino acids at their *N*-termini. Presence of these amino acids can alter biological activity and imposes significant problems in the purification of such peptides. To overcome this limitation two main strategies were investigated. The first idea was to modify the leader sequence so it can be properly processed by host proteases. This would probably constitute the best option since then novel labyrinthopeptins can be directly isolated from bacterial cultures and do not need any further modifications. The second strategy is based on chemical degradation of incorrectly processed peptides. Although this method requires an additional step it is more predictable than the first one. The cloning strategy used to obtain vectors is presented in Figure 11. Unknown proteases from a heterologous strain *S. lividans* are able to cut Labyrinthopeptin Al leader sequence first between Ala-Ala and then between Ala-Asp. But they are not able to perform a cleavage between Asp-Ser, like it has been observed for the natural producer *A. namibiensis.* Based on this observation it was hypothesized that *S. lividans* would be able to cleave Ala-Ala bond. To investigate this hypothesis a synthetic gene SG3 was design where the last amino acid from a leader peptide (Asp) was substituted with Ala. SG3 was cloned to pLabAmp to create a vector pLab_SG3 (according to the cloning strategy shown above in Figure 11). Expression of pLab_SG3 in *S. lividans* shown that the use of this construct allow expression of AA-LabA1 and A-LabA1. After 2-3 weeks of growth the ratio between AA-LabA1 and A-LabA1 shifts to give A-LabA1 as a main product. Interestingly in older cultures also the molecular mass corresponding to LabA1 could be found. Although the use of pLab_SG3 allows expression of LabA1 without any additional amino acids the amount of LabA1 was so low that this construct could not be used for further studies. Because no other data allowing a prediction of possible cleavage site was available, a strategy of obtaining Labyrinthopeptins completely processed in *S. lividans* was rejected at this stage of the project.

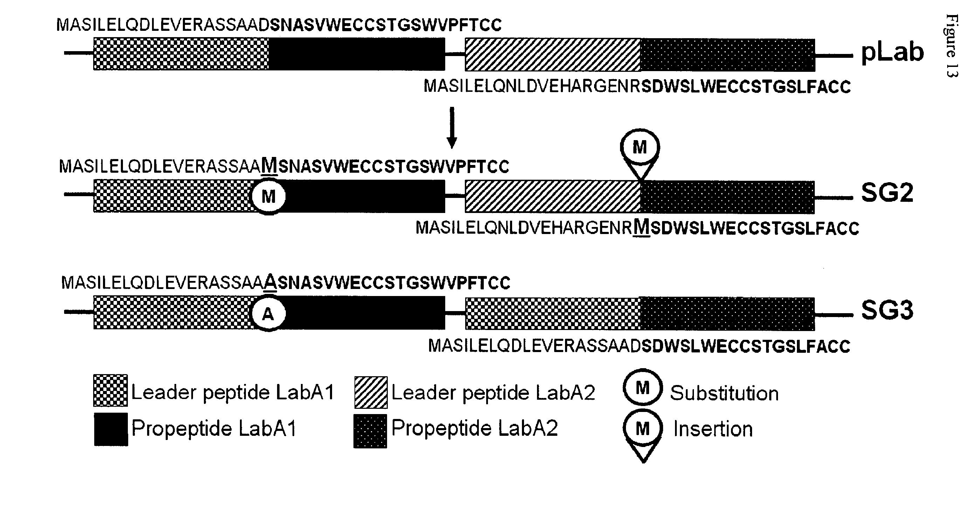

Another strategy was to isolate Labyrinthopeptins with additional amino acids allowing later reaction with CNBr, which removes N terminal methionine (*60*). To follow this method first Labyrinthopeptins with Met as the last, C-terminal amino acid in the leader peptide need to be expressed. A synthetic gene SG2 was ordered where in *labA1* gene Asp codon was substituted with Met, and in *labA2* gene Met codon was inserted between the last, C-terminal amino acid from a leader peptide and the first amino acid from a prepeptide (Figure 13). Peptides observed in *S. lividans*/ SG2 cultures where surprising (see Figure 12): In one week old cultures AM-LabA1, M-LabA1 and also LabA1 could be detected. In older cultures (2-3 weeks old) almost all AM-LabA1 and M-LabA1 were converted to LabA1. Interestingly, no expression of LabA2 was detected. It seems that addition of Met completely abolish an expression of LabA2. Similar results were obtained in *in vitro* assays.

The use of pLab_SG2 allows an expression of LabA1 in *S. lividans* in the same way like in *A. namibiensis.* Another advantage of that construct is that LabA2 is not present in a *S. lividans*/ pLab_SG2 culture, which makes isolation of LabA1 much easier. SG2 was used as a template for the generation of almost all LabA1 derivatives by means of site-directed mutagenesis below.

Peptides observed in a supernatant of *S. lividans*/pLab_SG2 and *S. lividans*/ pLab_SG3 (ND-not detected):

| | | Peptide masses | (Da) | |
|---|---|---|---|---|
| Construct/ Labyrinthopeptin derivate | Formula | Expected molecular mass | Expected [M+2H]²⁺ | Observed [M+2H]²⁺ |
| pLab_SG2/ LabA1 | C₉₂H₁₁₉N₂₃O₂₅S₄ | 2073.76 | 1037.88 | 1037.99 |
| pLab_SG2/ M- LabA1 | C₉₇H₁₂₈N₂₄O₂₆S₅ | 2204.96 | 1103.48 | 1103.38 |
| pLab_SG2/ AM- LabA1 | C₁₀₀H₁₃₃N₂₅O₂₇S₅ | 2276.04 | 1139.02 | 1138.87 |
| pLab_SG3/ LabA1 | C₉₂H₁₁₉N₂₃O₂₅S₄ | 2073.76 | 1037.88 | ND |
| pLab_SG3/ A-LabA1 | C₉₅H₁₂₄N₂₄O₂₆S₄ | 2144.84 | 1073.42 | 1073.9 |
| pLab_SG3/ AA_LabA1 | C₉₈H₁₂₉N₂₅O₂₇S₄ | 2215.92 | 1108.96 | 1109.7 |

### Optimization of a vector template for engineering of LabA2

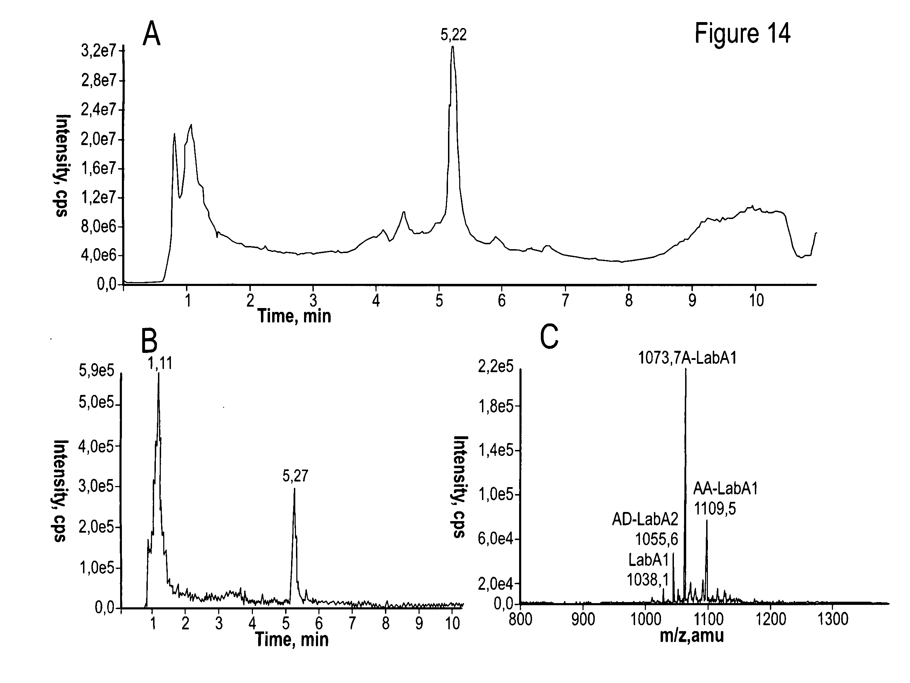

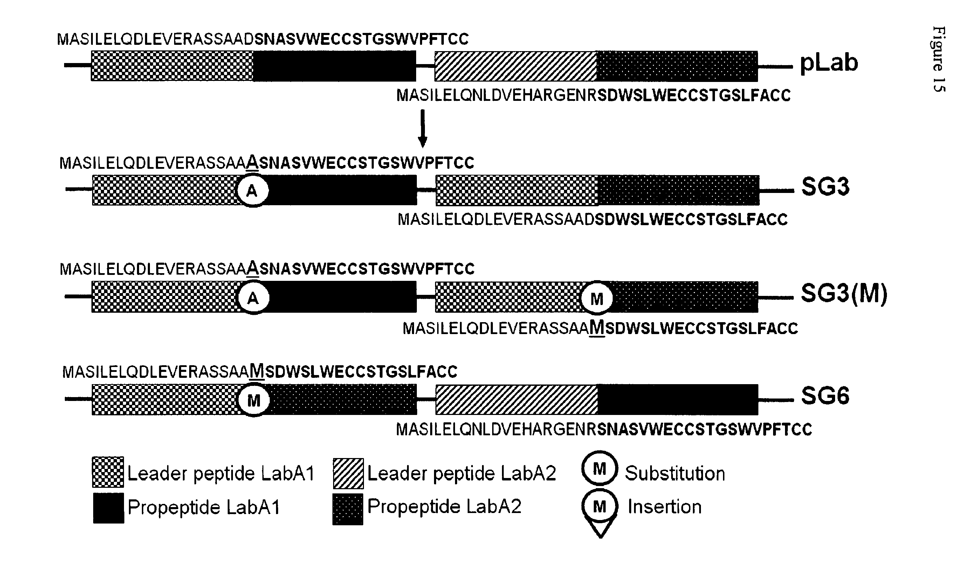

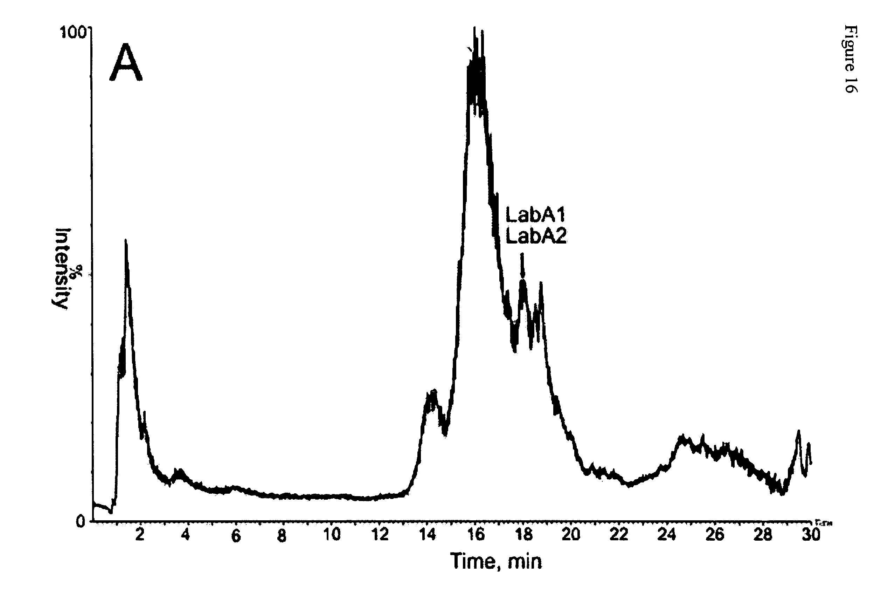

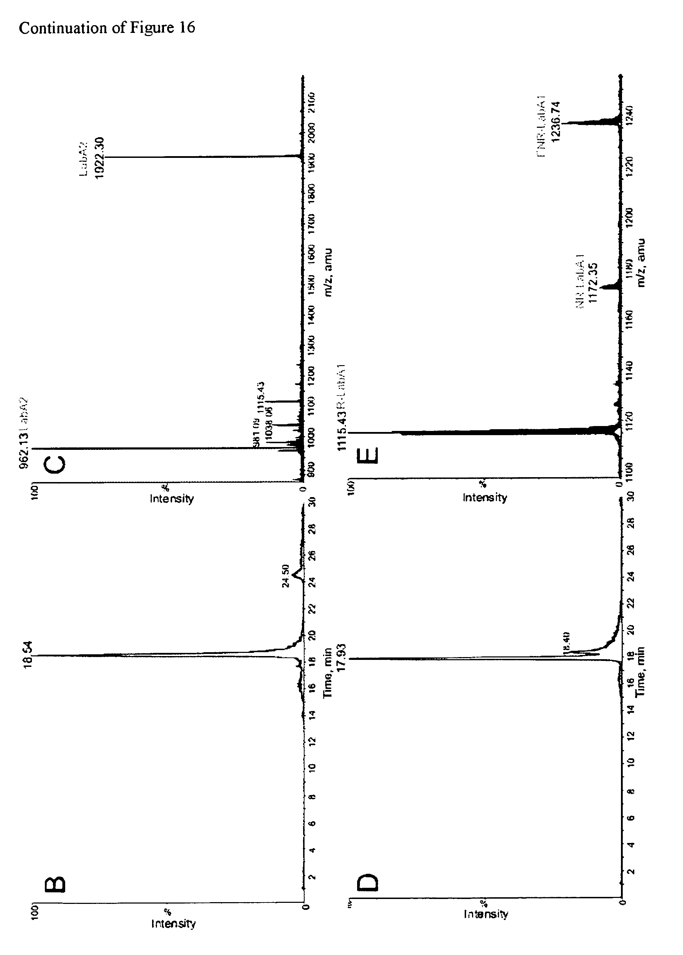

It was important to verify if the method established for proteolytic processing of LabA1 leader peptide could be applied to LabA2. The use of a gene SG3 (Figures 13, 14 and 15) showed that the expression of the peptide AD-LabA2 is possible when LabA2 prepeptide is attached to the LabA1 leader peptide. Another step was to generate SG3(M) by substitution of Asp with Met (like in SG2 for LabA1). This was accomplished by site-directed mutagenesis with primers D20M_fw (5'-CAGCTCGGCCGCCATGTCCGACTGGAGC-3') and D20M_rv (5'-GCTCCAGTCGGACATGGCGGCCGAGCTG-3') and SG3 as a template for PCR. *S. lividans*/pLab_SG3 was able to express LabA2 without additional amino acids from a leader sequence although expression levels were not high. It was observed for a wild type and also for heterologous strain that expression of LabA1 is higher than LabA2. It is possible that higher expression levels are due to the arrangement of genes in the *lan* cluster. In order to overcome a problem with low LabA2 expression, a gene SG6 was design where the order of *labA1* and *labA2* were swapped. Indeed LabA2 expression with the use of pLab_SG6 was higher than with pLab_SG3(M). *S. lividans*/ pLab_SG6 expressed not only LabA2 but also LabA1 derivatives, as shown in Figure 16. SG6 was used for generation of almost all LabA2 derivatives like it is described in an example below.

Peptides observed in a supernatant of *S. lividans*/ pLab_SG3, *S. lividans*/ pLab_SG6 and *S. lividans*/ pLab_SG3(M):

| | | Peptide masses (Da) | | |
|---|---|---|---|---|
| Construct/ Labyrinthopeptin derivate | Formula | Expected molecular mass | Expected [M+2H]²⁺ | Observed [M+2H]²⁺ |
| pLab_SG3/ AD-LabA2 | C₉₂H₁₂₀N₂₂O₂₈S₄ | 2108.86 | 1055.43 | 1055.7 |
| pLab_SG3(M)/ AM-LabA2 | C₉₃H₁₂₄N₂₂O₂₆S₅ | 2124.97 | 1063.48 | 1063.7 |
| pLab_SG3(M)/ M- LabA2 | C₉₀H₁₁₉N₂₁O₂₅S₅ | 2053.89 | 1027.94 | 1028.3 |
| pLab_SG3(M)/LabA2 | C₈₅H₁₁₀N₂₀O₂₄S₄ | 1922.69 | 962.34 | 963.0 |
| pLab_SG6/ M-LabA2 | C₉₀H₁₁₉N₂₁O₂₅S₅ | 2053.89 | 1027.94 | 1027.99 |
| pLab_SG6/ LabA2 | C₈₅H₁₁₀N₂₀O₂₄S₄ | 1922.69 | 962.34 | 962.52 |
| pLab_SG6/ ENR-LabA1 | C₁₀₇H₁₄₄N₃₀O₃₁S₄ | 2473.16 | 1237.58 | 1237.24 |
| pLab_SG6/ NR-LabA1 | C₁₀₂H₁₃₇N₂₉O₂₈S₄ | 2344.05 | 1173.02 | 1172.83 |
| pLab_SG6/ R-LabA1 | C₉₈H₁₃₁N₂₇O₂₆S₄ | 2229.95 | 1115.97 | 1115.89 |

### Expression of a single Labyrinthopeptin

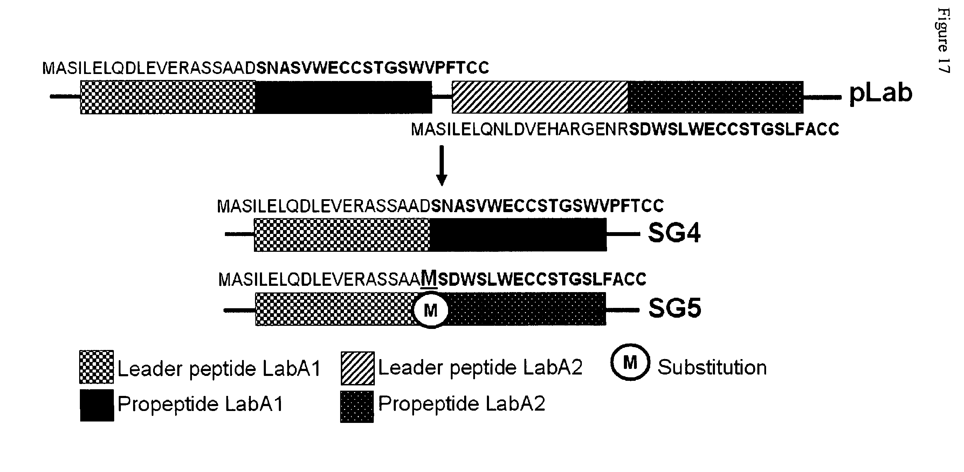

Since LabA1 and LabA2 are similar molecules it would be helpful for isolation and downstream processing purposes to have a system to express not a mix of LabA1 and LabA2 but only a single labyrinthopeptin derivative at a time. Such a possibility was tested on two different ways. First by the use of pLab/ SG4 where labA1 but not labA2 was present. The second construct was pLab/SG5, which contains only a sequence coding for LabA2 prepeptide attached to LabA1 leader peptide with Met as a C-terminal amino acid of the leader peptide (see Figure 17). No expression of LabA1 derivatives were detected in cultures of *S. lividans*/ pLab_SG4 as well as none LabA2 derivatives were observed in *S. lividans*/ pLab_SG5 cultures. These results show that both genes coding for prepropeptides are needed Labyrinthopeptin expression. This phenomenon is still not explained but probably it is due to the stabilization of mRNA. It seems that the best method for expressing of only one Labyrinthopeptin is to insert Met between sequence of leader and prepeptide like it was done for LabA2 in SG2 gene. This data demonstrates that the complementation strategy, which requires structural gene knock-out, would probably fail.

### Detection of Labyrinthopeptin derivatives in cultures of S. lividans/ pLab_SG4 and S. lividans/ pLab_SG5 (ND-not detected):

| | | Peptide masses (Da) | | |
|---|---|---|---|---|
| Construct/ Labyrinthopeptin derivate | Formula | Expected molecular mass | Expected [M+2H]²⁺ | Observed [M+2H]²⁺ |
| pLab_SG5/ AM-LabA2 | C₉₃H₁₂₄N₂₂O₂₆S₅ | 2124.97 | 1063.48 | ND |
| pLab_SG5/ M-LabA2 | C₉₀H₁₁₉N₂₁O₂₅S₅ | 2053.89 | 1027.94 | ND |
| pLab_SG5/ LabA2 | C₈₅H₁₁₀N₂₀O₂₄S₄ | 1922.69 | 962.34 | ND |
| pLab_SG4/ AD-LabA1 | C₉₉H₁₂₉O₂₉N₂₅S₄ | 2259.83 | 1130.91 | ND |
| pLab_SG4/ D-LabA1 | C₉₆H₁₂₄O₂₈N₂₄S₄ | 2188.79 | 1095.39 | ND |
| pLab_SG4/ LabA1 | C₉₂H₁₁₉N₂₃O₂₅S₄ | 2073.76 | 1037.88 | ND |

In summary, it was possible to establish a method for engineering of Labyrinthopeptins which overcomes three main problems very often found in related studies. First of all, because the designed method introduces as few changes as possible in the *lab* gene cluster, the high level of expression could be maintained. This is an extremely important aspect in terms of subsequent peptide isolations and biological activity tests. Additionally a cloning strategy was designed to be very efficient. The use of a vector pLabAmp allows fast and easy generation of labyrinthopeptin variants only by cloning of fragments containing mutated structural genes. *labA1* and *labA2* genes on a small plasmid (pMK or pMA) are easily manipulated by site-directed mutagenesis. Capabilities for creation of mutants by the use of a system proposed in this work are demonstrated by the number of generated novel labyrinthopeptins described herein.

The third and probably most challenging problem was to express labyrinthopeptins without any additional N-terminal overhangs. During this study a new processing site allowing correct leader peptide removal in the heterologous host was discovered. Most importantly the introduced modifications had no detectable impact on the processing by the LabKC enzyme. This strategy enabled to fully control wild type LabA1 and LabA2 productions by introducing a Met residue between leader and structural peptides and by exchanging leader peptides. It was also possible to engineer the construct to enforce production of only one labyrinthopeptin.

Presented results demonstrate further that establishing of a heterologous expression system for lantibiotic production, and *in vivo* engineering of these compounds is not an easy and straightforward task. What seems to be true also in similar studies is that the best strategy is to combine biochemical intuition with a trial and error approach.

### Example 4: Engineering of new Labyrithopeptins

It is always desirable to generate new variants of natural products displaying biological activities. Systematically introduced changes can be helpful in understanding the mode of action and even in the building of a pharmacophore model for a drug candidate. Moreover, rationally designed modifications can tailor some interesting properties like activity, stability or ADME profiles. Lantibiotics seem to be good targets for engineering with the possibility of accommodating numerous changes, because of their ribosomal origin and promiscuous substrate specificity (*61-62*) of enzymes involved in their biosynthesis. Modifications could be introduced by using different methods e.g. chemical synthesis, genetic modification of producing strain, chemical modifications on naturally produced compounds and semisynthesis. For the presented study, a biological approach was chosen, mainly as a consequence of difficulties associated with the synthetic attempts. However, there are reports of successful synthesis of lantibiotics e.g. total synthesis of nisin A (*63*) and lactocin S (*64*) were successfully conducted. Nevertheless, these methods are still expensive, multistep and not efficient enough for drug development purposes.

The lantibiotic engineering faces many obstacles mainly because of the fact that expression systems need to be developed not only for structural genes but also for genes encoding biosynthetic enzymes, immunity and regulatory proteins. Even the well-developed expression systems do not guarantee a successful expression of the final product. It has been shown that the number of mutations which can be applied is limited (*65*). Many designed mutants could not be produced, probably due to disturbed interactions of the peptides with biosynthetic enzymes or secretion machinery (*65*). In spite of all these limitations a large variety of different lantibiotics variants has been reported and characterized, the most prominent examples being: nisin (*50-51*), Pep5 (*54*), epidermin (*53*), mutacin II (*52*), actagardine (*49*), lacticin 3147 (*66*), mersacidin (*67*). Unfortunately, in most cases obtained derivatives did not exhibit improved properties. One of the most notable mutants was prepared for subtilin by substitution of glutamate (Glu4) with isoleucine. This derivative possesses approximately 3-4 fold higher activity than the natural peptide and displays a 57-fold increase in chemical and biological stability (*68*). Few spectacular mutants were prepared also for nisin. Nisin derivative which was prepared by conversion of Dha5 to Dhb has a greater resistance to acid catalyzed chemical degradation than wild-type nisin Z (*68*). Similarly the solubility of nisin at neutral pH has been enhanced through mutagenesis (*68*). Studies on this lantibiotic demonstrated that the use of genetic engineering enables modulating the antimicrobial activity of a compound, with respect to particular target organisms. A nisin mutant G18T/M17Q was approximately 4 fold less active than nisin against *B. cereus* and *S. thermophilus*, but it was twice more active than the wild type nisin against *M*. *flavus* (*68*).

In order to overcome problems connected with the generation of mutants *in vivo* (e.g. immunity and regulatory proteins must be functional, mutated peptides may be degraded by proteases) *in vitro* assays were developed. Another advantage of the *in vitro* assay is that lantibiotic biosynthetic enzymes used in it show even greater flexibility than in the *in vivo* method. Moreover, biosynthesis intermediates generated by an *in vitro* approach commonly can be more specifically characterized by bioanalytical methods, which allows elucidation of biosynthesis mechanisms on a molecular level (*69*). The successes in this area include the reconstitution of activity in *in vitro* assays for lacticin 481 (*61*), nukacin ISK-1 (*70*), mutacin II (*70*), ruminococcin A (*70*), nisin (*71*), haloduracin (*72*), Labyrinthopeptin (*73*), venezuelin (*74*) and lantipeptides from *Prochlorococcus* MIT9313 (*75*). In some cases it was not possible to reconstitute *in vitro* activity despite of multiple attempts (reported examples: RamC, NisA). Worth mentioning is also the fact that the benefits are often restricted by the limited quantities of the final product.

Another reason which makes lantibiotics engineering very interesting is the presence of lanthionine, a characteristic structural motif providing chemical, proteolytic and metabolic stability. Several studies have shown that cyclic lanthionines increase both stability and activity of non-lantibiotic peptides (*76-77*). This suggests that this motif may be used as the structural core for the design of new therapeutic peptides. Moreover, the recently reported labionin amino acid, which was identified in Labyrinthopeptin structures, provides even higher conformational constraint and seems to be responsible for novel biological activity against neuropathic pain and viral diseases. As described in the Example above, we succeeded in the construction of a vector pLab_SG2 and pLab_SG6 which can easily be used as a template for bioengineering of LabA1 and LabA2, respectively. This enables expressing Labyrinthopeptin-like peptides for SAR studies purposes and for testing substrate specificity of the modifying enzyme. To the best of my knowledge, this is the first report of engineering of a lantibiotic demonstrating a biological function different than antimicrobial activity. These are also the first *in vivo* studies on generation of mutants containing labionin.

### Strategy for the generation of mutants

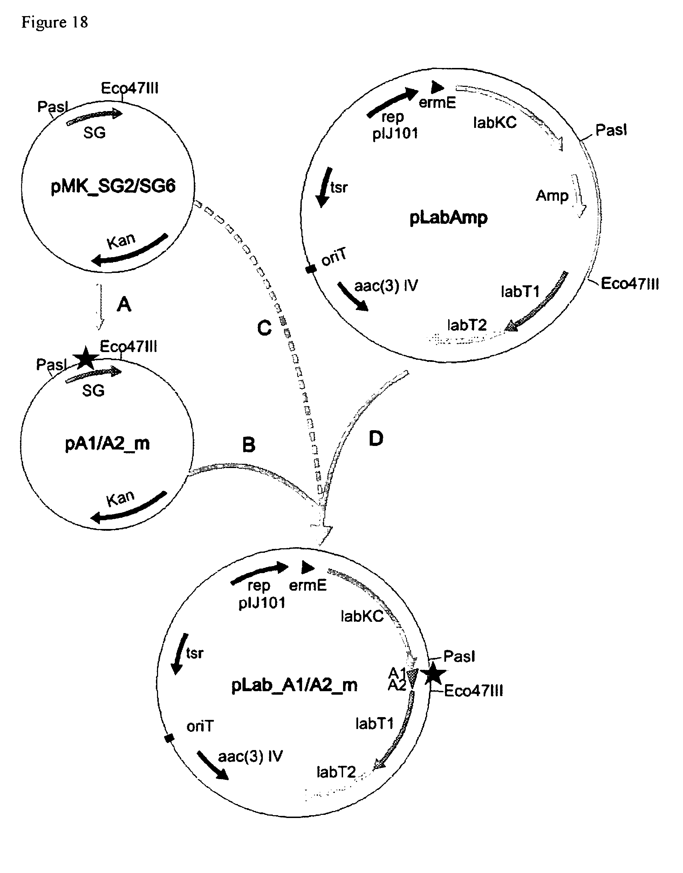

All Labyrinthopeptin derivatives were generated in the same way as shown in Figure 18. The first step was the preparation of a linearized vector pLabAmp by restriction digestion with PasI and Eco47III. Then, fragment of the vector containing the *lab* gene cluster (13.4 kpb) without structural genes was extracted from agarose gel. Since yields from DNA recovery after agarose gel isolation is normally very low, most of the cloning experiments were performed using the whole restriction mixture purified with the GeneJET™ PCR Purification Kit (Fermentas). The desired insert was provided by PCR reaction from an appropriate template plasmid using Infusion1 and Infusion_rv primers. Reaction conditions were optimized, to obtain only a single product which was directly used to prepare a final construct (pLab_A1_m or pLab_A2_m, where m indicates modifications in LabA1 or LabA2 respectively) with ligation independent cloning approach (LIC, (*13-14*)). All constructs were verified by sequencing and/or restriction digestion. Because of high GC content and the presence of secondary structures in many cases sequencing reaction with pLab_A1/2_m failed. To overcome this problem a region coding for Labyrinthopeptins prepropeptides was amplified by PCR with Infusion1 and Infusion_rev primers, isolated and sequenced with primer Infusion1.

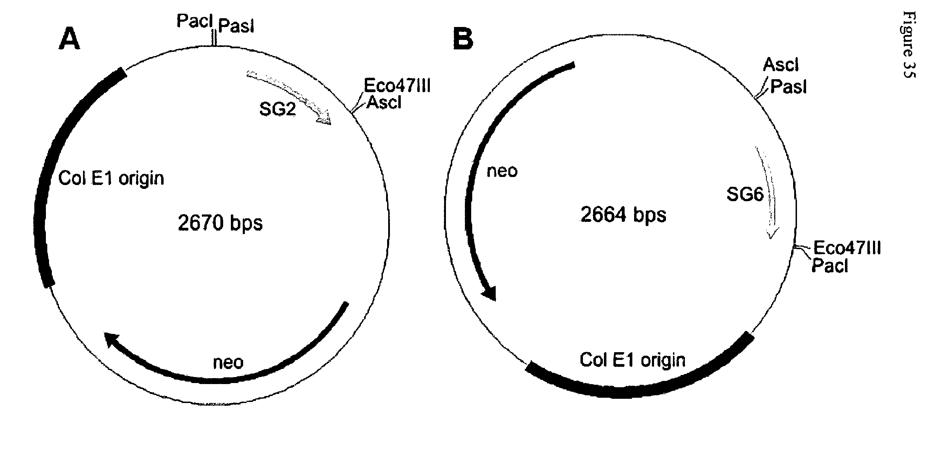

Two main types of template plasmids were used to generate mutants. In order to perform site-directed mutagenesis of *labA1* and *labA2* structural genes were provided on above described plasmids: pMK_SG2 and pMK_SG6. Small size of these vectors (pMK_SG2: 2670 bp, see Figure 35A; pMK_SG6: 2664 bp, see Figure 35B) allows an easy exchange of amino acids. While designing primers the codon usage of *Streptomyces* (see blow) was considered and most frequent codons were used. All constructs were confirmed by sequencing with pMA primer. Correct inserts were amplified by PCR and used for preparation of final constructs as described above. The second type of plasmid templates used to prepare more complicated variants were provided on plasmids pMK_SGx ("x" refers to variant number). In this case structural genes between PasI and Eco47III sites were prepared by a combination of enzymatic and chemical synthesis of DNA (78) and purchased from GENEART AG (Regensburg Germany) already cloned into pMK plasmid.

The codon usage (CU) of the genus *Streptomyces* (88). CU - codon usage in *Streptomyces* (%).

| Amino acid | Codon | CU | Amino acid | Codon | CU | Amino acid | Codon | CU |
|---|---|---|---|---|---|---|---|---|
| Phe | TTT | 1.6 | Ser | TCT | 1.1 | Arg | CGT | 7.3 |
| | TTC | 98.4 | | TCC | 39.1 | | CGC | 45.0 |
| | | | | TCA | 2.4 | | CGA | 3.7 |
| Glu | GAA | 17.7 | | TCG | 27.4 | | CGG | 37.7 |
| | GAG | 82.3 | | | | | | |
| | | | Cys | TGT | 12.0 | Gln | CAA | 6.1 |
| Leu | TTA | 0.4 | | TGC | 88.0 | | CAG | 93.9 |
| | TTG | 2.6 | | | | | | |
| | | | Stop | TGA | 81.0 | Ala | GCT | 2.3 |
| Tyr | TAT | 5.0 | | | | | GCC | 58.7 |
| | TAC | 95.0 | Trp | TGG | 100.0 | | GCA | 4.3 |
| | | | | | | | GCG | 34.7 |
| Stop | TAA | 4.0 | Pro | CCT | 2.7 | | | |
| | TAG | 15.0 | | CCC | 42.4 | Ile | ATT | 4.4 |
| | | | | CCA | 1.6 | | ATC | 92.0 |
| Leu | CTT | 2.0 | | CCG | 53.3 | | ATA | 3.6 |
| | CTC | 38.5 | | | | | | |
| | CTA | 0.3 | His | CAT | 6.5 | Asn | AAT | 4.6 |
| | CTG | 56.1 | | CAC | 93.5 | | AAC | 95.4 |
| Thr | ACT | 2.2 | Ser | AGT | 2.9 | Val | GTC | 56.0 |
| | ACC | 65.2 | | AGC | 27.1 | | GTA | 2.5 |
| | ACA | 2.4 | | | | | GTG | 38.8 |
| | ACG | 30.2 | Gly | GGT | 8.7 | | | |
| | | | | GGC | 64.2 | Met | ATG | 100.0 |
| Lys | AAA | 5.8 | | GGA | 8.9 | | | |
| | AAG | 94.2 | | GGG | 18.2 | | | |

### Explanation of the meaning of terms used to designate mutation codes in this study:

| Mutation type | Mutation code | Description |
|---|---|---|
| Substitution | E15P | Replaces a glutamic acid (E) at position 15 with a proline (P). |
| Deletion | E15del | Deletes a glutamic acid (E) at position 15. |
| | EF15del | |
| | | Deletes a glutamic acid and a phenylalanine (EF) at positions 15-16. |
| Insertion | E15insP | Inserts a proline (P) at position 15, pushing the glutamic acid (E) to position 16. |
| | EF15insPQ | Inserts a proline and a glutamine (PQ) at positions 15-16, pushing the glutamic acid and the phenylalanine (EF) to positions 17-18. |

### Alanine scanning mutagenesis of LabA1 and LabA2

Structure-activity relationship (SAR) studies evaluate importance and contributions of different parts of a target molecule to its bioactivity. This allows later rational modifications which may improve its properties. SAR studies may also be useful for building a pharmacophore model of lead compounds during drug development processes. The most important requirement for performing SAR studies is the availability of structural analogs. In the case of ribosomally synthesized peptides genetic manipulations leading to the generation of new peptides are very often more efficient than synthetic approaches. However, the most important issue is the recognition and proper processing by the post-translational modification machinery. One of the most widely used strategies for SAR studies of peptides and proteins is alanine scanning, when relevant residues are exchanged one by one with Ala. In most of the cases Ala is a reasonable choice since it neither disturb secondary structures nor introduce extreme steric or electrostatic changes (*79*).

Above presented strategy to generate mutants of Labyrinthopeptins was applied to alanine scanning procedure. Since investigated peptides display interesting antiviral activities and a remarkable activity against neuropathic pain (mouse model) this experiment is a starting point for subsequent SAR studies. Moreover, alanine scanning in this case will additionally provide information about importance of each residue for biosynthesis which is a key issue for isolation and further activity studies. Former studies showed that the production levels of lantibiotics mutants were significantly reduced or totally suppressed when residues involved in lanthionine formation were modified (*66*). This indicates that the presence of the lanthionine rings is important for recognition by the secretion systems (*53*, *80*) and it might provide proteolysis stability necessary suppress degradation. Based on those findings, in this study only amino acids which are not involved in formation of rings (lanthionine, labionin and disulphide according to the X-ray crystal structure) were substituted with Ala.

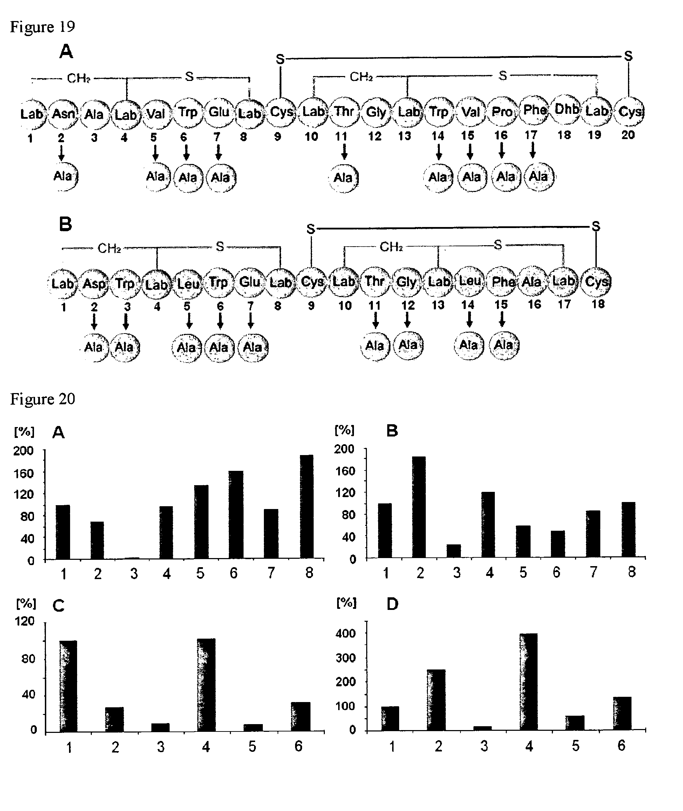

Generation of nine mutants for LabA1 and also nine mutants for LabA2 was accomplished. For two LabA1 mutants (LabA1_G12A and LabA1_T18A) it was impossible to introduce necessary mutation even though site-directed mutagenesis experiments were repeated several times. All performed mutations are summarized in Figure 19. LC-MS was used in order to detect production of mutated peptides. In each case presence of peptides in liquid cultures and in extracts from MS plates was verified. Interestingly, LabA1 and LabA2 mutants displayed considerable differences in productions observed in liquid cultures. Molecular masses corresponding to LabA1 mutants were found in all three tested media (YEME, R2YE, NZ Amine). LabA2 mutants were produced in sufficient amounts only in NZ Amine medium (exceptions were mutants where Trp was substituted with Ala). In all cases observed molecular masses were in agreement with theoretically calculated ones. Production yields of LabA1 and LabA2 mutants varied significantly which was in agreement with observations made for other engineered peptides (*81*). Figure 20 shows changes in production levels of LabA1 and LabA2 alanine mutants after 8 and 21 days of growth in NZ Amine medium. To eliminate additional factors, which could have an influence on Labyrinthopeptins production, all cultures were started in the same day and bacteria were grown in the same type of flask. Presented quantification was performed by means of LC-MS with assumption that introduced mutations do not change ionization efficiency in LC-ESI-MS investigation. Since measurements were performed in positive mode and none of mutated residues was basic this was quite a reasonable assumption. For LabA1 as well as for LabA2 all mutants where tryptophan was substituted with alanine were produced only in trace amounts. This result may indicate that these peptides are not efficiently recognized by the modification and/or secretion system. An alternative explanation might be that these residues are crucial for proper peptide conformation which is necessary for the ring formation. Another interesting finding concerns Trp3 of LabA2. As presented on Figure 19, substitution of this residue with alanine makes A ring of LabA2 very similar to that of LabA1 (dipeptides Asn-Ala for LabA1 and Asp-Ala for LabA2). However, even in this case production is significantly suppressed after Trp substitution which is even more surprising since both peptides are modified by the same enzyme.

### Structural characterization of a mutant peptide LabA2_L14A derived from LabA2 by NMR spectroscopy

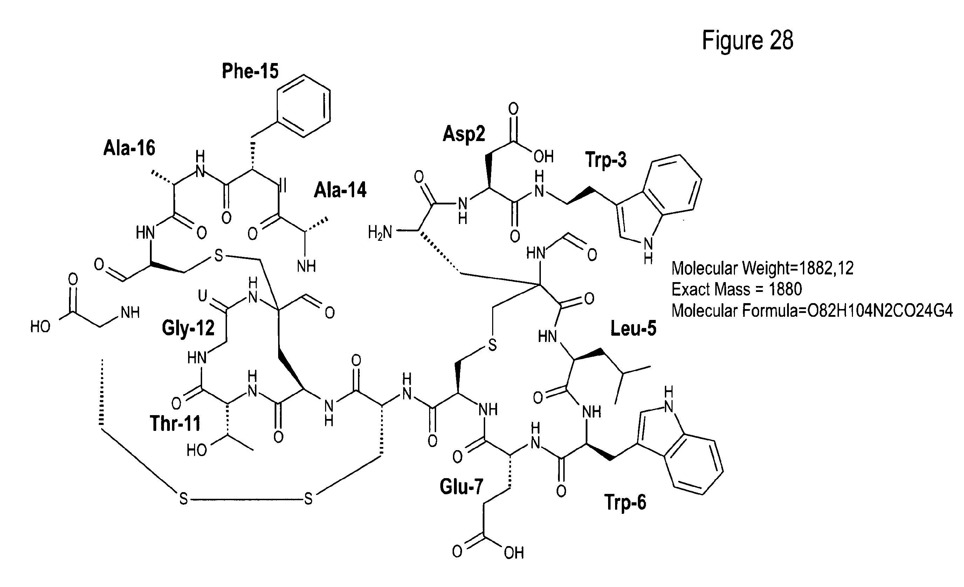

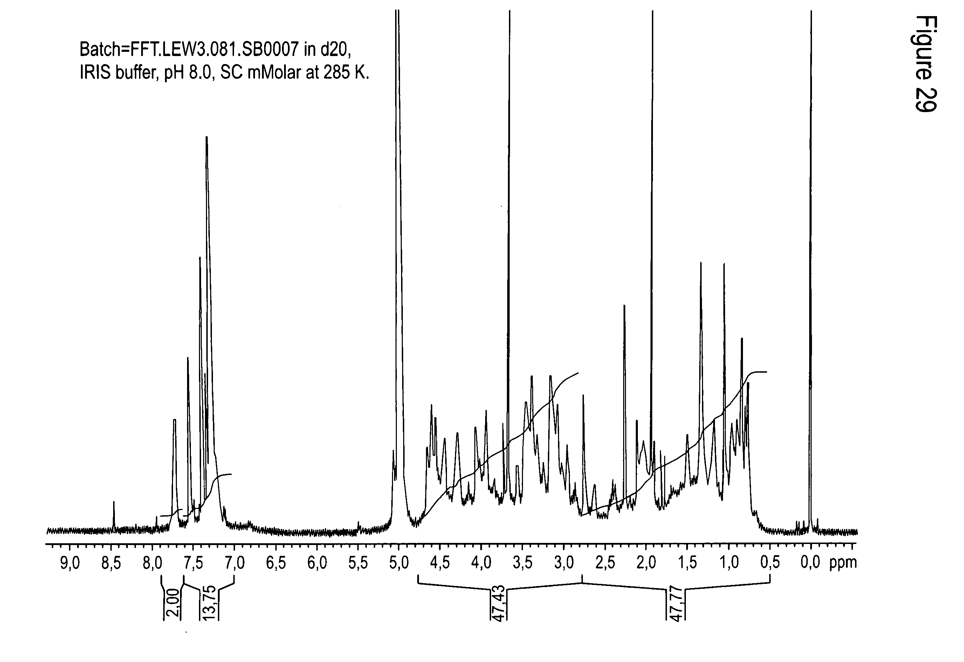

The position of substitution in the mutant peptide LabA2_L14A (Leu14 is substituted by Ala; see Figure 28) was identified by comparison with the NMR spectra (Figure 29) obtained for the natural Labyrinthopeptin A2. The analysis of the NMR spectra is hampered by partially extreme line broadening. A partial proton and carbon assignment is given in the following table, showing chemical shifts of LabA2_L14A and LabA2 in TRIS buffer (pH 8.0, 50 mMolar) at 285 K:

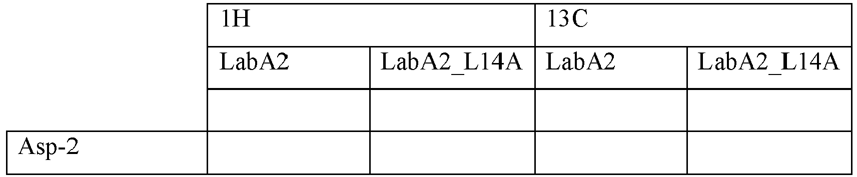

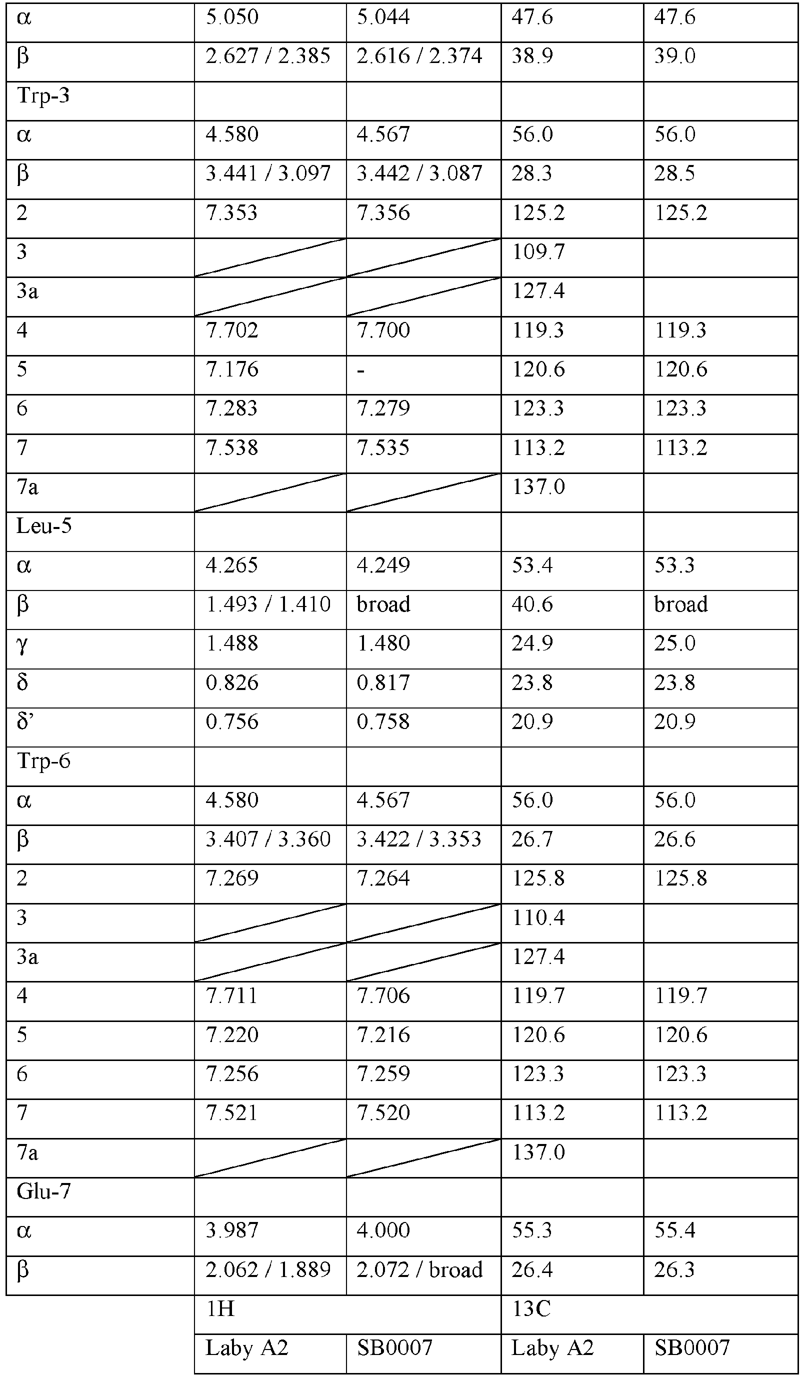

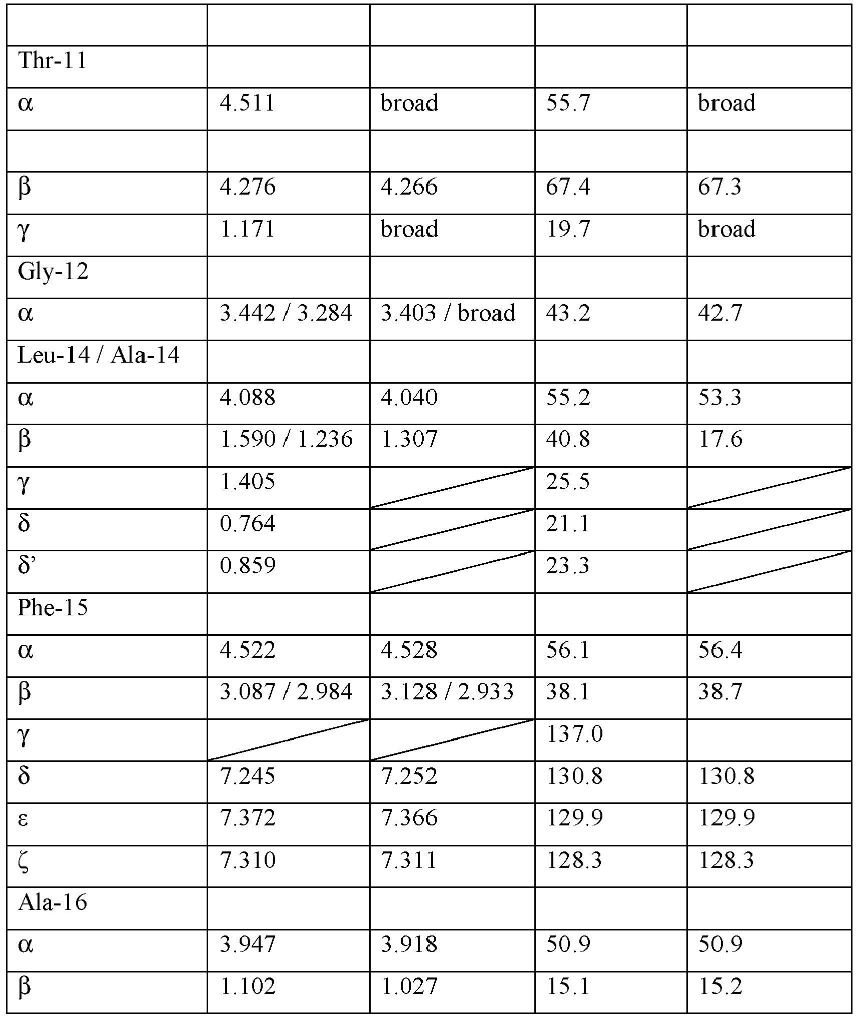

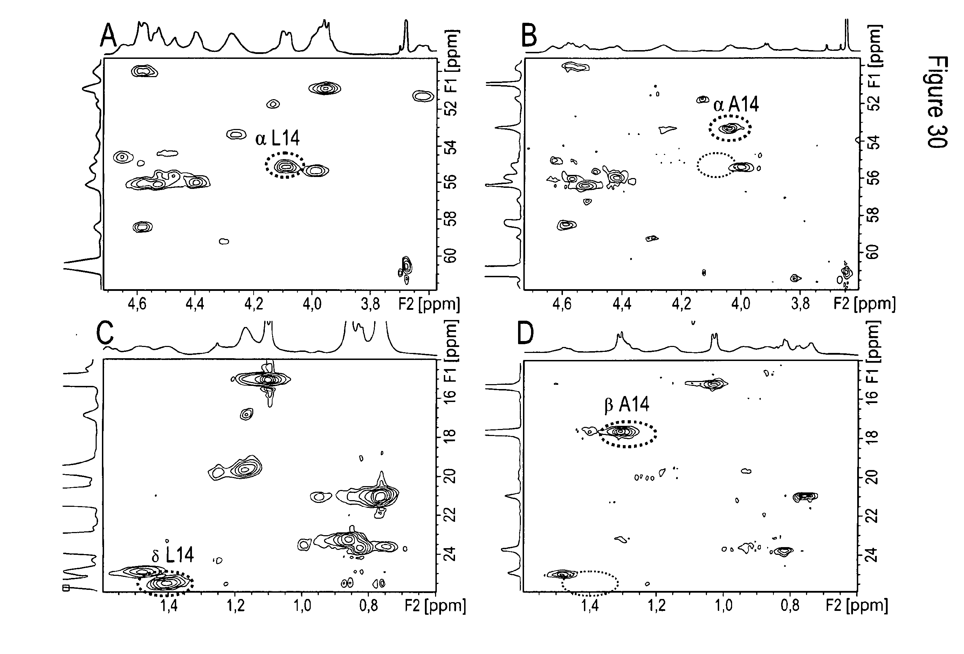

Carbon chemical shifts have been obtained from the HSQC spectrum. TSP has been used as internal standard (0.00 ppm for ¹H, -1.76 ppm for ¹³C). Quarternary carbons have not been assigned (no HMBC spectrum). Comparison of HSQC spectra for LabA2 and its mutant LabA2_L14A (Figure 30) revealed that LabA2_L14A is substituted in the position 14 (i.e. the leucine is replaced by an alanine).

### Modulation of the Ser/Ser/Cys motif

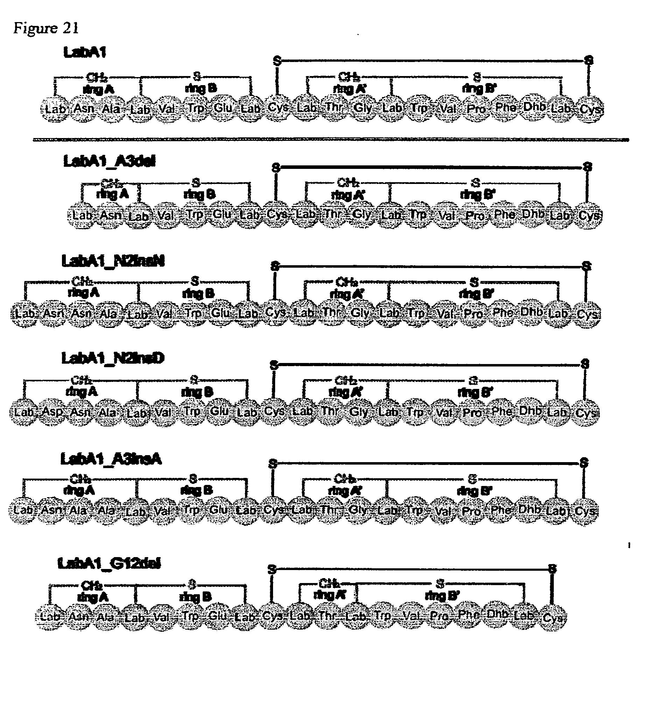

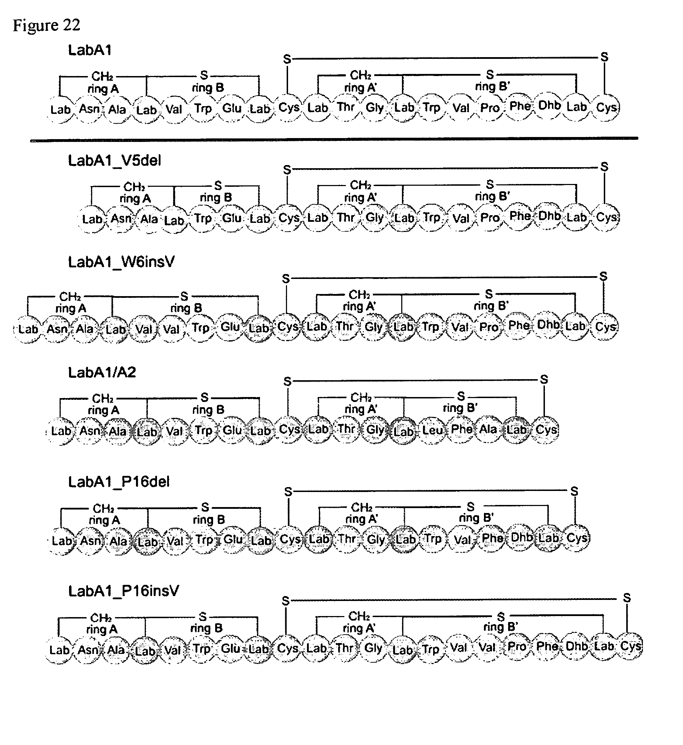

For any rational modifications of Labyrinthopeptins not only the knowledge about the importance of single residues but also the general information about the structure sensitivity to more drastic changes is significant. X-ray structure together with an alignment of structural peptides from gene clusters displaying homology to the *lab* gene cluster, reveals a number of conserved residues, representing a Ser-Xxx-Xxx-Ser-(Xxx)ₙ-Cys motif which appears twice in the peptide. This motif represents an essential ring forming core in the structure. It seems that Labyrinthopeptin-like peptides consist of variable B, B' rings and conserved A, A' rings. In order to determine to which extent rings are in fact amenable to changes, variants with extended or reduced ring sizes were generated. Only mutants with LabA1 as a model system were prepared as presented in Figures 21 and 22. The previously described strategy was chosen in which appropriate synthetic genes were ordered or the pMK_SG2 vector was modified by site-directed mutagenesis. In order to amplify the insert primers Infusion 1 and Infusion 2 were used. To verify if the size of A and A' ring can be increased or decreased, six different LabA1 variants were generated. To increase ring sizes three different amino acids (Asp, Asn, Ala) were chosen for insertion at two different positions for the A ring, and one amino acid (Ala) at single position for the A' ring. In order to decrease ring size Ala and Gly were remove in A and A' rings respectively. None of these peptides were observed in bacterial cultures by means of HPLC-MS analysis.

### Substitution of Ser involved in Lab formation by Thr

It was interesting to investigate if LabKC enzyme can accept Ser→Thr substitutions. We created LabA1 mutants in which Ser residues involved in ring formation (positions 1, 4 and 13) were substituted by Thr. Analysis of fermentation samples of Thr mutants at positions 1 and 13 by LC-MS showed no evidence of lantibiotic production, suggesting a high specificity for either dehydration or ring-forming machinery. For mutant LabA1_S4T only trace amounts were detected of the expected peptide.

### Expression of partial structures of LabA1

In general, peptides are poor drugs mainly because of problems with degradation and bioavailability (*82*). With respect to large molecular weight (2073.7 Da for LabA1 and 1922.7 Da for LabA2) it would be very desirable to reduce Labyrinthopeptins size with retaining bioactivity. Herein we present results concerning size reduction. To express LabA1_west, a stop codon was introduces after Cys8 by means of site-directed mutagenesis. Experiment was performed with pMK_SG2 as a template. To express LabA1_east, a synthetic gene SG7 was ordered. pLab_SG7 allows expression of LabA1_east with a leader peptide for LabA1 where the last, C-terminal amino acid from a leader peptide was substituted with Met. Surprisingly, neither LabA1_east nor LabA1_west peptide was expressed by heterologous host *S. lividans* carrying appropriate vector. To overcome these expression problems a synthetic gene SG20 was ordered. It allowed expression of LabA1_M and LabA2_M with an additional Met residue. In case of LabA1_M the Met residue was inserted between rings B and C. For LabA2_M ring C was removed by substitution of Cys with Met. With these constructs a chemical cleavage should be introduced in order to obtain a truncated variant. No expression of LabA1_M was observed. However, it is possible to express LabA2_M. This product can be subjected to cleavage by chemical reaction with CNBr (*89*), which cleaves C-terminally of Met.

### Ser/Ser/Ser/Cys motif

The biosynthetic machinery of labyrinthopeptins is able to generate the labionin motif with a Ser-Xxx-Xxx-Ser-(Xxx)ₙ-Cys core. In further experiments we were interested to verify if the same machinery is able to form a "double labionin ring" from Ser-Xxx-Xxx-Ser-Xxx-Xxx-Ser-Xxx-Xxx-Xxx-Cys. To answer this question the synthetic gene SG15 was used. pLab_SG15 codes for LabA1_W6insVSA where an additional Val-Ser-Ala tripeptide is introduced into ring B, and for LabA1_P16insS where an additional Ser is introduced into ring B'. Neither expression of LabA1_W6insVSA nor of LabA1_P16insS was observed which means that this motif is not only unable to form an additional labionin ring but also disturbs production of the native like bridged form.

### Additional substitutions

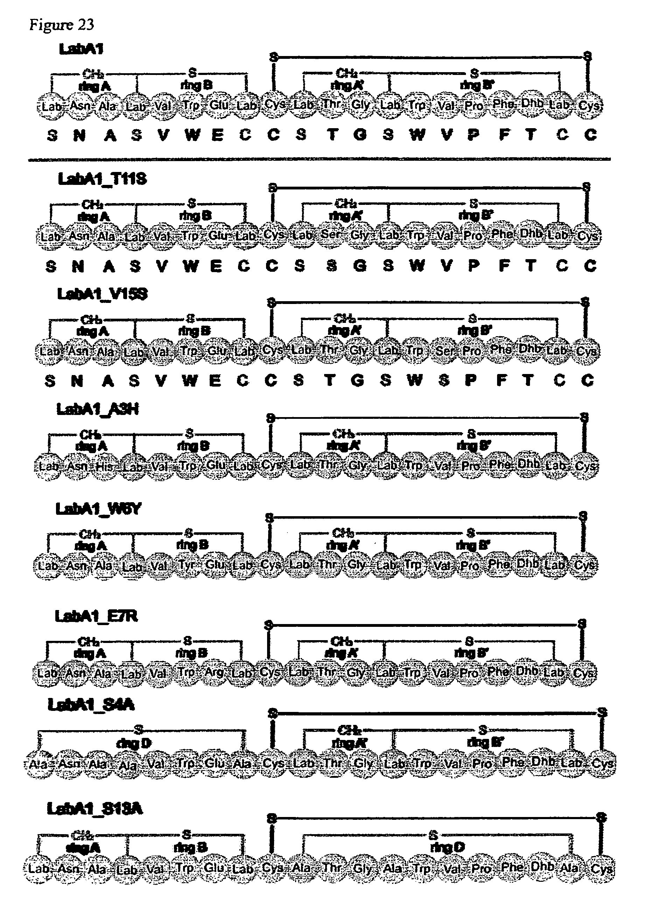

During the posttranslational modifications of Labyrinthopeptins all available Ser and Thr (except these which are located within the A'-rings) undergo dehydration. To address the question of whether the Labyrinthopeptins biosynthetic apparatus can dehydrate also additional Ser, mutants LabA1_T11S and LabA1_V15S were prepared. The main reason for conducting this study was to investigate the possibility of installation of dehydroamino acids in the structure (*83*). LabA1_T11S was expressed by the use of a pLab_SG16 construct. The vector coding for LabA1-V15S was prepared by means of site-directed mutagenesis with pMK_SG2 as a template and primers LabA1-V15S_fw/rv. MS analysis revealed that additional serines in LabA1_T11S as well as in LabA1_V15S were not dehydrated. Analysis of 37 lantibiotic primary structures in the study by Rink *et al.* (*84-85*). showed that serine residues escape dehydration more often than threonines. These findings are in agreement with results presented for Labyrinthopeptins. However, it also should be taken into consideration that LabKC may distinguish between the position of serine/threonine and more mutants would have to be investigated to draw final conclusions. The effects of additional substitutions were investigated as well (see Figure 23).

### Introduction of appropriate mutations to LabA1 to generate vectors

LabA1_A3HÆ7R/W6Y/S4A/S13A were performed by site-directed mutagenesis. As a template pMA_SG2 was used. Ala3 was substituted with His to verify if addition of ionizable amino acids can have a negative influence of Labyrinthopeptin biosynthesis. LabA1_A3H was detected which shows that such substitution is tolerated. However, the information obtained from that single experiment can not be generalized. Negatively charged Glu7 was substituted with positively charged Arg. Production of LabA1_E7R was not detected which suggest that this substitution had disturbed biosynthesis. Mutant with substitution of Trp6→Tyr (LabA1_W6Y) was produced in much higher yield than Trp6→Ala (LabA1_W6A) which indicates that it is possible to increase Labyrinthopeptins diversity by substitutions of tryphophans but not all substitutions are accepted. This finding shows that rational bioengineering of lantibiotics is not a straightforward task because it requires the understanding of the importance of each amino acid in the molecule for the posttranslational processing and the biosynthetic machinery as a whole.

Ser at position 4 and 13 were substituted with Ala to verify if the disruption of labionin formation will result in formation of lanthionine ring between both Ser1 and Cys8, and Ser10 and Cys19. Expression of LabA1_S4A and LabA1_S13A was not observed.

### Studies on ring C mutations

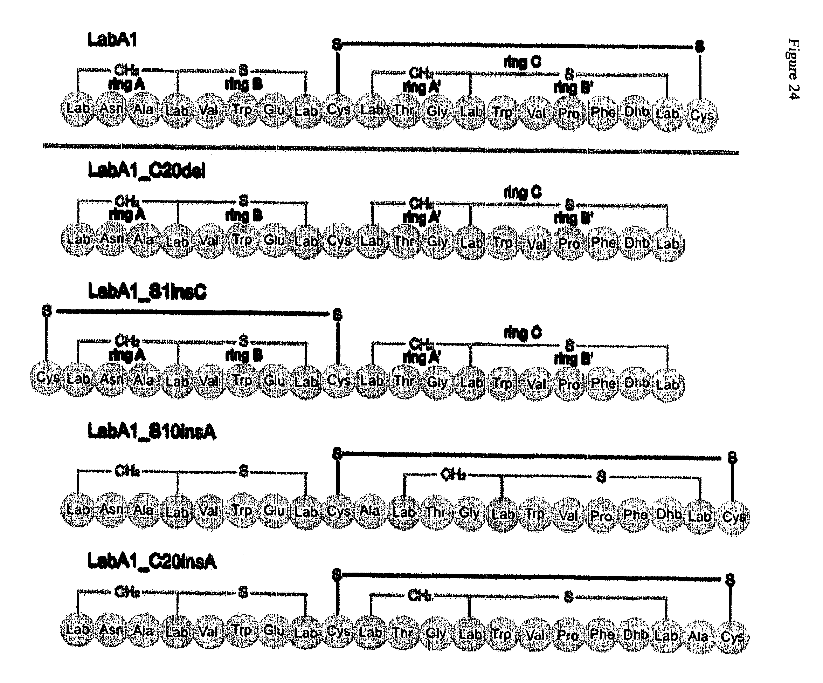

All the mutants used to confirm the flexibility of the C-ring were generated by site-directed mutagenesis with pMK_SG2 as a template. Expression of all peptides presented in Figure 24 was observed. A possibility to detect LabA1_C20del shows that the disulfide bridge is not necessary to obtain labyrinthopeptin which is stable in the proteolytic environment of *S. lividans.* LabA1_SlinsC presents a possibility to create a disulfide bridge also in the west part of a molecule. In this case occurrence of disulfide bond was deduced only on the basis of the observed molecular mass (-2 Da corresponding to two protons). Mutants LabA1_S10insA and LabA1_C20insA show that the size of ring C can be increased although the production levels of these variants were significantly lower.

### Generation of Labyrinthopeptin hybrids

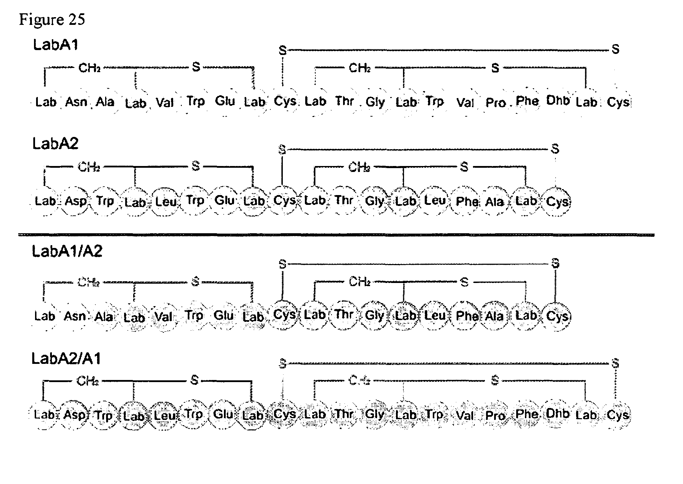

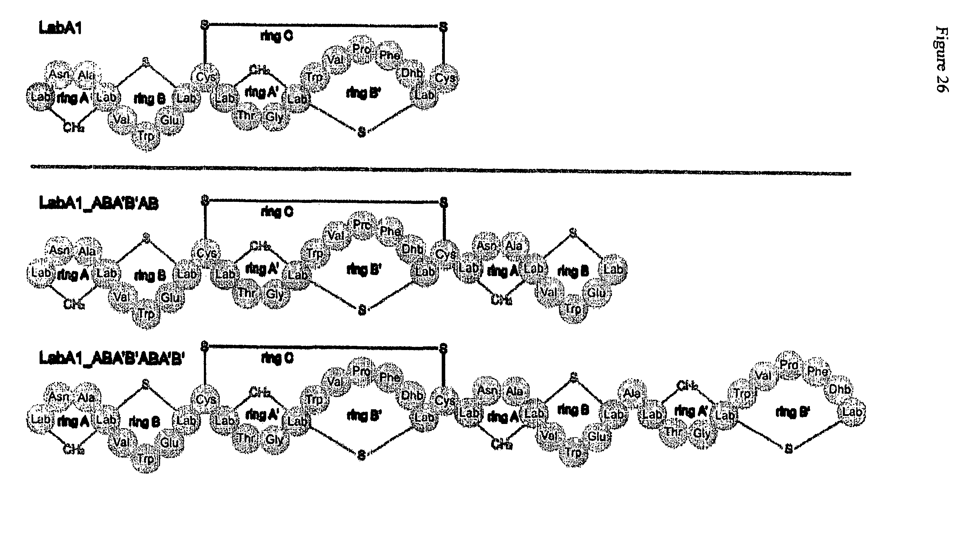

The majority of studies performed in this work concern LabA1. In this situation it was essential to evaluate if the knowledge collected for LabA1 can be also used for engineering of LabA2. In order to provide an answer to this question gene SG11 was synthesized. pLab_SG11 codes for LabA1/A2 (Figure 25) and LabA2/A1 (Figure 25) hybrid molecules which are expressed with unmodified LabA1 and LabA2 leader peptides, respectively. Both peptides were detected in cultures of *S. lividanslpLab_SG11* which suggested that observations made for LabA1 could also be applied to LabA2. Another interesting aspect investigated in this study was to evaluate if it is possible to extended Labyrinthopeptins repeatedly. This could be possible because of the polymeric nature of the substrate. Also distributive mode of action of the biosynthesis enzyme, which was presented for LanM (*69*), supports this possibility. Synthetic genes SG9 and SG10 were designed to generate peptides shown in Figure 26. Neither LabA1_ABA'B' ABA'B' nor LabA1_ABA'B' ABA'B' could be detected. To test if the Labyrinthopeptin biosynthetic machinery can be used for production of silent lantibiotics (those which are difficult to isolate from natural sources, for which structure and production conditions are not known) belonging to class III a synthetic gene SG8 was ordered. SG8 codes for a hypothetical lantibiotics RamS2. It was found during *in silico* analysis of *Streptomyces scabies* genomic DNA. The whole gene cluster responsible for biosynthesis of RamS2 show homology to the *lab* gene cluster. Analysis of a supernatant and also a cells extract from *S. lividans*/ pLab_SG8 didn't reveal a production of a peptide which could correspond to RamS2.

In summary, alanine-scanning studies showed that most of the amino acids not involved in rings formation can be easily substituted with Ala. Only exceptions are Trp residues for which Ala substitutions inhibited production of Labyrinthopeptins or resulted in trace amounts. However, production was detected when Trp residue was substituted with Tyr, which indicates that conservation of these positions might not be absolute. Substitution of Gln to Arg suppresses production. Another investigated aspect concerned size requirements for rings formation. It was demonstrated that any change in the size of rings A and A' is not accepted. Rings B and B' however are more elastic, 5,6,7,8-membered rings were created. It was also shown that disulphide bond ring C is not required or that it can be moved to western part of the molecule. Attempt to form double labionin (containing two quaternary carbon atoms) failed as well as any extension of the whole molecule.

### Summary of expected and observed molecular masses of bioengineered Labyrinthopeptins (ND - not detected):

| Mutant | Molecular formula | Peptide masses (Da) | | |
|---|---|---|---|---|
| | | Expected molecular mass | Expected [M+2H]²⁺ | Observed [M+2H]²⁺ |
| Alanine scanning mutagenesis | | | | |
| LabA1_N2A | C₉₀H₁₁₈N₂₂O₂₄S₄ | 2030.76 | 1016.38 | 1016.39 |
| LabA1_V5A | C₉₀H₁₁₅N₂₃O₂₅S₄ | 2045.73 | 1023.86 | 1023.88 |
| LabA1_W6A | C₈₄H₁₁₄N₂₂O₂₅S₄ | 1958.72 | 980.36 | 980.69 |
| LabA1_E7A | C₉₀H₁₁₇N₂₃O₂₃S₄ | 2015.76 | 1008.88 | 1008.89 |
| LabA1_T11A | C₉₁H₁₁₇N₂₃O₂₄S₄ | 2043.75 | 1022.87 | 1022.89 |
| LabA1_W14A | C₈₄H₁₁₄N₂₂O₂₅S₄ | 1958.72 | 980.36 | ND |
| LabA1_V15A | C₉₀H₁₁₅N₂₃O₂₅S₄ | 2045.73 | 1023.86 | 1023.88 |
| LabA1_P16A | C₉₀H₁₁₇N₂₃O₂₅S₄ | 2047.75 | 1024.87 | 1024.63 |
| LabA1_F17A | C₈₆H₁₁₅N₂₃O₂₅S₄ | 1997.73 | 999.86 | 999.88 |
| LabA2_D2A | C₈₄H₁₁₀N₂₀O₂₂S₄ | 1878.70 | 940.35 | 940.36 |
| LabA2_W3A | C₇₇H₁₀₅N₁₉O₂₄S₄ | 1807.65 | 904.82 | 904.84 |
| LabA2_L5A | C₈₂H₁₀₄N₂₀O₂₄S₄ | 1880.64 | 941.32 | 1881.91 [M+H]⁺ |
| LabA2_W6A | C₇₇H₁₀₅N₁₉O₂₄S₄ | 1807.65 | 904.82 | ND |
| LabA2_E7A | C₈₃H₁₀₈N₂₀O₂₂S₄ | 1864.68 | 933.34 | 933.36 |
| LabA2_T11A | C₈₄H₁₀₈N₂₀O₂₃S₄ | 1892.68 | 947.34 | 1892.36 [M+H]⁺ |
| LabA2_G12A | C₈₆H₁₁₂N₂₀O₂₄S₄ | 1936.70 | 969.35 | 969.36 |
| LabA2_L14A | C₈₂H₁₀₄N₂₀O₂₄S₄ | 1880.64 | 941.32 | 941.34 |
| LabA2_F15A | C₇₉H₁₀₆N₂₀O₂₄S₄ | 1846.66 | 924.33 | 924.35 |

| Ser/Ser/Cys motif, Ring A and A' | | | | |
|---|---|---|---|---|
| LabA1_N2insD | C₉₆H₁₂₄N₂₄O₂₈S₄ | 2188.79 | 1095.39 | ND |
| LabA1_N2insN | C₉₆H₁₂₅N₂₅O₂₇S₄ | 2187.81 | 1094.90 | ND |
| LabA1_A3insA | C₉₅H₁₂₄N₂₄O₂₆S₄ | 2144.80 | 1073.40 | ND |
| LabA1_A3del | C₈₉H₁₁₄N₂₂O₂₄S₄ | 2002.73 | 1002.36 | ND |
| LabA1_G12insA | C₉₅H₁₂₄O₂₆N₂₄S₄ | 2144.80 | 1073.40 | ND |
| LabA1_G12del | C₉₀H₁₁₆N₂₂O₂₄S₄ | 2016.74 | 1009.37 | ND |

| Ser/Ser/Cys motif, Ring B and B' | | | | |
|---|---|---|---|---|
| LabA1_V5del | C₈₇H₁₁₀N₂₂O₂₄S₄ | 1974.69 | 988.69 | 988.56 |
| LabA1_W6insV | C₉₇H₁₂₈N₂₄O₂₆5₄ | 2172.83 | 1087.41 | 1087.42 |
| LabA1_P16del | C₈₇H₁₁₂N₂₂O₂₄S₄ | 1976.71 | 989.35 | 989.36 |
| LabA1_P16insV | C₉₇H₁₂₈O₂₆N₂₄S₄ | 2172.83 | 1087.41 | 1087.50 |

| Substitution of serines involved in ring formation by threonines | | | | |
|---|---|---|---|---|
| LabA1_S4T | C₉₃H₁₂₁N₂₃O₂₅S₄ | 2087.78 | 1044.89 | 1045.11 |
| M-LabA1_S4T | C₉₈H₁₃₀N₂₄O₂₆S₅ | 2218.98 | 1110.49 | 1110.58 |
| AM-LabA1_S4T | C₁₀₁H₁₃₅N₂₅O₂₇S₅ | 2290.06 | 1146.03 | 1146.07 |
| LabA1_S13T | C₉₃H₁₂₁N₂₃O₂₅S₄ | 2087.78 | 1044.89 | ND |
| LabA1_S1T | C₉₃H₁₂₁N₂₃O₂₅S₄ | 2087.78 | 1044.89 | ND |

| Express east or west part of labyrinthopeptin | | | | |
|---|---|---|---|---|
| LabA1_west (LabA1_C9tga) | C₃₇H₅₀N₁₀O₁₂S | 858.33 | 859.33 [M+H⁺] | ND |
| LabA1_east (SG7) | C₅₅H₇₁N₁₃O₁₄S₃ | 1233.44 | - | ND |
| LabA1_M (SG20) | C₉₇H₁₂₈N₂₄O₂₆S₅ | 2204.96 | 1103.48 | ND |
| NR-LabA2_M (SG20) | C₉₄H₁₂₉N₂₅O₂₆S₃ | 2120.02 | 1061.01 | 1060.92 |
| R-LabA2_M (SG20) | C₉₀H₁₂₃N₂₃O₂₄S₃ | 2005.92 | 1003.96 | 1003.97 |

| Mutant | Molecular formula | Peptide masses (Da) | | |
|---|---|---|---|---|
| | | Expected molecular mass | Expected [M+2H]²⁺ | Observed [M+2H]²⁺ |
| Ser/Ser/Ser/Cys motif | | | | |
| LabA1_P16insS (Ser) | C₉₅H₁₂₄N₂₄O₂₇S₄ | 2160.84 | 1081.42 | ND |
| LabA1_P16insS (Dha) | C₉₅H₁₂₂N₂₄O₂₆S₄ | 2142.84 | 1072.42 | ND |
| LabA1_ W6insVSA (Ser) | C₁₀₃H₁₃₈N₂₆O₂₉S₄ | 2331.05 | 1166.52 | ND |
| LabA1_ W6insVSA (Dha) | C₁₀₃H₁₃₆N₂₆O₂₈S₄ | 2313.05 | 1157.52 | ND |

| Additional substitutions | | | | |
|---|---|---|---|---|
| LabA1_V15S | C₉₀H₁₁₅N₂₃O₂₆S₄ | 2061.73 | 1031.86 | 1031.93 |
| LabA1_T11S (Ser) | C₉₁H₁₁₇N₂₃O₂₅S₄ | 2059.74 | 1030.87 | 1030.67 |
| LabA1_T11S (Dha) | C₉₁H₁₁₇N₂₃O₂₅S₄ | 2041.74 | 1021.87 | ND |
| LabA1_W6Y | C₉₀H₁₁₈N₂₂O₂₆S₄ | 2050.75 | 1026.37 | 1026.38 |
| LabA1_A3H | C₉₅H₁₂₁O₂₅N₂₅S₄ | 2139.78 | 1070.89 | 1070.91 |
| LabA1_E7R | C₉₃H₁₂₄O₂₃N₂₆S₄ | 2100.82 | 1051.41 | ND |
| LabA1_S4A | C₉₂H₁₂₃N₂₃O₂₆S₄ | 2093.79 | 1047.89 | ND |
| LabA1_S13A | C₉₂H₁₂₃N₂₃S₂₆S₄ | 2093.79 | 1047.89 | ND |

| Ring C | | | | |
|---|---|---|---|---|
| LabA1_C20insA | C₉₅H₁₂₄N₂₄O₂₆S₄ | 2144.80 | 1073.40 | 1073.41 |
| LabA1_S10insA | C₉₅H₁₂₄N₂₄O₂₆S₄ | 2144.80 | 1073.40 | 1073.52 |
| LabAl-C20del | C₈₉H₁₁₆O₂₄N₂₂S₃ | 1972.77 | 987.38 | 987.49 |
| LabA1_SlinsC | C₉₂H₁₁₉N₂₃O₂₅S₄ | 2073.76 | 1037.88 | 1037.98 |

| Spacer | | | | |
|---|---|---|---|---|
| LabA1_C9insV | C₉₇H₁₂₈O₂₆N₂₄S₄ | 2172.83 | 1087.41 | ND |
| LabA1_C9insVN | C₁₀₁H₁₃₅O₂₈N₂₆S₄ | 2286.93 | 1144.46 | ND |

| Hybrids | | | | |
|---|---|---|---|---|
| LabA1_ABA'B' AB (SG9) | C₁₂₉H₁₆₇O₃₆N₃₃S₅ | 2914.09 | 1458.04 | ND |
| D-LabA1/A2 (SG11) | C₈₀H₁₀₉O₂₆N₂₁S₄ | 1907.74 | 954.87 | 954.99 |
| AD-LabA1/A2 (SG11) | C₈₃H₁₁₄O₂₇N₂₂S₄ | 1978.82 | 990.41 | 990.47 |
| R-LabA2/A1 (SG11) | C₁₀₇H₁₃₇O₂₇N₂₇S₄ | 2359.99 | 1180.99 | 1180.90 |
| NR-LabA2/A1 (SG11) | C₁₁₁H₁₄₃O₂₉N₂₉S₄ | 2474.09 | 1238.04 | 1237.85 |
| ENR-LabA2/A1 (SG11) | C₁₁₆H₁₅₀O₃₂N₃₀S₄ | 2603.20 | 1302.60 | 1302.30 |

Evaluation of anti-pain activity of labyrinthopeptin A2 and its structural analogs LabA2_L14A and LabA2_F15A in an *in vitro* assay (IC50 determination of LabA2, LabA2_L14A and LabA2_F15A on hP2X4_HEK-FITR-cell line)

The use of labyrinthopeptin A2 and its derivatives, in particular LabA2_L 14A and LabA2_F15A, as analgesics was investigated for neuropathic pain based on their inhibitory effects on P2X4 receptors (Nagata K *et al*., 2009).

Recombinant human P2X4 receptor was expressed in HEK cells (hP2X4_HEK-FITR). hP2X4 expression was induced with doxycyclin 1 µg/ml for 24h to 48h. The patch clamp experiment has been performed under standard conditions using an axopatch-200B amplifier and pClamp v10 acquisition software (Axon Instruments Inc, Foster City, CA, USA). Cells were continuously superfused with an external solution consisting of (in mM) 130 CsCl, 4 NaCl, 1 MgCl₂, 1 CaCl₂, 10 EGTA (free Ca²⁺ = 6.7 nM) and 10 HEPES (pH 7.4 with CsOH). This was replaced with a low divalent external solution consisting of (in mM) 145 NaCl, 4 KCl, 2 CaCl₂, 1 MgCl₂ and 10 HEPES (pH 7.4 with NaOH). 250 ms application of 10 µM ATP (Sigma) every 10 sec was performed. When current stabilization was achieved increasing concentrations of labyrinthopeptins (LabA2, LabA2_L14A and LabA2_F15A) was perfused (from 0.3 to 90 µM). Currents were measured at a holding potential of -60 mV.

Results were calculated using Origin7.5 software (Figure 27). It was found that LabA2 and LabA2_L14A inhibit human P2X4 receptor function with IC50 values of 6.2 µM and 30 µM respectively. IC50 value for LabA2_F 15A could not be determined in used conditions. This result suggests that LabA2_F 15A can not block P2X4 receptors or it can block them but with high IC50 values which discriminate it for a use as an anti-pain drug. LabA2 and LabA2_L14A were shown to be useful for the treatment of pain, in particular neuropathic pain.

References
1. Hanahan, D. (1983) Studies on transformation of Escherichia coli with plasmids., J Mol Biol 166, 557-580.
2. Datsenko, K. A., Wanner, B.L. (2000) One-step inactivation of chromosomal genes in Escherichia coli K-12 using PCR products., Proc Natl Acad Sci USA 97, 6640-6645.
3. Gewain, K. M., Occi, J.L., Foor, F., MacNeil, D.J. (1992) Vectors for generating nested deletions and facilitating subcloning G+C-rich DNA between Escherichia coli and Streptomyces sp., Gene 119, 149-150.
4. Kirchner, O., Tauch, A. (2003) Tools for genetic engineering in the amino acid-producing bacterium Corynebacterium glutamicum., J Biotechnol 104, 287-299.
5. Gust, B., Challis, G.L., Fowler, K., Kieser, T., Chater, K.F. (2003) PCR-targeted Streptomyces gene replacement identifies a protein domain needed for biosynthesis of the sesquiterpene soil odor geosmin., Proc Natl Acad Sci USA 100, 1541-1546.
6. Paget, M.S., Chamberlin, L., Atrih, A., Foster, S.J., Buttner, M.J. (1999) Evidence that the extracytoplasmic function sigma factor sigmaE is required for normal cell wall structure in Streptomyces coelicolor A3(2). J Bacteriol 181, 204-211.
7. Bierman, M., Logan, R., O'Brien, K., Seno, E.T., Rao, R.N., Schoner, B.E. (1992) Plasmid cloning vectors for the conjugal transfer of DNA from Escherichia coli to Streptomyces spp, Gene 116, 43-49.
8. Wehmeier, U.F. (1995) New multifunctional Escherichia coli-Streptomyces shuttle vectors allowing blue-white screening on XGal plates., Gene 165, 149-150.
9. Feitelson, J.S., Englenwood, N.J. (1991) Multifunctional plasmid vectors from *Actinomadura* and *Escherichia coli.,* US Patent no. 5,002,891.
10. Kieser, T., Bibb, M.J., Buttner, M.J., Chater, K.F., Hopwood, D.A. (2000) Practical Streptomyces genetics (2nd ed.)., John Innes Foundation, Norwich, UK.
11. Madoń, J., Hütter, R. (1991) Transformation system for Amycolatopsis (Nocardia) mediterranei: direct transformation of mycelium with plasmid DNA., J Bacteriol 173, 6325-6331.
12. Wang, W., Malcolm, B.A. (1999) Two-stage PCR protocol allowing introduction of multiple mutations, deletions and insertions using QuikChange site-directed mutagenesis., Biotechniques 26, 680-682.
13. Rashtchian, A. (1995) Novel methods for cloning and engineering genes using the polymerase chain reaction., Curr Opin Biotechnol 6, 30-36.
14. Aslanidis, C., de Jong, P.J. (1990) Ligation-independent cloning of PCR products (LIC-PCR). Nucleic Acids Res 18, 6069-6074*.*
15. Schägger, H., von Jagow, G. (1987) Tricine-sodium dodecyl sulfate-polyacrylamide gel electrophoresis for the separation of proteins in the range from 1 to 100 kDa., Anal Biochem 166, 368-379*.*
16. Fukase, K., Kitazawa, M., Sano, A., Shimbo, K., Horimoto, S., Fujita, H., Kubo, A., Wakamiya, T., Shiba, T. (1992) ChemInform Abstract: Synthetic Study on Peptide Antibiotic Nisin. Part 5. Total Synthesis of Nisin, ChemInform 23*.*
17. Dairi, T., Hamano, Y., Furumai, Tamotsu, Oki, T. (1999) Development of a self-cloning system for Actinomadura verrucosospora and identification of polyketide synthase genes essential for production of the angucyclic antibiotic pradimicin., Appl Environ Microbiol 65, 2703-2709.
18. Combes, P., Till, R., Bee, S., Smith, M.C.M. (2002) The Streptomyces genome contains multiple pseudo-attB sites for the (phi)C31-encoded site-specific recombination system., J Bacteriol 184, 5746-5752.
19. Thompson, C. J., Ward, J.M., Hopwood, D.A. (1982) Cloning of antibiotic resistance and nutritional genes in streptomycetes., J Bacteriol 151, 668-677.
20. Okanishi, M., Katagiri, K., Furumai, T., Takeda, K., Kawaguchi, K., Saitoh, M., Nabeshima, S. (1983) Basic techniques for DNA cloning and conditions required for streptomycetes as a host., JAntibiot (Tokyo) 36, 99-108.
21. Rau, A., Hogg, T., Marquardt, R., Hilgenfeld, R. (2001) A new lysozyme fold. Crystal structure of the muramidase from Streptomyces coelicolor at 1.65 A resolution., J Biol Chem 2 76, 31994-31999.
22. Bibb, M.J., Ward, J.M., Hopwood, D.A. (1978) Transformation of plasmid DNA into Streptomyces at high frequency., Nature 274, 398-400.
23. Baltz, R.H., Hosted, T.J. (1996) Molecular genetic methods for improving secondary-metabolite production in actinomycetes., Trends Biotechnol 14, 245-250.
24. Trieu-Cuot, P., Carlier, C., Martin, P., Courvalin, P. (1987) Plasmid transfer by conjugation from Escherichia coli to Gram-positive bacteria., FEMS Microbiol Lett 48, 289-294.
25. Mazodier, P., Petter, R., Thompson, C. (1989) Intergeneric conjugation between Escherichia coli and Streptomyces species., J Bacteriol 171, 3583-3585.
26. Smokvina, T., Mazodier, P., Boccard, F., Thompson, C.J., Guérineau, M. (1990) Construction of a series of pSAM2-based integrative vectors for use in actinomycetes., Gene 94, 53-59.
27. Stegmann, E., Pelzer, S., Wilken, K., Wohlleben, W. (2001) Development of three different gene cloning systems for genetic investigation of the new species Amycolatopsis japonicum MG417-CF 17, the ethylenediaminedisuccinic acid producer., J Biotechnol 92, 195-204.
28. Heinzelmann, E., Berger, S., Puk, O., Reichenstein, B., Wohlleben, W., Schwartz, D. (2003) A glutamate mutase is involved in the biosynthesis of the lipopeptide antibiotic friulimicin in Actinoplanes friuliensis., Antimicrob Agents Chemother 47, 447-457.
29. Stinchi, S., Azimonti, S., Donadio, S., Sosio, M. (2003) A gene transfer system for the glycopeptide producer Nonomuraea sp. ATCC39727., FEMS Microbiol Lett 225, 53-57.
30. Voeykova, T., Emelyanova, L., Tabakov, V., Mkrtumyan, N. (1998) Transfer of plasmid pTO1 from Escherichia coli to various representatives of the order Actinomycetales by intergeneric conjugation, FEMS Microbiol Lett 162, 47-52.
31. Wink J., K., R.M., Seibert, G., Stackebrandt, E. (2003) Actinomadura namibiensis sp. nov., Int J Syst Evol Microbiol 53, 721-724.
32. Vrijbloed, J.W., Madon, J., Dijkhuizen, L.. (1995) Transformation of the methylotrophic actinomycete Amycolatopis methanolica with plasmid DNA: stimulatory effect of a pMEA300-encoded gene., Plasmid 34, 96-104.
33. Kumar, C.V., Coque, J.J., Martin, J.F. (1994) Efficient transformation of the cephamycin C producer Nocardia lactamdurans and development of shuttle and promoter-probe cloning vectors., Appl Environ Microbiol 60, 4086-4093.
34. Yeung, M. K., Kozelsky, C. S. (1994) Transformation of Actinomyces spp. by a gram-negative broad-host-range plasmid., J Bacteriol 176, 4173-4176.
35. Pigac, J., Schrempf, H. (1995) A simple and rapid method of transformation of Streptomyces rimosus R6 and other Streptomycetes by electroporation., Appl Environ Microbiol 61, 352-356.
36. Mazy-Servais, C., Baczkowski, D., Dusart, J. (1997) Electroporation of intact cells of Streptomyces parvulus and Streptomyces vinaceus., FEMS Microbiol Lett 151, 135-138.
37. MacNeil, D.J. (1987) Introduction of plasmid DNA into Streptomyces lividans by electroporation., FEMS Microbiol Lett 42, 239-244*.*
38. Tyurin, M., Starodubtseva, L., Kudryavtseva, H., Voeykova, T., Livshits, V. (1995) Electrotransformation of germinating spores of Streptomyces spp., Biotechnol Tech 9, 737-740.
39. Flinspach, K., Westrich, L., Kaysser, L., Siebenberg, S., Juan Pablo Gomez-Escribano, J.P., Bibb, M., Gust, B., Heide, L. (2010) Heterologous expression of the biosynthetic gene clusters of coumermycin A1, clorobiocin and caprazamycins in genetically modified Streptomyces coelicolor strains., Biopolymers 93, 823-832.
40. Widdick, D.A., Dodd, H.M., Barraille, P., White, J., Stein, T.H., Chater, K.F., Gasson, M.J., Bibb, M.J. (2003) Cloning and engineering of the cinnamycin biosynthetic gene cluster from Streptomyces cinnamoneus cinnamoneus DSM 40005., Proc Natl Acad Sci U S A 100, 4316-4321*.*
41. Zhang, H., Wang, Y., Pfeifer, B.A. (2008) Bacterial hosts for natural product production., Mol Pharm 5, 212-225.
42. Van Lanen, S.G., Oh, T., Wen Liu, W., Wendt-Pienkowski, E., Shen, B. (2007) Characterization of the maduropeptin biosynthetic gene cluster from Actinomadura madurae ATCC 39144 supporting a unifying paradigm for enediyne biosynthesis., J Am Chem Soc 129, 13082-13094.
43. Corvey, C., Stein, T., Duesterhus, S., Karas, M., Entian, K.D. (2003) Activation of subtilin precursor by Bacillus subtilis extracellular serine proteases subtilisin (AprE), WprA, and Vpr., Biochem Biophys Res Commun 304, 48-54.
44. Schmiederer, T. (2008) Biosynthese der Labyrinthopeptine A1, A2 und A3, einer neuen Klasse von Lantibiotika aus Actinomadura namibiensis. Dissertation, TU Berlin.
45. Kuhstoss, S., Rao, R.N. (1991) Analysis of the integration function of the streptomycete bacteriophage ΦC31., J Mol Biol 222, 897-908.
46. Combes, P., Till, R., Sally Bee, S., Smith, M.C.M. (2002) The Streptomyces genome contains multiple Pseudo-attB Sites for the phi C31-encoded site-specific recombination system, Journal of Bacteriology 184, 5746-5752.
47. Wilkinson, C.J., Hughes-Thomas, Z.A., Martin, C.J., Böhm, I., Mironenko, T., Deacon, M., Wheatcroft, M., Wirtz, G., Staunton, J., Leadlay, P.F. (2002) Increasing the efficiency of heterologous promoters in actinomycetes., J Mol Microbiol Biotechnol. 4, 417-426.
48. Keijser, B.J., van Wezel, G.P., Canters, G.W., Kieser, T., Vijgenboom, E. (2000) The ram-dependence of Streptomyces lividans differentiation is bypassed by copper., J Mol Microbiol Biotechnol 2, 565-574.
49. Boakes, S., Cortés, J., Appleyard, A.N., Rudd, B.A.M., Dawson, M. J. (2009) Organization of the genes encoding the biosynthesis of actagardine and engineering of a variant generation system., Mol Microbiol 72, 1126-1136.
50. Dodd, H.M., Horn, N., Hao, Z., Gasson, M.J. (1992) A lactococcal expression system for engineered nisins., Appl Environ Microbiol 58, 3683-3693.
51. Dodd, H.M., Horn, N., Giffard, C.J., Gasson, M.J. (1996) A gene replacement strategy for engineering nisin., Microbiology 142, 47-55.
52. Chen, P., Novak, J., Kirk, M., Barnes, S., Qi, F., Caufield, P.W. (1998) Structure-activity study of the lantibiotic mutacin II from Streptococcus mutans T8 by a gene replacement strategy., Appl Environ Microbiol 64, 2335-2340.
53. Ottenwalder, B., Kupke, T., Brecht, S., Gnau, V., Metzger, J., Jung, G., Gotz, F. (1995) Isolation and characterization of genetically engineered gallidermin and epidermin analogs., Appl Environ Microbiol 61, 3894-3903.
54. Bierbaum, G., Reis, M., Szekat, C., Sahl, H.G. (1994) Construction of an expression system for engineering of the lantibiotic Pep5., Appl Environ Microbiol 60, 4332-4338.
55. Kuipers, O.P., Bierbaum, G., Ottenwälder, B., Dodd, H.M., Horn, N., Metzger, J., Kupke, T., Gnau, V., Bongers, R., van den Bogaard, P., Kosters, H., Rollema, H.S., de Vos, W.M., Siezen, R.J., Jung, G., Götz, F., Sahl, H.G., Gasson, M.J. (1996) Protein engineering of lantibiotics., Antonie Van Leeuwenhoek 69, 161-169.
56. Lin, Y., Teng, K., Huan, L., Zhong, J. (2011) Dissection of the bridging pattern of bovicin HJ50, a lantibiotic containing a characteristic disulfide bridge., Microbiol Res 166, 146-154.
57. Altena, K., Guder, A., Cramer, C., Bierbaum, G. (2000) Biosynthesis of the lantibiotic mersacidin: organization of a type B lantibiotic gene cluster., Appl Environ Microbiol 66, 2565-2571.
58. Kuipers, O.P., Beerthuyzen, M.M., Siezen, R.J., De Vos, W.M. (1993) Characterization of the nisin gene cluster nisABTCIPR of Lactococcus lactis., Eur JBiochem 216, 281-291.
59. Pag, U., Heidrich, C., Bierbaum, G., Sahl, H.G. (1999) Molecular analysis of expression of the lantibiotic pep5 immunity phenotype., Appl Environ Microbiol 65, 591-598.
60. Kaiser, R., Metzka, L. (1999) Enhancement of cyanogen bromide cleavage yields for methionyl-serine and methionyl-threonine peptide bonds., Anal Biochem 266, 1-8.
61. Xie, L., Miller, L.M., Chatterjee, C., Averin, O., Kelleher, N.L., van der Donk, W.A. (2004) Lacticin 481: in vitro reconstitution of lantibiotic synthetase activity., Science 303, 679-681.
62. Levengood, M.R., Knerr, P.J., Oman, T.J., van der Donk, W.A. (2009) In vitro mutasynthesis of lantibiotic analogues containing nonproteinogenic amino acids., J Am Chem Soc 131, 12024-12025.
63. Fukase, K., Kitazawa, M., Sano, A., Shimbo, K., Horimoto, S., Fujita, H., Kubo, A., Wakamiya, T., Shiba, T. (1988) Total synthesis of peptide antibiotic nisin., Tetrahedron Lett 29, 795-798.
64. Ross, A.C., Liu, H., Pattabiraman, V.R., Vederas, J.C. (2009) Synthesis of the lantibiotic lactocin S using peptide cyclizations on solid phase., J Am Chem Soc 132, 462-463.
65. van Kraaij, C., de Vos, W.M., Siezena, R.J., Kuipers, O.P. (1999) Lantibiotics: biosynthesis, mode of action and applications., Nat Prod Rep 16, 575-587.
66. Cotter, P.D., Deegan, L.H., Lawton, E.M., Draper, L.A., O'Connor, P.M., Hill, C., Ross, R.P. (2006) Complete alanine scanning of the two-component lantibiotic lacticin 3147: generating a blueprint for rational drug design., Mol Microbiol 62, 735-747.
67. Appleyard, A.N., Choi, S., Read, D.M., Lightfoot, A., Boakes, S., Hoffmann, A., Chopra, I., Bierbaum, G., Rudd, B.A.M., Dawson, M.J., Cortes, J. (2009) Dissecting structural and functional diversity of the lantibiotic mersacidin., Chem Biol 16, 490-498.
68. Piper, C., Cotter, P.D., Ross, R.P., Hill, C. (2009) Discovery of medically significant lantibiotics. , Curr Drug Discov Technol 6, 1-18.
69. Lee, M.V., Ihnken, L.A.F., You, Y.O., McClerren, A.L., van der Donk, W.A., Kelleher, N.L. (2009) Distributive and directional behavior of lantibiotic synthetases revealed by high-resolution tandem mass spectrometry., J Am Chem Soc 131, 12258-12264.
70. Patton, G.C., Paul, M., Cooper, L.E., Chatterjee, C., van der Donk, W.A. (2008) The importance of the leader sequence for directing lanthionine formation in lacticin 481., Biochemistry 47, 7342-7351.
71. Cheng, F., Takala, T.M., Saris, P.E.J. (2007) Nisin biosynthesis in vitro., J Mol Microbiol Biotechnol 13, 248-254.
72. McClerren, A.L., Cooper, L.E., Quan, C., Thomas, P.M., Kelleher, N.L., van der Donk, W.A. (2006) Discovery and in vitro biosynthesis of haloduracin, a two-component lantibiotic., Proc Natl Acad Sci USA. 103, 17243-17248.
73. Müller, W.M., Schmiederer, T., Ensle, P., Süssmuth, R.D. (2010) In vitro biosynthesis of the prepeptide of type-III lantibiotic labyrinthopeptin A2 including formation of a C-C bond as a post-translational modification. , Angew Chem Int Ed Engl 49, 2436-2440.
74. Goto, Y., Li, B., Claesen, J., Shi, Y., Bibb, M.J., van der Donk, W.A. (2010) Discovery of unique lanthionine synthetases reveals new mechanistic and evolutionary insights PLoS Biology 8, e1000339.
75. Li, B., Sher, D., Kelly, L., Shi, Y., Huang, K., Knerr, P.J., Joewono, I., Rusch, D., Chisholm, S.W., van der Donk, W.A. (2010) Catalytic promiscuity in the biosynthesis of cyclic peptide secondary metabolites in planktonic marine cyanobacteria., Proc Natl Acad Sci U S A 107, 10430-10435.
76. Osapay, G., Prokai, L., Kim, H.-S., Medzihradszky, K.F., Coy, D.H., Liapakis, G., Reisine, T., Melacini, G., Zhu, Q., Wang, S.H.H., Mattern, R.-H., Goodman, M. (1997) Lanthionine-somatostatin analogs: synthesis, characterization, biological activity, and enzymatic stability studies., J Med Chem 40, 2241-2251.
77. Rew, Y., Malkmus, S., Svensson, C., Yaksh, T.L., Chung, N.N., Schiller, P.W., Cassel, J.A., DeHaven, R.N., Taulane, J.P., Goodman, M. (2002) Synthesis and biological activities of cyclic lanthionine enkephalin analogues: delta-opioid receptor selective ligands., J Med Chem 45, 3746-3754.
78. Hegemann, P. (2002) Method for producing nucleic acid polymers. US Patent no. 6,472,184.
79. Lefèvre, F., Rémy, M.-H., Masson, J.-M. (1997) Alanine-stretch scanning mutagenesis: a simple and efficient method to probe protein structure and function., Nucleic Acids Res 25, 447-448.
80. Bierbaum, G., Szekat, C., Josten, M., Heidrich, C., Kempter, C., Jung, G., Sahl, H.G. (1996) Engineering of a novel thioether bridge and role of modified residues in the lantibiotic Pep5., Appl Environ Microbiol 62, 4332-4338.
81. Szekat, C., Jack, R.W., Skutlarek, D., Farber, H., Bierbaum, G. (2003) Construction of an expression system for site-directed mutagenesis of the lantibiotic mersacidin., Appl Environ Microbiol 69, 3777-3783
82. Groner, B., (Ed.) (2009) Peptides as drugs: discovery and development., WILEY-VCH, Weinheim.
83. Moll, G., Kuipers, A., Rink, R. (2010) Microbial engineering of dehydro-amino acids and lanthionines in non-lantibiotic peptides., Antonie Van Leeuwenhoek 97, 319-333.
84. Rink, R., Kuipers, A., de Boef, E., Leenhouts, K.J., Driessen, A.J., Moll, G.N., Kuipers, O.P. (2005) Lantibiotic structures as guidelines for the design of peptides that can be modified by lantibiotic enzymes., Biochemistry 44, 8873-8882.
85. Rink, R., Wierenga, J., Kuipers, A., Kluskens, L.D., Driessen, A.J., Kuipers, O.P., Moll, G.N. (2007) Dissection and modulation of the four distinct activities of nisin by mutagenesis of rings A and B and by C-terminal truncation., Appl Environ Microbiol 73, 5809-5816.
86. Meindl, K., Schmiederer, T., Schneider, K., Reicke, A., Butz, D., Keller, S., Gühring, H., Vértesy, L., Wink, J., Hoffmann, H., Brönstrup, M., Sheldrick, G.M., Süssmuth, R.D. (2010) Labyrinthopeptins: a new class of carbacyclic lantibiotics., Angew Chem Int Ed Engl 49, 1151.
87. Sambrook, J., Fritsch, E.F., Maniatis, T. (1989) Molecular Cloning. A Laboratory Manual (2nd ed.), Cold Spring Harbor Laboratory Press, Cold Spring Harbor, NY
88. Wright, F., Bibb, M.J. (1992) Codon usage in the G+C-rich Streptomyces genome, Gene 113, 55-65.
89. Kaiser, R., Metzka, L. (1999) Enhancement of cyanogen bromide cleavage yields for methionyl-serine and methionyl-threonine peptide bonds., Anal Biochem 266, 1-8.
90. Feitelson, J. S., Englenwood, N.J. (1991) Multifunctional plasmid vectors from Actinomadura and Escherichia coli., US Patent no. 5,002,891.
91. Bierman, M., Logan, R., O'Brien, K., Seno, E. T., Nagaraj a Rao, R., Schoner, B. E. (1992) Plasmid cloning vectors for the conjugal transfer of DNA from Escherichia coli to Streptomyces spp., Gene 116, 43-49.
92. Combes, P., Till, R., Bee, S., Smith, M.C.M. (2002) The Streptomyces genome contains multiple pseudo-attB sites for the (phi)C31-encoded site-specific recombination system., J Bacteriol 184, 5746-5752.

## Claims

1. A nucleic acid comprising at least one Labyrinthopeptin *(lab)* gene cluster, in particular a gene cluster having a sequence according to SEQ ID No. 139, of an actinomycete, in particular of *Actinomaduras namibiensis,* wherein:
(a) the nucleic acid further comprises a constitutive promoter for expression in *Streptomyces* cells, in particular an *ermE* promotor,
and wherein the gene cluster is under control of said promoter,
and/or
(b) the nucleic acid encoding LabA1 and/or LabA2 or a functional derivative thereof is mutated, such that the C-terminal amino acid of the leader peptide of LabA1 and/or LabA2 is substituted with a methionine,
and/or
(c) the nucleic acid further comprises at least one hybrid gene sequence, wherein the hybrid gene sequence:
(i) encodes a pre-pro-peptide comprising a LabA2 leader sequence and LabA1 pre-peptide or functional derivative thereof and/or
(ii) encodes a pre-pro-peptide comprising a LabA1 leader sequence and LabA2 pre-peptide or functional derivative thereof.

2. The nucleic acid according to claim 1,
(a) wherein the Labyrinthopeptin *(lab)* gene cluster comprises the genes *labKC*, *labA1*, *labA2*, *labT1* and *labT2*, and/or
(b) wherein the nucleic acid encoding LabA1 and/or LabA2 has a sequence according to SEQ ID No. 140 or SEQ ID No. 141, or has a sequence exhibiting at least 85%, preferably at least 90%, even more preferably at least 95% sequence identity to SEQ ID No. 140 and/or SEQ ID No. 141.

3. A vector comprising a nucleic acid according to claim 1 or 2.

4. The vector according to claim 4, wherein the vector is:
(a) pLab, having a sequence according to SEQ ID No. 143, or pUWLab, having a sequence according to SEQ ID No. 143, or pLabAmp, having a sequence according to SEQ ID No. 144, and/or
(b) obtainable from pUWLoriT by inserting at least one *lab* gene cluster, such that the gene cluster is under control of the constitutive promotor.

5. A bacterial cell, in particular a *Streptomyces* or *E. coli* cell, more preferably a *Streptomyces lividans* or *Streptomyces albus* cell, transformed with a vector according to claim 4.

6. A method for producing at least one Labyrinthopeptin or functional derivative thereof, comprising the steps:
(a) culturing a *Streptomyces* cell, preferably a *Streptomyces lividans* or *Streptomyces albus* cell, which cell is transformed with a vector according to claim 4, in culture medium,
(b) harvesting the culture medium comprising at least one Labyrinthopeptin or functional derivative thereof, and
(c) optionally purifying at least one Labyrinthopeptin or functional derivative thereof from the culture medium.

7. The method according to claim 6, wherein:
(a) the vector of claim 4 or 5 comprises a nucleic acid encoding LabA1 and/or LabA2 or functional derivative thereof, wherein the nucleic acid is mutated such that the C-terminal amino acid the leader peptide of LabA1 and/or LabA2 is substituted with a methionine, and/or
(b) the vector of claim 4 or 5 comprises at least one hybrid nucleic acid selected from:
(i) a hybrid nucleic acid encoding a pre-pro-peptide
comprising a LabA2 leader sequence and LabA1 pre-peptide or functional derivative thereof, and/or
(ii) a hybrid nucleic acid encoding a pre-pro-peptide
comprising a LabA1 leader sequence and LabA2 pre-peptide or functional derivative thereof,
preferably (ii).

8. A mixture of more than one Labyrinthopeptin or functional derivatives thereof, obtainable by the method according to claim 6 or 7.

9. A compound selected from:
(a) a Labyrinthopeptin pre-pro-peptide Labyrinthopeptin or a functional derivative thereof, wherein the C-terminal amino acid of the leader sequence is a methionine,
or
(b) a nucleic acid encoding a Labyrinthopeptin pre-pro-peptide or a functional derivative thereof, wherein the C-terminal amino acid of the leader sequence of the pre-pro-peptide is a methionine.

10. A compound selected from:
(a) a nucleic acid comprising a sequence according to any of SEQ ID No. 1 to 24, and/or
(b) a functional derivative of a Labyrinthopeptin pre-pro-peptide
encoded by a nucleic acid of (a), and which has a sequence different from LabA1 pre-pro- peptide according to SEQ ID No. 132 or LabA2 pre-pro-peptide according to SEQ ID No. 135, and/or
(c) a functional derivative of a Labyrinthopeptin pre-peptide encoded by
a nucleic acid of (a), and which has a sequence different from LabA1 pre-peptide according to SEQ ID No. 134 or LabA2 pre-peptide according to SEQ ID No. 137, or LabA3 pre-peptide according to SEQ ID No. 138 and which lacks the leader sequence or at least the N-terminal 80%, in particular 90%, more preferably 95% of the leader sequence, in particular a leader sequence according to SEQ ID No. 133 or SEQ ID No. 136, and/or
(d) a functional derivative of a Labyrinthopeptin peptide selected from:
LabA1_N2A, LabA1_V5A, LabA1_W6A, LabA1_E7A, LabA1_T11A, LabA1_V15A, LabA1_P16A, LabA1_F17A, LabA2_D2A, LabA2_W3A, LabA2_L5A, LabA2_E7A,
LabA2_T11A, LabA2_G12A, LabA2_L14A, LabA2_F15A, LabA1_V5del, LabA1_W6insV, LabA1_P16del, LabA1_P16insV, LabA1-S4T, M-LabA1_S4T, AM-LabA1_S4T, NR-LabA2_M (SG20), R-LabA2_M (SG20), LabA1_V15S, M-LabA1_V15S, LabA1_T11S (Ser), LabA1_W6Y, LabA1_A3H, LabA1_C20insA, LabA1_ S10insA LabA1_C20del, LabA1_SlinsC, D-LabA1/A2
(SG11), AD-LabA1/A2 (SG11), R-LabA2/A1 (SG11), NR-LabA2/A1 (SG11), and ENR-LabA2/A1 (SG11).

11. Use of at least one *Streptomyces lividans* and/or *Streptomyces albus* cell for the recombinant expression of at least one Labyrinthopeptin or a functional derivative thereof.

12. A mixture or a compound according to any of claims 8 to 10, for use in the treatment and/or prevention of bacterial infections, HIV infections or pain, in particular neuropathic pain.


SEQUENCE LISTING

<110>  SANOFI

<120>  Method for recombinant production of Labyrinthopeptins and
       functional derivatives thereof

<130>  DE2011/284

<160>  162

<170>  PatentIn version 3.5

<210>  1
<211>  379
<212>  DNA
<213>  Artificial Sequence

<220>
<223>  SG2

<400>  1
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gcaacgccag cgtctgggag tgctgcagca     180

cgggcagctg ggttcccttc acctgctgct gacgcccgca caccgttcca ccgatgagag     240

gtgacagtcc catggcgtca atattggaac tccagaacct ggacgtcgag cacgcccgcg     300

gcgagaaccg catgtccgac tggagcctgt gggagtgctg tagcacggga agcctgttcg     360

cctgctgctg aacagcgct                                                  379

<210>  2
<211>  376
<212>  DNA
<213>  Artificial Sequence

<220>
<223>  SG3

<400>  2
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccgcca gcaacgccag cgtctgggag tgctgcagca     180

cgggcagctg ggttcccttc acctgctgct gacgcccgca caccgttcca ccgatgagag     240

gtgacagtcc catggcatcc atccttgagc tccaggacct ggaggtcgag cgcgccagct     300

cggccgccga ctccgactgg agcctgtggg agtgctgtag cacgggaagc ctgttcgcct     360

gctgctgaac agcgct                                                     376

<210>  3
<211>  220
<212>  DNA
<213>  Artificial Sequence

<220>
<223>  SG4

<400>  3
   ccctgggcgg ccaccccctg agactcccct tcctccatcc ggaggaaggg gcgagcacgc      60
   gaccccgggg gaggaggtga acatccacca tggcatccat ccttgagctc caggacctgg     120
   aggtcgagcg cgccagctcg gccgccgaca gcaacgccag cgtctgggag tgctgcagca     180
   cgggcagctg ggttcccttc acctgctgct gaacagcgct                           220


<210>  4
<211>  214
<212>  DNA
<213>  Artificial Sequence

<220>
<223>  SG5

<400>  4
   ccctgggcgg ccaccccctg agactcccct tcctccatcc ggaggaaggg gcgagcacgc      60
   gaccccgggg gaggaggtga acatccacca tggcatccat ccttgagctc caggacctgg     120
   aggtcgagcg cgccagctcg gccgccatgt ccgactggag cctgtgggag tgctgtagca     180
   cgggaagcct gttcgcctgc tgctgaacag cgct                                 214


<210>  5
<211>  376
<212>  DNA
<213>  Artificial Sequence

<220>
<223>  SG6

<400>  5
   ccctgggcgg ccaccccctg agactcccct tcctccatcc ggaggaaggg gcgagcacgc      60
   gaccccgggg gaggaggtga acatccacca tggcatccat ccttgagctc caggacctgg     120
   aggtcgagcg cgccagctcg gccgccatgt ccgactggag cctgtgggag tgctgtagca     180
   cgggaagcct gttcgcctgc tgctgacgcc cgcacaccgt tccaccgatg agaggtgaca     240
   gtcccatggc gtcgatcctg gaactccaga acctggacgt cgagcacgcc cgcggcgaga     300
   accgcagcaa cgccagcgtc tgggagtgct gcagcacggg cagctgggtt cccttcacct     360
   gctgctgaac agcgct                                                     376


<210>  6
<211>  355
<212>  DNA
<213>  Artificial Sequence

<220>
<223>  SG7

<400>  6
   ccctgggcgg ccaccccctg agactcccct tcctccatcc ggaggaaggg gcgagcacgc      60
   gaccccgggg gaggaggtga acatccacca tggcatccat ccttgagctc caggacctgg     120
   aggtcgagcg cgccagctcg gccgccatgt gcagcacggg cagctgggtt cccttcacct     180
   gctgctgacg cccgcacacc gttccaccga tgagaggtga cagtcccatg gcgtcgatcc     240
   tggaactcca gaacctggac gtcgagcacg cccgcggcga gaaccgcatg tccgactgga     300
   gcctgtggga gtgctgtagc acgggaagcc tgttcgcctg ctgctgaaca gcgct          355


<210>  7
<211>  373
<212>  DNA
<213>  Artificial Sequence

<220>
<223>  SG8

<400>  7
ccctgggcgg ccaccccctg agactcccct tcctccatcc ggaggaaggg gcgagcacgc      60

gaccccgggg gaggaggtga acatccacca tggcacttct cgacctgcag acgatggaag     120

ccgacgagac gaccggtacc ggcgggccca gctccctgag cgtgctgtcc tgtgtgagcg     180

cggccagcat cacgctctgc ctctgacgcc cgcacaccgt tccaccgatg agaggtgaca     240

gtcccatggc gtcaatattg gaactccaga acctggacgt cgagcacgcc cgcggcgaga     300

accgcatgtc cgactggagc ctgtgggagt gctgtagcac gggaagcctg ttcgcctgct     360

gctgaacagc gct                                                        373


<210>  8
<211>  403
<212>  DNA
<213>  Artificial Sequence

<220>
<223>  SG9

<400>  8
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gcaacgccag cgtctgggag tgctgcagca     180

cgggcagctg ggttcccttc acctgctgca gcaacgccag cgtctgggag tgctgacgcc     240

cgcacaccgt tccaccgatg agaggtgaca gtcccatggc gtcaatattg gaactccaga     300

acctggacgt cgagcacgcc cgcggcgaga accgcatgtc cgactggagc ctgtgggagt     360

gctgtagcac gggaagcctg ttcgcctgct gctgaacagc gct                       403


<210>  9
<211>  439
<212>  DNA
<213>  Artificial Sequence

<220>
<223>  SG10

<400>  9
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gcaacgccag cgtctgggag tgctgcagca     180

cgggcagctg ggttcccttc acctgctgca gcaacgccag cgtctgggag tgcgccagca     240

cgggcagctg ggttcccttc acctgcgcct gacgcccgca caccgttcca ccgatgagag 300

gtgacagtcc catggcgtca atattggaac tccagaacct ggacgtcgag cacgcccgcg 360

gcgagaaccg catgtccgac tggagcctgt gggagtgctg tagcacggga agcctgttcg 420

cctgctgctg aacagcgct 439

<210> 10
<211> 376
<212> DNA
<213> Artificial Sequence

<220>
<223> SG11

<400> 10
ccctgggcgg ccaccccctg agactcccct tcctccatcc ggaggaaggg gcgagcacgc 60

gaccccgggg gaggaggtga acatccacca tggcatccat ccttgagctc caggacctgg 120

aggtcgagcg cgccagctcg gccgccgaca gcaacgccag cgtctgggag tgctgtagca 180

cgggaagcct gttcgcctgc tgctgacgcc cgcacaccgt tccaccgatg agaggtgaca 240

gtcccatggc gtcgatcctg gaactccaga acctggacgt cgagcacgcc cgcggcgaga 300

accgctccga ctggagcctg tgggagtgct gcagcacggg cagctgggtt cccttcacct 360

gctgctgaac agcgct 376

<210> 11
<211> 382
<212> DNA
<213> Artificial Sequence

<220>
<223> SG12

<400> 11
ccctgggcgg ccaccccctg agactcccct tcctccatcc ggaggaaggg gcgagcacgc 60

gaccccgggg gaggaggtga acatccacca tggcatccat ccttgagctc caggacctgg 120

aggtcgagcg cgccagctcg gccgccatga gcaacgccag ctggtgctgc agcacgggca 180

gctgggttcc cttcacctgc tgctgacgcc cgcacaccgt tccaccgatg agaggtgaca 240

gtcccatggc gtcgatcctg gaactccaga acctggacgt cgagcacgcc cgcggcgaga 300

accgcagcaa cgccagcgtc gtcgcctggg agtgctgcag cacgggcagc tgggttccct 360

tcacctgctg ctgaacagcg ct 382

<210> 12
<211> 379
<212> DNA
<213> Artificial Sequence

<220>
<223> SG13

<400> 12
ccctgggcgg ccaccccctg agactcccct tcctccatcc ggaggaaggg gcgagcacgc 60

gaccccgggg gaggaggtga acatccacca tggcatccat ccttgagctc caggacctgg 120

aggtcgagcg cgccagctcg gccgccatga gcaacgccag cgtctgggag tgctgcagca 180

cgggcagctg gacctgctgc tgacgcccgc acaccgttcc accgatgaga ggtgacagtc 240

ccatggcgtc gatcctggaa ctccagaacc tggacgtcga gcacgcccgc ggcgagaacc 300

gcagcaacgc cagcgtctgg gagtgctgca gcacgggcag ctgggttgcc gtccccttca 360

cctgctgctg aacagcgct 379

<210> 13
<211> 382
<212> DNA
<213> Artificial Sequence

<220>
<223> SG14

<400> 13
ccctgggcgg ccaccccctg agactcccct tcctccatcc ggaggaaggg gcgagcacgc 60

gaccccgggg gaggaggtga acatccacca tggcatccat ccttgagctc caggacctgg 120

aggtcgagcg cgccagctcg gccgccatga gcaacgccag cgtctgggag tgcgccagca 180

cgggcagctg ggttcccttc acctgcgcct gacgcccgca caccgttcca ccgatgagag 240

gtgacagtcc catggcgtcg atcctggaac tccagaacct ggacgtcgag cacgcccgcg 300

gcgagaaccg ctgcaacgcc gtcagctggg agagctgcag cacgggcagc tgggttccct 360

tcacctgctg ctgaacagcg ct 382

<210> 14
<211> 394
<212> DNA
<213> Artificial Sequence

<220>
<223> SG15

<400> 14
ccctgggcgg ccaccccctg agactcccct tcctccatcc ggaggaaggg gcgagcacgc 60

gaccccgggg gaggaggtga acatccacca tggcatccat ccttgagctc caggacctgg 120

aggtcgagcg cgccagctcg gccgccatga gcaacgccag cgtcgtcagc gcctgggagt 180

gctgcagcac gggcagctgg gttcccttca cctgctgctg acgcccgcac accgttccac 240

cgatgagagg tgacagtccc atggcgtcga tcctggaact ccagaacctg gacgtcgagc 300

acgcccgcgg cgagaaccgc agcaacgcca gcgtctggga gtgctgcagc acgggcagct 360

gggttagccc cttcacctgc tgctgaacag cgct 394

<210> 15
<211> 373
<212> DNA
<213> Artificial Sequence

<220>
<223> SG16

<400>  15
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gcaacgccag cgtctgggag tgctgcagca     180

gcggcagctg ggttcccttc acctgctgct gacgcccgca caccgttcca ccgatgagag     240

gtgacagtcc catggcgtcg atcctggaac tccagaacct ggacgtcgag cacgcccgcg     300

gcgagaaccg cagcaacgcc agctgctgca gcacgggcag ctgggttccc ttcacctgct     360

gctgaacagc gct                                                        373

<210>  16
<211>  370
<212>  DNA
<213>  Artificial Sequence

<220>
<223>  SG17

<400>  16
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gcaacgccag cgtctgggag tgctgcagca     180

cgggcagctg ggttcccttc agctgctgct gacgcccgca caccgttcca ccgatgagag     240

gtgacagtcc catggcgtcg atcctggaac tccagaacct ggacgtcgag cacgcccgcg     300

gcgagaaccg cagcaacgcc agcgtctggg agtgctgcag cacgggcagc tggtgctgct     360

gaacagcgct                                                            370

<210>  17
<211>  370
<212>  DNA
<213>  Artificial Sequence

<220>
<223>  SG18

<400>  17
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gctccctgag cgtgctgtcc tgtgtgagcg     180

cggccagcat cacgctctgc ctctgacgcc cgcacaccgt tccaccgatg agaggtgaca     240

gtcccatggc gtcgatcctg gaactccaga acctggacgt cgagcacgcc cgcggcgaga     300

accgcagctc cctgagcgtg ctgtcctgtt gcagcgcggc cagcatcacg ctctgctgct     360

gaacagcgct                                                            370

<210>  18
<211>  385
<212>  DNA
<213>  Artificial Sequence

<220>
<223>  SG19

<400>  18
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga ccggcagccg cgcgagcctc ctgctctgcg     180

gcgacagcag cctgagcatc accacctgta actgacgccc gcacaccgtt ccaccgatga     240

gaggtgacag tcccatggcg tcgatcctgg aactccagaa cctggacgtc gagcacgccc     300

gcggcgagaa ccgcaccggc agccgcgcga gcctcctgct ctgctgcagc agcctgagca     360

tcaccacctg ttgctgaaca gcgct                                           385


<210>  19
<211>  376
<212>  DNA
<213>  Artificial Sequence

<220>
<223>  SG20

<400>  19
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gcaacgccag cgtctgggag tgcatgtgca     180

gcacgggcag ctgggttccc ttcacctgct gctgacgccc gcacaccgtt ccaccgatga     240

gaggtgacag tcccatggcg tcaatattgg aactccagaa cctggacgtc gagcacgccc     300

gcggcgagaa ccgctccgac tggagcctgt gggagtgcat gagcacggga agcctgttcg     360

cctgctgaac agcgct                                                     376


<210>  20
<211>  376
<212>  DNA
<213>  Artificial Sequence

<220>
<223>  LabA2_T11A

<400>  20
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatgt ccgactggag cctgtgggag tgctgtagcg     180

ccggaagcct gttcgcctgc tgctgacgcc cgcacaccgt tccaccgatg agaggtgaca     240

gtcccatggc gtcgatcctg gaactccaga acctggacgt cgagcacgcc cgcggcgaga     300

accgcagcaa cgccagcgtc tgggagtgct gcagcacggg cagctgggtt cccttcacct     360

gctgctgaac agcgct                                                     376


<210>  21
<211>  379

<212>  DNA
<213>  Artificial Sequence

<220>
<223>  LabA1_G12A

<400>  21
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gcaacgccag cgtctgggag tgctgcagca     180

cggccagctg ggttcccttc acctgctgct gacgcccgca caccgttcca ccgatgagag     240

gtgacagtcc catggcgtca atattggaac tccagaacct ggacgtcgag cacgcccgcg     300

gcgagaaccg catgtccgac tggagcctgt gggagtgctg tagcacggga agcctgttcg     360

cctgctgctg aacagcgct                                                  379


<210>  22
<211>  379
<212>  DNA
<213>  Artificial Sequence

<220>
<223>  LabA1_W14A

<400>  22
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gcaacgccag cgtctgggag tgctgcagca     180

cgggcagcgc cgttcccttc acctgctgct gacgcccgca caccgttcca ccgatgagag     240

gtgacagtcc catggcgtca atattggaac tccagaacct ggacgtcgag cacgcccgcg     300

gcgagaaccg catgtccgac tggagcctgt gggagtgctg tagcacggga agcctgttcg     360

cctgctgctg aacagcgct                                                  379


<210>  23
<211>  379
<212>  DNA
<213>  Artificial Sequence

<220>
<223>  LabA1_P16A

<400>  23
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gcaacgccag cgtctgggag tgctgcagca     180

cgggcagctg ggttgccttc acctgctgct gacgcccgca caccgttcca ccgatgagag     240

gtgacagtcc catggcgtca atattggaac tccagaacct ggacgtcgag cacgcccgcg     300

gcgagaaccg catgtccgac tggagcctgt gggagtgctg tagcacggga agcctgttcg     360

cctgctgctg aacagcgct                                                  379

<210>  24
<211>  379
<212>  DNA
<213>  Artificial Sequence

<220>
<223>  LabA1_T18A

<400>  24
ccctgggcgg ccaccccctg agactcccct tcctccatcc ggaggaaggg gcgagcacgc      60

gaccccgggg gaggaggtga acatccacca tggcatccat ccttgagctc caggacctgg     120

aggtcgagcg cgccagctcg gccgccatga gcaacgccag cgtctgggag tgctgcagca     180

cgggcagctg ggttcccttc gcctgctgct gacgcccgca caccgttcca ccgatgagag     240

gtgacagtcc catggcgtca atattggaac tccagaacct ggacgtcgag cacgcccgcg     300

gcgagaaccg catgtccgac tggagcctgt gggagtgctg tagcacggga agcctgttcg     360

cctgctgctg aacagcgct                                                  379


<210>  25
<211>  1393
<212>  DNA
<213>  Artificial Sequence

<220>
<223>  aac(3)IV with changed PasI (CCCTCGG) and Eco47III (AGCGGT)
       restriction sites

<400>  25
acgcgtcgat tatctcgaga atgaccactg ctgtgagcgg tttgccttgg cggacaggtg      60

gctcaaggag aagagccttc agaaggaagg tccagtcggt catgcctttg ctcggttgat     120

ccgctcccgc gacattgtgg cgacagccct cggtcaactg ggccgagatc cgttgatctt     180

cctgcatccg ccagaggcgg gatgcgaaga atgcgatgcc gctcgccagt cgattggctg     240

agctcatgag cggagaacga gatgacgttg gaggggcaag gtcgcgctga ttgctggggc     300

aacacgtgga gcggatcggg gattgtcttt cttcagctcg ctgatgatat gctgacgctc     360

aatgcgcctc actgattaag cattggtaac tgtcagacca agtttactca tatatacttt     420

agattgattt aaaacttcat ttttaattta aaaggatcta ggtgaagatc ctttttgata     480

atctcatgac caaaatccct taacgtgagt tttcgttcca ctgagcgtca gaccccgtag     540

aaaagatcaa aggatcttct tgagatcctt tttttctgcg cgtaatctgc tgcttgcaaa     600

caaaaaaacc accgctacca gcggtggttt gtttgccgga tcaagagcta ccaactcttt     660

ttccgaaggt aactggcttc agcagagcgc agataccaaa tactgttctt ctagtgtagc     720

cgtagttagg ccaccacttc aagaactctg tagcaccgcc tacatacctc gctctgctaa     780

tcctgttacc agtggctgct gccagtggcg ataagtcgtg tcttaccggg tggactcaag     840

acgatagtta ccggataagg cgcagcggtc gggctgaacg gggggttcgt gcacacagcc     900

cagcttggag cgaacgacct acaccgaact gagataccta cagcgtgagc tatgagaaag     960

cgccacgctt cccgaaggga gaaaggcgga caggtatccg gtaagcggca gggtcggaac    1020

aggagagcgc acgagggagc ttccaggggg aaacgcctgg tatctttata gtcctgtcgg 1080

gtttcgccac ctctgacttg agcgtcgatt tttgtgatgc tcgtcagggg ggcggagcct 1140

atggaaaaac gccagcaacg cggccttttt acggttcctg gccttttgct ggccttttgc 1200

tcacatgttc tttcctgcgt tatcccctga ttctgtggat aaccgtatta ccgcctttga 1260

gtgagctgat accgctcgcc gcagccgaac gaccgagcgc agcgagtcag tgagcgagga 1320

agcggaagag cgcccaatac gcaaaccgcc tctccccgcg cgttggccga ttcattaatg 1380

cagagcttct aga 1393

<210> 26
<211> 26
<212> DNA
<213> Artificial Sequence

<220>
<223> LabA1_N2A_fw

<400> 26
ccgccatgag cgcggccagc gtctgg 26

<210> 27
<211> 26
<212> DNA
<213> Artificial Sequence

<220>
<223> LabA1_N2A_rv

<400> 27
ccagacgctg gccgcgctca tggcgg 26

<210> 28
<211> 25
<212> DNA
<213> Artificial Sequence

<220>
<223> LabA1_V5A_fw

<400> 28
gcaacgccag cgcctgggag tgctg 25

<210> 29
<211> 25
<212> DNA
<213> Artificial Sequence

<220>
<223> LabA1_V5A_rv

<400> 29
cagcactccc aggcgctggc gttgc 25

<210> 30
<211> 27
<212> DNA
<213> Artificial Sequence

<220>

<223>  LabA1_W6A_fw

<400>  30
caacgccagc gtcgcggagt gctgcag                                          27


<210>  31
<211>  27
<212>  DNA
<213>  Artificial Sequence

<220>
<223>  LabA1_W6A_rv

<400>  31
ctgcagcact ccgcgacgct ggcgttg                                          27


<210>  32
<211>  25
<212>  DNA
<213>  Artificial Sequence

<220>
<223>  LabA1_E7A_fw

<400>  32
cagcgtctgg gcctgctgca gcacg                                            25


<210>  33
<211>  25
<212>  DNA
<213>  Artificial Sequence

<220>
<223>  LabA1_E7A_rv

<400>  33
cgtgctgcag caggcccaga cgctg                                            25


<210>  34
<211>  25
<212>  DNA
<213>  Artificial Sequence

<220>
<223>  LabA1_T11A_fw

<400>  34
gagtgctgca gcgcgggcag ctggg                                            25


<210>  35
<211>  25
<212>  DNA
<213>  Artificial Sequence

<220>
<223>  LabA1_T11A_rv

<400>  35
cccagctgcc cgcgctgcag cactc                                            25


<210>  36
<211>  30
<212>  DNA

<213>  Artificial Sequence

<220>
<223>  LabA1_V15A_fw

<400>  36
cacgggcagc tgggcaccct tcacctgctg                                        30


<210>  37
<211>  30
<212>  DNA
<213>  Artificial Sequence

<220>
<223>  LabA1_V15A_rv

<400>  37
cagcaggtga agggtgccca gctgcccgtg                                        30


<210>  38
<211>  25
<212>  DNA
<213>  Artificial Sequence

<220>
<223>  LabA1_F17A_fw

<400>  38
gctgggttcc cgccacctgc tgctg                                             25


<210>  39
<211>  25
<212>  DNA
<213>  Artificial Sequence

<220>
<223>  LabA1_F17A_rv

<400>  39
cagcagcagg tggcgggaac ccagc                                             25


<210>  40
<211>  25
<212>  DNA
<213>  Artificial Sequence

<220>
<223>  LabA2_D2A_fw

<400>  40
ccgccatgtc cgcctggagc ctgtg                                             25


<210>  41
<211>  25
<212>  DNA
<213>  Artificial Sequence

<220>
<223>  LabA2_D2A_rv

<400>  41
cacaggctcc aggcggacat ggcgg                                             25

<210>  42
<211>  28
<212>  DNA
<213>  Artificial Sequence

<220>
<223>  LabA2_W3A_fw

<400>  42
cgccatgtcc gacgccagcc tgtgggag                                          28


<210>  43
<211>  28
<212>  DNA
<213>  Artificial Sequence

<220>
<223>  LabA2_W3A_rv

<400>  43
ctcccacagg ctggcgtcgg acatggcg                                          28


<210>  44
<211>  25
<212>  DNA
<213>  Artificial Sequence

<220>
<223>  LabA2_L5A_fw

<400>  44
ccgactggag cgcgtgggag tgctg                                             25


<210>  45
<211>  25
<212>  DNA
<213>  Artificial Sequence

<220>
<223>  LabA2_L5A_rv

<400>  45
cagcactccc acgcgctcca gtcgg                                             25


<210>  46
<211>  30
<212>  DNA
<213>  Artificial Sequence

<220>
<223>  LabA2_W6A_fw

<400>  46
cgactggagc ctggccgagt gctgtagcac                                        30


<210>  47
<211>  30
<212>  DNA
<213>  Artificial Sequence

<220>
<223>  LabA2_W6A_rv

<400>  47

gtgctacagc actcggccag gctccagtcg                                    30


<210>  48
<211>  26
<212>  DNA
<213>  Artificial Sequence

<220>
<223>  LabA2_E7A_fw

<400>  48
ggagcctgtg ggcctgctgt agcacg                                        26


<210>  49
<211>  26
<212>  DNA
<213>  Artificial Sequence

<220>
<223>  LabA2_E7A_rv

<400>  49
cgtgctacag caggcccaca ggctcc                                        26


<210>  50
<211>  26
<212>  DNA
<213>  Artificial Sequence

<220>
<223>  LabA2_G12A_fw

<400>  50
gctgtagcac ggccagcctg ttcgcc                                        26


<210>  51
<211>  26
<212>  DNA
<213>  Artificial Sequence

<220>
<223>  LabA2_G1A_rv

<400>  51
ggcgaacagg ctggccgtgc tacagc                                        26


<210>  52
<211>  25
<212>  DNA
<213>  Artificial Sequence

<220>
<223>  LabA2_L14A_fw

<400>  52
gcacgggaag cgcgttcgcc tgctg                                         25


<210>  53
<211>  25
<212>  DNA
<213>  Artificial Sequence

<220>

<223>  LabA2_L14A_rv

<400>  53
cagcaggcga acgcgcttcc cgtgc                                          25


<210>  54
<211>  27
<212>  DNA
<213>  Artificial Sequence

<220>
<223>  LabA2_F15A_fw

<400>  54
cacgggaagc ctggccgcct gctgctg                                        27


<210>  55
<211>  27
<212>  DNA
<213>  Artificial Sequence

<220>
<223>  LabA2_F15A_rv

<400>  55
cagcagcagg cggccaggct tcccgtg                                        27


<210>  56
<211>  26
<212>  DNA
<213>  Artificial Sequence

<220>
<223>  LabA1_N2insD_fw

<400>  56
ccgccatgag cgacaacgcc agcgtc                                         26


<210>  57
<211>  26
<212>  DNA
<213>  Artificial Sequence

<220>
<223>  LabA1_N2insD_rv

<400>  57
gacgctggcg ttgtcgctca tggcgg                                         26


<210>  58
<211>  25
<212>  DNA
<213>  Artificial Sequence

<220>
<223>  LabA1_N2insN_fw

<400>  58
ccatgagcaa caacgccagc gtctg                                          25


<210>  59
<211>  25
<212>  DNA

<213>  Artificial Sequence

<220>
<223>  LabA1_N2insN_rv

<400>  59
cagacgctgg cgttgttgct catgg                                          25


<210>  60
<211>  28
<212>  DNA
<213>  Artificial Sequence

<220>
<223>  LabA1_A3insA_fw

<400>  60
catgagcaac gccgccagcg tctgggag                                       28


<210>  61
<211>  28
<212>  DNA
<213>  Artificial Sequence

<220>
<223>  LabA1_A3insA_rv

<400>  61
ctcccagacg ctggcggcgt tgctcatg                                       28


<210>  62
<211>  25
<212>  DNA
<213>  Artificial Sequence

<220>
<223>  LabA1_A3del_fw

<400>  62
cgccatgagc aacagcgtct gggag                                          25


<210>  63
<211>  25
<212>  DNA
<213>  Artificial Sequence

<220>
<223>  LabA1_A3del_rv

<400>  63
ctcccagacg ctgttgctca tggcg                                          25


<210>  64
<211>  29
<212>  DNA
<213>  Artificial Sequence

<220>
<223>  LabA1_G12insA_fw

<400>  64
gtgctgcagc acggccggca gctgggttc                                      29

<210>  65
<211>  29
<212>  DNA
<213>  Artificial Sequence

<220>
<223>  LabA1_G12insA_rv

<400>  65
gaacccagct gccggccgtg ctgcagcac                                          29


<210>  66
<211>  25
<212>  DNA
<213>  Artificial Sequence

<220>
<223>  LabA1_G12del_fw

<400>  66
gtgctgcagc acgagctggg ttccc                                              25


<210>  67
<211>  25
<212>  DNA
<213>  Artificial Sequence

<220>
<223>  LabA1_G12del_rv

<400>  67
gggaacccag ctcgtgctgc agcac                                              25


<210>  68
<211>  28
<212>  DNA
<213>  Artificial Sequence

<220>
<223>  LabA1_W6insV_fw

<400>  68
caacgccagc gtcgtctggg agtgctgc                                           28


<210>  69
<211>  28
<212>  DNA
<213>  Artificial Sequence

<220>
<223>  LabA1_W6insV_rv

<400>  69
gcagcactcc cagacgacgc tggcgttg                                           28


<210>  70
<211>  28
<212>  DNA
<213>  Artificial Sequence

<220>
<223>  LabA1_W6insL_fw

<400>  70

caacgccagc gtcctgtggg agtgctgc                                          28


<210>  71
<211>  28
<212>  DNA
<213>  Artificial Sequence

<220>
<223>  LabA1_W6insL_rv

<400>  71
gcagcactcc cacaggacgc tggcgttg                                          28


<210>  72
<211>  25
<212>  DNA
<213>  Artificial Sequence

<220>
<223>  LabA1_V5del_fw

<400>  72
gagcaacgcc agctgggagt gctgc                                             25


<210>  73
<211>  25
<212>  DNA
<213>  Artificial Sequence

<220>
<223>  LabA1_V5del_rv

<400>  73
gcagcactcc cagctggcgt tgctc                                             25


<210>  74
<211>  25
<212>  DNA
<213>  Artificial Sequence

<220>
<223>  LabA1_P16del_fw

<400>  74
gggcagctgg gttttcacct gctgc                                             25


<210>  75
<211>  25
<212>  DNA
<213>  Artificial Sequence

<220>
<223>  LabA1_P16del_rv

<400>  75
gcagcaggtg aaaacccagc tgccc                                             25


<210>  76
<211>  25
<212>  DNA
<213>  Artificial Sequence

<220>

<223>  LabA1_T18del_fw

<400>  76
ctgggttccc ttctgctgct gacgc                                          25


<210>  77
<211>  25
<212>  DNA
<213>  Artificial Sequence

<220>
<223>  LabA1_T18del_rv

<400>  77
gcgtcagcag cagaagggaa cccag                                          25


<210>  78
<211>  25
<212>  DNA
<213>  Artificial Sequence

<220>
<223>  LabA1_P16insV_fw

<400>  78
gcagctgggt tgtccccttc acctg                                          25


<210>  79
<211>  25
<212>  DNA
<213>  Artificial Sequence

<220>
<223>  LabA1_P16insV_rv

<400>  79
caggtgaagg ggacaaccca gctgc                                          25


<210>  80
<211>  25
<212>  DNA
<213>  Artificial Sequence

<220>
<223>  LabA1_VP15del_fw

<400>  80
cacgggcagc tggttcacct gctgc                                          25


<210>  81
<211>  25
<212>  DNA
<213>  Artificial Sequence

<220>
<223>  LabA1_VP15del_rv

<400>  81
gcagcaggtg aaccagctgc ccgtg                                          25


<210>  82
<211>  25
<212>  DNA

<213>  Artificial Sequence

<220>
<223>  LabA1_S1T_fw

<400>  82
ggccgccatg acgaacgcca gcgtc                                              25


<210>  83
<211>  25
<212>  DNA
<213>  Artificial Sequence

<220>
<223>  LabA1_S1T_rv

<400>  83
gacgctggcg ttcgtcatgg cggcc                                              25


<210>  84
<211>  28
<212>  DNA
<213>  Artificial Sequence

<220>
<223>  LabA1_S10T_fw

<400>  84
catgagcaac gccaccgtct gggagtgc                                           28


<210>  85
<211>  28
<212>  DNA
<213>  Artificial Sequence

<220>
<223>  LabA1_S10T_rv

<400>  85
gcactcccag acggtggcgt tgctcatg                                           28


<210>  86
<211>  26
<212>  DNA
<213>  Artificial Sequence

<220>
<223>  LabA1_S13T_fw

<400>  86
gcagcacggg cacctgggtt cccttc                                             26


<210>  87
<211>  26
<212>  DNA
<213>  Artificial Sequence

<220>
<223>  LabA1_S13T_rv

<400>  87
gaagggaacc caggtgcccg tgctgc                                             26

<210>  88
<211>  30
<212>  DNA
<213>  Artificial Sequence

<220>
<223>  LabA1_C9tga_fw

<400>  88
cgtctgggag tgctgaagca cgggcagctg                                        30


<210>  89
<211>  30
<212>  DNA
<213>  Artificial Sequence

<220>
<223>  LabA1_C9tga_rv

<400>  89
cagctgcccg tgcttcagca ctcccagacg                                        30


<210>  90
<211>  27
<212>  DNA
<213>  Artificial Sequence

<220>
<223>  LabA1_S4A_fw

<400>  90
catgagcaac gccgccgtct gggagtg                                           27


<210>  91
<211>  27
<212>  DNA
<213>  Artificial Sequence

<220>
<223>  LabA1_S4A_rv

<400>  91
cactcccaga cggcggcgtt gctcatg                                           27


<210>  92
<211>  33
<212>  DNA
<213>  Artificial Sequence

<220>
<223>  LabA1_S13A_fw

<400>  92
gtgctgcagc acgggcgcct gggttccctt cac                                    33


<210>  93
<211>  33
<212>  DNA
<213>  Artificial Sequence

<220>
<223>  LabA1_S13A_rv

<400>  93

gtgaagggaa cccaggcgcc cgtgctgcag cac                                    33


<210>  94
<211>  27
<212>  DNA
<213>  Artificial Sequence

<220>
<223>  LabA1_V5T_fw

<400>  94
gagcaacgcc agcacctggg agtgctg                                          27


<210>  95
<211>  27
<212>  DNA
<213>  Artificial Sequence

<220>
<223>  LabA1_V5T_rv

<400>  95
cagcactccc aggtgctggc gttgctc                                          27


<210>  96
<211>  28
<212>  DNA
<213>  Artificial Sequence

<220>
<223>  LabA1_V15S_fw

<400>  96
   cacgggcagc tggtccccct tcacctgc                                          28


<210>  97
<211>  28
<212>  DNA
<213>  Artificial Sequence

<220>
<223>  LabA1_V15S_rv

<400>  97
gcaggtgaag ggggaccagc tgcccgtg                                         28


<210>  98
<211>  30
<212>  DNA
<213>  Artificial Sequence

<220>
<223>  LabA1_W6Y_fw

<400>  98
caacgccagc gtctacgagt gctgcagcac                                       30


<210>  99
<211>  30
<212>  DNA
<213>  Artificial Sequence

<220>

<223>  LabA1_W6Y_rv

<400>  99
gtgctgcagc actcgtagac gctggcgttg                                        30


<210>  100
<211>  27
<212>  DNA
<213>  Artificial Sequence

<220>
<223>  LabA1_A3H_fw

<400>  100
gccatgagca accacagcgt ctgggag                                           27


<210>  101
<211>  27
<212>  DNA
<213>  Artificial Sequence

<220>
<223>  LabA1_A3H_rv

<400>  101
ctcccagacg ctgtggttgc tcatggc                                           27


<210>  102
<211>  25
<212>  DNA
<213>  Artificial Sequence

<220>
<223>  LabA1_E7R_fw

<400>  102
ccagcgtctg gcggtgctgc agcac                                             25


<210>  103
<211>  25
<212>  DNA
<213>  Artificial Sequence

<220>
<223>  LabA1_E7R_rv

<400>  103
gtgctgcagc accgccagac gctgg                                             25


<210>  104
<211>  25
<212>  DNA
<213>  Artificial Sequence

<220>
<223>  LabA1_C20insA_fw

<400>  104
ccttcacctg cgcctgctga cgccc                                             25


<210>  105
<211>  25
<212>  DNA

<213>  Artificial Sequence

<220>
<223>  LabA1_C20insA_rv

<400>  105
gggcgtcagc aggcgcaggt gaagg                                          25


<210>  106
<211>  25
<212>  DNA
<213>  Artificial Sequence

<220>
<223>  LabA1_S10insA_fw

<400>  106
gggagtgctg cgccagcacg ggcag                                          25


<210>  107
<211>  25
<212>  DNA
<213>  Artificial Sequence

<220>
<223>  LabA1_S10insA_rv

<400>  107
ctgcccgtgc tggcgcagca ctccc                                          25


<210>  108
<211>  29
<212>  DNA
<213>  Artificial Sequence

<220>
<223>  LabA1_C20del_fw

<400>  108
gttcccttca cctgctgacg cccgcacac                                      29


<210>  109
<211>  29
<212>  DNA
<213>  Artificial Sequence

<220>
<223>  LabA1_C20del_rv

<400>  109
gtgtgcgggc gtcagcaggt gaagggaac                                      29


<210>  110
<211>  25
<212>  DNA
<213>  Artificial Sequence

<220>
<223>  LabA1_S1insC_fw

<400>  110
cggccgccat gtgcagcaac gccag                                          25

<210>  111
<211>  25
<212>  DNA
<213>  Artificial Sequence

<220>
<223>  LabA1_S1insC_rv

<400>  111
ctggcgttgc tgcacatggc ggccg                                              25


<210>  112
<211>  26
<212>  DNA
<213>  Artificial Sequence

<220>
<223>  LabA1_C9insV_fw

<400>  112
gtctgggagt gcgtctgcag cacggg                                             26


<210>  113
<211>  26
<212>  DNA
<213>  Artificial Sequence

<220>
<223>  LabA1_C9insV_rv

<400>  113
cccgtgctgc agacgcactc ccagac                                             26


<210>  114
<211>  25
<212>  DNA
<213>  Artificial Sequence

<220>
<223>  LabA1_C9insVN_fw

<400>  114
gggagtgcgt caactgcagc acggg                                              25


<210>  115
<211>  25
<212>  DNA
<213>  Artificial Sequence

<220>
<223>  LabA1_C9insVN_rv

<400>  115
cccgtgctgc agttgacgca ctccc                                              25


<210>  116
<211>  20
<212>  DNA
<213>  Artificial Sequence

<220>
<223>  attB_For

<400>  116

cggtctcgaa gccgcggtgc                                                    20

<210>  117
<211>  19
<212>  DNA
<213>  Artificial Sequence

<220>
<223>  attB_Rev

<400>  117
gcccgccgtg accgtcgag                                                     19

<210>  118
<211>  27
<212>  DNA
<213>  Artificial Sequence

<220>
<223>  p18mob_EcoRI_for

<400>  118
catctcgaat tccgctcatg agctcag                                            27

<210>  119
<211>  29
<212>  DNA
<213>  Artificial Sequence

<220>
<223>  p18mob_EcoRI_rev

<400>  119
agttatcgag atctgcagga gctctttgg                                          29

<210>  120
<211>  20
<212>  DNA
<213>  Artificial Sequence

<220>
<223>  pSETproof_for

<400>  120
cgagccggaa gcataaagtg                                                    20

<210>  121
<211>  20
<212>  DNA
<213>  Artificial Sequence

<220>
<223>  pSETproof_rev

<400>  121
gcttggagcg aacgacctac                                                    20

<210>  122
<211>  20
<212>  DNA
<213>  Artificial Sequence

<220>

<223>  STK_RTPCR_fw

<400>  122
agcagcaagt acgccgaacg                                                    20


<210>  123
<211>  20
<212>  DNA
<213>  Artificial Sequence

<220>
<223>  STK_RTPCR_rv

<400>  123
gcgaagtgga gctggttgag                                                    20


<210>  124
<211>  20
<212>  DNA
<213>  Artificial Sequence

<220>
<223>  pMA_seq

<400>  124
tgtgctgcaa ggcgattaag                                                    20


<210>  125
<211>  34
<212>  DNA
<213>  Artificial Sequence

<220>
<223>  Infusion 1 (fw)

<400>  125
gtcgaggcgg ccctgggcgg ccaccccctg agac                                    34


<210>  126
<211>  39
<212>  DNA
<213>  Artificial Sequence

<220>
<223>  Infusion 2 (rv)

<400>  126
gcggcctcgg tcagcgctgt tcagcagcag gcgaacagg                               39


<210>  127
<211>  36
<212>  DNA
<213>  Artificial Sequence

<220>
<223>  Infusion 3 (rv)

<400>  127
tcggcggcct cggtcagcgc tgttcagcag caggtg                                  36


<210>  128
<211>  35
<212>  DNA

<213>  Artificial Sequence

<220>
<223>  Infusion SG17 (rv)

<400>  128
cggcggcctc ggtcagcgct gttcagcagc accag                                35


<210>  129
<211>  35
<212>  DNA
<213>  Artificial Sequence

<220>
<223>  Infusion SG18 (rv)

<400>  129
tcggcggcct cggtcagcgc tgttcagcag cagag                                35


<210>  130
<211>  35
<212>  DNA
<213>  Artificial Sequence

<220>
<223>  Infusion SG19 (rv)

<400>  130
ttcggcggcc tcggtcagcg ctgttcagca acagg                                35


<210>  131
<211>  35
<212>  DNA
<213>  Artificial Sequence

<220>
<223>  Infusion SG20 (rv)

<400>  131
cggcggcctc ggtcagcgct gttcagcagg cgaac                                35


<210>  132
<211>  40
<212>  PRT
<213>  Actinomadura namibiensis

<400>  132

Met Ala Ser Ile Leu Glu Leu Gln Asp Leu Glu Val Glu Arg Ala Ser
1               5                   10                  15


Ser Ala Ala Asp Ser Asn Ala Ser Val Trp Glu Cys Cys Ser Thr Gly
            20                  25                  30


Ser Trp Val Pro Phe Thr Cys Cys
        35                  40


<210>  133
<211>  20
<212>  PRT
<213>  Actinomadura namibiensis

<400>  133

Met Ala Ser Ile Leu Glu Leu Gln Asp Leu Glu Val Glu Arg Ala Ser
1               5                   10                  15

Ser Ala Ala Asp
            20

<210>  134
<211>  20
<212>  PRT
<213>  Actinomadura namibiensis

<400>  134

Ser Asn Ala Ser Val Trp Glu Cys Cys Ser Thr Gly Ser Trp Val Pro
1               5                   10                  15

Phe Thr Cys Cys
            20

<210>  135
<211>  38
<212>  PRT
<213>  Actinomadura namibiensis

<400>  135

Met Ala Ser Ile Leu Glu Leu Gln Asn Leu Asp Val Glu His Ala Arg
1               5                   10                  15

Gly Glu Asn Arg Ser Asp Trp Ser Leu Trp Glu Cys Cys Ser Thr Gly
            20                  25                  30

Ser Leu Phe Ala Cys Cys
        35

<210>  136
<211>  20
<212>  PRT
<213>  Actinomadura namibiensis

<400>  136

Met Ala Ser Ile Leu Glu Leu Gln Asn Leu Asp Val Glu His Ala Arg
1               5                   10                  15

Gly Glu Asn Arg
            20

<210>  137
<211>  18
<212>  PRT
<213>  Actinomadura namibiensis

<400>  137

Ser Asp Trp Ser Leu Trp Glu Cys Cys Ser Thr Gly Ser Leu Phe Ala
1               5                   10                  15

Cys Cys

<210> 138
<211> 21
<212> PRT
<213> Actinomadura namibiensis

<400> 138

Asp Ser Asn Ala Ser Val Trp Glu Cys Cys Ser Thr Gly Ser Trp Val
1               5                   10                  15

Pro Phe Thr Cys Cys
            20

<210> 139
<211> 6400
<212> DNA
<213> Actinomadura namibiensis

<220>
<221> misc_feature
<222> (1)..(2589)
<223> labKC gene

<220>
<221> misc_feature
<222> (2599)..(2721)
<223> labA1 gene

<220>
<221> misc_feature
<222> (2761)..(2877)
<223> labA2 gene

<220>
<221> misc_feature
<222> (2988)..(4688)
<223> labT1 gene

<220>
<221> misc_feature
<222> (4685)..(6400)
<223> labT2 gene

<400> 139
   atggatctgc ggtaccacgc ctatgcgatg gccgatcccg tcttttatga ctcgccgtcc 60
   agcgacaccc gggagaccga cggctactcc gacgacctcc ccctgccgga cggctgggaa 120
   cgtcggcgcg tgggggtctg ggtgatgcag ggccacgacg ggttgaccat gcctgaccag 180
   ggctggaaga tccatgtgtc ggccggtctg gacaacgcct ggcccgtcct cgaactggtc 240
   gccaaatact gcgtcgagca ggaaatgcct ttcaagttcc tgcgcagcag aaggacgttg 300
   ctggcccgca gcagcaagta cgccgaacgc ggcggcagcg gaaagttcat cacgatctac 360
   cccgccgacg aaggcgctct ggaaaagacc ctccatgaac tcggtggaat gctggaaggg 420
   cagcccggcc cctatatctt gagcgatctg cgctggcgtt cggggccgct gttcgtgcgc 480
   tacggcgctt tcaaggagaa attctgccgg gacgggcgcg gcgagatggt gcccgcgatc 540
   gcgcgcccgg acggcgtgct ggtgcccgac gccagggacc cggtgttccg ggtgcccgcg 600
   tgggtggagg tgcccggctt cctgcgggag gccatcgacg cccgggagaa cgggaccgtc 660
   gaggacttcc cctaccggat cgagaaggcg ctgcacttct ccaacggcgg cggcctctac 720
   cgcgccgtgg acgagcgcac cggccgcagg gtcctggtga aggaggcgcg gccgatggcg 780
   ggcctggacc gcgccgagga cgacgccgtc gtccggctcg aacgcgagca cggcctgctg 840
   ctccgcctcg ccgacctgga ctgcatcccc gacctggtcg agtaccggag ctggtgggag 900
   caccgcttcc tcgtgcggga gtacgtcgag ggcgagaccc tcacccacca catggtgcgc 960
   cgcaacccga tgctgcacta cggcgcgacg ccgcaggagg tcgccggcta caccgagtgg 1020
   gcgctcggcg tcgtcgaccg ggtggagagc gcgctcggcc ggctgcacga acgcggcgtg 1080
   gtcttcggcg acctgcaccc cggcaacatc atcgtccggg acgacgactc catcgtgttc 1140
   gtcgacttcg agctggtcgc cgaggcggag gaggcgacgc acccggcgct cggcgcgccc 1200
   ggctaccagg ccccgcccga ctacaccggg ttcgccatcg accgctacgc gctcggctgc 1260
   atcaggctcg cggtcttcac ctccctcacc gcgacgctgc actggacgga ccgcaaggtc 1320
   gagcagttcc tcgacgtgat ctgcgagtcc ttcccgctgc cgccggacta cgccgaccgg 1380
   atccgccgcg acctcgcccg cccggcgccg gcggacggcg cgccgccgat ctggcgcgag 1440
   ccgacgcccg ccacctggcc ggacacgcgg gccgggatcg ccgccgcgat cctcgacacg 1500
   gccacgcccg agcgcgccga ccggctcttc ccgggcgaca tcgagcagtt cgcgaccagc 1560
   gtcggtggga tcgggttcgg ccacggcgcg gcgggcgtgc tgtgggcgct ggccgaggcg 1620
   ggcgccggcc gcttccccga ccacgaggac tgggtgcggg acgccgtcgc cagggcgcaa 1680
   cggccgccgc ccggcttcta cgacggcgtc gcgggcgtcg cccacgtcct ggaccggctc 1740
   ggccgcgccg acgaggcccg cgagctcatg gagcacgcgc ccgccgcgac gggggcgacc 1800
   gacaacagcc tctaccgggg gctggcgggc atcggcctca accagctcca cttcgcccgc 1860
   gtcacgggcg aggcgtcgtt cgccgcggcg gccgaggaga ccgccggccg ggtggtcgcg 1920
   aacctgcgcc gcaagacgga gggcgcctac cgggcgggcc tgatgtacgg ctcctccggc 1980
   ccggccctgt tcctcgtccg gatgttcgag gcgacgggcg acggccactg gctggacgag 2040
   gccgaacgcg ccctgcaccg cgagctggac gcctgcaagt ggacgcagaa ggacagcacg 2100
   ctccaggtcg acgagggctg gcgggtcctt ccgtacgtcg ccaccggcag cgtgggcatc 2160
   ggcatcgcgc tgcacgagtt cctgcggcac cgcccggcgc cgcgcttcac cgaggcgcag 2220
   gaggggatcc ggagggcggc ggctcccgcg tacttcgtgc agagcggcct gctcaacgga 2280
   cggtccggca tcctcgccta cctgctgcac gtcggggccg gccgggagga cccggtcgtc 2340
   cggacgcacc tgcgcaacct cggctggcac gccgtcccgt accccggccg gggcgaggac 2400
   gccccggcgc ccggcgcgcg gcggaccgcg ttcatcgggg accagctgct gcgcctgtcc 2460
   atggacctcg ccacgggctc cgccggggtc ctggccacgg tcgaggcggc cctgggcggc 2520
   caccccctga gactcccctt cctccatccg gaggaagggg cgagcacgcg accccggggg 2580
   aggaggtgaa catccaccat ggcatccatc cttgagctcc aggacctgga ggtcgagcgc 2640
   gccagctcgg ccgccgacag caacgccagc gtctgggagt gctgcagcac gggcagctgg 2700
   gttcccttca cctgctgctg acgcccgcac accgttccac cgatgagagg tgacagtccc 2760
   atggcgtcga tcctggaact ccagaacctg gacgtcgagc acgcccgcgg cgagaaccgc 2820
   tccgactgga gcctgtggga gtgctgtagc acgggaagcc tgttcgcctg ctgctgaaca 2880
   gcgctgaccg aggccgccga agcggcaccc atgtgaaacg accgcccggg gcggtgggga 2940
   ctcccaccgc cccgggcgga ccaccatcag cgaggagcac gacaggaatg cacgccgacc 3000
   ggctgctcgt ccaggcgctg cgcgccggcc ccgggtggac ggccctcctg gtcgccgcga 3060
   cgctgctgaa cgcggtgtgc gcgctcgccc tgcccgccgc gatcggcgcg gtcaccgacg 3120
   cggtgctcgc ccgcgacgcg ggccgcggcg ccggggcccc gctgggctgg ctggcggcgg 3180
   cgctcggcgg cgtggcgctg ggcacggcgc tgtcccggct cgccgaggtg tactgcggca 3240
   cggccgcgac cagggagctg cggacgcgcc tggtcacgca cgtgctggcc ctcggggtgc 3300
   cggggacgcg cgcgttcacg cccggcgacc tggccagccg cgccgtcacc ggcgcgccgc 3360
   aggccggcgc ggtcgcgcgg tcggtggtca gcgcggtcgc ggggctgctg atgtcggtcg 3420
   gcggcgtggt cgcgctgtgg ctgatcgact ggcggctggt ggcggcggtg ggcggcgcgg 3480
   tcccgatcgg gctgctgctc atgcgcgtct tcgtccggga cgcctccgac ctggtcaccg 3540
   agtaccagga ggcgcagggc gagatcgccg cgcggatgac cgacgcgctc accggcatcc 3600
   gcaccatccg cgccgccggg acctggcggc gcgaggccga ccgggtcctg gccccgctgc 3660
   cgggcctgtc ggactccggg cggcggctct ggcacgccta cgggcggatg caggggcagg 3720
   gccgcctgat cgtcccgctg gccgagatcg cggcgctggc cgtcgccggg cggggcgtcc 3780
   tgaccggtcg gctgtcggcg ggggagatgc tcgcggcggc gggctacgcc ccgatggcgc 3840
   tgggcctggt cggccagatc cccctgctgc tggccctggc gcggctgcgg gcgggggcgc 3900
   gccgcctggc cgaggtgctg tcggtccccg cccccggcgg cggcgaccgc gcgctgccgc 3960
   ccggccccgg cgcgctggtc ctgcgcgggg tgaccgtgcg gacgccggac gggccgctgc 4020
   tcgacgcggt cgacctgacc gtcccgcccg gccggacggt ggcggtggtc ggccggtccg 4080
   gcgcgggcaa gacgacgctc gccgccgtgg ccgggcgcct cctcgacccc gacgaggggc 4140
   gggtgctcct ggacggggtc ccgctgcgcg agctcgcgcc cgccgcgctg cgcgcgcagg 4200
   tcgcctacgc cttcgaacgc cccgacctcc aggggaccac gatcgccgac gccatcgcct 4260
   acggctgccc gtccgcgccg ccccgcgcgg tcgaacgggc ggccgcgctc gcgcgggcgg 4320
   acggcttcgt ccggctgctc ccggcgggct tcgacacgcc cgtcgcggac acgcccctgt 4380
   ccggcgggga acggcagcgg ctcgggctgg cccgggcctt cgtccggaac gcccgcctga 4440
   tcatcctcga cgacgcgacc tccagcctcg actcggtgac cgaggcgcag gtggcggccg 4500
   cgctcgcgga ggccgccgcg acccgcaccc ggctggtggt cgcgcaccgc gccgggaccg 4560
   ccgcgcgcgc cgacctggtc ctgtggctgg actccggccg ggtgcgcgcg ctccgcccgc 4620
   accgcgagct gtgggccgat ccggactacc gggcgatgtt cgagccggcg ccggcggagc 4680
   gcgcgtgacg gggggcgcgg cgcgctggtt cgccgcccag ctgcggaccg aacggcgcgg 4740
   cctggccggg gtgctggcgt ggtcggtcgc ggcggcgctc ccggcgctgg tgtccggacg 4800
   gctgatcgcg ctcgccgtcg accagggctt cctgcgcgga cgcggcacgg tcggcctggc 4860
   gtggctgggg gcgctcgcgg cggcgacggc ggtcggcgcg ctgggcgcgc ggcagatccc 4920
   gcgcgcgctc ggcggggccc tggagccggt gcgcgaccgg ctcgtccgcc ggatcgtggc 4980
   cggcgcgctg cgccgcgcgc tggccggccg gcccgatccg ggcgtggtcg cgaagctgac 5040
   cgagcagacc gagaccgtcc gggacaccgg cgcggggctg ctgctcggga tgcagcaggt 5100
   cggcatggcg gtggtcgcgg ccgcgatcgg gctgctgctg ctcgcgcccg tcacggcgct 5160
   gctggtcctc ccgccggtgc tggcggcgct gctgctgctc tcccggctgc tgccggagct 5220
   gatggcgcgg caccgggccc tgctggcggc cgaggaccgc gtcgcggcga ccgtcggcgg 5280
   cctgaccgcg gcggtgcgcg acgtggtcgc gtgcggggcc gggccccggg ccggggccga 5340
   ggcggcggcg gtcttcgccg agcaggccgc ggccgaacgc gccgtcgccc gcgccgacgc 5400
   gctgcgcacc gcgctcggca tcgtcggcgg ccagctgtcg ctggtcgtcc tgctggccgc 5460
   cgcgccctgg ctggtggcct ccgggcggct cagccccggc gaggtgctcg gcgcgctcac 5520
   ctacgtgacg accggcctgc aaccggcgct gcgcgcggcc gtgcagagca ccgggggttc 5580
   cggcgtgcgg ctggcggtga ccctgcaacg cctcctggac ggctccgcgg acgaacccga 5640
   ccccgcgccc gccggagggc gcgtcccggg ccgcctcgac ctgtcggtgg aggggctcac 5700
   gttcgcctac ggcgccggtg ccgaaccggt cgtgcgcgac ctggacctga ccgtgccgca 5760
   cgggacgcac ctggccgtcg tcggccccag cgggatcggc aagtcgaccc tggccgacct 5820
   gctcaccggg gtcgccgtcc cgcaggcggg ccgggtccgc gtcggcggcg tgccgctcgc 5880
   cgagaccgat cccgcctgga ggcgccggtc ggtcgcgatc atcccgcagg aggcgtacgt 5940
   cttcagcggc acgctcgccg agaacctcgc ctacctgcgc ccggacgccg ccgaggccga 6000
   gctcgacgcc gccgtcgccg cgctgggcct ggcgccgctg cgcgcccggc tcggcgggta 6060
   cggggccgcg ctcgggccgc gttcgggcct gacgccgggg gagcggcagc tcgtcgcgct 6120
   cgcccgcgtc tggctcagcc ccgcgcggat cgtggtgctg gacgaggcga cctgccacct 6180
   cgaccccgcc gccgaggccc gcgtggagca cgccttccgg gaccggcccg gcaccctggt 6240
   cgtcatcgcg caccggctca gctcggcgcg ccgcgccgac cgcgtcctgg tgatggacgg 6300
   gacccgcccc cggctgggca cccacgggga actcctccgg gactccccgc tctacgccga 6360
   cctggtcggg gcctggaccg ttcaccccgc ggcgcggtag 6400

<210> 140
<211> 123
<212> DNA
<213> Actinomadura namibiensis

<400> 140
atggcatcca tccttgagct ccaggacctg gaggtcgagc gcgccagctc ggccgccgac 60

agcaacgcca gcgtctggga gtgctgcagc acgggcagct gggttccctt cacctgctgc 120

tga                                                                 123

<210>  141
<211>  117
<212>  DNA
<213>  Actinomadura namibiensis

<400>  141
atggcgtcga tcctggaact ccagaacctg gacgtcgagc acgcccgcgg cgagaaccgc      60

tccgactgga gcctgtggga gtgctgtagc acgggaagcc tgttcgcctg ctgctga        117

<210>  142
<211>  13799
<212>  DNA
<213>  Artificial Sequence

<220>
<223>  Vector pUWLab

<220>
<221>  misc_feature
<222>  (537)..(581)
<223>  ermE

<220>
<221>  misc_feature
<222>  (618)..(3206)
<223>  labKC

<220>
<221>  misc_feature
<222>  (3216)..(3338)
<223>  labA1

<220>
<221>  misc_feature
<222>  (3378)..(3494)
<223>  labA2

<220>
<221>  misc_feature
<222>  (3605)..(5305)
<223>  labT1

<220>
<221>  misc_feature
<222>  (5302)..(7017)
<223>  labT2

<220>
<221>  misc_feature
<222>  (7272)..(7859)
<223>  ori for E. coli

<220>
<221>  misc_feature
<222>  (8954)..(8178)
<223>  aac(3) IV

<220>
<221>  misc_feature
<222>  (9350)..(9471)
<223>  oriT

<220>
<221>  misc_feature
<222>  (11300)..(10491)
<223>  tsr

<220>
<221>  misc_feature
<222>  (12566)..(13682)
<223>  replicon pIJ101

<400>  142
tcgcccagcg ccgcgaggaa gcaactgcac gccgacggcg agagcaggac gacgaccagg      60

acgacgacgc cgacgaccgc caggagcggg ccgcccggca catcgcccgg ctcgcaagtg     120

ggcccacttc gcactaactc gctccccccc gccgtacgtc atcccggtga cgtacggcgg     180

gggtcggtga cgtacgcggc gacggcggcc ggggtcgaag ccgcgggagt aatcctggga     240

ttactcgccc ggggtcggcc ccgccggcac ttcgtgcagg cggtaccggg ccccccctcg     300

aggtccagcc cgacccgagc acgcgccggc acgcctggtc gatgtcggac cggagttcga     360

ggtacgcggc ttgcaggtcc aggaagggga cgtccatgcg agtgtccgtt cgagtggcgg     420

cttgcgcccg atgctagtcg cggttgatcg gcgatcgcag gtgcacgcgg tcgatcttga     480

cggctggcga gaggtgcggg gaggatctga ccgacgcggt ccacacgtgg caccgcgatg     540

ctgttgtggg cacaatcgtg ccggttggta ggatcgacgg tatcgataag cttgatatcg     600

aattcttgga ggagtggatg gatctgcggt accacgccta tgcgatggcc gatcccgtct     660

tttatgactc gccgtccagc gacacccggg agaccgacgg ctactccgac gacctccccc     720

tgccggacgg ctgggaacgt cggcgcgtgg gggtctgggt gatgcagggc cacgacgggt     780

tgaccatgcc tgaccagggc tggaagatcc atgtgtcggc cggtctggac aacgcctggc     840

ccgtcctcga actggtcgcc aaatactgcg tcgagcagga aatgcctttc aagttcctgc     900

gcagcagaag gacgttgctg gcccgcagca gcaagtacgc cgaacgcggc ggcagcggaa     960

agttcatcac gatctacccc gccgacgaag gcgctctgga aaagaccctc catgaactcg    1020

gtggaatgct ggaagggcag cccggcccct atatcttgag cgatctgcgc tggcgttcgg    1080

ggccgctgtt cgtgcgctac ggcgctttca aggagaaatt ctgccgggac gggcgcggcg    1140

agatggtgcc cgcgatcgcg cgcccggacg gcgtgctggt gcccgacgcc agggacccgg    1200

tgttccgggt gcccgcgtgg gtggaggtgc ccggcttcct gcgggaggcc atcgacgccc    1260

gggagaacgg gaccgtcgag gacttcccct accggatcga gaaggcgctg cacttctcca    1320

acggcggcgg cctctaccgc gccgtggacg agcgcaccgg ccgcagggtc ctggtgaagg    1380

aggcgcggcc gatggcgggc ctggaccgcg ccgaggacga cgccgtcgtc cggctcgaac    1440

gcgagcacgg cctgctgctc cgcctcgccg acctggactg catccccgac ctggtcgagt    1500

accggagctg gtgggagcac cgcttcctcg tgcgggagta cgtcgagggc gagaccctca    1560

cccaccacat ggtgcgccgc aacccgatgc tgcactacgg cgcgacgccg caggaggtcg    1620

ccggctacac cgagtgggcg ctcggcgtcg tcgaccgggt ggagagcgcg ctcggccggc    1680

tgcacgaacg cggcgtggtc ttcggcgacc tgcaccccgg caacatcatc gtccgggacg    1740

acgactccat cgtgttcgtc gacttcgagc tggtcgccga ggcggaggag gcgacgcacc 1800
cggcgctcgg cgcgcccggc taccaggccc cgcccgacta caccgggttc gccatcgacc 1860
gctacgcgct cggctgcatc aggctcgcgg tcttcacctc cctcaccgcg acgctgcact 1920
gggacgaccg caaggtcgag cagttcctcg acgtgatctg cgagtccttc ccgctgccgc 1980
cggactacgc cgaccggatc cgccgcgacc tcgcccgccc ggcgccggcg gacggcgcgc 2040
cgccgatctg gcgcgagccg acgcccgcca cctggccgga cacgcgggcc gggatcgccg 2100
ccgcgatcct cgacacggcc acgcccgagc gcgccgaccg gctcttcccg ggcgacatcg 2160
agcagttcgc gaccagcgtc ggtgggatcg ggttcggcca cggcgcggcg ggcgtgctgt 2220
gggcgctggc cgaggcgggc gccggccgct tccccgacca cgaggactgg gtgcgggacg 2280
ccgtcgccag ggcgcaacgg ccgccgcccg gcttctacga cggcgtcgcg ggcgtcgccc 2340
acgtcctgga ccggctcggc cgcgccgacg aggcccgcga gctcatggag cacgcgcccg 2400
ccgcgacggg ggcgaccgac aacagcctct accgggggct ggcgggcatc ggcctcaacc 2460
agctccactt cgcccgcgtc acgggcgagg cgtcgttcgc cgcggcggcc gaggagaccg 2520
ccggccgggt ggtcgcgaac ctgcgccgca agacggaggg cgcctaccgg gcgggcctga 2580
tgtacggctc ctccggcccg gccctgttcc tcgtccggat gttcgaggcg acgggcgacg 2640
gccactggct ggacgaggcc gaacgcgccc tgcaccgcga gctggacgcc tgcaagtgga 2700
cgcagaagga cagcacgctc caggtcgacg agggctggcg ggtccttccg tacgtcgcca 2760
ccggcagcgt gggcatcggc atcgcgctgc acgagttcct gcggcaccgc ccggcgccgc 2820
gcttcaccga ggcgcaggag gggatccgga gggcggcggc tcccgcgtac ttcgtgcaga 2880
gcggcctgct caacggacgg tccggcatcc tcgcctacct gctgcacgtc ggggccggcc 2940
gggaggaccc ggtcgtccgg acgcacctgc gcaacctcgg ctggcacgcc gtcccgtacc 3000
ccggccgggg cgaggacgcc ccggcgcccg gcgcgcggcg gaccgcgttc atcggggacc 3060
agctgctgcg cctgtccatg gacctcgcca cgggctccgc cggggtcctg gccacggtcg 3120
aggcggccct gggcggccac cccctgagac tccccttcct ccatccggag gaaggggcga 3180
gcacgcgacc ccgggggagg aggtgaacat ccaccatggc atccatcctt gagctccagg 3240
acctggaggt cgagcgcgcc agctcggccg ccgacagcaa cgccagcgtc tgggagtgct 3300
gcagcacggg cagctgggtt cccttcacct gctgctgacg cccgcacacc gttccaccga 3360
tgagaggtga cagtcccatg gcgtcgatcc tggaactcca gaacctggac gtcgagcacg 3420
cccgcggcga gaaccgctcc gactggagcc tgtgggagtg ctgtagcacg ggaagcctgt 3480
tcgcctgctg ctgaacagcg ctgaccgagg ccgccgaagc ggcacccatg tgaaacgacc 3540
gcccggggcg gtggggactc ccaccgcccc gggcggacca ccatcagcga ggagcacgac 3600
aggaatgcac gccgaccggc tgctcgtcca ggcgctgcgc gccggccccg ggtggacggc 3660
cctcctggtc gccgcgacgc tgctgaacgc ggtgtgcgcg ctcgccctgc ccgccgcgat 3720
cggcgcggtc accgacgcgg tgctcgcccg cgacgcgggc cgcggcgccg gggccccgct 3780
gggctggctg gcggcggcgc tcggcggcgt ggcgctgggc acggcgctgt cccggctcgc 3840

cgaggtgtac tgcggcacgg ccgcgaccag ggagctgcgg acgcgcctgg tcacgcacgt 3900
gctggccctc ggggtgccgg ggacgcgcgc gttcacgccc ggcgacctgg ccagccgcgc 3960
cgtcaccggc gcgccgcagg ccggcgcggt cgcgcggtcg gtggtcagcg cggtcgcggg 4020
gctgctgatg tcggtcggcg gcgtggtcgc gctgtggctg atcgactggc ggctggtggc 4080
ggcggtgggc ggcgcggtcc cgatcgggct gctgctcatg cgcgtcttcg tccgggacgc 4140
ctccgacctg gtcaccgagt accaggaggc gcagggcgag atcgccgcgc ggatgaccga 4200
cgcgctcacc ggcatccgca ccatccgcgc cgccgggacc tggcggcgcg aggccgaccg 4260
ggtcctggcc ccgctgccgg gcctgtcgga ctccgggcgg cggctctggc acgcctacgg 4320
gcggatgcag gggcagggcc gcctgatcgt cccgctggcc gagatcgcgg cgctggccgt 4380
cgccgggcgg ggcgtcctga ccggtcggct gtcggcgggg gagatgctcg cggcggcggg 4440
ctacgccccg atggcgctgg gcctggtcgg ccagatcccc ctgctgctgg ccctggcgcg 4500
gctgcgggcg ggggcgcgcc gcctggccga ggtgctgtcg gtccccgccc ccggcggcgg 4560
cgaccgcgcg ctgccgcccg gccccggcgc gctggtcctg cgcggggtga ccgtgcggac 4620
gccggacggg ccgctgctcg acgcggtcga cctgaccgtc ccgcccggcc ggacggtggc 4680
ggtggtcggc cggtccggcg cgggcaagac gacgctcgcc gccgtggccg ggcgcctcct 4740
cgaccccgac gaggggcggg tgctcctgga cggggtcccg ctgcgcgagc tcgcgcccgc 4800
cgcgctgcgc gcgcaggtcg cctacgcctt cgaacgcccc gacctccagg ggaccacgat 4860
cgccgacgcc atcgcctacg gctgcccgtc cgcgccgccc cgcgcggtcg aacgggcggc 4920
cgcgctcgcg cgggcggacg gcttcgtccg gctgctcccg gcgggcttcg acacgcccgt 4980
cgcggacacg cccctgtccg gcggggaacg gcagcggctc gggctggccc gggccttcgt 5040
ccggaacgcc cgcctgatca tcctcgacga cgcgacctcc agcctcgact cggtgaccga 5100
ggcgcaggtg gcggccgcgc tcgcggaggc cgccgcgacc cgcacccggc tggtggtcgc 5160
gcaccgcgcc gggaccgccg cgcgcgccga cctggtcctg tggctggact ccggccgggt 5220
gcgcgcgctc cgcccgcacc gcgagctgtg ggccgatccg gactaccggg cgatgttcga 5280
gccggcgccg gcggagcgcg cgtgacgggg ggcgcggcgc gctggttcgc cgcccagctg 5340
cggaccgaac ggcgcggcct ggccggggtg ctggcgtggt cggtcgcggc ggcgctcccg 5400
gcgctggtgt ccggacggct gatcgcgctc gccgtcgacc agggcttcct gcgcggacgc 5460
ggcacggtcg gcctggcgtg gctgggggcg ctcgcggcgg cgacggcggt cggcgcgctg 5520
ggcgcgcggc agatcccgcg cgcgctcggc ggggccctgg agccggtgcg cgaccggctc 5580
gtccgccgga tcgtggccgg cgcgctgcgc cgcgcgctgg ccggccggcc cgatccgggc 5640
gtggtcgcga agctgaccga gcagaccgag accgtccggg acaccggcgc ggggctgctg 5700
ctcgggatgc agcaggtcgg catggcggtg gtcgcggccg cgatcgggct gctgctgctc 5760
gcgcccgtca cggcgctgct ggtcctcccg ccggtgctgg cggcgctgct gctgctctcc 5820
cggctgctgc cggagctgat ggcgcggcac cgggccctgc tggcggccga ggaccgcgtc 5880

gcggcgaccg tcggcggcct gaccgcggcg gtgcgcgacg tggtcgcgtg cggggccggg 5940
ccccgggccg gggccgaggc ggcggcggtc ttcgccgagc aggccgcggc cgaacgcgcc 6000
gtcgcccgcg ccgacgcgct gcgcaccgcg ctcggcatcg tcggcggcca gctgtcgctg 6060
gtcgtcctgc tggccgccgc gccctggctg gtggcctccg ggcggctcag ccccggcgag 6120
gtgctcggcg cgctcaccta cgtgacgacc ggcctgcaac cggcgctgcg cgcggccgtg 6180
cagagcaccg ggggttccgg cgtgcggctg gcggtgaccc tgcaacgcct cctggacggc 6240
tccgcggacg aacccgaccc cgcgcccgcc ggagggcgcg tcccgggccg cctcgacctg 6300
tcggtggagg ggctcacgtt cgcctacggc gccggtgccg aaccggtcgt gcgcgacctg 6360
gacctgaccg tgccgcacgg gacgcacctg gccgtcgtcg gccccagcgg gatcggcaag 6420
tcgaccctgg ccgacctgct caccggggtc gccgtcccgc aggcgggccg ggtccgcgtc 6480
ggcggcgtgc cgctcgccga gaccgatccc gcctggaggc gccggtcggt cgcgatcatc 6540
ccgcaggagg cgtacgtctt cagcggcacg ctcgccgaga acctcgccta cctgcgcccg 6600
gacgccgccg aggccgagct cgacgccgcc gtcgccgcgc tgggcctggc gccgctgcgc 6660
gcccggctcg gcgggtacgg ggccgcgctc gggccgcgtt cgggcctgac gccgggggag 6720
cggcagctcg tcgcgctcgc ccgcgtctgg ctcagccccg cgcggatcgt ggtgctggac 6780
gaggcgacct gccacctcga ccccgccgcc gaggcccgcg tggagcacgc cttccgggac 6840
cggcccggca ccctggtcgt catcgcgcac cggctcagct cggcgcgccg cgccgaccgc 6900
gtcctggtga tggacgggac ccgcccccgg ctgggcaccc acggggaact cctccgggac 6960
tccccgctct acgccgacct ggtcggggcc tggaccgttc accccgcggc gcggtagccg 7020
ctcagtctag aagctctgca ttaatgaatc ggccaacgcg cggggagagg cggtttgcgt 7080
attgggcgct cttccgcttc ctcgctcact gactcgctgc gctcggtcgt tcggctgcgg 7140
cgagcggtat cagctcactc aaaggcggta atacggttat ccacagaatc aggggataac 7200
gcaggaaaga acatgtgagc aaaaggccag caaaaggcca ggaaccgtaa aaaggccgcg 7260
tgcctggcgt ttttccatag gctccgcccc cctgacgagc atcacaaaaa tcgacgctca 7320
agtcagaggt ggcgaaaccc gacaggacta taaagatacc aggcgtttcc ccctggaagc 7380
tccctcgtgc gctctcctgt tccgaccctg ccgcttaccg gatacctgtc cgcctttctc 7440
ccttcgggaa gcgtggcgct ttctcatagc tcacgctgta ggtatctcag ttcggtgtag 7500
gtcgttcgct ccaagctggg ctgtgtgcac gaaccccccg ttcagcccga ccgctgcgcc 7560
ttatccggta actatcgtct tgagtccacc cggtaagaca cgacttatcg ccactggcag 7620
cagccactgg taacaggatt agcagagcga ggtatgtagg cggtgctaca gagttcttga 7680
agtggtggcc taactacggc tacactagaa gaacagtatt tggtatctgc gctctgctga 7740
agccagttac cttcggaaaa agagttggta gctcttgatc cggcaaacaa accaccgctg 7800
gtagcggtgg tttttttgtt tgcaagcagc agattacgcg cagaaaaaaa ggatctcaag 7860
aagatccttt gatcttttct acggggtctg acgctcagtg gaacgaaaac tcacgttaag 7920
ggattttggt catgagatta tcaaaaagga tcttcaccta gatcctttta aattaaaaat 7980

gaagttttaa atcaatctaa agtatatatg agtaaacttg gtctgacagt taccaatgct 8040
taatcagtga ggcgcattga gcgtcagcat atcatcagcg agctgaagaa agacaatccc 8100
cgatccgctc cacgtgttgc cccagcaatc agcgcgacct tgcccctcca acgtcatctc 8160
gttctccgct catgagctca gccaatcgac tggcgagcgg catcgcattc ttcgcatccc 8220
gcctctggcg gatgcaggaa gatcaacgga tctcggccca gttgacccag ggctgtcgcc 8280
acaatgtcgc gggagcggat caaccgagca aaggcatgac cgactggacc ttccttctga 8340
aggctcttct ccttgagcca cctgtccgcc aaggcaaagc gctcacagca gtggtcattc 8400
tcgagataat cgacgcgtac caacttgcca tcctgaagaa tggtgcagtg tctcggcacc 8460
ccatagggaa cctttgccat caactcggca agatgcagcg tcgtgttggc atcgtgtccc 8520
acgccgagga gaagtacctg cccatcgagt tcatggacac gggcgaccgg gcttgcaggc 8580
gagtgaggtg gcaggggcaa tggatcagag atgatctgct ctgcctgtgg ccccgctgcc 8640
gcaaaggcaa atggatgggc gctgcgcttt acatttggca ggcgccagaa tgtgtcagag 8700
acaactccaa ggtccggtgt aacgggcgac gtggcaggat cgaacggctc gtcgtccaga 8760
cctgaccacg agggcatgac gagcgtccct cccggaccca gcgcagcacg cagggcctcg 8820
atcagtccaa gtggcccatc ttcgaggggc cggacgctac ggaaggagct gtggaccagc 8880
agcacaccgc cgggggtaac cccaaggttg agaagctgac cgatgagctc ggcttttcgc 8940
cattcgtatt gcacgacatt gcactccacc gctgatgaca tcagtcgatc atagcacgat 9000
caacggcact gttgcaaata gtcggtggtg ataaacttat catccccttt tgctgatgga 9060
gctgcacatg aaccaaaagg atctaggtga agatcctttt tgataatctc atgaccaaaa 9120
tcccttaacg tgagttttcg ttccactgag cgtcagacca ataagggcga cacggaaatg 9180
ttgaatactc atactcttcc tttcaatggg ctgcaggtcg acggatcttt tccgctgcat 9240
aaccctgctt cggggtcatt atagcgattt tttcggtata tccatccttt ttcgcacgat 9300
atacaggatt ttgccaaagg gttcgtgtag actttccttg gtgtatccaa cggcgtcagc 9360
cgggcaggat aggtgaagta ggcccacccg cgagcgggtg ttccttcttc actgtccctt 9420
attcgcacct ggcggtgctc aacgggaatc ctgctctgcg aggctggccg gctaccgccg 9480
gcgtaacaga tgagggcaag cggatggctg atgaaaccaa gccaaccagg aagggcagcc 9540
cacctatcaa ggtgtactgc cttccagacg aacgaagagc gattgaggaa aaggcggcgg 9600
cggccggcat gagcctgtcg gcctacctgc tggccgtcgg ccagggctac aaaatcacgg 9660
gcgtcgtgga ctatgagcac gtccgcgagc tggcccgcat caatggcgac ctgggccgcc 9720
tgggcggcct gctgaaactc tggctcaccg acgacccgcg cacggcgcgg ttcggtgatg 9780
ccacgatcct cgccctgctg gcgaagatcg aagagaagca ggacgagctt ggcaaggtca 9840
tgatgggcgt ggtccgcccg agggcagagc catgactttt ttagccgcta aaacggccgg 9900
ggggtgcgcg tgattgccaa gcacgtcccc atgcgctcca tcaagaagag cgacttcgcg 9960
gagctggtga agtacatcac cgacgagcaa ggcaagaccg atccccatta acgcttacaa 10020

tttccattcg ccattcaggc tgcgcaactg ttgggaaggg cgatcggtgc gggcctcttc 10080
gctattacgc cagagcttgg ccggatctaa agttttgtcg tctttccaga cgttagtaaa 10140
tgaattttct gtatgaggtt ttgctaaaca actttcaaca gtttcagcgg agtgagaata 10200
gaaaggaaca actaaaggaa ttgcgaataa taattttttc acgttgaaaa tctccaaaaa 10260
aaaaggctcc aaaaggagcc tttaattgta tcggtttatc agcttgcttt cgaggtgaat 10320
ttcttaaaca gcttgatacc gatagttgcg ccgacaatga caacaaccat cgcccacgca 10380
taaccgatat attcggtcgc tgaggcttgc agggagtcaa aggccgcttt tgcgggatca 10440
tcactgacga atcgaggtcg aggaaccgag cgtccgagga acagaggcgc ttatcggttg 10500
gccgcgagat tcctgtcgat cctctcgtgc agcgcgattc cgagggaaac ggaaacgttg 10560
agagactcgg tctggctcat catggggatg gaaaccgagg cggaagacgc ctcctcgaac 10620
aggtcggaag gcccaccctt ttcgctgccg aacagcaagg ccagccgatc cggattgtcc 10680
ccgagttcct tcacggaaat gtcgccatcc gccttgagcg tcatcagctg cataccgctg 10740
tcccgaatga aggcgatggc ctcctcgcga ccggagagaa cgacgggaag ggagaagacg 10800
taacctcggc tggccctttg gagacgccgg tccgcgatgc tggtgatgtc actgtcgacc 10860
aggatgatcc ccgacgctcc gagcgcgagc gacgtgcgta ctatcgcgcc gatgttcccg 10920
acgatcttca ccccgtcgag aacgacgacg tccccacgcc ggctcgcgat atcgccgaac 10980
ctggccgggc gagggacgcg ggcgatgccg aatgtcttgg ccttccgctc ccccttgaac 11040
aactggttga cgatcgagga gtcgatgagg cggaccggta tgttctgccg cccgcacaga 11100
tccagcaact cagatggaaa aggactgctg tcgctgccgt agacctcgat gaactccacc 11160
ccggccgcga tgctgtgcat gaggggctcg acgtcctcga tcaacgttgt ctttatgttg 11220
gatcgcgacg gcttggtgac atcgatgatc cgctgcaccg cgggatcgga cggatttgcg 11280
atggtgtcca actcagtcat ggtcgtccta ccggctgctg tgttcagtga cgcgattcct 11340
ggggtgtgac accctacgcg acgatggcgg atggctgccc tgaccggcaa tcaccaacgc 11400
aaggggaagt cgtcgctctc tggcaaagct ccccgctctt ccccgtccgg gacccgcgcg 11460
gtcgatcccc gcatatgaag tattcgcctt gatcagtccc ggtggacgcg ccagcggccc 11520
gccggagcga cggactcccc gacctcgatc gtgtcgccct gagcgtccac gtagacgttg 11580
cgtgagagca ggactgggcc gccgccgacc gcaccgccct caccaccgac cgcgaccgcg 11640
ccatggccgc cgccgacggc ctggtcgccg ccgccgcccg ccggttcggc gcctgacccg 11700
accaaccccc gcggggcgcc ggcacttcgt gctggcgccc cgcccccacc caccaggaga 11760
ccgaccatga ccgacttcga cggacgcctg accgagggga ccgtgaacct ggtccaggac 11820
cccaacggcg gtggctggtc cgcccactgc gctgagcccg gttgcgactg ggccgacttc 11880
gccggaccgc tcggcttcca gggcctcgtg gccatcgctc gccgacacac gcactgaccg 11940
cacgtcaaag ccccgccgga tcaccggcgg ggctctcttc ggccctccaa gtcacaccag 12000
ccccaagggg cgtcgggagt ggcggaggga acctctggcc cgattggtgc caggattccc 12060
accagaccaa agagcaacgg gccggacttc gcacctccga cccgtccgct cccagactcg 12120

cgccccttag ccgggcgaga caggaacgtt gctcgtgccc agagtacgga gcgatgccga 12180

ggcattgcca gatcggcccg ccgggccccg ctgccactgc gggaccgcaa ttgcccacac 12240

accgggcaaa cggccgcgta tctactgctc agaccgctgc cggatggcag cgaagcgggc 12300

gatcgcgcgt gtgacgcgag atgccgcccg aggcaaaagc gaacaccttg ggaaagaaac 12360

aacagagttt cccgcacccc tccgacctgc ggtttctccg gacggggtgg atggggagag 12420

cccgagaggc gacagcctct cggaagtagg aagcacgtcg cggagcgacg ctgcccgact 12480

gcggaaagcc gcccggtaca gccgccgccg gacgctgtgg cggatcagcg gggacgccgc 12540

gtgcaagggc tgcggccgcg ccctgatgga ccctgcctcc ggcgtaatcg tcgcccagac 12600

ggcggccgga acgtccgtgg tcctgggcct gatgcggtgc gggcggatct ggctctgccc 12660

ggtctgcgcc gccacgatcc ggcacaagcg ggccgaggag atcaccgccg ccgtggtcga 12720

gtggatcaag cgcgggggga ccgcctacct ggtcaccttc acggcccgcc atgggcacac 12780

ggaccggctc gcggacctca tggacgccct ccagggcacc cggaagacgc cggacagccc 12840

ccggcggccg ggcgcctacc agcgactgat cacgggcggc acgtgggccg gacgccgggc 12900

caaggacggg caccgggccg ccgaccgcga gggcatccga gaccggatcg ggtacgtcgg 12960

catgatccgc gcgaccgaag tcaccgtggg gcagatcaac ggctggcacc cgcacatcca 13020

cgcgatcgtc ctggtcggcg gccggaccga gggggagcgg tccgcgaagc agatcgtcgc 13080

caccttcgag ccgaccggcg ccgcgctcga cgagtggcag gggcactggc ggtccgtgtg 13140

gaccgccgcc ctgcgcaagg tcaaccccgc cttcacgccc gacgaccggc acggcgtcga 13200

cttcaagcgg ctggagaccg agcgcgacgc caacgacctc gccgagtaca tcgccaagac 13260

ccaggacggg aaggcgcccg ccctcgaact cgcccgcgcc gacctcaaga cggcgaccgg 13320

cgggaacgtc gccccgttcg aactcctcgg acggatcggg gacctgaccg gcggcatgac 13380

cgaggacgac gccgccgggg tcggctcgct ggagtggaac ctctcgcgct ggcacgagta 13440

cgagcgggca acccggggac gccgggccat cgaatggacc cgctacctgc ggcagatgct 13500

cgggctcgac ggcggcgaca ccgaggccga cgacctcgat ctgctcctgg cggccgacgc 13560

cgacggcggg gagctgcggg ccggggtcgc cgtgaccgag gacggatggc acgcggtcac 13620

ccgccgcgcc ctcgacctcg aggcgacccg ggccgccgaa ggcaaggacg gcaacgagga 13680

ttcggcggcc gtgggcgaac gggtgcggga ggtcctggcg ctggccgacg cggccgacac 13740

agtggtggtg ctcacggcgg gggaggtggc cgaggcgtac gccgacatgc tcgccgccc 13799

<210> 143
<211> 13799
<212> DNA
<213> Artificial Sequence

<220>
<223> Vector pLab


<220>
<221> misc_feature

<222>  (68)..(13746)
<223>  oriT

<220>
<221>  misc_feature
<222>  (464)..(998)
<223>  aac(3) IV'

<220>
<221>  misc_feature
<222>  (999)..(1240)
<223>  aac(3) IV

<220>
<221>  misc_feature
<222>  (4116)..(2401)
<223>  labT2

<220>
<221>  misc_feature
<222>  (5813)..(4113)
<223>  labT1

<220>
<221>  misc_feature
<222>  (6040)..(5924)
<223>  labA2

<220>
<221>  misc_feature
<222>  (6202)..(6080)
<223>  labA1

<220>
<221>  misc_feature
<222>  (8800)..(6212)
<223>  labkc

<220>
<221>  misc_feature
<222>  (8881)..(8837)
<223>  ermE

<220>
<221>  misc_feature
<222>  (10651)..(9535)
<223>  rep pIJ101   (replicon pIJ101)

<220>
<221>  misc_feature
<222>  (11917)..(12726)
<223>  tsr (Thiostrepton Resistence gene)

<400>  143
   ggacagtgaa gaaggaacac ccgctcgcgg gtgggcctac ttcacctatc ctgcccggct      60
   gacgccgttg gatacaccaa ggaaagtcta cacgaaccct ttggcaaaat cctgtatatc     120
   gtgcgaaaaa ggatggatat accgaaaaaa tcgctataat gaccccgaag cagggttatg     180
   cagcggaaaa gatccgtcga cctgcagccc attgaaagga agagtatgag tattcaacat     240
   ttccgtgtcg cccttattgg tctgacgctc agtggaacga aaactcacgt taagggattt     300
   tggtcatgag attatcaaaa aggatcttca cctagatcct tttggttcat gtgcagctcc     360
   atcagcaaaa ggggatgata agtttatcac caccgactat ttgcaacagt gccgttgatc     420
   gtgctatgat cgactgatgt catcagcggt ggagtgcaat gtcgtgcaat acgaatggcg     480
   aaaagccgag ctcatcggtc agcttctcaa ccttggggtt acccccggcg gtgtgctgct 540
   ggtccacagc tccttccgta gcgtccggcc cctcgaagat gggccacttg gactgatcga 600
   ggccctgcgt gctgcgctgg gtccgggagg gacgctcgtc atgccctcgt ggtcaggtct 660
   ggacgacgag ccgttcgatc ctgccacgtc gcccgttaca ccggaccttg gagttgtctc 720
   tgacacattc tggcgcctgc caaatgtaaa gcgcagcgcc catccatttg cctttgcggc 780
   agcggggcca caggcagagc agatcatctc tgatccattg cccctgccac ctcactcgcc 840
   tgcaagcccg gtcgcccgtg tccatgaact cgatgggcag gtacttctcc tcggcgtggg 900
   acacgatgcc aacacgacgc tgcatcttgc cgagttgatg gcaaaggttc cctatggggt 960
   gccgagacac tgcaccattc ttcaggatgg caagttggta cgcgtcgatt atctcgagaa 1020
   tgaccactgc tgtgagcggt ttgccttggc ggacaggtgg ctcaaggaga agagccttca 1080
   gaaggaaggt ccagtcggtc atgcctttgc tcggttgatc cgctcccgcg acattgtggc 1140
   gacagccctc ggtcaactgg gccgagatcc gttgatcttc ctgcatccgc cagaggcggg 1200
   atgcgaagaa tgcgatgccg ctcgccagtc gattggctga gctcatgagc ggagaacgag 1260
   atgacgttgg aggggcaagg tcgcgctgat tgctggggca acacgtggag cggatcgggg 1320
   attgtctttc ttcagctcgc tgatgatatg ctgacgctca atgcgcctca ctgattaagc 1380
   attggtaact gtcagaccaa gtttactcat atatacttta gattgattta aaacttcatt 1440
   tttaatttaa aaggatctag gtgaagatcc tttttgataa tctcatgacc aaaatccctt 1500
   aacgtgagtt ttcgttccac tgagcgtcag accccgtaga aaagatcaaa ggatcttctt 1560
   gagatccttt ttttctgcgc gtaatctgct gcttgcaaac aaaaaaacca ccgctaccag 1620
   cggtggtttg tttgccggat caagagctac caactctttt tccgaaggta actggcttca 1680
   gcagagcgca gataccaaat actgttcttc tagtgtagcc gtagttaggc caccacttca 1740
   agaactctgt agcaccgcct acatacctcg ctctgctaat cctgttacca gtggctgctg 1800
   ccagtggcga taagtcgtgt cttaccgggt ggactcaaga cgatagttac cggataaggc 1860
   gcagcggtcg ggctgaacgg ggggttcgtg cacacagccc agcttggagc gaacgaccta 1920
   caccgaactg agatacctac agcgtgagct atgagaaagc gccacgcttc ccgaagggag 1980
   aaaggcggac aggtatccgg taagcggcag ggtcggaaca ggagagcgca cgagggagct 2040
   tccaggggga aacgcctggt atctttatag tcctgtcggg tttcgccacc tctgacttga 2100
   gcgtcgattt ttgtgatgct cgtcaggggg gcggagccta tggaaaaacg ccagcaacgc 2160
   ggccttttta cggttcctgg ccttttgctg gccttttgct cacatgttct ttcctgcgtt 2220
   atcccctgat tctgtggata accgtattac cgcctttgag tgagctgata ccgctcgccg 2280
   cagccgaacg accgagcgca gcgagtcagt gagcgaggaa gcggaagagc gcccaatacg 2340
   caaaccgcct ctccccgcgc gttggccgat tcattaatgc agagcttcta gactgagcgg 2400
   ctaccgcgcc gcggggtgaa cggtccaggc cccgaccagg tcggcgtaga gcggggagtc 2460
   ccggaggagt tccccgtggg tgcccagccg ggggcgggtc ccgtccatca ccaggacgcg 2520
   gtcggcgcgg cgcgccgagc tgagccggtg cgcgatgacg accagggtgc cgggccggtc 2580
   ccggaaggcg tgctccacgc gggcctcggc ggcggggtcg aggtggcagg tcgcctcgtc 2640
   cagcaccacg atccgcgcgg ggctgagcca gacgcgggcg agcgcgacga gctgccgctc 2700
   ccccggcgtc aggcccgaac gcggcccgag cgcggccccg tacccgccga gccgggcgcg 2760
   cagcggcgcc aggcccagcg cggcgacggc ggcgtcgagc tcggcctcgg cggcgtccgg 2820
   gcgcaggtag gcgaggttct cggcgagcgt gccgctgaag acgtacgcct cctgcgggat 2880
   gatcgcgacc gaccggcgcc tccaggcggg atcggtctcg gcgagcggca cgccgccgac 2940
   gcggacccgg cccgcctgcg ggacggcgac cccggtgagc aggtcggcca gggtcgactt 3000
   gccgatcccg ctggggccga cgacggccag gtgcgtcccg tgcggcacgg tcaggtccag 3060
   gtcgcgcacg accggttcgg caccggcgcc gtaggcgaac gtgagcccct ccaccgacag 3120
   gtcgaggcgg cccgggacgc gccctccggc gggcgcgggg tcgggttcgt ccgcggagcc 3180
   gtccaggagg cgttgcaggg tcaccgccag ccgcacgccg gaaccccggg tgctctgcac 3240
   ggccgcgcgc agcgccggtt gcaggccggt cgtcacgtag gtgagcgcgc cgagcacctc 3300
   gccggggctg agccgcccgg aggccaccag ccagggcgcg gcggccagca ggacgaccag 3360
   cgacagctgg ccgccgacga tgccgagcgc ggtgcgcagc gcgtcggcgc gggcgacggc 3420
   gcgttcggcc gcggcctgct cggcgaagac cgccgccgcc tcggccccgg cccggggccc 3480
   ggccccgcac gcgaccacgt cgcgcaccgc cgcggtcagg ccgccgacgg tcgccgcgac 3540
   gcggtcctcg gccgccagca gggcccggtg ccgcgccatc agctccggca gcagccggga 3600
   gagcagcagc agcgccgcca gcaccggcgg gaggaccagc agcgccgtga cgggcgcgag 3660
   cagcagcagc ccgatcgcgg ccgcgaccac cgccatgccg acctgctgca tcccgagcag 3720
   cagccccgcg ccggtgtccc ggacggtctc ggtctgctcg gtcagcttcg cgaccacgcc 3780
   cggatcgggc cggccggcca gcgcgcggcg cagcgcgccg gccacgatcc ggcggacgag 3840
   ccggtcgcgc accggctcca gggccccgcc gagcgcgcgc gggatctgcc gcgcgcccag 3900
   cgcgccgacc gccgtcgccg ccgcgagcgc ccccagccac gccaggccga ccgtgccgcg 3960
   tccgcgcagg aagccctggt cgacggcgag cgcgatcagc cgtccggaca ccagcgccgg 4020
   gagcgccgcc gcgaccgacc acgccagcac cccggccagg ccgcgccgtt cggtccgcag 4080
   ctgggcggcg aaccagcgcg ccgcgccccc cgtcacgcgc gctccgccgg cgccggctcg 4140
   aacatcgccc ggtagtccgg atcggcccac agctcgcggt gcgggcggag cgcgcgcacc 4200
   cggccggagt ccagccacag gaccaggtcg gcgcgcgcgg cggtcccggc gcggtgcgcg 4260
   accaccagcc gggtgcgggt cgcggcggcc tccgcgagcg cggccgccac ctgcgcctcg 4320
   gtcaccgagt cgaggctgga ggtcgcgtcg tcgaggatga tcaggcgggc gttccggacg 4380
   aaggcccggg ccagcccgag ccgctgccgt tccccgccgg acaggggcgt gtccgcgacg 4440
   ggcgtgtcga agcccgccgg gagcagccgg acgaagccgt ccgcccgcgc gagcgcggcc 4500
   gcccgttcga ccgcgcgggg cggcgcggac gggcagccgt aggcgatggc gtcggcgatc 4560
   gtggtcccct ggaggtcggg gcgttcgaag gcgtaggcga cctgcgcgcg cagcgcggcg 4620
   ggcgcgagct cgcgcagcgg gaccccgtcc aggagcaccc gcccctcgtc ggggtcgagg 4680
   aggcgcccgg ccacggcggc gagcgtcgtc ttgcccgcgc cggaccggcc gaccaccgcc 4740
   accgtccggc cgggcgggac ggtcaggtcg accgcgtcga gcagcggccc gtccggcgtc 4800
   cgcacggtca ccccgcgcag gaccagcgcg ccggggccgg gcggcagcgc gcggtcgccg 4860
   ccgccggggg cggggaccga cagcacctcg gccaggcggc gcgcccccgc ccgcagccgc 4920
   gccagggcca gcagcagggg gatctggccg accaggccca gcgccatcgg ggcgtagccc 4980
   gccgccgcga gcatctcccc cgccgacagc cgaccggtca ggacgccccg cccggcgacg 5040
   gccagcgccg cgatctcggc cagcgggacg atcaggcggc cctgcccctg catccgcccg 5100
   taggcgtgcc agagccgccg cccggagtcc gacaggcccg gcagcggggc caggacccgg 5160
   tcggcctcgc gccgccaggt cccggcggcg cggatggtgc ggatgccggt gagcgcgtcg 5220
   gtcatccgcg cggcgatctc gccctgcgcc tcctggtact cggtgaccag gtcggaggcg 5280
   tcccggacga agacgcgcat gagcagcagc ccgatcggga ccgcgccgcc caccgccgcc 5340
   accagccgcc agtcgatcag ccacagcgcg accacgccgc cgaccgacat cagcagcccc 5400
   gcgaccgcgc tgaccaccga ccgcgcgacc gcgccggcct gcggcgcgcc ggtgacggcg 5460
   cggctggcca ggtcgccggg cgtgaacgcg cgcgtccccg gcaccccgag ggccagcacg 5520
   tgcgtgacca ggcgcgtccg cagctccctg gtcgcggccg tgccgcagta cacctcggcg 5580
   agccgggaca gcgccgtgcc cagcgccacg ccgccgagcg ccgccgccag ccagcccagc 5640
   ggggcccggg cgccgcggcc cgcgtcgcgg gcgagcaccg cgtcggtgac cgcgccgatc 5700
   gcggcgggca gggcgagcgc gcacaccgcg ttcagcagcg tcgcggcgac caggagggcc 5760
   gtccacccgg ggccggcgcg cagcgcctgg acgagcagcc ggtcggcgtg cattcctgtc 5820
   gtgctcctcg ctgatggtgg tccgcccggg gcggtgggag tccccaccgc cccgggcggt 5880
   cgtttcacat gggtgccgct tcggcggcct cggtcagcgc tgttcagcag caggcgaaca 5940
   ggcttcccgt gctacagcac tcccacaggc tccagtcgga gcggttctcg ccgcgggcgt 6000
   gctcgacgtc caggttctgg agttccagga tcgacgccat gggactgtca cctctcatcg 6060
   gtggaacggt gtgcgggcgt cagcagcagg tgaagggaac ccagctgccc gtgctgcagc 6120
   actcccagac gctggcgttg ctgtcggcgg ccgagctggc gcgctcgacc tccaggtcct 6180
   ggagctcaag gatggatgcc atggtggatg ttcacctcct cccccggggt cgcgtgctcg 6240
   ccccttcctc cggatggagg aaggggagtc tcagggggtg gccgcccagg gccgcctcga 6300
   ccgtggccag gaccccggcg gagcccgtgg cgaggtccat ggacaggcgc agcagctggt 6360
   ccccgatgaa cgcggtccgc cgcgcgccgg gcgccggggc gtcctcgccc cggccggggt 6420
   acgggacggc gtgccagccg aggttgcgca ggtgcgtccg gacgaccggg tcctcccggc 6480
   cggccccgac gtgcagcagg taggcgagga tgccggaccg tccgttgagc aggccgctct 6540
   gcacgaagta cgcgggagcc gccgccctcc ggatcccctc ctgcgcctcg gtgaagcgcg 6600
   gcgccgggcg gtgccgcagg aactcgtgca gcgcgatgcc gatgcccacg ctgccggtgg 6660
   cgacgtacgg aaggacccgc cagccctcgt cgacctggag cgtgctgtcc ttctgcgtcc 6720
   acttgcaggc gtccagctcg cggtgcaggg cgcgttcggc ctcgtccagc cagtggccgt 6780
   cgcccgtcgc ctcgaacatc cggacgagga acagggccgg gccggaggag ccgtacatca 6840
   ggcccgcccg gtaggcgccc tccgtcttgc ggcgcaggtt cgcgaccacc cggccggcgg 6900
   tctcctcggc cgccgcggcg aacgacgcct cgcccgtgac gcgggcgaag tggagctggt 6960
   tgaggccgat gcccgccagc ccccggtaga ggctgttgtc ggtcgccccc gtcgcggcgg 7020
   gcgcgtgctc catgagctcg cgggcctcgt cggcgcggcc gagccggtcc aggacgtggg 7080
   cgacgcccgc gacgccgtcg tagaagccgg gcggcggccg ttgcgccctg gcgacggcgt 7140
   cccgcaccca gtcctcgtgg tcggggaagc ggccggcgcc cgcctcggcc agcgcccaca 7200
   gcacgcccgc cgcgccgtgg ccgaacccga tcccaccgac gctggtcgcg aactgctcga 7260
   tgtcgcccgg gaagagccgg tcggcgcgct cgggcgtggc cgtgtcgagg atcgcggcgg 7320
   cgatcccggc ccgcgtgtcc ggccaggtgg cgggcgtcgg ctcgcgccag atcggcggcg 7380
   cgccgtccgc cggcgccggg cgggcgaggt cgcggcggat ccggtcggcg tagtccggcg 7440
   gcagcgggaa ggactcgcag atcacgtcga ggaactgctc gaccttgcgg tcgtcccagt 7500
   gcagcgtcgc ggtgagggag gtgaagaccg cgagcctgat gcagccgagc gcgtagcggt 7560
   cgatggcgaa cccggtgtag tcgggcgggg cctggtagcc gggcgcgccg agcgccgggt 7620
   gcgtcgcctc ctccgcctcg gcgaccagct cgaagtcgac gaacacgatg gagtcgtcgt 7680
   cccggacgat gatgttgccg gggtgcaggt cgccgaagac cacgccgcgt tcgtgcagcc 7740
   ggccgagcgc gctctccacc cggtcgacga cgccgagcgc ccactcggtg tagccggcga 7800
   cctcctgcgg cgtcgcgccg tagtgcagca tcgggttgcg gcgcaccatg tggtgggtga 7860
   gggtctcgcc ctcgacgtac tcccgcacga ggaagcggtg ctcccaccag ctccggtact 7920
   cgaccaggtc ggggatgcag tccaggtcgg cgaggcggag cagcaggccg tgctcgcgtt 7980
   cgagccggac gacggcgtcg tcctcggcgc ggtccaggcc cgccatcggc cgcgcctcct 8040
   tcaccaggac cctgcggccg gtgcgctcgt ccacggcgcg gtagaggccg ccgccgttgg 8100
   agaagtgcag cgccttctcg atccggtagg ggaagtcctc gacggtcccg ttctcccggg 8160
   cgtcgatggc ctcccgcagg aagccgggca cctccaccca cgcgggcacc cggaacaccg 8220
   ggtccctggc gtcgggcacc agcacgccgt ccgggcgcgc gatcgcgggc accatctcgc 8280
   cgcgcccgtc ccggcagaat ttctccttga aagcgccgta gcgcacgaac agcggccccg 8340
   aacgccagcg cagatcgctc aagatatagg ggccgggctg cccttccagc attccaccga 8400
   gttcatggag ggtcttttcc agagcgcctt cgtcggcggg gtagatcgtg atgaactttc 8460
   cgctgccgcc gcgttcggcg tacttgctgc tgcgggccag caacgtcctt ctgctgcgca 8520
   ggaacttgaa aggcatttcc tgctcgacgc agtatttggc gaccagttcg aggacgggcc 8580
   aggcgttgtc cagaccggcc gacacatgga tcttccagcc ctggtcaggc atggtcaacc 8640
   cgtcgtggcc ctgcatcacc cagaccccca cgcgccgacg ttcccagccg tccggcaggg 8700
   ggaggtcgtc ggagtagccg tcggtctccc gggtgtcgct ggacggcgag tcataaaaga 8760
   cgggatcggc catcgcatag gcgtggtacc gcagatccat ccactcctcc aagaattcga 8820
   tatcaagctt atcgataccg tcgatcctac caaccggcac gattgtgccc acaacagcat 8880
   cgcggtgcca cgtgtggacc gcgtcggtca gatcctcccc gcacctctcg ccagccgtca 8940
   agatcgaccg cgtgcacctg cgatcgccga tcaaccgcga ctagcatcgg gcgcaagccg 9000
   ccactcgaac ggacactcgc atggacgtcc ccttcctgga cctgcaagcc gcgtacctcg 9060
   aactccggtc cgacatcgac caggcgtgcc ggcgcgtgct cgggtcgggc tggacctcga 9120
   ggggggcccc ggtaccgcct gcacgaagtg ccggcggggc cgaccccggg cgagtaatcc 9180
   caggattact cccgcggctt cgaccccggc cgccgtcgcc gcgtacgtca ccgacccccg 9240
   ccgtacgtca ccgggatgac gtacggcggg ggggagcgag ttagtgcgaa gtgggcccac 9300
   ttgcgagccg ggcgatgtgc cgggcggccc gctcctggcg gtcgtcggcg tcgtcgtcct 9360
   ggtcgtcgtc ctgctctcgc cgtcggcgtg cagttgcttc ctcgcggcgc tgggcgaggg 9420
   cggcgagcat gtcggcgtac gcctcggcca cctcccccgc cgtgagcacc accactgtgt 9480
   cggccgcgtc ggccagcgcc aggacctccc gcacccgttc gcccacggcc gccgaatcct 9540
   cgttgccgtc cttgccttcg gcggcccggg tcgcctcgag gtcgagggcg cggcgggtga 9600
   ccgcgtgcca tccgtcctcg gtcacggcga ccccggcccg cagctccccg ccgtcggcgt 9660
   cggccgccag gagcagatcg aggtcgtcgg cctcggtgtc gccgccgtcg agcccgagca 9720
   tctgccgcag gtagcgggtc cattcgatgg cccggcgtcc ccgggttgcc cgctcgtact 9780
   cgtgccagcg cgagaggttc cactccagcg agccgacccc ggcggcgtcg tcctcggtca 9840
   tgccgccggt caggtccccg atccgtccga ggagttcgaa cggggcgacg ttcccgccgg 9900
   tcgccgtctt gaggtcggcg cgggcgagtt cgagggcggg cgccttcccg tcctgggtct 9960
   tggcgatgta ctcggcgagg tcgttggcgt cgcgctcggt ctccagccgc ttgaagtcga 10020
   cgccgtgccg gtcgtcgggc gtgaaggcgg ggttgacctt gcgcagggcg gcggtccaca 10080
   cggaccgcca gtgcccctgc cactcgtcga gcgcggcgcc ggtcggctcg aaggtggcga 10140
   cgatctgctt cgcggaccgc tccccctcgg tccggccgcc gaccaggacg atcgcgtgga 10200
   tgtgcgggtg ccagccgttg atctgcccca cggtgacttc ggtcgcgcgg atcatgccga 10260
   cgtacccgat ccggtctcgg atgccctcgc ggtcggcggc ccggtgcccg tccttggccc 10320
   ggcgtccggc ccacgtgccg cccgtgatca gtcgctggta ggcgcccggc cgccgggggc 10380
   tgtccggcgt cttccgggtg ccctggaggg cgtccatgag gtccgcgagc cggtccgtgt 10440
   gcccatggcg ggccgtgaag gtgaccaggt aggcggtccc cccgcgcttg atccactcga 10500
   ccacggcggc ggtgatctcc tcggcccgct tgtgccggat cgtggcggcg cagaccgggc 10560
   agagccagat ccgcccgcac cgcatcaggc ccaggaccac ggacgttccg gccgccgtct 10620
   gggcgacgat tacgccggag gcagggtcca tcagggcgcg gccgcagccc ttgcacgcgg 10680
   cgtccccgct gatccgccac agcgtccggc ggcggctgta ccgggcggct ttccgcagtc 10740
   gggcagcgtc gctccgcgac gtgcttccta cttccgagag gctgtcgcct ctcgggctct 10800
   ccccatccac cccgtccgga gaaaccgcag gtcggagggg tgcgggaaac tctgttgttt 10860
   ctttcccaag gtgttcgctt ttgcctcggg cggcatctcg cgtcacacgc gcgatcgccc 10920
   gcttcgctgc catccggcag cggtctgagc agtagatacg cggccgtttg cccggtgtgt 10980
   gggcaattgc ggtcccgcag tggcagcggg gcccggcggg ccgatctggc aatgcctcgg 11040
   catcgctccg tactctgggc acgagcaacg ttcctgtctc gcccggctaa ggggcgcgag 11100
   tctgggagcg gacgggtcgg aggtgcgaag tccggcccgt tgctctttgg tctggtggga 11160
   atcctggcac caatcgggcc agaggttccc tccgccactc ccgacgcccc ttggggctgg 11220
   tgtgacttgg agggccgaag agagccccgc cggtgatccg gcggggcttt gacgtgcggt 11280
   cagtgcgtgt gtcggcgagc gatggccacg aggccctgga agccgagcgg tccggcgaag 11340
   tcggcccagt cgcaaccggg ctcagcgcag tgggcggacc agccaccgcc gttggggtcc 11400
   tggaccaggt tcacggtccc ctcggtcagg cgtccgtcga agtcggtcat ggtcggtctc 11460
   ctggtgggtg ggggcggggc gccagcacga agtgccggcg ccccgcgggg gttggtcggg 11520
   tcaggcgccg aaccggcggg cggcggcggc gaccaggccg tcggcggcgg ccatggcgcg 11580
   gtcgcggtcg gtggtgaggg cggtgcggtc ggcggcggcc cagtcctgct ctcacgcaac 11640
   gtctacgtgg acgctcaggg cgacacgatc gaggtcgggg agtccgtcgc tccggcgggc 11700
   cgctggcgcg tccaccggga ctgatcaagg cgaatacttc atatgcgggg atcgaccgcg 11760
   cgggtcccgg acggggaaga gcggggagct ttgccagaga gcgacgactt ccccttgcgt 11820
   tggtgattgc cggtcagggc agccatccgc catcgtcgcg tagggtgtca caccccagga 11880
   atcgcgtcac tgaacacagc agccggtagg acgaccatga ctgagttgga caccatcgca 11940
   aatccgtccg atcccgcggt gcagcggatc atcgatgtca ccaagccgtc gcgatccaac 12000
   ataaagacaa cgttgatcga ggacgtcgag cccctcatgc acagcatcgc ggccggggtg 12060
   gagttcatcg aggtctacgg cagcgacagc agtccttttc catctgagtt gctggatctg 12120
   tgcgggcggc agaacatacc ggtccgcctc atcgactcct cgatcgtcaa ccagttgttc 12180
   aagggggagc ggaaggccaa gacattcggc atcgcccgcg tccctcgccc ggccaggttc 12240
   ggcgatatcg cgagccggcg tggggacgtc gtcgttctcg acggggtgaa gatcgtcggg 12300
   aacatcggcg cgatagtacg cacgtcgctc gcgctcggag cgtcggggat catcctggtc 12360
   gacagtgaca tcaccagcat cgcggaccgg cgtctccaaa gggccagccg aggttacgtc 12420
   ttctcccttc ccgtcgttct ctccggtcgc gaggaggcca tcgccttcat tcgggacagc 12480
   ggtatgcagc tgatgacgct caaggcggat ggcgacattt ccgtgaagga actcggggac 12540
   aatccggatc ggctggcctt gctgttcggc agcgaaaagg gtgggccttc cgacctgttc 12600
   gaggaggcgt cttccgcctc ggtttccatc cccatgatga gccagaccga gtctctcaac 12660
   gtttccgttt ccctcggaat cgcgctgcac gagaggatcg acaggaatct cgcggccaac 12720
   cgataagcgc ctctgttcct cggacgctcg gttcctcgac ctcgattcgt cagtgatgat 12780
   cccgcaaaag cggcctttga ctccctgcaa gcctcagcga ccgaatatat cggttatgcg 12840
   tgggcgatgg ttgttgtcat tgtcggcgca actatcggta tcaagctgtt taagaaattc 12900
   acctcgaaag caagctgata aaccgataca attaaaggct ccttttggag cctttttttt 12960
   tggagatttt caacgtgaaa aaattattat tcgcaattcc tttagttgtt cctttctatt 13020
   ctcactccgc tgaaactgtt gaaagttgtt tagcaaaacc tcatacagaa aattcattta 13080
   ctaacgtctg gaaagacgac aaaactttag atccggccaa gctctggcgt aatagcgaag 13140
   aggcccgcac cgatcgccct tcccaacagt tgcgcagcct gaatggcgaa tggaaattgt 13200
   aagcgttaat ggggatcggt cttgccttgc tcgtcggtga tgtacttcac cagctccgcg 13260
   aagtcgctct tcttgatgga gcgcatgggg acgtgcttgg caatcacgcg caccccccgg 13320
   ccgttttagc ggctaaaaaa gtcatggctc tgccctcggg cggaccacgc ccatcatgac 13380
   cttgccaagc tcgtcctgct tctcttcgat cttcgccagc agggcgagga tcgtggcatc 13440
   accgaaccgc gccgtgcgcg ggtcgtcggt gagccagagt ttcagcaggc cgcccaggcg 13500
   gcccaggtcg ccattgatgc gggccagctc gcggacgtgc tcatagtcca cgacgcccgt 13560
   gattttgtag ccctggccga cggccagcag gtaggccgac aggctcatgc cggccgccgc 13620
   cgccttttcc tcaatcgctc ttcgttcgtc tggaaggcag tacaccttga taggtgggct 13680
   gcccttcctg gttggcttgg tttcatcagc catccgcttg ccctcatctg ttacgccggc 13740
   ggtagccggc cagcctcgca gagcaggatt cccgttgagc accgccaggt gcgaataag 13799

<210> 144
<211> 15787
<212> DNA
<213> Artificial Sequence

<220>
<223> Vector pLab Amp


<220>
<221> misc_feature
<222> (594)..(638)
<223> ermE

<220>
<221> misc_feature
<222> (675)..(3188)
<223> LabKC

<220>
<221> misc_feature
<222> (3692)..(4552)
<223> AmpR

<220>
<221> misc_feature
<222> (5650)..(7350)
<223> labT1

<220>
<221> misc_feature
<222> (7347)..(9062)
<223> labT2

<220>
<221> misc_feature
<222> (10464)..(10223)

<223>  aac(3) IV

<220>
<221>  misc_feature
<222>  (10999)..(10465)
<223>  aac(3) IV'

<220>
<221>  misc_feature
<222>  (11395)..(11516)
<223>  oriT

<220>
<221>  misc_feature
<222>  (13345)..(12536)
<223>  tsr

<220>
<221>  misc_feature
<222>  (14611)..(15727)
<223>  replicon pIJ101

<400>  144
tggtggtgct cacggcgggg gaggtggccg aggcgtacgc cgacatgctc gccgccctcg      60

cccagcgccg cgaggaagca actgcacgcc gacggcgaga gcaggacgac gaccaggacg     120

acgacgccga cgaccgccag gagcgggccg cccggcacat cgcccggctc gcaagtgggc     180

ccacttcgca ctaactcgct cccccccgcc gtacgtcatc ccggtgacgt acggcggggg     240

tcggtgacgt acgcggcgac ggcggccggg gtcgaagccg cgggagtaat cctgggatta     300

ctcgcccggg gtcggccccg ccggcacttc gtgcaggcgg taccgggccc cccctcgagg     360

tccagcccga cccgagcacg cgccggcacg cctggtcgat gtcggaccgg agttcgaggt     420

acgcggcttg caggtccagg aaggggacgt ccatgcgagt gtccgttcga gtggcggctt     480

gcgcccgatg ctagtcgcgg ttgatcggcg atcgcaggtg cacgcggtcg atcttgacgg     540

ctggcgagag gtgcggggag gatctgaccg acgcggtcca cacgtggcac cgcgatgctg     600

ttgtgggcac aatcgtgccg gttggtagga tcgacggtat cgataagctt gatatcgaat     660

tcttggagga gtggatggat ctgcggtacc acgcctatgc gatggccgat cccgtctttt     720

atgactcgcc gtccagcgac acccgggaga ccgacggcta ctccgacgac ctccccctgc     780

cggacggctg ggaacgtcgg cgcgtggggg tctgggtgat gcagggccac gacgggttga     840

ccatgcctga ccagggctgg aagatccatg tgtcggccgg tctggacaac gcctggcccg     900

tcctcgaact ggtcgccaaa tactgcgtcg agcaggaaat gcctttcaag ttcctgcgca     960

gcagaaggac gttgctggcc cgcagcagca agtacgccga acgcggcggc agcggaaagt    1020

tcatcacgat ctaccccgcc gacgaaggcg ctctggaaaa gaccctccat gaactcggtg    1080

gaatgctgga agggcagccc ggcccctata tcttgagcga tctgcgctgg cgttcggggc    1140

cgctgttcgt gcgctacggc gctttcaagg agaaattctg ccgggacggg cgcggcgaga    1200

tggtgcccgc gatcgcgcgc ccggacggcg tgctggtgcc cgacgccagg gacccggtgt    1260

tccgggtgcc cgcgtgggtg gaggtgcccg gcttcctgcg ggaggccatc gacgcccggg    1320

agaacgggac cgtcgaggac ttcccctacc ggatcgagaa ggcgctgcac ttctccaacg    1380

gcggcggcct ctaccgcgcc gtggacgagc gcaccggccg cagggtcctg gtgaaggagg 1440

cgcggccgat ggcgggcctg gaccgcgccg aggacgacgc cgtcgtccgg ctcgaacgcg 1500

agcacggcct gctgctccgc ctcgccgacc tggactgcat ccccgacctg gtcgagtacc 1560

ggagctggtg ggagcaccgc ttcctcgtgc gggagtacgt cgagggcgag accctcaccc 1620

accacatggt gcgccgcaac ccgatgctgc actacggcgc gacgccgcag gaggtcgccg 1680

gctacaccga gtgggcgctc ggcgtcgtcg accgggtgga gagcgcgctc ggccggctgc 1740

acgaacgcgg cgtggtcttc ggcgacctgc accccggcaa catcatcgtc cgggacgacg 1800

actccatcgt gttcgtcgac ttcgagctgg tcgccgaggc ggaggaggcg acgcacccgg 1860

cgctcggcgc gcccggctac caggccccgc ccgactacac cgggttcgcc atcgaccgct 1920

acgcgctcgg ctgcatcagg ctcgcggtct tcacctccct caccgcgacg ctgcactggg 1980

acgaccgcaa ggtcgagcag ttcctcgacg tgatctgcga gtccttcccg ctgccgccgg 2040

actacgccga ccggatccgc cgcgacctcg cccgcccggc gccggcggac ggcgcgccgc 2100

cgatctggcg cgagccgacg cccgccacct ggccggacac gcgggccggg atcgccgccg 2160

cgatcctcga cacggccacg cccgagcgcg ccgaccggct cttcccgggc gacatcgagc 2220

agttcgcgac cagcgtcggt gggatcgggt tcggccacgg cgcggcgggc gtgctgtggg 2280

cgctggccga ggcgggcgcc ggccgcttcc ccgaccacga ggactgggtg cgggacgccg 2340

tcgccagggc gcaacggccg ccgcccggct tctacgacgg cgtcgcgggc gtcgcccacg 2400

tcctggaccg gctcggccgc gccgacgagg cccgcgagct catggagcac gcgcccgccg 2460

cgacgggggc gaccgacaac agcctctacc gggggctggc gggcatcggc ctcaaccagc 2520

tccacttcgc ccgcgtcacg ggcgaggcgt cgttcgccgc ggcggccgag gagaccgccg 2580

gccgggtggt cgcgaacctg cgccgcaaga cggagggcgc ctaccgggcg ggcctgatgt 2640

acggctcctc cggcccggcc ctgttcctcg tccggatgtt cgaggcgacg ggcgacggcc 2700

actggctgga cgaggccgaa cgcgccctgc accgcgagct ggacgcctgc aagtggacgc 2760

agaaggacag cacgctccag gtcgacgagg gctggcgggt ccttccgtac gtcgccaccg 2820

gcagcgtggg catcggcatc gcgctgcacg agttcctgcg gcaccgcccg gcgccgcgct 2880

tcaccgaggc gcaggagggg atccggaggg cggcggctcc cgcgtacttc gtgcagagcg 2940

gcctgctcaa cggacggtcc ggcatcctcg cctacctgct gcacgtcggg gccggccggg 3000

aggacccggt cgtccggacg cacctgcgca acctcggctg gcacgccgtc ccgtaccccg 3060

gccggggcga ggacgccccg gcgcccggcg cgcggcggac cgcgttcatc ggggaccagc 3120

tgctgcgcct gtccatggac ctcgccacgg gctccgccgg ggtcctggcc acggtcgagg 3180

cggccctggg cgcgcctagg ccttgacggc cttccgccaa ttcgccctat agtgagtcgt 3240

attacgtcgc gctcactggc cgtcgtttta caacgtcgtg actgggaaaa ccctggcgtt 3300

acccaactta atcgccttgc agcacatccc cctttcgcca gctggcgtaa tagcgaagag 3360

gcccgcaccg aaacgccctt cccaacagtt gcgcagcctg aatggcgaat gggagcgccc 3420

tgtagcggcc actcaaccct atctcggtct attcttttga tttataaggg attttgccga 3480

tttcggccta ttggttaaaa aatgagctga tttaacaaaa atttaacgcg aattttaaca 3540
aaatattaac gcttacaatt taggtggcac ttttcgggga aatgtgcgcg gaacccctat 3600
ttgtttattt ttctaaatac attcaaatat gtatccgctc atgagacaat aaccctgata 3660
aatgcttcaa taatattgaa aaaggaagag tatgagtatt caacatttcc gtgtcgccct 3720
tattcccttt tttgcggcat tttgccttcc tgtttttgct cacccagaaa cgctggtgaa 3780
agtaaaagat gctgaagatc agttgggtgc acgagtgggt tacatcgaac tggatctcaa 3840
cagcggtaag atccttgaga gttttcgccc cgaagaacgt tttccaatga tgagcacttt 3900
taaagttctg ctatgtggcg cggtattatc ccgtattgac gccgggcaag agcaactcgg 3960
tcgccgcata cactattctc agaatgactt ggttgagtac tcaccagtca cagaaaagca 4020
tcttacggat ggcatgacag taagagaatt atgcagtgct gccataacca tgagtgataa 4080
cactgcggcc aacttacttc tgacaacgat cggaggaccg aaggagctaa ccgctttttt 4140
gcacaacatg ggggatcatg taactcgcct tgatcgttgg gaaccggagc tgaatgaagc 4200
cataccaaac gacgagcgtg acaccacgat gcctgtagca atggcaacaa cgttgcgcaa 4260
actattaact ggcgaactac ttactctagc ttcccggcaa caattaatag actggatgga 4320
ggcggataaa gttgcaggac cacttctgcg ctcggccctt ccggctggct ggtttattgc 4380
tgataaatct ggagccggtg agcgtggttc tcgcggtatc attgcagcac tggggccaga 4440
tggtaagccc tcccgtatcg tagttatcta cacgacgggg agtcaggcaa ctatggatga 4500
acgaaataga cagatcgctg agataggtgc ctcactgatt aagcattggt aactgtcaga 4560
ccaagtttac tcatatatac tttagattga tttaaaactt catttttaat ttaaaaggat 4620
ctaggtgaag atcctttttg ataatctcat gaccaaaatc ccttaacgtg agttttcgtt 4680
ccactgagcg tcagaccccg tagaaaagat caaaggatct tcttgagatc ctttttttct 4740
gcgcgtaatc tgctgcttgc aaacaaaaaa accaccgcta ccagcggtgg tttgtttgcc 4800
ggatcaagag ctaccaactc tttttccgaa ggtaactggc ttcagcagag cgcagatacc 4860
aaatactgtt cttctagtgt agccgtagtt aggccaccac ttcaagaact ctgtagcacc 4920
gcctacatac ctcgctctgc taatcctgtt accagtggct gctgccagtg gcgataagtc 4980
gtgtcttacc gggttggact caagacgata gttaccggat aaggcgcagc ggtcgggctg 5040
aacggggggt tcgtgcacac agcccagctt ggagcgaacg acctacaccg aactgagata 5100
cctacagcgt gagctatgag aaagcgccac gcttcccgaa gggagaaagg cggacaggta 5160
tccggtaagc ggcagggtcg gaacaggaga gcgcacgagg gagcttccag ggggaaacgc 5220
ctggtatctt tatagtcctg tcgggtttcg ccacctctga cttgagcgtc gatttttgtg 5280
atgctcgtca ggggggcgga gcctatggaa aaacgccagc aacgcggcct ttttacggtt 5340
cctggccttt tgctggcctt ttgctcatta ggcaccccag gctttacccg aacgaccgag 5400
cgcagcgagt cagtgagcga ggaagcggag agcgcccaat acgcaaggaa acagctatga 5460
ccatgttaat gcagctggca cgacaggttt cccgactgga aagcgggcag tgagcggaag 5520

gcccatgagg ccagttaatt aagcgctgac cgaggccgcc gaagcggcac ccatgtgaaa 5580
cgaccgcccg gggcggtggg gactcccacc gccccgggcg gaccaccatc agcgaggagc 5640
acgacaggaa tgcacgccga ccggctgctc gtccaggcgc tgcgcgccgg ccccgggtgg 5700
acggccctcc tggtcgccgc gacgctgctg aacgcggtgt gcgcgctcgc cctgcccgcc 5760
gcgatcggcg cggtcaccga cgcggtgctc gcccgcgacg cgggccgcgg cgccggggcc 5820
ccgctgggct ggctggcggc ggcgctcggc ggcgtggcgc tgggcacggc gctgtcccgg 5880
ctcgccgagg tgtactgcgg cacggccgcg accagggagc tgcggacgcg cctggtcacg 5940
cacgtgctgg ccctcggggt gccggggacg cgcgcgttca cgcccggcga cctggccagc 6000
cgcgccgtca ccggcgcgcc gcaggccggc gcggtcgcgc ggtcggtggt cagcgcggtc 6060
gcggggctgc tgatgtcggt cggcggcgtg gtcgcgctgt ggctgatcga ctggcggctg 6120
gtggcggcgg tgggcggcgc ggtcccgatc gggctgctgc tcatgcgcgt cttcgtccgg 6180
gacgcctccg acctggtcac cgagtaccag gaggcgcagg gcgagatcgc cgcgcggatg 6240
accgacgcgc tcaccggcat ccgcaccatc cgcgccgccg ggacctggcg gcgcgaggcc 6300
gaccgggtcc tggccccgct gccgggcctg tcggactccg ggcggcggct ctggcacgcc 6360
tacgggcgga tgcaggggca gggccgcctg atcgtcccgc tggccgagat cgcggcgctg 6420
gccgtcgccg ggcggggcgt cctgaccggt cggctgtcgg cgggggagat gctcgcggcg 6480
gcgggctacg ccccgatggc gctgggcctg gtcggccaga tccccctgct gctggccctg 6540
gcgcggctgc gggcgggggc gcgccgcctg gccgaggtgc tgtcggtccc cgcccccggc 6600
ggcggcgacc gcgcgctgcc gcccggcccc ggcgcgctgg tcctgcgcgg ggtgaccgtg 6660
cggacgccgg acgggccgct gctcgacgcg gtcgacctga ccgtcccgcc cggccggacg 6720
gtggcggtgg tcggccggtc cggcgcgggc aagacgacgc tcgccgccgt ggccgggcgc 6780
ctcctcgacc ccgacgaggg gcgggtgctc ctggacgggg tcccgctgcg cgagctcgcg 6840
cccgccgcgc tgcgcgcgca ggtcgcctac gccttcgaac gccccgacct ccaggggacc 6900
acgatcgccg acgccatcgc ctacggctgc ccgtccgcgc cgccccgcgc ggtcgaacgg 6960
gcggccgcgc tcgcgcgggc ggacggcttc gtccggctgc tcccggcggg cttcgacacg 7020
cccgtcgcgg acacgcccct gtccggcggg gaacggcagc ggctcgggct ggcccgggcc 7080
ttcgtccgga acgcccgcct gatcatcctc gacgacgcga cctccagcct cgactcggtg 7140
accgaggcgc aggtggcggc cgcgctcgcg gaggccgccg cgacccgcac ccggctggtg 7200
gtcgcgcacc gcgccgggac cgccgcgcgc gccgacctgg tcctgtggct ggactccggc 7260
cgggtgcgcg cgctccgccc gcaccgcgag ctgtgggccg atccggacta ccgggcgatg 7320
ttcgagccgg cgccggcgga gcgcgcgtga cggggggcgc ggcgcgctgg ttcgccgccc 7380
agctgcggac cgaacggcgc ggcctggccg gggtgctggc gtggtcggtc gcggcggcgc 7440
tcccggcgct ggtgtccgga cggctgatcg cgctcgccgt cgaccagggc ttcctgcgcg 7500
gacgcggcac ggtcggcctg gcgtggctgg gggcgctcgc ggcggcgacg gcggtcggcg 7560
cgctgggcgc gcggcagatc ccgcgcgcgc tcggcggggc cctggagccg gtgcgcgacc 7620

ggctcgtccg ccggatcgtg gccggcgcgc tgcgccgcgc gctggccggc cggcccgatc 7680
cgggcgtggt cgcgaagctg accgagcaga ccgagaccgt ccgggacacc ggcgcggggc 7740
tgctgctcgg gatgcagcag gtcggcatgg cggtggtcgc ggccgcgatc gggctgctgc 7800
tgctcgcgcc cgtcacggcg ctgctggtcc tcccgccggt gctggcggcg ctgctgctgc 7860
tctcccggct gctgccggag ctgatggcgc ggcaccgggc cctgctggcg gccgaggacc 7920
gcgtcgcggc gaccgtcggc ggcctgaccg cggcggtgcg cgacgtggtc gcgtgcgggg 7980
ccgggccccg ggccggggcc gaggcggcgg cggtcttcgc cgagcaggcc gcggccgaac 8040
gcgccgtcgc ccgcgccgac gcgctgcgca ccgcgctcgg catcgtcggc ggccagctgt 8100
cgctggtcgt cctgctggcc gccgcgccct ggctggtggc ctccgggcgg ctcagccccg 8160
gcgaggtgct cggcgcgctc acctacgtga cgaccggcct gcaaccggcg ctgcgcgcgg 8220
ccgtgcagag caccgggggt tccggcgtgc ggctggcggt gaccctgcaa cgcctcctgg 8280
acggctccgc ggacgaaccc gaccccgcgc ccgccggagg gcgcgtcccg ggccgcctcg 8340
acctgtcggt ggaggggctc acgttcgcct acggcgccgg tgccgaaccg gtcgtgcgcg 8400
acctggacct gaccgtgccg cacgggacgc acctggccgt cgtcggcccc agcgggatcg 8460
gcaagtcgac cctggccgac ctgctcaccg gggtcgccgt cccgcaggcg ggccgggtcc 8520
gcgtcggcgg cgtgccgctc gccgagaccg atcccgcctg gaggcgccgg tcggtcgcga 8580
tcatcccgca ggaggcgtac gtcttcagcg gcacgctcgc cgagaacctc gcctacctgc 8640
gcccggacgc cgccgaggcc gagctcgacg ccgccgtcgc cgcgctgggc ctggcgccgc 8700
tgcgcgcccg gctcggcggg tacggggccg cgctcgggcc gcgttcgggc ctgacgccgg 8760
gggagcggca gctcgtcgcg ctcgcccgcg tctggctcag ccccgcgcgg atcgtggtgc 8820
tggacgaggc gacctgccac ctcgaccccg ccgccgaggc ccgcgtggag cacgccttcc 8880
gggaccggcc cggcaccctg gtcgtcatcg cgcaccggct cagctcggcg cgccgcgccg 8940
accgcgtcct ggtgatggac gggacccgcc cccggctggg cacccacggg gaactcctcc 9000
gggactcccc gctctacgcc gacctggtcg gggcctggac cgttcacccc gcggcgcggt 9060
agccgctcag tctagaagct ctgcattaat gaatcggcca acgcgcgggg agaggcggtt 9120
tgcgtattgg gcgctcttcc gcttcctcgc tcactgactc gctgcgctcg gtcgttcggc 9180
tgcggcgagc ggtatcagct cactcaaagg cggtaatacg gttatccaca gaatcagggg 9240
ataacgcagg aaagaacatg tgagcaaaag gccagcaaaa ggccaggaac cgtaaaaagg 9300
ccgcgtgcct ggcgtttttc cataggctcc gcccccctga cgagcatcac aaaaatcgac 9360
gctcaagtca gaggtggcga aacccgacag gactataaag ataccaggcg tttccccctg 9420
gaagctccct cgtgcgctct cctgttccga ccctgccgct taccggatac ctgtccgcct 9480
ttctcccttc gggaagcgtg gcgctttctc atagctcacg ctgtaggtat ctcagttcgg 9540
tgtaggtcgt tcgctccaag ctgggctgtg tgcacgaacc ccccgttcag cccgaccgct 9600
gcgccttatc cggtaactat cgtcttgagt ccacccggta agacacgact tatcgccact 9660

ggcagcagcc actggtaaca ggattagcag agcgaggtat gtaggcggtg ctacagagtt 9720
cttgaagtgg tggcctaact acggctacac tagaagaaca gtatttggta tctgcgctct 9780
gctgaagcca gttaccttcg gaaaaagagt tggtagctct tgatccggca aacaaaccac 9840
cgctggtagc ggtggttttt ttgtttgcaa gcagcagatt acgcgcagaa aaaaaggatc 9900
tcaagaagat cctttgatct tttctacggg gtctgacgct cagtggaacg aaaactcacg 9960
ttaagggatt ttggtcatga gattatcaaa aaggatcttc acctagatcc ttttaaatta 10020
aaaatgaagt tttaaatcaa tctaaagtat atatgagtaa acttggtctg acagttacca 10080
atgcttaatc agtgaggcgc attgagcgtc agcatatcat cagcgagctg aagaaagaca 10140
atccccgatc cgctccacgt gttgccccag caatcagcgc gaccttgccc ctccaacgtc 10200
atctcgttct ccgctcatga gctcagccaa tcgactggcg agcggcatcg cattcttcgc 10260
atcccgcctc tggcggatgc aggaagatca acggatctcg gcccagttga ccgagggctg 10320
tcgccacaat gtcgcgggag cggatcaacc gagcaaaggc atgaccgact ggaccttcct 10380
tctgaaggct cttctccttg agccacctgt ccgccaaggc aaaccgctca cagcagtggt 10440
cattctcgag ataatcgacg cgtaccaact tgccatcctg aagaatggtg cagtgtctcg 10500
gcaccccata gggaaccttt gccatcaact cggcaagatg cagcgtcgtg ttggcatcgt 10560
gtcccacgcc gaggagaagt acctgcccat cgagttcatg gacacgggcg accgggcttg 10620
caggcgagtg aggtggcagg ggcaatggat cagagatgat ctgctctgcc tgtggccccg 10680
ctgccgcaaa ggcaaatgga tgggcgctgc gctttacatt tggcaggcgc cagaatgtgt 10740
cagagacaac tccaaggtcc ggtgtaacgg gcgacgtggc aggatcgaac ggctcgtcgt 10800
ccagacctga ccacgagggc atgacgagcg tccctcccgg acccagcgca gcacgcaggg 10860
cctcgatcag tccaagtggc ccatcttcga ggggccggac gctacggaag gagctgtgga 10920
ccagcagcac accgccgggg gtaaccccaa ggttgagaag ctgaccgatg agctcggctt 10980
ttcgccattc gtattgcacg acattgcact ccaccgctga tgacatcagt cgatcatagc 11040
acgatcaacg gcactgttgc aaatagtcgg tggtgataaa cttatcatcc ccttttgctg 11100
atggagctgc acatgaacca aaaggatcta ggtgaagatc ctttttgata atctcatgac 11160
caaaatccct taacgtgagt tttcgttcca ctgagcgtca gaccaataag ggcgacacgg 11220
aaatgttgaa tactcatact cttcctttca atgggctgca ggtcgacgga tcttttccgc 11280
tgcataaccc tgcttcgggg tcattatagc gattttttcg gtatatccat cctttttcgc 11340
acgatataca ggattttgcc aaagggttcg tgtagacttt ccttggtgta tccaacggcg 11400
tcagccgggc aggataggtg aagtaggccc acccgcgagc gggtgttcct tcttcactgt 11460
cccttattcg cacctggcgg tgctcaacgg gaatcctgct ctgcgaggct ggccggctac 11520
cgccggcgta acagatgagg gcaagcggat ggctgatgaa accaagccaa ccaggaaggg 11580
cagcccacct atcaaggtgt actgccttcc agacgaacga agagcgattg aggaaaaggc 11640
ggcggcggcc ggcatgagcc tgtcggccta cctgctggcc gtcggccagg gctacaaaat 11700
cacgggcgtc gtggactatg agcacgtccg cgagctggcc cgcatcaatg gcgacctggg 11760

ccgcctgggc ggcctgctga aactctggct caccgacgac ccgcgcacgg cgcggttcgg 11820
tgatgccacg atcctcgccc tgctggcgaa gatcgaagag aagcaggacg agcttggcaa 11880
ggtcatgatg ggcgtggtcc gcccgagggc agagccatga cttttttagc cgctaaaacg 11940
gccggggggt gcgcgtgatt gccaagcacg tccccatgcg ctccatcaag aagagcgact 12000
tcgcggagct ggtgaagtac atcaccgacg agcaaggcaa gaccgatccc cattaacgct 12060
tacaatttcc attcgccatt caggctgcgc aactgttggg aagggcgatc ggtgcgggcc 12120
tcttcgctat tacgccagag cttggccgga tctaaagttt tgtcgtcttt ccagacgtta 12180
gtaaatgaat tttctgtatg aggttttgct aaacaacttt caacagtttc agcggagtga 12240
gaatagaaag gaacaactaa aggaattgcg aataataatt ttttcacgtt gaaaatctcc 12300
aaaaaaaaag gctccaaaag gagcctttaa ttgtatcggt ttatcagctt gctttcgagg 12360
tgaatttctt aaacagcttg ataccgatag ttgcgccgac aatgacaaca accatcgccc 12420
acgcataacc gatatattcg gtcgctgagg cttgcaggga gtcaaaggcc gcttttgcgg 12480
gatcatcact gacgaatcga ggtcgaggaa ccgagcgtcc gaggaacaga ggcgcttatc 12540
ggttggccgc gagattcctg tcgatcctct cgtgcagcgc gattccgagg gaaacggaaa 12600
cgttgagaga ctcggtctgg ctcatcatgg ggatggaaac cgaggcggaa gacgcctcct 12660
cgaacaggtc ggaaggccca cccttttcgc tgccgaacag caaggccagc cgatccggat 12720
tgtccccgag ttccttcacg gaaatgtcgc catccgcctt gagcgtcatc agctgcatac 12780
cgctgtcccg aatgaaggcg atggcctcct cgcgaccgga gagaacgacg ggaagggaga 12840
agacgtaacc tcggctggcc ctttggagac gccggtccgc gatgctggtg atgtcactgt 12900
cgaccaggat gatccccgac gctccgagcg cgagcgacgt gcgtactatc gcgccgatgt 12960
tcccgacgat cttcaccccg tcgagaacga cgacgtcccc acgccggctc gcgatatcgc 13020
cgaacctggc cgggcgaggg acgcgggcga tgccgaatgt cttggccttc cgctccccct 13080
tgaacaactg gttgacgatc gaggagtcga tgaggcggac cggtatgttc tgccgcccgc 13140
acagatccag caactcagat ggaaaaggac tgctgtcgct gccgtagacc tcgatgaact 13200
ccaccccggc cgcgatgctg tgcatgaggg gctcgacgtc ctcgatcaac gttgtcttta 13260
tgttggatcg cgacggcttg gtgacatcga tgatccgctg caccgcggga tcggacggat 13320
ttgcgatggt gtccaactca gtcatggtcg tcctaccggc tgctgtgttc agtgacgcga 13380
ttcctggggt gtgacaccct acgcgacgat ggcggatggc tgccctgacc ggcaatcacc 13440
aacgcaaggg gaagtcgtcg ctctctggca aagctccccg ctcttccccg tccgggaccc 13500
gcgcggtcga tccccgcata tgaagtattc gccttgatca gtcccggtgg acgcgccagc 13560
ggcccgccgg agcgacggac tccccgacct cgatcgtgtc gccctgagcg tccacgtaga 13620
cgttgcgtga gagcaggact gggccgccgc cgaccgcacc gccctcacca ccgaccgcga 13680
ccgcgccatg gccgccgccg acggcctggt cgccgccgcc gcccgccggt tcggcgcctg 13740
acccgaccaa cccccgcggg gcgccggcac ttcgtgctgg cgccccgccc ccacccacca 13800

ggagaccgac catgaccgac ttcgacggac gcctgaccga ggggaccgtg aacctggtcc 13860
aggaccccaa cggcggtggc tggtccgccc actgcgctga gcccggttgc gactgggccg 13920
acttcgccgg accgctcggc ttccagggcc tcgtggccat cgctcgccga cacacgcact 13980
gaccgcacgt caaagccccg ccggatcacc ggcggggctc tcttcggccc tccaagtcac 14040
accagcccca aggggcgtcg ggagtggcgg agggaacctc tggcccgatt ggtgccagga 14100
ttcccaccag accaaagagc aacgggccgg acttcgcacc tccgacccgt ccgctcccag 14160
actcgcgccc cttagccggg cgagacagga acgttgctcg tgcccagagt acggagcgat 14220
gccgaggcat tgccagatcg gcccgccggg ccccgctgcc actgcgggac cgcaattgcc 14280
cacacaccgg gcaaacggcc gcgtatctac tgctcagacc gctgccggat ggcagcgaag 14340
cgggcgatcg cgcgtgtgac gcgagatgcc gcccgaggca aaagcgaaca ccttgggaaa 14400
gaaacaacag agtttcccgc acccctccga cctgcggttt ctccggacgg ggtggatggg 14460
gagagcccga gaggcgacag cctctcggaa gtaggaagca cgtcgcggag cgacgctgcc 14520
cgactgcgga aagccgcccg gtacagccgc cgccggacgc tgtggcggat cagcggggac 14580
gccgcgtgca agggctgcgg ccgcgccctg atggaccctg cctccggcgt aatcgtcgcc 14640
cagacggcgg ccggaacgtc cgtggtcctg ggcctgatgc ggtgcgggcg gatctggctc 14700
tgcccggtct gcgccgccac gatccggcac aagcgggccg aggagatcac cgccgccgtg 14760
gtcgagtgga tcaagcgcgg ggggaccgcc tacctggtca ccttcacggc ccgccatggg 14820
cacacggacc ggctcgcgga cctcatggac gccctccagg gcacccggaa gacgccggac 14880
agcccccggc ggccgggcgc ctaccagcga ctgatcacgg gcggcacgtg ggccggacgc 14940
cgggccaagg acgggcaccg ggccgccgac cgcgagggca tccgagaccg gatcgggtac 15000
gtcggcatga tccgcgcgac cgaagtcacc gtggggcaga tcaacggctg gcacccgcac 15060
atccacgcga tcgtcctggt cggcggccgg accgaggggg agcggtccgc gaagcagatc 15120
gtcgccacct tcgagccgac cggcgccgcg ctcgacgagt ggcaggggca ctggcggtcc 15180
gtgtggaccg ccgccctgcg caaggtcaac cccgccttca cgcccgacga ccggcacggc 15240
gtcgacttca agcggctgga gaccgagcgc gacgccaacg acctcgccga gtacatcgcc 15300
aagacccagg acgggaaggc gcccgccctc gaactcgccc gcgccgacct caagacggcg 15360
accggcggga acgtcgcccc gttcgaactc ctcggacgga tcggggacct gaccggcggc 15420
atgaccgagg acgacgccgc cggggtcggc tcgctggagt ggaacctctc gcgctggcac 15480
gagtacgagc gggcaacccg gggacgccgg gccatcgaat ggacccgcta cctgcggcag 15540
atgctcgggc tcgacggcgg cgacaccgag gccgacgacc tcgatctgct cctggcggcc 15600
gacgccgacg gcggggagct gcgggccggg gtcgccgtga ccgaggacgg atggcacgcg 15660
gtcacccgcc gcgccctcga cctcgaggcg acccgggccg ccgaaggcaa ggacggcaac 15720
gaggattcgg cggccgtggg cgaacgggtg cgggaggtcc tggcgctggc cgacgcggcc 15780
gacacag 15787

<210>  145
<211>  20
<212>  DNA
<213>  Artificial Sequence

<220>
<223>  Iko-Fw1

<400>  145
gttcgttcga cggaccaatg                                                   20


<210>  146
<211>  20
<212>  DNA
<213>  Artificial Sequence

<220>
<223>  Iko Rev1

<400>  146
cctgctcgac gcagtatttg                                                   20


<210>  147
<211>  20
<212>  DNA
<213>  Artificial Sequence

<220>
<223>  Iko Fw2

<400>  147
cgcaggacga acggtttcag                                                   20


<210>  148
<211>  20
<212>  DNA
<213>  Artificial Sequence

<220>
<223>  Iko Rev2

<400>  148
ccatgggact gtcacctctc                                                   20


<210>  149
<211>  20
<212>  DNA
<213>  Artificial Sequence

<220>
<223>  Iko Fw3

<400>  149
catccaccat ggcatccatc                                                   20


<210>  150
<211>  20
<212>  DNA
<213>  Artificial Sequence

<220>
<223>  Iko Rev3'

<400>  150

gcgtcgtcga ggatgatcag                                                    20


<210>  151
<211>  20
<212>  DNA
<213>  Artificial Sequence

<220>
<223>  Iko Fw3'

<400>  151
actaccgggc gatgttcgag                                                    20


<210>  152
<211>  20
<212>  DNA
<213>  Artificial Sequence

<220>
<223>  Iko Rev3

<400>  152
agcagccggg agagcagcag                                                    20


<210>  153
<211>  20
<212>  DNA
<213>  Artificial Sequence

<220>
<223>  Iko Fw4

<400>  153
actaccgggc gatgttcgag                                                    20


<210>  154
<211>  20
<212>  DNA
<213>  Artificial Sequence

<220>
<223>  Iko Rev4'

<400>  154
ctgaagacgt acgcctcctg                                                    20


<210>  155
<211>  20
<212>  DNA
<213>  Artificial Sequence

<220>
<223>  Iko Fw4'

<400>  155
caggaggcgt acgtcttcag                                                    20


<210>  156
<211>  20
<212>  DNA
<213>  Artificial Sequence

<220>

<223>  Iko Rev4

<400>  156
agatgaagcg ggcgatcgag                                                    20


<210>  157
<211>  20
<212>  DNA
<213>  Artificial Sequence

<220>
<223>  LabKC_Fw2

<400>  157
cctgccggac ggctgggaac                                                    20


<210>  158
<211>  20
<212>  DNA
<213>  Artificial Sequence

<220>
<223>  LabKC_Fw3

<400>  158
gggagaacgg gaccgtcgag                                                    20


<210>  159
<211>  20
<212>  DNA
<213>  Artificial Sequence

<220>
<223>  LabKC_Fw4

<400>  159
cgcccgacta caccgggttc                                                    20


<210>  160
<211>  20
<212>  DNA
<213>  Artificial Sequence

<220>
<223>  LabKC_Fw5

<400>  160
cccgcgagct catggagcac                                                    20


<210>  161
<211>  20
<212>  DNA
<213>  Artificial Sequence

<220>
<223>  LabKC_Fw6

<400>  161
ccggcatcct cgcctacctg                                                    20


<210>  162
<211>  7403
<212>  DNA

<213>  Artificial Sequence

<220>
<223>  pUWLoriT plasmid


<220>
<221>  misc_feature
<222>  (203)..(247)
<223>  ermE

<220>
<221>  misc_feature
<222>  (2224)..(1448)
<223>  aac(3) IV

<220>
<221>  misc_feature
<222>  (2620)..(2741)
<223>  oriT

<220>
<221>  misc_feature
<222>  (4570)..(3761)
<223>  tsr thiostrepton resistence gene

<220>
<221>  misc_feature
<222>  (5836)..(6952)
<223>  replicon pIJ101

<400>  162
ctggtcgatg tcggaccgga gttcgaggta cgcggcttgc aggtccagga aggggacgtc      60

catgcgagtg tccgttcgag tggcggcttg cgcccgatgc tagtcgcggt tgatcggcga      120

tcgcaggtgc acgcggtcga tcttgacggc tggcgagagg tgcggggagg atctgaccga      180

cgcggtccac acgtggcacc gcgatgctgt tgtgggcaca atcgtgccgg ttggtaggat      240

cgacggtatc gataagcttg atatcgaatt cctgcagccc gggggatcca ctagttctag      300

aagctctgca ttaatgaatc ggccaacgcg cggggagagg cggtttgcgt attgggcgct      360

cttccgcttc ctcgctcact gactcgctgc gctcggtcgt tcggctgcgg cgagcggtat      420

cagctcactc aaaggcggta atacggttat ccacagaatc aggggataac gcaggaaaga      480

acatgtgagc aaaaggccag caaaaggcca ggaaccgtaa aaaggccgcg ttgctggcgt      540

ttttccatag gctccgcccc cctgacgagc atcacaaaaa tcgacgctca agtcagaggt      600

ggcgaaaccc gacaggacta taaagatacc aggcgtttcc ccctggaagc tccctcgtgc      660

gctctcctgt tccgaccctg ccgcttaccg gatacctgtc cgcctttctc ccttcgggaa      720

gcgtggcgct ttctcatagc tcacgctgta ggtatctcag ttcggtgtag gtcgttcgct      780

ccaagctggg ctgtgtgcac gaaccccccg ttcagcccga ccgctgcgcc ttatccggta      840

actatcgtct tgagtccacc cggtaagaca cgacttatcg ccactggcag cagccactgg      900

taacaggatt agcagagcga ggtatgtagg cggtgctaca gagttcttga agtggtggcc      960

taactacggc tacactagaa gaacagtatt tggtatctgc gctctgctga agccagttac      1020

cttcggaaaa agagttggta gctcttgatc cggcaaacaa accaccgctg gtagcggtgg      1080

tttttttgtt tgcaagcagc agattacgcg cagaaaaaaa ggatctcaag aagatccttt      1140

gatcttttct acggggtctg acgctcagtg gaacgaaaac tcacgttaag ggattttggt 1200
catgagatta tcaaaaagga tcttcaccta gatcctttta aattaaaaat gaagttttaa 1260
atcaatctaa agtatatatg agtaaacttg gtctgacagt taccaatgct taatcagtga 1320
ggcgcattga gcgtcagcat atcatcagcg agctgaagaa agacaatccc cgatccgctc 1380
cacgtgttgc cccagcaatc agcgcgacct tgcccctcca acgtcatctc gttctccgct 1440
catgagctca gccaatcgac tggcgagcgg catcgcattc ttcgcatccc gcctctggcg 1500
gatgcaggaa gatcaacgga tctcggccca gttgacccag ggctgtcgcc acaatgtcgc 1560
gggagcggat caaccgagca aaggcatgac cgactggacc ttccttctga aggctcttct 1620
ccttgagcca cctgtccgcc aaggcaaagc gctcacagca gtggtcattc tcgagataat 1680
cgacgcgtac caacttgcca tcctgaagaa tggtgcagtg tctcggcacc ccatagggaa 1740
cctttgccat caactcggca agatgcagcg tcgtgttggc atcgtgtccc acgccgagga 1800
gaagtacctg cccatcgagt tcatggacac gggcgaccgg gcttgcaggc gagtgaggtg 1860
gcaggggcaa tggatcagag atgatctgct ctgcctgtgg ccccgctgcc gcaaaggcaa 1920
atggatgggc gctgcgcttt acatttggca ggcgccagaa tgtgtcagag acaactccaa 1980
ggtccggtgt aacgggcgac gtggcaggat cgaacggctc gtcgtccaga cctgaccacg 2040
agggcatgac gagcgtccct cccggaccca gcgcagcacg cagggcctcg atcagtccaa 2100
gtggcccatc ttcgaggggc cggacgctac ggaaggagct gtggaccagc agcacaccgc 2160
cgggggtaac cccaaggttg agaagctgac cgatgagctc ggcttttcgc cattcgtatt 2220
gcacgacatt gcactccacc gctgatgaca tcagtcgatc atagcacgat caacggcact 2280
gttgcaaata gtcggtggtg ataaacttat catccccttt tgctgatgga gctgcacatg 2340
aaccaaaagg atctaggtga agatcctttt tgataatctc atgaccaaaa tcccttaacg 2400
tgagttttcg ttccactgag cgtcagacca ataagggcga cacggaaatg ttgaatactc 2460
atactcttcc tttcaatggg ctgcaggtcg acggatcttt tccgctgcat aaccctgctt 2520
cggggtcatt atagcgattt tttcggtata tccatccttt ttcgcacgat atacaggatt 2580
ttgccaaagg gttcgtgtag actttccttg gtgtatccaa cggcgtcagc cgggcaggat 2640
aggtgaagta ggcccacccg cgagcgggtg ttccttcttc actgtccctt attcgcacct 2700
ggcggtgctc aacgggaatc ctgctctgcg aggctggccg gctaccgccg gcgtaacaga 2760
tgagggcaag cggatggctg atgaaaccaa gccaaccagg aagggcagcc cacctatcaa 2820
ggtgtactgc cttccagacg aacgaagagc gattgaggaa aaggcggcgg cggccggcat 2880
gagcctgtcg gcctacctgc tggccgtcgg ccagggctac aaaatcacgg gcgtcgtgga 2940
ctatgagcac gtccgcgagc tggcccgcat caatggcgac ctgggccgcc tgggcggcct 3000
gctgaaactc tggctcaccg acgacccgcg cacggcgcgg ttcggtgatg ccacgatcct 3060
cgccctgctg gcgaagatcg aagagaagca ggacgagctt ggcaaggtca tgatgggcgt 3120
ggtccgcccg agggcagagc catgactttt ttagccgcta aaacggccgg ggggtgcgcg 3180

tgattgccaa gcacgtcccc atgcgctcca tcaagaagag cgacttcgcg gagctggtga 3240
agtacatcac cgacgagcaa ggcaagaccg atccccatta acgcttacaa tttccattcg 3300
ccattcaggc tgcgcaactg ttgggaaggg cgatcggtgc gggcctcttc gctattacgc 3360
cagagcttgg ccggatctaa agttttgtcg tctttccaga cgttagtaaa tgaattttct 3420
gtatgaggtt ttgctaaaca actttcaaca gtttcagcgg agtgagaata gaaaggaaca 3480
actaaaggaa ttgcgaataa taattttttc acgttgaaaa tctccaaaaa aaaaggctcc 3540
aaaaggagcc tttaattgta tcggtttatc agcttgcttt cgaggtgaat ttcttaaaca 3600
gcttgatacc gatagttgcg ccgacaatga caacaaccat cgcccacgca taaccgatat 3660
attcggtcgc tgaggcttgc agggagtcaa aggccgcttt tgcgggatca tcactgacga 3720
atcgaggtcg aggaaccgag cgtccgagga acagaggcgc ttatcggttg gccgcgagat 3780
tcctgtcgat cctctcgtgc agcgcgattc cgagggaaac ggaaacgttg agagactcgg 3840
tctggctcat catggggatg gaaaccgagg cggaagacgc ctcctcgaac aggtcggaag 3900
gcccaccctt ttcgctgccg aacagcaagg ccagccgatc cggattgtcc ccgagttcct 3960
tcacggaaat gtcgccatcc gccttgagcg tcatcagctg cataccgctg tcccgaatga 4020
aggcgatggc ctcctcgcga ccggagagaa cgacgggaag ggagaagacg taacctcggc 4080
tggccctttg gagacgccgg tccgcgatgc tggtgatgtc actgtcgacc aggatgatcc 4140
ccgacgctcc gagcgcgagc gacgtgcgta ctatcgcgcc gatgttcccg acgatcttca 4200
ccccgtcgag aacgacgacg tccccacgcc ggctcgcgat atcgccgaac ctggccgggc 4260
gagggacgcg ggcgatgccg aatgtcttgg ccttccgctc ccccttgaac aactggttga 4320
cgatcgagga gtcgatgagg cggaccggta tgttctgccg cccgcacaga tccagcaact 4380
cagatggaaa aggactgctg tcgctgccgt agacctcgat gaactccacc ccggccgcga 4440
tgctgtgcat gaggggctcg acgtcctcga tcaacgttgt ctttatgttg gatcgcgacg 4500
gcttggtgac atcgatgatc cgctgcaccg cgggatcgga cggatttgcg atggtgtcca 4560
actcagtcat ggtcgtccta ccggctgctg tgttcagtga cgcgattcct ggggtgtgac 4620
accctacgcg acgatggcgg atggctgccc tgaccggcaa tcaccaacgc aaggggaagt 4680
cgtcgctctc tggcaaagct ccccgctctt ccccgtccgg gacccgcgcg gtcgatcccc 4740
gcatatgaag tattcgcctt gatcagtccc ggtggacgcg ccagcggccc gccggagcga 4800
cggactcccc gacctcgatc gtgtcgccct gagcgtccac gtagacgttg cgtgagagca 4860
ggactgggcc gccgccgacc gcaccgccct caccaccgac cgcgaccgcg ccatggccgc 4920
cgccgacggc ctggtcgccg ccgccgcccg ccggttcggc gcctgacccg accaaccccc 4980
gcggggcgcc ggcacttcgt gctggcgccc cgcccccacc caccaggaga ccgaccatga 5040
ccgacttcga cggacgcctg accgagggga ccgtgaacct ggtccaggac cccaacggcg 5100
gtggctggtc cgcccactgc gctgagcccg gttgcgactg ggccgacttc gccggaccgc 5160
tcggcttcca gggcctcgtg gccatcgctc gccgacacac gcactgaccg cacgtcaaag 5220
ccccgccgga tcaccggcgg ggctctcttc ggccctccaa gtcacaccag ccccaagggg 5280

cgtcgggagt ggcggaggga acctctggcc cgattggtgc caggattccc accagaccaa 5340
agagcaacgg gccggacttc gcacctccga cccgtccgct cccagactcg cgccccttag 5400
ccgggcgaga caggaacgtt gctcgtgccc agagtacgga gcgatgccga ggcattgcca 5460
gatcggcccg ccgggccccg ctgccactgc gggaccgcaa ttgcccacac accgggcaaa 5520
cggccgcgta tctactgctc agaccgctgc cggatggcag cgaagcgggc gatcgcgcgt 5580
gtgacgcgag atgccgcccg aggcaaaagc gaacaccttg ggaaagaaac aacagagttt 5640
cccgcacccc tccgacctgc ggtttctccg gacggggtgg atggggagag cccgagaggc 5700
gacagcctct cggaagtagg aagcacgtcg cggagcgacg ctgcccgact gcggaaagcc 5760
gcccggtaca gccgccgccg gacgctgtgg cggatcagcg gggacgccgc gtgcaagggc 5820
tgcggccgcg ccctgatgga ccctgcctcc ggcgtaatcg tcgcccagac ggcggccgga 5880
acgtccgtgg tcctgggcct gatgcggtgc gggcggatct ggctctgccc ggtctgcgcc 5940
gccacgatcc ggcacaagcg ggccgaggag atcaccgccg ccgtggtcga gtggatcaag 6000
cgcgggggga ccgcctacct ggtcaccttc acggcccgcc atgggcacac ggaccggctc 6060
gcggacctca tggacgccct ccagggcacc cggaagacgc cggacagccc ccggcggccg 6120
ggcgcctacc agcgactgat cacgggcggc acgtgggccg gacgccgggc caaggacggg 6180
caccgggccg ccgaccgcga gggcatccga gaccggatcg ggtacgtcgg catgatccgc 6240
gcgaccgaag tcaccgtggg gcagatcaac ggctggcacc cgcacatcca cgcgatcgtc 6300
ctggtcggcg gccggaccga gggggagcgg tccgcgaagc agatcgtcgc caccttcgag 6360
ccgaccggcg ccgcgctcga cgagtggcag gggcactggc ggtccgtgtg gaccgccgcc 6420
ctgcgcaagg tcaaccccgc cttcacgccc gacgaccggc acggcgtcga cttcaagcgg 6480
ctggagaccg agcgcgacgc caacgacctc gccgagtaca tcgccaagac ccaggacggg 6540
aaggcgcccg ccctcgaact cgcccgcgcc gacctcaaga cggcgaccgg cgggaacgtc 6600
gccccgttcg aactcctcgg acggatcggg gacctgaccg gcggcatgac cgaggacgac 6660
gccgccgggg tcggctcgct ggagtggaac ctctcgcgct ggcacgagta cgagcggcca 6720
acccggggac gccgggccat cgaatggacc cgctacctgc ggcagatgct cgggctcgac 6780
ggcggcgaca ccgaggccga cgacctcgat ctgctcctgg cggccgacgc cgacggcggg 6840
gagctgcggg ccggggtcgc cgtgaccgag gacggatggc acgcggtcac ccgccgcgcc 6900
ctcgacctcg aggcgacccg ggccgccgaa ggcaaggacg gcaacgagga ttcggcggcc 6960
gtgggcgaac gggtgcggga ggtcctggcg ctggccgacg cggccgacac agtggtggtg 7020
ctcacggcgg gggaggtggc cgaggcgtac gccgacatgc tcgccgccct cgcccagcgc 7080
cgcgaggaag caactgcacg ccgacggcga gagcaggacg acgaccagga cgacgacgcc 7140
gacgaccgcc aggagcgggc cgcccggcac atcgcccggc tcgcaagtgg gcccacttcg 7200
cactaactcg ctcccccccg ccgtacgtca tcccggtgac gtacggcggg ggtcggtgac 7260
gtacgcggcg acggcggccg gggtcgaagc cgcgggagta atcctgggat tactcgcccg 7320

gggtcggccc cgccggcact tcgtgcaggc ggtaccgggc cccccctcga ggtccagccc 7380
gacccgagca cgcgccggca cgc 7403